(19) Europäisches Patentamt
European Patent Office
Office européen des brevets

(11) EP 0 596 015 B1

(12) EUROPEAN PATENT SPECIFICATION

(45) Date of publication and mention of the grant of the patent: **01.10.1997 Bulletin 1997/40**

(21) Application number: **92916790.6**

(22) Date of filing: **21.07.1992**

(51) Int Cl.$^6$: **C07D 205/08**, C07D 205/085, C07D 401/04, C07D 403/04, C07D 405/10, C07D 405/04, C07D 409/04, A61K 31/395

(86) International application number: **PCT/US92/05972**

(87) International publication number: **WO 93/02048 (04.02.1993 Gazette 1993/04)**

(54) **SUBSTITUTED BETA-LACTAM COMPOUNDS USEFUL AS HYPOCHOLESTEROLEMIC AGENTS AND PROCESSES FOR THE PREPARATION THEREOF**

SUBSTITUIERTE BETA-LACTAM-VERBINDUNGEN ALS HYPOCHOLESTEROLEMISCHE MITTEL UND VERFAHREN ZU DEREN HERSTELLUNG

COMPOSES DE BETA-LACTAME SUBSTITUES UTILISES COMME AGENTS D'HYPOCHOLESTEROLEMIE, ET PROCEDES DE PREPARATION

(84) Designated Contracting States: **AT BE CH DE DK ES FR GB GR IT LI LU MC NL PT SE**

(30) Priority: **23.07.1991 US 734426**
**23.07.1991 US 734652**

(43) Date of publication of application: **11.05.1994 Bulletin 1994/19**

(73) Proprietor: **SCHERING CORPORATION Kenilworth New Jersey 07033 (US)**

(72) Inventors:
- **BURNETT, Duane, A.**
  **Fanwood, NJ 07023 (US)**
- **CLADER, John, W.**
  **Cranford, NJ 07016 (US)**
- **THIRUVENGADAM, Tiruvettipuram, K.**
  **Edison, NJ 08820 (US)**
- **TANN, Chou-Hong**
  **Berkeley Heights, NJ 07922 (US)**
- **LEE, Junning**
  **Springfield, NJ 07081 (US)**
- **MC ALLISTER, Timothy**
  **Fords, NJ 08863 (US)**
- **COLON, Cesar**
  **Rahway, NJ 07065 (US)**
- **BARTON, Derek, H., R.**
  **College Station, TX 77840 (US)**
- **BRESLOW, Ronald**
  **Englewood, NJ 07631 (US)**
- **DUGAR, Sundeep**
  **Parlin, NJ 08859 (US)**
- **VACCARO, Wayne**
  **Princeton, NJ 08540 (US)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) References cited:
**EP-A- 0 199 630**
**EP-A- 0 264 231**
**EP-A- 0 337 549**
**EP-A- 0 365 364**
**EP-A- 0 375 527**
**EP-A- 0 462 667**
**EP-A- 0 481 671**

Remarks:
Consolidated with 92112425.1/0524595 (European application No./publication No.) by decision dated 09.09.94.

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### BACKGROUND OF THE INVENTION

The present invention relates to substituted β-lactams useful as hypocholesterolemic agents in the treatment and prevention of atherosclerosis and to processses for preparing β-lactams.

Atherosclerotic coronary heart disease represents the major cause for death and cardiovascular morbidity in the western world. Risk factors for atherosclerotic coronary heart disease include hypertension, diabetes mellitus, family history, male sex, cigarette smoking and serum cholesterol. A total cholesterol level in excess of 225-250 mg/dl is associated with significant elevation of risk.

Cholesteryl esters are a major component of atherosclerotic lesions and the major storage form of cholesterol in arteria! wall cells. Formation of cholesteryl esters is also a key step in the intestinal absorption of dietary cholesterol. The intracellular esterification of cholesterol is catalyzed by the enzyme acyl CoA:cholesterol acyl transferase (ACAT, EC 2.3.1.26). Thus, inhibition of ACAT is likely to inhibit the progression of atherosclerotic lesion formation, decrease the accumulation of cholesteryl esters in the arterial wall, and block the intestinal absorption of dietary cholesterol.

A few β-lactam compounds have been reported as being useful in lowering cholesterol and/or in inhibiting the formation of cholesterol-containing lesions in mammalian arterial walls. U.S. 4,983,597 and European Patent Application 462,667 disclose N-sulfonyl-2-azetidinones as anticholesterolemic agents and Ram, et al., in Indian J Chem.. Sect. B. 29B, 12 (1990), p. 1134-7, disclose ethyl 4-(2-oxoazetidin-4-yl)phenoxy-alkanoates as hypolipidemic agents. European Patent Publication 264,231 discloses 1-substituted-4-phenyl-3-(2-oxoalkylidene)-2-azetidinones as blood platelet aggregation inhibitors.

European Patent 0,199,630, European Patent Application 337,549 and European Patent Application 481,671 disclose elastase inhibitory substituted azetidinones said to be useful in treating inflammatory conditions resulting in tissue destruction which are associated with various disease states, e.g. atherosclerosis. European Patent Application 365,364 discloses blood platelet aggregation inhibitory azetidinone derivatives having an (aminoalkoxyimino)propylidene substituent. European Patent Application 375,527 discloses elastase inhibitory azetidinones which are unsubstituted at the 4-position.

### SUMMARY OF THE INVENTION

Novel hypocholesterolemic compounds of the present invention are represented by the formula I

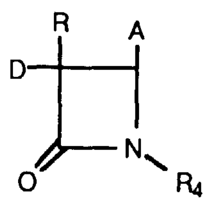

wherein

A is

- -CH=CH-B;
- -C≡C-B;
- $-(CH_2)p$-X-B, wherein p is 0, 1 or 2 and X is a bond, -NH- or $-S(O)_{0-2}$-;
- heteroaryl, benzofused heteroaryl, W-substituted heteroaryl or W-substituted benzofused heteroaryl, wherein heteroaryl is selected from the group consisting of pyrrolyl, pyridinyl, pyrimidinyl, pyrazinyl, triazinyl, imidazolyl thiazolyl, pyrazolyl, thienyl, oxazolyl and furanyl, and for nitrogen-containing heteroaryls, the N-oxides thereof, and wherein W is 1-3 substituents on the ring carbon atoms selected from the group consisting of lower alkyl, hydroxy lower alkyl, lower alkoxy, alkoxyalkyl, alkoxyalkoxy, alkoxycarbonylalkoxy, (lower alkoxy-imino)lower alkyl, lower alkanedioyl, lower alkyl lower alkanedioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $-NR_{10}R_{11}$, $NR_{10}R_{11}$(lower alkyl)-, $NR_{10}R_{11}$(lower alkoxy)-, OH, halogeno, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-butyldimethylsilyloxymethyl, $-C(O)R_{12}$ and

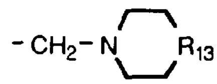,

and wherein the substituents on the substituted heteroaryl ring nitrogen atoms, when present, are selected from the group consisting of lower alkyl, lower alkoxy, $-C(O)OR_5$, $-C(O)R_5$, OH, $NR_{10}R_{11}$(lower alkyl)-, $NR_{10}R_{11}$(lower alkoxy)-, $-S(O)_2NH_2$ and 2-(trimethylsilyl)ethoxymethyl;
-C(O)-B: or

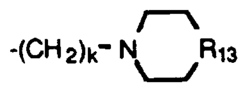,

wherein k is 1 or 2;

D is

$B'-(CH_2)_mC(O)-$, wherein m is 1, 2, 3, 4 or 5;
$B'-(CH_2)_q-$, wherein q is 2, 3, 4, 5 or 6;
$B'-(CH_2)_e-Z-(CH_2)_r$, wherein Z is -O-, -C(O)-, phenylene, $-NR_8-$ or $-S(O)_{0-2}-$, e is 0, 1, 2, 3, 4 or 5 and r is 1, 2, 3, 4 or 5, provided that the sum of e and r is 1, 2, 3, 4, 5 or 6;
$B'-(C_2-C_6$ alkenylene)-; $B'-(C_4-C_6$ alkadienylene)-;
$B'-(CH_2)_t-Z-(C_2-C_6$ alkenylene)-, wherein Z is as defined above, and wherein t is 0, 1, 2 or 3, provided that the sum of t and the number of carbon atoms in the alkenylene chain is 2, 3, 4, 5 or 6;
$R'-(CH_2)_f-V-(CH_2)_g-$. wherein V is $C_3-C_6$ cycloalkylene, f is 1, 2, 3, 4 or 5 and g is 0, 1, 2, 3, 4 or 5, provided that the sum of f and g is 1, 2, 3, 4, 5 or 6;
$B'-(CH_2)_t-V-(C_2-C_6$ alkenylene)- or $B'-(C_2-C_6$ alkenylene)$-V-(CH_2)_t-$, wherein V and t are as defined above, provided that the sum of t and the number of carbon atoms in the alkenylene chain is 2, 3, 4, 5 or 6;
$B'-(CH_2)_a-Z-(CH_2)_b-V-(CH_2)_d-$, wherein Z and V are as defined above and a ,b and d are independently 0, 1, 2, 3, 4, 5 or 6, provided that the sum of a, b and d is 0, 1, 2, 3, 4, 5 or 6;
$T-(CH_2)_s-$, wherein T is cycloalkyl of 3-6 carbon atoms and s is 1, 2, 3, 4, 5 or 6; or
naphthylmethyl, heteroarylmethyl, or W-substituted heteroarylmethyl, wherein heteroaryl and W are as defined above;

B is

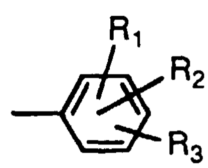

B' is naphthyl, heteroaryl or W-substituted heteroaryl, wherein heteroaryl is as defined above, or

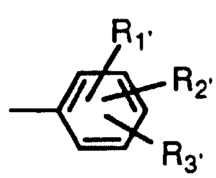

R is hydrogen, fluoro, $C_1-C_{15}$ alkyl, $C_1-C_{15}$ alkenyl, $C_1-C_{15}$ alkynyl, or $B-(CH_2)_h-$, wherein h is 0, 1, 2, or 3;
$R_1$, $R_2$ and $R_3$ are independently selected from the group consisting of H, lower alkyl, hydroxy lower alkyl,

lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $-NR_{10}R_{11}$, $NR_{10}R_{11}$(lower alkyl)-, $NR_{10}R_{11}$(lower alkoxy)-, OH, o-halogeno, m-halogeno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldimethylsilyloxymethyl, $-C(O)R_{12}$,

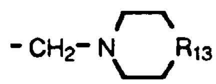

and

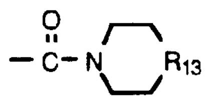
,

or $R_1$ is hydrogen and $R_2$ and $R_3$, together with adjacent carbon atoms to which they are attached, form a dioxolanyl ring;

$R_{1'}$, $R_{2'}$ and $R_{3'}$ are independently selected from the group consisting of H, lower alkyl, hydroxy lower alkyl, lower alkoxy, alkoxyalkyl, alkoxyalkoxy, alkoxycarbonylalkoxy, (lower alkoxyimino)lower alkyl, lower alkanedioyl, lower alkyl alkanedioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $-NR_{10}R_{11}$, $NR_{10}R_{11}$ (lower alkyl)-, $NR_{10}R_{11}$(lower alkoxy)-, OH, halogeno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldimethylsilyloxymethyl, $-C(O)R_{12}$,

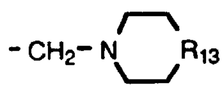
and
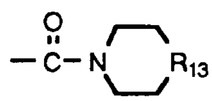
,

or $R_{1'}$ is hydrogen and $R_{2'}$ and $R_{3'}$, together with adjacent carbon atoms to which they are attached, form a dioxolanyl ring;

$R_4$ is

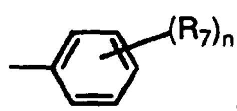
,

wherein n is 0, 1, 2 or 3, indanyl, benzofuranyl, benzodioxolyl, tetrahydronaphthyl, pyridyl, pyrazinyl, pyrimidinyl or quinolyl;

$R_5$ is lower alkyl, phenyl, $R_{14}$-Phenyl, benzyl or $R_{14}$-benzyl;

$R_6$ is OH, lower alkyl, phenyl, benzyl, $R_{14}$-phenyl or $R_{14}$-benzyl;

$R_7$ is lower alkyl, lower alkoxy, OH, halogeno, $-NR_{10}R_{11}$, $-NHC(O)OR_5$, $-NHC(O)R_5$, $NO_2$, -CN, $-N_3$, -SH, $-S(O)_{0-2}$-(lower alkyl), $-COOR_9$, $-CONR_{10}R_{11}$, $-COR_{12}$, phenoxy, benzyloxy, $-OCF_3$, or tert-butyldimethylsilyloxy, and where n is 2 or 3, the $R_7$ groups can be the same or different;

$R_8$ is H, lower alkyl, phenyl lower alkyl, or $-C(O)R_9$;

$R_9$ is H, lower alkyl, phenyl or phenyl lower alkyl;

$R_{10}$ and $R_{11}$ are independently selected from H and lower alkyl;

$R_{12}$ is H, OH, alkoxy, phenoxy, benzyloxy,

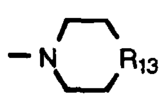
,

$-NR_{10}R_{11}$, lower alkyl, phenyl or $R_{14}$-phenyl;
$R_{13}$ is -O-, $-CH_2-$, -NH- or -N(lower alkyl)-; and
$R_{14}$ is 1-3 groups independently selected from the group consisting of lower alkyl, lower alkoxy, -COOH, $NO_2$, $-NR_{10}R_{11}$, OH or halogeno;

or a pharmaceutically acceptable salt thereof.

Preferred are compounds of formula I wherein R is H. Another group of preferred compounds of formula I is that wherein D is $B'-(CH_2)_q-$, $B'-(CH_2)_e-Z-(CH_2)_r$, $B'-(C_2-C_6$ alkenylene)-, or $B'-(CH_2)_f-V-(CH_2)_g-$, wherein B', Z, V, q, e, r, f, and g are as defined above. A third group of preferred compounds of formula I is that wherein $R_4$ is phenyl, $R_7$-substituted phenyl or indanyl. Still another group of preferred compounds of formula I is that wherein A is $-(CH_2)_p-X-B$, wherein X, B and p are as defined above.

Especially preferred are compounds of formula I wherein D is: $B'-(CH_2)_q-$, wherein B' is phenyl and q is 3 or 4; $B'-(CH_2)_e-Z-(CH_2)_r-$, wherein B' is p-fluorophenyl or p-methoxyphenyl, e is zero, Z is -O-, and r is 2; $B'-(C_2-C_6$ alkenylene)- is 3-phenyl-1-propenyl; or $B'-(CH_2)_f-V-(CH_2)_g-$, wherein B' is phenyl, f is 1, V is cyclopropylene, and g is zero. Also especially preferred are compounds of formula I wherein A is $-(CH_2)_p-X-B$ wherein p is zero and X is a bond. Preferably $R_1$, $R_2$ and $R_3$ are selected from H, OH, $-NO_2$, lower alkoxy, alkoxyalkoxy, lower alkyl lower alkandioyl, m-halogeno, $NR_{10}R_{11}$(lower alkoxy)-, allyloxy, phenoxy, alkoxycarbonylalkoxy and $-C(O)R_{12}$. Compounds wherein $R_1$ and $R_3$ are each H and $R_2$ is in the para-position are more preferred.

$R_7$ is preferably selected from lower alkyl, lower alkoxy, halogeno, $-OCF_3$, lower alkylthio, $-NR_{10}R_{11}$, -CN, OH, and $-COR_{12}$. More preferred are compounds wherein n is 1 and $R_7$ is in the para-position.

Especially preferred compounds of formula I, wherein R is hydrogen, are shown in the following Table 1:

| D | A | $R_4$ |
|---|---|---|
| $C_6H_5-(CH_2)_3-$ | $p-CH_3O-C_6H_4-$ | $p-CH_3O-C_6H_4-$ |
| $C_6H_5-(CH_2)_4-$ | $p-CH_3O-C_6H_4-$ | $p-CH_3O-C_6H_4-$ |
| $C_6H_5-(CH_2)_3-$ | $p-CH_3O-C_6H_4-$ | $C_6H_5-$ |
| $C_6H_5-(CH_2)_3-$ | $p-OH-C_6H_4-$ | $p-CH_3O-C_6H_4-$ |
| $C_6H_5-(CH_2)_3-$ | $p-CH_3O-C_6H_4-$ | $p-Cl-C_6H_4-$ |
| $C_6H_5-(CH_2)_3-$ | $p-CH_3CH_2-O-C_6H_4-$ | $p-CH_3O-C_6H_4-$ |
| $C_6H_5-(CH_2)_3-$ | $p-CH_3O-C_6H_4-$ | $p-CH_3CH_2O-C_6H_4-$ |
| $p-F-C_6H_4-O-(CH_2)_2-$ | $p-CH_3O-C_6H_4-$ | $p-CH_3O-C_6H_4-$ |
| $C_6H_5-(CH_2)_3-$ | $p-CH_3O-C_6H_4-$ | $p-F-C_6H_4-$ |
| $C_6H_5-(CH_2)_3-$ | $p-CH_3O-C_6H_4-$ | $m-CH_3O-C_6H_4-$ |
| $C_6H_5-(CH_2)_3-$ | $p-CH_3O-C_6H_4-$ | $p-CF_3O-C_6H_4-$ |
| $C_6H_5-(CH_2)_3-$ | $p-CH_3O-C_6H_4-$ | $p-CH_3-C_6H_4-$ |
| $C_6H_5-CH_2-CH=CH-$ | $p-CH_3O-C_6H_4-$ | $p-CH_3O-C_6H_4-$ |
| $C_6H_5-(CH_2)_3-$ | $p-(CH_3(CH_2)_3)-O-C_6H_4-$ | $p-CH_3O-C_6H_4-$ |
| $C_6H_5-(CH_2)_3-$ | $p-CH_3O-C_6H_4-$ | $p-CH_3S-C_6H_4-$ |
| $C_6H_5-(CH_2)_3-$ | $p-(CH_2=CH-CH_2-O)-C_6H_4-$ | $p-CH_3O-C_6H_4-$ |
| $C_6H_5-(CH_2)_3-$ | $p-(C_6H_5-O)-C_6H_4-$ | $p-CH_3O-C_6H_4-$ |
| $C_6H_5-(CH_2)_3-$ | $p-(CH_3CH_2O_2C)-C_6H_4-$ | $p-CH_3O-C_6H_4-$ |

| D | A | $R_4$ |
|---|---|---|
| $C_6H_5-(CH_2)_3-$ | $p-CH_3O-C_6H_4-$ | 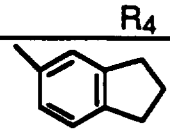 |
| $C_6H_5-(CH_2)_3-$ | $p-(CH_3CH_2CH_2-O)-C_6H_4-$ | $p-CH_3O-C_6H_4-$ |
| $C_6H_5-(CH_2)_5-$ | $p-CH_3O-C_6H_4-$ | $p-CH_3O-C_6H_4-$ |
| $C_6H_5-(CH_2)_3-$ | $p-CH_3O-C_6H_4-$ | $p-((CH_3CH_2)_2N)-C_6H_4-$ |
| $C_6H_5-(CH_2)_3-$ | $p-(CH_3CH_2O)-C_6H_4-$ | $C_6H_5-$ |
| 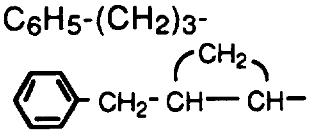 | $p-CH_3O-C_6H_4-$ | $p-CH_3O-C_6H_4-$ |
| $C_6H_5-(CH_2)_3-$ | $p-((CH_3)_2CH-O)-C_6H_4-$ | $p-CH_3O-C_6H_4-$ |
| $p-F-C_6H_4-O-(CH_2)_2-$ | $p-CH_3O-C_6H_4-$ | $C_6H_5-$ |
| $C_6H_5-(CH_2)_3-$ | $p-CH_3O-C_6H_4-$ | 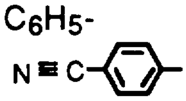 |
| $C_6H_5-(CH_2)_3-$ | $p-(CH_3O_2C)-C_6H_4-$ | $C_6H_5-$ |
| $C_6H_5-(CH_2)_3-$ | $p-CH_3O-C_6H_4-$ | 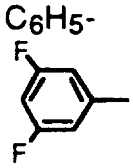 |
| $C_6H_5-(CH_2)_3-$ | 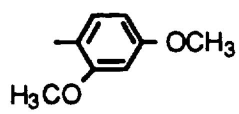 | $p-CH_3O-C_6H_4-$ |
| $C_6H_5-(CH_2)_3-$ | $p-CH_3O-C_6H_4-$ | $p-(H_3CC(O))-C_6H_4-$ |
| $C_6H_5-(CH_2)_3-$ | 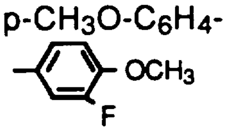 | $p-CH_3O-C_6H_4-$ |
| $C_6H_5-(CH_2)_3-$ | $p-(CH_3OCH_2O)-C_6H_4-$ | $C_6H_5-$ |
| $p-CH_3O-C_6H_4-O-(CH_2)_2-$ | $p-CH_3O-C_6H_4-$ | $C_6H_5-$ |
| $C_6H_5-(CH_2)_3-$ | $p-(CH_3CH_2OC(O)CH_2O)-C_6H_4-$ | $p-CH_3O-C_6H_4-$ |
| $C_6H_5-(CH_2)_3-$ | $p-(CH_3OC(O)-(CH_2)_2-C(O)-O)-C_6H_4-$ | $p-CH_3O-C_6H_4-$ |
| $C_6H_5-(CH_2)_3-$ | $p-((CH_3)_2N-(CH_2)_3-O)-C_6H_4-$ | $p-CH_3O-C_6H_4-$ |
| $C_6H_5-(CH_2)_3-$ | $p-HO-C_6H_4-$ | $p-HO-C_6H_4-$ |
| 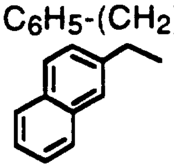 | $p-CH_3O-C_6H_4-$ | $C_6H_5-$ |

The first-listed compound in the above table having the (3R,4S) absolute stereochemistry is more preferred.

This invention also relates to the use of a novel compound of formula I of the present invention as a hypocholesterolemic agent in a mammal in need of such treatment.

In another aspect, the invention relates to a pharmaceutical composition comprising a novel β-lactam of formula I of the present invention in a pharmaceutically acceptable carrier.

In still another aspect, the present invention relates to the use of a pharmaceutical composition for use in the manufacture of a medicament for lowering serum cholesterol, which composition comprises a compound having the structural formula II

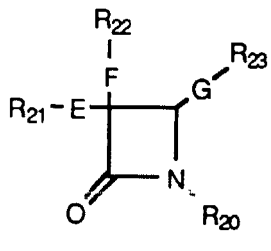

II

wnerein

$R_{20}$ is phenyl, W-substituted phenyl, naphthyl, W-substituted naphthyl, benzodioxolyl, heteroaryl, W-substituted heteroaryl, benzofused heteroaryl and W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof;

$R_{21}$, $R_{22}$ and $R_{23}$ are independently selected from H or $R_{20}$;

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)lower alkyl, lower alkanedioyl, lower alkyl lower alkanedioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $-NR_{10}R_{11}$, $NR_{10}R_{11}$(lower alkyl)-, $NR_{10}R_{11}$(lower alkoxy)-, OH, halogeno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldimethylsilyloxymethyl, $-C(O)R_{12}$,

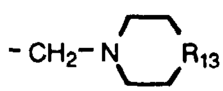 and 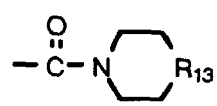 ;

E, F and G are independently a bond; $C_3$-$C_6$ cycloalkylene; $C_1$-$C_{10}$ alkylene; $C_1$-$C_{10}$ alkenylene; $C_1$-$C_{10}$ alkynylene; an alkylene, alkenylene or alkynylene chain as defined substituted by one or more substituents independently selected from the group consisting of phenyl, W-substituted phenyl, heteroaryl and W-substituted heteroaryl, wherein heteroaryl is as defined above; an alkylene, alkenylene or alkynylene chain as defined interrupted by one or more groups independently selected from the group consisting of -O-, -S-, -SO-, $-SO_2-$, $-NR_8$, -C(O)-, $C_3$-$C_6$ cycloalkylene, phenylene, W-substituted phenylene, heteroarylene and W-substituted heteroarylene; or an interrupted alkylene, alkenylene or alkynylene chain as defined substituted by one or more substituents independently selected from the group consisting of phenyl, W-substituted phenyl, heteroaryl and W-substituted heteroaryl; or one of $R_{21}$-E and $R_{22}$-F is selected from the group consisting of halogeno, OH, lower alkoxy, $-OC(O)R_5$, $-NR_{10}R_{11}$, -SH or -S(lower alkyl);

$R_5$ is lower alkyl, phenyl, $R_{14}$-phenyl, benzyl or $R_{14}$-benzyl;

$R_6$ is OH, lower alkyl, phenyl, benzyl, $R_{14}$-phenyl or $R_{14}$-benzyl;

$R_8$ is H, lower alkyl, phenyl lower alkyl or $-C(O)R_9$;

$R_9$ is H, lower alkyl, phenyl or phenyl lower alkyl;

$R_{10}$ and $R_{11}$ are independently selected from H and lower alkyl;

$R_{12}$ is H, OH, alkoxy, phenoxy, benzyloxy,

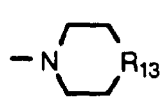

,

$-NR_{10}R_{11}$, lower alkyl, phenyl or $R_{14}$-phenyl;
$R_{13}$ is -O-, $-CH_2-$, -NH- or -N(lower alkyl)-;
$R_{14}$ is 1-3 groups independently selected from the group consisting of lower alkyl, lower alkoxy, -COOH, $NO_2$, $-NR_{10}R_{11}$, OH or halogeno;
provided that when G is a bond, $R_{23}$ is not H, and provided that when $R_{23}$ is W-substituted phenyl, W is not p-halogeno;
or a pharmaceutically acceptable salt thereof;
in a pharmaceutically acceptable carrier.

It is noted that novel compounds of formula I are included within the scope of formula II.

Preferred compounds of formula II are those wherein $R_{21}$ is H and E is a bond or lower alkylene, and those wherein $R_{21}$ is phenyl and E is lower alkylene. Also preferred are compounds of formula II wherein $R_{22}$ is H and F is a bond. Another group of preferred compounds of formula II is that wherein G is a bond and $R_{23}$ is phenyl substituted by OH, $-NO_2$, lower alkoxy, alkoxyalkoxy, m-halogeno, lower alkyl lower alkandioyl, $NR_{10}R_{11}$(lower alkoxy)-, allyloxy, phenoxy, alkoxycarbonylalkoxy, and $-C(O)R_{12}$. Still another group of preferred compounds of formula II is that wherein $R_{20}$ is phenyl or phenyl substituted by lower alkyl, lower alkoxy, halogeno, $-OCF_3$, lower alkylthio, $-NR_{10}R_{11}$, -CN, OH or acetyl.

Certain compounds not within the scope of formula I but within the scope of formula II are novel compounds. Examples of such compounds are represented in the following Table 2

| $R_{21}$-E- | $R_{22}$-F- | $R_{23}$-G- | $R_{20}$- |
|---|---|---|---|
| $C_{10}H_{21}$- | H | $C_6H_5$- | 4-$CH_3O$-$C_6H_4$- |
| $C_{10}H_{21}$- | H | $C_6H_5$- | 2,4,6-tri-$CH_3O$-$C_6H_2$- |
| $C_{10}H_{21}$- | $CH_3CH_2$- | $C_6H_5$- | 4-$CH_3O$-$C_6H_4$- |
| $C_{10}H_{21}$- | $C_6H_5$-$(CH_2)_3$- | $C_6H_5$- | 4-$CH_3O$-$C_6H_4$- |
| $C_{10}H_{21}$- | $CH_3CH_2$- | $C_6H_5$- | 2,4,6-tri-$CH_3O$-$C_6H_2$- |
| $C_{10}H_{21}$- | $CH_3CH_2$- | $C_6H_5$-CH=CH- | 4-$CH_3O$-$C_6H_4$- |
| $C_{10}H_{21}$- | $CH_3$- | $C_6H_5$- | 4-$CH_3O$-$C_6H_4$- |

The present invention also relates to the method of lowering serum cholesterol in a mammal in need of such treatment comprising administering a pharmaceutical composition comprising a compound of formula II in a pharmaceutically acceptable carrier.

The present invention also relates to the use of a compound of formula I or II as an ACAT inhibitor.

The present invention also relates to a stereoselective process for producing β-lactams of formula I, wherein R is hydrogen ,and D and A have trans relative stereochemistry, from a hydroxyamide of the formula

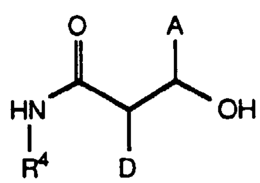

wherein D, A and $R^4$ are as defined above, by cyclizing the hydroxyamide, and wherein the hydroxyamide is prepared from a carboxylic acid $DCH_2COOH$, an aldehyde A-CHO and an amine $R^4NH_2$, wherein D, A and $R^4$ are as defined above, in a process utilizing as a chiral auxiliary an oxazolidinone of the formula

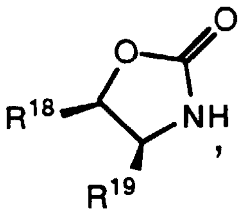, 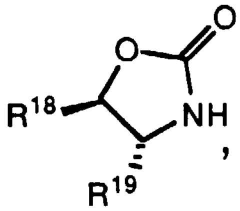, 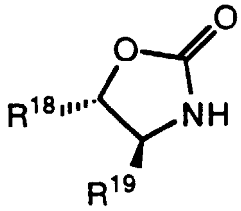 or 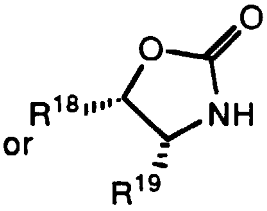

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl, wherein said chiral auxiliary is preferably R-(+)-4-benzyl-oxazolidinone.

This process, designated Method D, for producing compounds of formula I, wherein R is hydrogen, and D and A have trans relative stereochemistry, comprises the steps:

(a) reacting a carboxylic acid of the formula
$D$-$CH_2COOH$, wherein D is as defined above, with a chlorinating agent;
(b) deprotonating a chiral oxazolidinone, as described above, preferably R-(+)-4-benzyloxazolidinone, with a strong base or a tertiary amine base and treating the resulting anion with the product of step (a);
(c) enolizing the product of step (b) with either:

(i) a dialkylboron triflate and a tertiary amine base; or
(ii) $TiCl_4$ and tetramethylethylenediamine (TMEDA) or a mixture of TMEDA and triethylamine;

then condensing with an aldehyde of the formula A-CHO, wherein A is as defined above;
(d) hydrolyzing the product of step (c) with a base and hydrogen peroxide;
(e) condensing the product of step (d) with an amine of the formula $R^4NH_2$, wherein $R^4$ is as defined above, by treating with a dehydrative coupling agent, optionally adding an activating agent;
(f) cyclizing the product of step (e) by reacting the product of step (e) with:

(i) a dialkylazodicarboxylate and a trialkylphosphine; or
(ii) a di- or tri-chlorobenzoyl chloride, an aqueous solution of a base and a phase transfer catalyst, then treating the resulting di- or tri-chlorobenzoate with an aqueous solution of a base and a phase transfer catalyst; or
(iii) a dialkylchlorophosphate, an aqueous solution of a base and a phase transfer catalyst; or
(iv) a di- or tri-chlorobenzoyl chloride and a metal hydride.

In another embodiment, the process of this invention provides the steps of producing a β-lactam of formula I, wherein R is hydrogen, and D and A have trans relative stereochemistry, from a β-aminoamide derivative of the formula

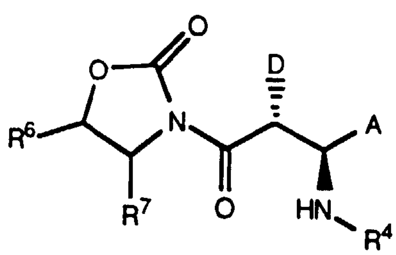

by cyclizing the β-aminoamide, wherein the β-aminoamide is prepared from a carboxylic acid $DCH_2COOH$, and an imine $ACH{=}N$-$R^4$, wherein D, A and $R^4$ are as defined above, in a process utilizing as a chiral auxiliary an oxazolidinone of the formula

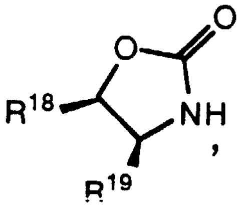 , 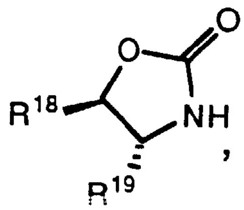 , 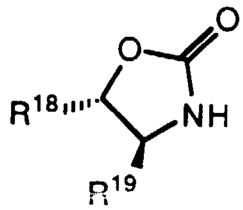 or 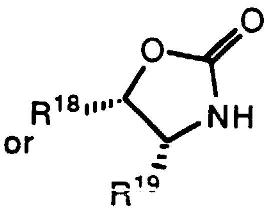

wherein $R^{18}$ and $R^{19}$ are as defined above, and wherein said chiral auxiliary is preferably R-(+)-4-benzyl-oxazolidinone.

This process, designated Method F, for producing compounds of formula I, wherein R is hydrogen, and D and A have trans relative stereochemistry, comprises the steps:

(a) enolizing the product of Method D, step (b) with $TiCl_4$ and tetramethylethylenediamine (TMEDA), then condensing with an imine of the formula A-CH=N-$R^4$, wherein A and $R^4$ are as defined above;
(b) cyclizing the product of step (a) by treating with a strong non-nucleophilic base, preferably sodium bistrimethylsilylamide.

DETAILED DESCRIPTION :

As used herein, the term "lower alkyl" means straight or branched alkyl chains of 1 to 6 carbon atoms and "lower alkyoxy" similarly refers to alkoxy groups having 1 to 6 carbon atoms.

"Alkenyl" means straight or branched carbon chains having one or more double bonds in the chain, conjugated or unconjugated, and alkadienyl refers to chains having two double bonds in the chain. Similarly, "alkynyl" means straight or branched carbon chains having one or more triple bonds in the chain.

Where an alkyl, alkenyl or alkynyl chain joins two other variables and is therefore bivalent, the terms alkylene, alkenylene and alkynylene are used.

"Cycloalkyl" means a saturated carbon ring of 3 to 6 carbon atoms, while "cycloalkylene" refers to a corresponding bivalent ring, wherein the points of attachment to other groups include all positional isomers.

"Halogeno" refers to fluorine, chlorine, bromine or iodine radicals.

"Heteroaryl" includes all positional isomers for a given heteroaryl group as defined above, for example 2-pyridyl, 3-pyridyl and 4-pyridyl. Benzofused heteroaryl refers to radicals formed by the bonding of a benzene radical to adjacent carbon atoms on a heteroaryl ring; examples are indolyl, quinolyl, quinazolinyl, quinoxalinyl, benzotriazolyl, indazolyl, benzoxazolyl, benzothienyl and benzofuranyl.

"Phenylene" means a bivalent phenyl group, including ortho, meta and para-substitution and "heteroarylene" similarly means a bivalent heteroaryl group, ioncluding all positional isomers.

"(Lower alkoxyimino)lower alkyl" refers to the group ($C_1$-$C_6$ lower alkoxy)-N=CH-($C_1$-$C_5$ lower alkyl). "Lower alkanedioyl" means radicals of the formula $-OC(O)(CH_2)_{1-4}C(O)OH$, while "lower alkyl lower alkanedioyl" means radicals of the formula $-OC(O)(CH_2)_{1-4}C(O)O$-(lower alkyl).

$R_{14}$-benzyl and $R_{14}$-benzyloxy refer to benzyl and benzyloxy radicals which are substituted on the phenyl ring.

"Tertiary amine base" means a trialkylamine, such as triethylamine or diisopropylethylamine, or a nitrogen containing heterocycle, such as pyridine.

"Base" means a metal hydroxide base such as lithium, sodium or potassium hydroxide

"Strong base" means a non-aqueous base such as a metal hydride or an alkyllithium.

"Metal hydride" means a commercially available metal hydride such as lithium, sodium or potassium hydride

"Alkyllithium" means a alkyllithium reagent such as n-butyllithium, s-butyllithium, t-butyllithium or methyllithium.

""Dehydrative coupling agent" means a carbodiimide such as 1-(3'-dimethylaminopropyl)-3-ethylcarbodiimide hydrochloride (DEC) or dicyclohexylcarbodiimide (DCC).

"Activating agent" means an agent used to facilitate the formation of amide bonds such as 1-hydroxybenzotriazole (HOBT) or N-hydroxysuccinimide.

"Halide salt" means a metal salt of a halogen such as sodium, lithium or potassium bromide.

The carbon chains as defined in E, F, and G. when substituted by optionally substituted phenyl or heteroaryl groups, may include independent substitution on different carbon atoms, disubstitution on one carbon atom, or both. One skilled in the art will recognize that the number of double or triple bonds present, the replacement of carbon atoms in the chain and the presence of substitutents on the carbon atoms in the chain are all dependent on the length of the chain: shorter carbon chains cannot accommodate as many double or triple bonds, carbon replacements or substituents as longer carbon chains can. In general, unsaturated carbon chains contain 1 to 4 double or triple bonds, conjugated or non-conjugated. Where carbon atoms are replaced, 1 to 4 replacement groups can be present. Similarly, when carbon atoms in the chain are substituted, 1 to 4 substituents can be present.

Examples of alkyl chains are methyl, ethyl, propyl, butyl and decyl.

Examples of unsaturated E, F and G groups are ethylene and acetylene.

Examples of E, F and G groups wherein the carbon atoms in the chain are replaced are $-CH_2CH_2O-$, $-OCH_2CH_2-$, $-CH_2O-$, $-CH_2CH_2CH_2O-$, $-CH_2-O-CH_2-$, $-CH_2CH_2-O-CH_2$, $-CH_2CH_2-NH-$, $-CH_2CH_2-N(CH_3)-$ and $-O-CH_2C(O)-NH-$.

Compounds of the invention have at least one asymmetrical carbon atom and therefore all isomers, including diastereomers and rotational isomers are contemplated as being part of this invention. The invention includes d and l isomers in both pure form and in admixture, including racemic mixtures. Isomers can be prepared using conventional techniques, either by reacting enantiomeric starting materials or by separating isomers of a compound of formula I or II.

Isomers may include geometric isomers, e.g. when E, F or G contains a double bond. All such isomers are contemplated for this invention.

Those skilled in the art will appreciate that for some compounds of formulae I and II, one isomer will show greater pharmacological activity than another isomer.

Compounds of the invention with an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

Compounds of formulae I and II can be prepared by several known methods, and in particular compounds having trans stereochemistry can be prepared by a novel method D, as disclosed in U.S. Serial No. 07/734,426, filed July 23, 1991, or novel method F.

Method A:

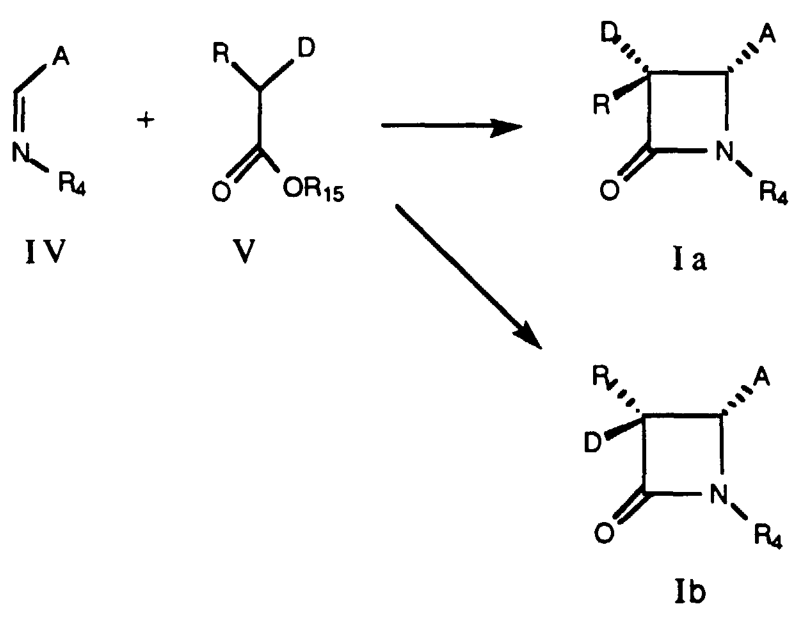

Compounds of formula Ia and Ib, wherein A, D, R and $R_4$ are as defined above, can be prepared by treatment of an ester of formula V, wherein $R_{15}$ is lower alkyl such as ethyl or a chiral moiety such as menthyl or 10-(diisopropylsulfonamido)-isobornyl, with a strong base such as lithium diisopropylamide in a suitable solvent such as tetrahydrofuran (THF) at -78°C. Hexamethylphosphoric triamide (HMPA) may optionally be added as a cosolvent. An imine of formula IV is added and the reaction mixture is warmed to room temperature and the product isolated using conventional

purification techniques. When a chiral ester of formula V is used, the resulting compound of formula Ia or Ib is not racemic.

Method B:

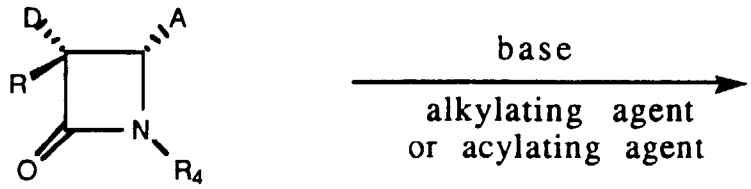


Ia

(R ≠ H)

Ic (R=H)

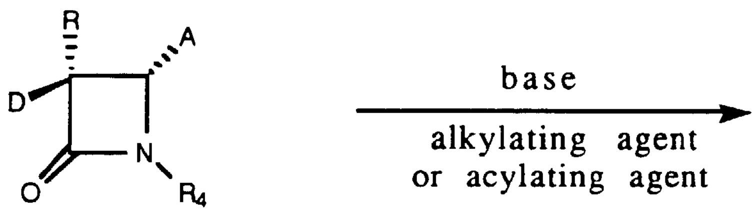


Ib

(R ≠ H)

Id (R=H)

Compounds of formula Ic or Id can be converted to compounds of formula Ia or Ib by treatment with a strong base such as lithium diisopropylamide in a suitable solvent such as THF in the presence or absence of HMPA at -78°C, followed by the addition of an alkylating agent R-$R_{17}$, or an acylating agent such as R-C(O)O-alkyl or R-C(O)Cl, wherein R is as defined above, except that R is not hydrogen, and $R_{17}$ is a leaving group such as bromo or iodo.

Method B':

Trans compounds of formula Id can be converted to the corresponding cis compounds of formula Ic by using Method B, above, but using a proton source such as acetic acid in place of the alkylating agent.

Method C:

Trans compounds of formula Id, wherein R is hydrogen and A, D and $R_4$ are as defined above can be prepared by treating cis compounds of formula Ic with a strong base such as lithium diisopropylamide or potassium t-butoxide in a suitable solvent such as THF. Those skilled in the art will appreciate that since the reaction conditions of Method C can be similar to those of Method A, the conversion of cis to trans sometimes occurs in situ under the conditions of Method A.

Method D

Step D1

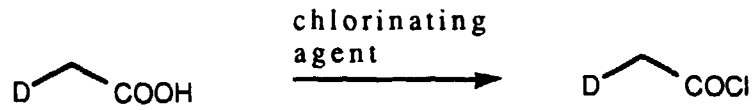


XIII XIV

Step D2

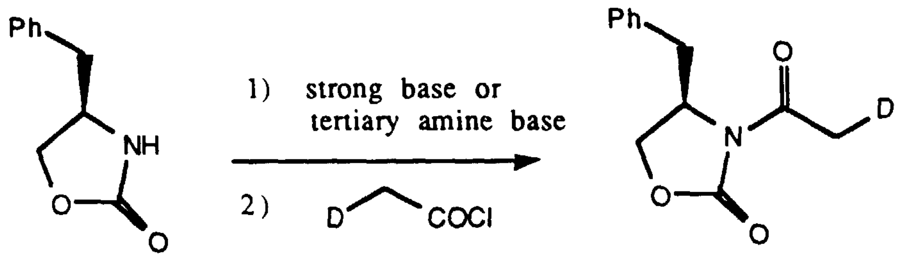


XV XIV XVI

Step D3

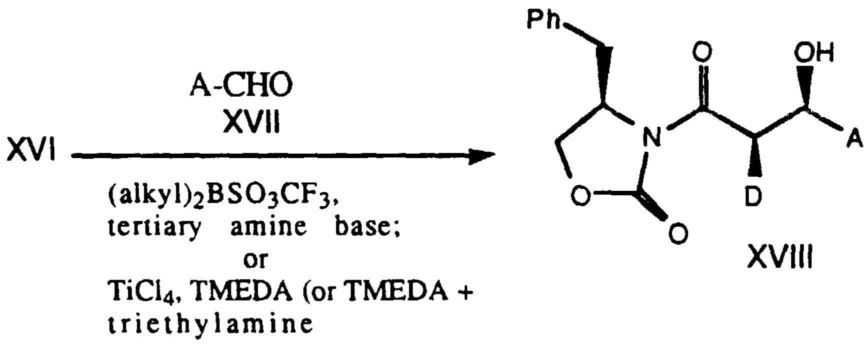


XVIII

Step D4

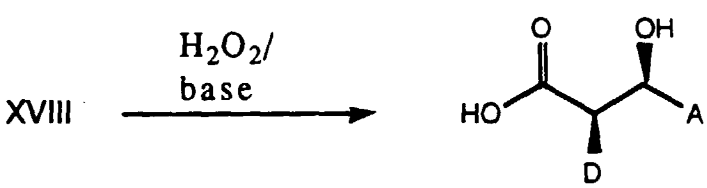


XIX

Step D5

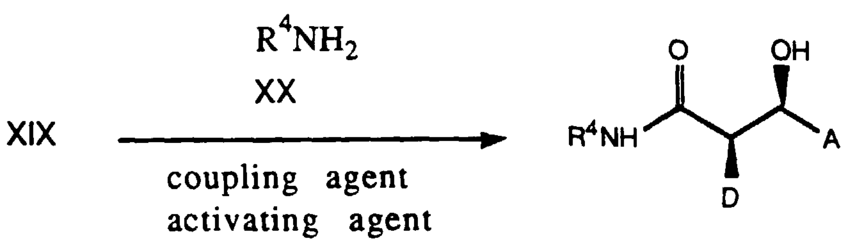


XXI

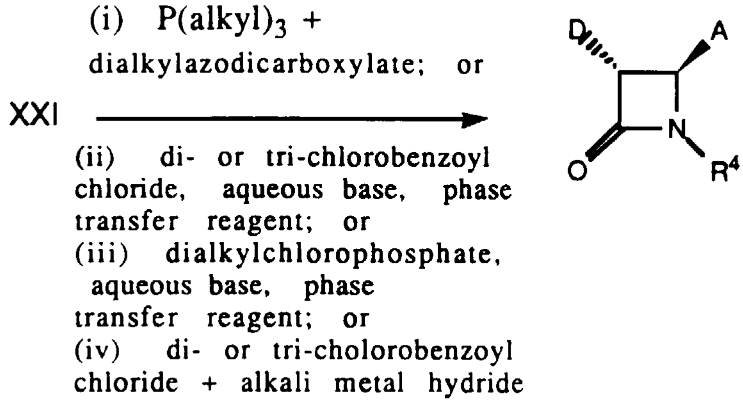


Step D6

In step D1 of Method D, the carboxylic acid XIII is treated with a chlorinating agent, e.g., thionyl chloride or oxalyl chloride, under a dry atmosphere, neat or in a suitable inert organic solvent, e.g., toluene, at 70°C to produce compound XIV.

In step D2, compound XV is converted to compound XVI in a two step reaction, first by deprotonating with a strong base, such as an alkyllithium, e.g., n-butyllithium, or metal hydride, e.g., sodium hydride, or a tertiary amine base, such triethylamine, in a suitable anhydrous organic solvent, e.g., dry THF, under a dry, inert atmosphere, e.g., nitrogen, at about 0°C to about -85°C, preferably about -78°C, over a period of about 30 to about 90 minutes, preferably about 60 minutes, and second by reacting the resulting anion, without isolation, with compound XIV in a suitable anhydrous organic solvent, e.g., dry THF, under a dry, inert atmosphere, e.g., nitrogen, at about -50°C to about -85°C, preferably about -78°C, over a period of about 30 to about 60 minutes, preferably about 45 minutes, followed by continued reaction at about -10°C to about 10°C, preferably about 0°C, for a period of about 30 to about 90 minutes, preferably about 60 minutes, then isolating the product, compound XVI, by extraction.

In step D3, compound XVI is treated with a dialkylboron triflate, e.g., di-n-butylborontriflate ($Bu_2BSO_3CF_3$), in a suitable inert organic solvent, e.g. $CH_2Cl_2$, under a dry, inert atmosphere, e.g., nitrogen, at about -60°C to about 10°C, preferably about -10°C to about 0°C, for a period of about 10 minutes. A tertiary amine base, e.g., diisopropylethylamine, is added at about -10°C to about 0°C, preferably about -6°C to about-3°C, for about 20 to about 40 minutes, preferably about 30 minutes. The mixture is stirred at about -50°C to about -85°C, preferably about -78°C, for about 20 to about 40 minutes, preferably about 30 minutes, then treated with compound XVII, at about -50°C to about -85°C, preferably about -78°C, for about 20 to about 40 minutes, preferably about 30 minutes. The mixture is stirred at about -10°C to about 5°C, preferably about 0°C for about 30 to about 90 minutes, preferably about 60 minutes, then quenched with an aqueous pH 7 buffer solution, e.g., an aqueous solution of KH2P04 and NaOH, and treated with methanol, and hydrogen peroxide, preferably 30% hydrogen peroxide, at about -5°C to about 5°C, preferably about 0°C, for about 1 hour. The product is isolated by extraction and crystallized from a suitable solvent, e.g., hexane/ethyl acetate, to obtain compound XVIII.

Alternatively, step D3 comprises treating compound XVI with titanium tetrachloride ($TiCl_4$), in a suitable inert organic solvent. e.g. $CH_2Cl_2$, at about -60°C to about 0°C, preferably about -25°C to about -15°C, and most preferably at about -20°C, for a period of about 10 minutes. Tetramethylethylenediamine (TMEDA) or a combination of TMEDA and triethylamine is added slowly over a period of about 10 minutes, while maintaining the temperature at about about -25°C to about -10°C. The mixture is stirred at about -25°C to about -10°C, preferably about -15°C to about -10°C, for a period of 30 to 90 minutes, preferably about 60 minutes, then treated with a compound of the formula XVII. The mixture is stirred at about -25°C to about -10°C, preferably about -15°C to about -10°C, for a period of 30 to 90 minutes, preferably about 60 minutes, then stirred for 30 to 60 minutes, preferably about 40 minutes, while warming to about 0°C to about 10°C, preferably about 10°C. The mixture is quenched with an aqueous solution of tartaric acid, preferably a solution of about 10% tartaric acid in water. The product is then isolated by extraction with a suitable solvent, e.g. ethyl acetate, and recrystallized from a suitable solvent, such as ethyl actetate/hexane, to obtain compound XVIII.

In step D4, compound XVIII is treated with hydrogen peroxide, preferably 30% hydrogen peroxide, in a inert organic solvent, e.g., THF/ water, at about -5°C to about 5°C, preferably about 0°C, for about 10 to about 20 minutes, preferably about 15 minutes, then treated with a base, e.g., lithium hydroxide, at about -5°C to about 5°C, preferably about 0°C, until no starting material remains, as determined by thin layer chromatography (TLC), in about 2 hours to about 4 hours, preferably about 3 hours. The excess peracid is reduced by slowly adding a solution of sodium sulfite in water to the mixture over a period of about 30 to about 90 minutes, preferably about 70 minutes. The bulk of the solvent is removed

under vacuum and the residue diluted with water. Compound IV is recovered from the mixture by extraction with a suitable inert organic solvent, e.g., toluene. The remaining aqueous solution is acidified to a pH of about 2.0 to about 3.0, preferably pH of about 2.4, using hydrochloric acid. The product is isolated by extraction using a suitable inert organic solvent, e.g., ethyl acetate, to provide compound XIX.

In step D5, compound XIX is reacted with compound XX, a dehydrative coupling agent, e.g., dicyclohexylcarbodiimide (DCC), and an activating agent, e.g., 1-hydroxybenzotriazole (HOBT), in a suitable inert organic solvent, e.g., dimethylformamide or acetonitrile, at about 25°C to about 50°C, preferably about 40°C. The reaction is continued until the starting material is consumed, as determined by TLC, in about 4 hours. The product is isolated by extraction to obtain compound XXI.

In step D6, compound XXI is cyclized by treating with triphenylphosphine or preferably a trialkylphosphine, e.g., tri-n-butyl-phosphine, and a dialkylazodicarboxylate, e.g., diethylazodicarboxylate (DEAD), in a suitable anhydrous organic solvent, e.g., dry THF, under a dry, inert atmosphere, e.g. nitrogen, at about -50°C to about -85°C, preferably about -70°C, for about 1 to about 3 hours, preferably about 2 hours. The reaction is then continued at about room temperature for about 12 to about 24 hours. The product is purified by preparative high performance liquid chromatography (HPLC) to obtain a compound of formula I, having trans relative stereochemistry. The use of tributylphosphine in this step gives reaction yields significantly higher than the yields obtained using triphenylphosphine.

Alternatively, step D6 comprises combining compound XXI and a suitable phase transfer catalyst, e.g. tetra-n-butylammonium hydrogen sulfate, in a suitable solvent, such as methylene chloride. The mixture is stirred while cooling to about 0°C to 20°C, preferably about 10°C to about 20°C, then treated with an aqueous base, such as an alkali metal hydroxide, preferably 50% aqueous sodium hydroxide. A di- or tri-chlorobenzoyl chloride, preferably 2,6-dichlorobenzoyl chloride or 2,4,6-trichlorobenzoyl chloride, is slowly added over a period of 20 to 60 minutes, preferably about 30 minutes. The mixture is stirred at about 0°C to about 25°C, preferably about 15°C to about 20°C, for a period of 2 to 4 hours, preferably about 3 hours, then poured into cold water. The organic layer is separated and washed with water to neutral pH. The di- or tri-chlorobenzoate product is isolated by crystallization from methylene chloride/heptane. The di- or tri-chlorobenzoate product is combined with a suitable phase transfer catalyst, e.g benzyltriethylammonium chloride, in a suitable solvent, such as a mixture methylene chloride and methyl t-butyl ether. The mixture is stirred at about 0°C to about 25°C, preferably about 15°C to about 20°C, and treated with an aqueous base, e.g. an alkali metal hydroxide, preferably 50% aqueous sodium hydroxide. After stirring for a period of 2 to 6 hours, preferably about 4 hours, the mixture is poured into ice water. The organic layer is washed with water to neutral pH. The product is isolated by removing the solvent, then purified by chromatography and recrystallization from a suitable solvent to give a compound of formula I, having trans relative stereochemistry.

A third alternative for step D6 comprises treating compound XXI in a suitable solvent, such as $CH_2Cl_2$, with a dialkylchlorophosphate, preferably diethylchlorophosphate, and an aqueous base, such as an alkali metal hydroxide, preferably 50% aqueous sodium hydroxide, in the presence of phase transfer catalyst, such as tetra-n-butylammonium hydrogen sulfate or benzyltriethylammonium chloride.

Another alternative for step D6 comprises treating compound XXI with a di- or tri-chlorobenzoyl chloride, preferably 2,6-dichlorobenzoyl chloride or 2,4,6-trichlorobenzoyl chloride, and a suitable base, such as sodium hydride in a suitable solvent, such as $CH_2Cl_2$, dimethylformamide, or a combination thereof. The product is isolated and purified by chromatography followed by crystallization from a suitable solvent, e.g. ether/hexane.

Starting compounds XIII, XV, XVII, and XX are all either commercially available or well known in the art and can be prepared via known methods.

<u>Method E:</u>

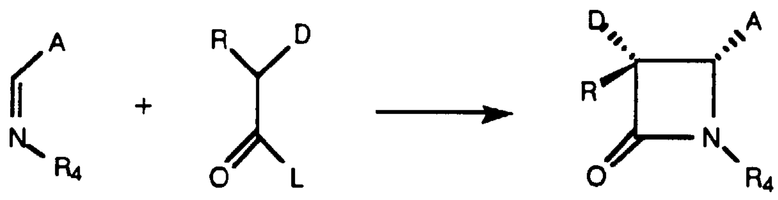

IV XII Ia

Compounds of formula Ia can also be prepared by treatment of an imine of formula IV with an activated carboxylic acid derivative of formula XII in the presence of a base such as triethylamine, tributylamine or diethylisopropylamine in an inert solvent such as $CH_2Cl_2$, heptane or toluene. Examples of activated carboxylic acid derivatives of formula XII include acid chlorides (L=Cl), mixed anhydrides formed with phenyl phosphorodichloridate (L=OP(O)(Cl)OPh), and N-methylpyridinium esters formed from the reaction of an acid with N-methyl-2-chloropyridinium iodide (L=2-oxy-N-methylpyridinium iodide).

Starting materials of formulae IV and V are known or can be prepared by methods well known in the art.

Method F:

**Step F1**

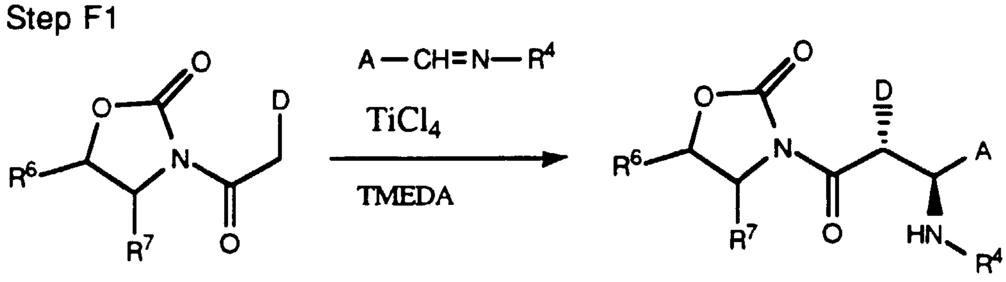


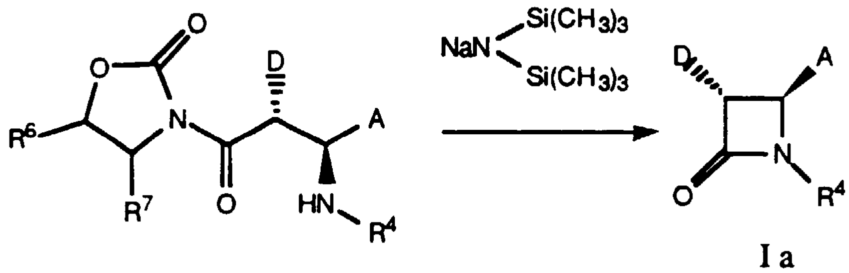


**Step F2**

I a

In step F1, compound XVI (from Method D, step 2) is dissolved in a suitable solvent, e.g. methylene chloride, then treated with titanium tetrachloride at about -60°C to about 0°C, preferably about -25°C to about -15°C, under a dry, inert atmosphere, preferably nitrogen, for a period of about 5 min. TMEDA is added and the mixture stirred at about -60°C to about -10°C, preferably about -25°C to about -20°C, for a period of about 1 hour. The imine (A-CH=N-$R^4$) is slowly over a period of 20 to 40 min., preferably about 30 min., and the mixture is stirred at about -60°C to about 0°C, preferably about -25°C to about -15°C, for 20 to 40 min., preferably about 30 min. The mixture is then warmed to about 0°C and the reaction monitored by high pressure liquid chromatography (HPLC) until complete. The mixture is then poured into a solution of tartaric acid in water, preferably 10% tartaric acid. The product is isolated by extraction with a suitable solvent, e.g. ethyl acetate, then purified by crystallization.

In step F2, the product of step F1 is treated with a strong non-nucleophilic base, such as sodium or lithium bistrimethylsilylamide, in a suitable inert organic solvent, e.g. $CH_2Cl_2$, at about -20°C to about 10°C, preferably about 0°C. The mixture is stirred while gradually warming to about 20° to about 25°C, then monitored by HPLC until the starting material is gone, typically after a period of 1 to 2 hours. The reaction mixture is poured into aqueous tartaric acid, preferably 10% tartaric acid, and the product isolated from the organic layer.

Imines of the formula A-CH=N-$R^4$ can be prepared from aldehydes of the formula A-CHO and amines of the formula $R^4$-$NH_2$ by procedures well known in the art. Aldehydes of formula A-CHO and amines of formula $R^4$-$NH_2$ are commercially available or can be prepared via known procedures.

Compounds of formula II can be prepared by methods similar to those described for compounds of formula I.

It will also be apparent to those skilled in the art, and by reference to the examples which follow, that compounds of formulae I and II can be converted into different compounds of formula I or II by we known methods. For example, a compound of formula I wherein A comprises a double or triple bond, or a compound of formula II wherein G comprises a double or triple bond can be converted to the corresponding saturated compound by treatment with hydrogen gas in the presence of a catalyst such as palladium or platinum on carbon.

Reactive groups not involved in the above processes can be protected during the reactions with conventional protecting groups which can be removed by standard procedures after the reaction. The following Table 3 shows some typical protecting groups:

Table 3

| Group to be Protected | Group to be Protected and Protecting Group |
|---|---|
| $-COOH$ | $-COOalkyl$, $-COObenzyl$, $-COOphenyl$ |
| 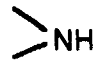 | 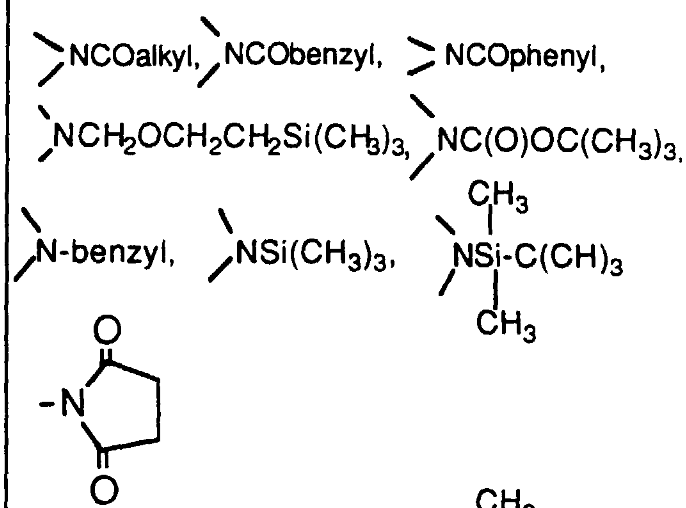 |
| $-NH_2$ | |
| $-OH$ | $-OCH_3$, $-OSi(CH_3)_3$, 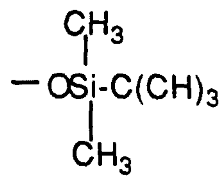 |

We have found that the compounds of this invention lower serum lipid levels, in particular serum cholesterol levels. Compounds of this invention have been found to inhibit the intestinal absorbtion of cholesterol and to significantly reduce the formation of liver cholesteryl esters in animal models. Some compounds also inhibit ACAT in vitro. Thus, compounds of this invention are hypocholesterolemic agents by virtue of their ability to inhibit the esterification and/or intestinal absorption of cholesterol; they are therefore useful in the treatment and prevention of atherosclerosis in mammals, in particular in humans.

In addition to the compound aspect, the present invention therefore also relates to a method of lowering serum cholesterol levels, which method comprises administering to a mammal in need of such treatment a hypocholesterolemic effective amount of a compound of formula I or II of this invention. The compound is preferably administered in a pharmaceutically acceptable carrier suitable for oral administration.

The in vitro and in vivo activity of the compounds of formulae I or II can be determined by the following procedures.

ACAT Assay (in vitro)

This assay measures the activity of ACAT by measuring the ACAT-mediated transfer of tritiated oleic acid from acyl-CoA to cholesterol to give labelled cholesteryl oleate. Rat liver microsomes are used as the source of ACAT. Assays are performed in round bottom microtiterplates using a total incubation volume of 50 µL. Each incubation well receives 10 µL assay buffer (0.5M $KHPO_4$, 10 µM dithiothreitol, pH 7.4), 7.5 µL of 40 mg/mL BSA (Bovine Serum Albumin) and 12.5 µg of microsomal protein. The test compound (in sufficient amount to bring the final concentration to from 0.1 to 25 µM), reference compound, or vehicle control is added and the final volume brought to 47 µL. The microtiterplate is then floated on the surface of a 37°C water bath for fifteen minutes. Incubations are started by the addition of 3 µL 3H-acyl CoA (1µCi/well, final concentration of 10 µM acyl CoA). The plate is then returned to the water bath for 15 minutes. The incubations are then terminated by application of 15 µL from each incubation to individual lanes on a thin layer plate (Silica Gel GF 20 x 20 cm). Standards are applied to several lanes so that the cholesteryl ester band can be identified. After drying, the plates are eluted with 90:10:1 petroleum ether:diethyl ether:acetic acid. The standards are visualized via iodine vapor, and the regions corresponding to cholesteryl ester are scraped into 7 mL scintillation vials. 4 mL of scintillant are added to each vial, and the radioactivity quantified. Background count is determined by the boiled controls. Full activity is determined by activity in the presence of vehicle. The percent inhibition

is calculated by subtracting the background from both control and test samples, and the test value is calculated as a percentage of the control. For $IC_{50}$ determinations, the inhibition is plotted against drug does on a log scale and the concentration at which 50% inhibition is obtained is determined.

In Vivo Assay of Hypolipidemic Agents Using the Hyperlipidemic Hamster

Hamsters are separated into groups of six and given a controlled cholesterol diet (Purina Chow #5001 containing 0.5% cholesterol) for seven days. Diet consumption is monitored to determine dietary cholesterol exposure in the face of test compounds. The animals are dosed with the test compound once daily beginning with the initiation of diet. Dosing is by oral gavage of 0.2mL of corn oil alone (control group) or solution (or suspension) of test compound in corn oil. All animals moribund or in poor physical condition are euthanized. After seven days, the animals are anesthetized by IM injection of ketamine and sacrificed by decapitation. Blood is collected into vacutainer tubes containing EDTA for plasma lipid analysis and the liver excised for tissue lipid analysis. Data is reported as percent reduction of lipid versus control.

The present invention also relates to a pharmaceutical composition comprising a compound of formula I or II of this invention and a pharmaceutically acceptable carrier. The compounds of formula I or II can be administered in any conventional oral dosage form such as capsules, tablets, powders, cachets, suspensions or solutions. The formulations and pharmaceutical compositions can be prepared using conventional pharmaceutically acceptable excipients and additives and conventional techniques. Such pharmaceutically acceptable excipients and additives include non-toxic compatible fillers, binders, disintegrants, buffers, preservatives, anti-oxidants, lubricants, flavorings, thickeners, coloring agents, emulsifiers and the like.

The daily hypocholesteremic dose of a compound of formula I or II is about 7 to about 30 mg/kg of body weight per day. For an average body weight of 70kg, the dosage level is therefore from about 500 to about 2000 mg of drug per day, given in a single dose or 2-4 divided doses . The exact dose, however, is determined by the attending clinician and is dependent on the potency of the compound administered, the age, weight, condition and response of the patient.

Following are examples of preparing compounds of formulae I and II. The stereochemistry listed is relative stereochemistry unless otherwise noted. The terms cis/trans refer to the relative orientations at the beta-lactam 3- and 4-positions when each is monosubstituted (i.e.. R=H).

EXAMPLE 1

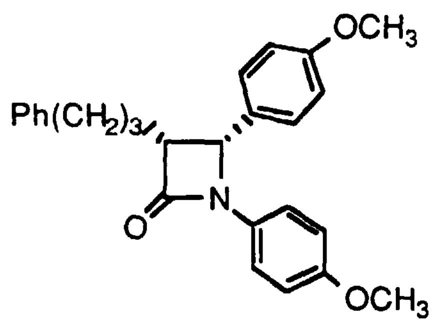

Freshly prepare lithium diisopropyl amide (LDA) by dissolving 23.96mL (17.39g, 172mmol) diisopropylamine in 230mL dry THF at -78°C under nitrogen. Add 103.9mL (166 mmol @ 1.6M in hexanes) of *n*-butyl lithium and stir at -78°C for 1h. To this cold solution add 32.58g (158mmol) of 5-phenyl valeric acid ethyl ester in 195mL dry THF over ~1h, keeping the reaction temperature below -65°C. Stir for 1h at -78°C, then add 38.13g (158mmol) of 4-methoxybenzylidine anisidine in 350mL dry $CH_2Cl_2$. Allow the reaction to slowly come to room temperature and the precipitate that forms will begin to go into solution. Stir the reaction mixture for 16h at room temperature. Partition the mixture between 1.2liter of 1N aqueous hydrochloric acid (HCl) and 1 liter of ether. Wash the ether layer with 300 mL 1N HCl. Combine the acid layers and extract with 1 liter of ether. Combine the ether extracts, dry over $MgSO_4$ and concentrate in vacuo. Crystallize the residue (35.08g, 55%) from ~200mL ethyl acetate-hexane (1:1) to give 32.05g of the title racemic compound as an off-white crystal, mp = 90-93°C.

Using a similar procedure, the following compounds shown in Tables 4 and 4A can also be prepared:

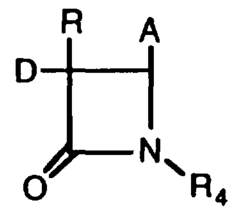

| Ex. | D | R | A | Relative Stereo-chemistry | $R_4$ | Data |
|---|---|---|---|---|---|---|
| 1A | $C_6H_5$-$CH_2C(O)$- | $C_6H_5$- | $C_6H_5$- | 3R,4S | p-MeO-$C_6H_4$- | mp=120.5-122.0°C |
| 1B | $C_6H_5$-$(CH_2)_3$- | H | $C_6H_5$- | 3R,4R | p-MeO-$C_6H_4$- | mp=120.5-121.5°C |
| 1C | $C_6H_5$-$(CH_2)_3$- | H | $C_6H_5$- | 3S,4R | p-MeO-$C_6H_4$- | mp=92.0-92.5°C |
| 1D | $C_6H_5$-$(CH_2)_2$- | H | $C_6H_5$- | 3S,4S | p-MeO-$C_6H_4$- | mp=151-152°C |
| 1E | $C_6H_5$-$(CH_2)_2$- | H | $C_6H_5$- | 3S,4R | p-MeO-$C_6H_4$- | mp=156-158°C |
| 1F | $C_6H_5$-$(CH_2)_3$- | H | $C_6H_5$- | 4:1 cis to trans | 2,4,6-tri-MeO-$C_6H_4$- | CI MS: (M+1)432 |
| 1G | $C_6H_5$-$(CH_2)_3$- | H | p-$NO_2$-$C_6H_4$- | 3S,4R | p-MeO-$C_6H_4$- | mp=116-117°C |
| 1H | $C_6H_5$-$(CH_2)_3$- | H | p-$NO_2$-$C_6H_4$- | 3R,4R | p-MeO-$C_6H_4$- | mp=120.5-122.0°C |
| 1I | $C_6H_5$-$(CH_2)_3$- | $CH_3CH_2$- | -$C_6H_5CH$=CH- | 3S,4S | 2,4,6-tri-MeO-$C_6H_4$- | FAB MS: (M+1)486.2 |
| 1J | $C_6H_5$-$(CH_2)_3$- | $CH_3CH_2$- | $C_6H_5$-CH=CH- | 3R,4S | 2,4,6-tri-MeO-$C_6H_4$- | FAB MS: (M+1)486.2 |

| | | | | | | |
|---|---|---|---|---|---|---|
| 1K | $C_6H_5$-$(CH_2)_3$- | H | $C_6H_5$-CH=CH- | 3S,4S | p-MeO-$C_6H_4$- | mp=119.5-120.5°C |
| 1L | $C_6H_5$-$(CH_2)_3$- | H | p-OH-$C_6H_4$- | 3S,4S | p-MeO-$C_6H_4$- | mp=152.5-155.0°C |
| 1M | $C_6H_5$-$(CH_2)_3$- | H | p-MeO-$C_6H_4$-CH=CH- | 3S,4S | p-MeO-$C_6H_4$- | mp=86-88°C |
| 1P | $C_6H_5$-$(CH_2)_3$- | H | 3-MeO-$C_6H_4$- | 3S,4S | p-MeO-$C_6H_4$- | mp=90.5-91.0°C |
| 1AM | $C_6H_5$-$(CH_2)_3$- | H | p-MeO-$C_6H_4$- | trans | 3-pyridyl- | 1H NMR (CDC13) δ 8.41 (s,1H), 8.28(s, 1H), 7.79(d, 1H), 7.20(m, 8H), 6.90(d, 2H), 4.63(s, 1H), 3.80(s, 3H), 3.20(t, 1H), 1.85(m, 4H) |
| 1AN | $C_6H_5$-$(CH_2)_3$- | H | p-MeO-$C_6H_4$- | trans | p-$CF_3$O-$C_6H_4$- | elemental analysis: calc'd C=68.56, H=5.31, N=3.08; found C=68.32, H=5.12, N=2.97 |
| 1AO | $C_6H_5$-$(CH_2)_3$- | H | 4-MeO-$C_6H_4$- | cis | p-Cl-$C_6H_4$- | elemental analysis: calc'd C=73.97, H=5.96, N=3.45, Cl=8.73; found C=73.63, H=5.92, N=3.52, Cl=9.12 |

| | | | | | | |
|---|---|---|---|---|---|---|
| 1AP | $C_6H_5$-$(CH_2)_3$- | H | 4-MeO-$C_6H_4$- | cis | p-$CH_3$-$C_6H_4$- | elemental analysis: calc'd C=81.01, H=7.06, N=3.63; found C=80.97, H=7.06, N=3.74 |
| 1AQ | $C_6H_5$-$(CH_2)_3$- | H | 4-$CH_3O$-$C_6H_4$- | cis | p-$C_6H_5O$-$C_6H_4$- | elemental analysis: calc'd C=80.32, H=6.31, N=3.02; found C=80.26, H=6.27, N=3.18 |
| 1AR | $C_6H_5$-$(CH_2)_3$- | H | 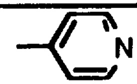 | trans | p-$CH_3O$-$C_6H_4$- | mp=101.5-102.5°C |
| 1AS | $C_6H_5$-$(CH_2)_3$- | H | $(CH_3)_3CSi(CH_3)_2$-$OCH_2$-$C_6H_4$- | trans | p-$CH_3O$-$C_6H_4$- | EIMS (M+1)=516 |
| 1AT | $C_6H_5$-$(CH_2)_3$- | H | $(CH_3)_3CSi(CH_3)_2$-$OCH_2$-$C_6H_4$- | cis | p-$CH_3O$-$C_6H_4$- | mp=84-85°C |
| 1AU | $C_6H_5$-$(CH_2)_3$- | H | 4-$CH_3S$-$C_6H_4$- | cis | p-$CH_3O$-$C_6H_4$- | mp=103-104°C |
| 1AV | $C_6H_5$-$(CH_2)_3$- | H | 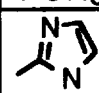 $CH_2O(CH_2)_2Si(CH_3)_3$ | trans | p-$CH_3O$-$C_6H_4$- | FABMS (M+1)=492 |
| 1AW | $C_6H_5$-$(CH_2)_3$- | H | 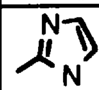 $CH_2O(CH_2)_2Si(CH_3)_3$ | cis | p-$CH_3O$-$C_6H_4$- | mp=108.5-110°C |

| | | | | | | |
|---|---|---|---|---|---|---|
| 1AX | $C_6H_5$-$(CH_2)_3$- | H | 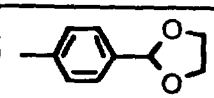 | | p-$CH_3O$-$C_6H_4$- | EIMS (M)=443 |
| 1AY | $C_6H_5$-$(CH_2)_3$- | H | 4-($(CH_3)_2CH$-O)-$C_6H_4$- | cis | p-$CH_3O$-$C_6H_4$- | mp=96-97°C |
| 1AZ | $C_6H_5$-$(CH_2)_3$- | H | 4-($CH_3(CH)_2$-O)-$C_6H_4$- | cis | p-$CH_3O$-$C_6H_4$- | mp=96-97°C |
| 1BA | $C_6H_5$-$(CH_2)_3$- | H | 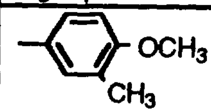 | cis | p-$CH_3O$-$C_6H_4$- | mp=91.5-92.5°C |
| 1BB | $C_6H_5$-$(CH_2)_3$- | H | 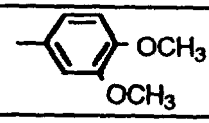 | cis | p-$CH_3O$-$C_6H_4$- | mp=86.0-87.0°C |
| 1BC | $C_6H_5$-$(CH_2)_3$- | H | 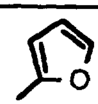 | cis | p-$CH_3O$-$C_6H_4$- | mp=130.0-131.0°C |
| 1BD | $C_6H_5$-$(CH_2)_3$- | H | 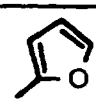 | trans | p-$CH_3O$-$C_6H_4$- | mp=111.0-112.5°C |
| 1BE | $C_6H_5$-$(CH_2)_3$- | H | 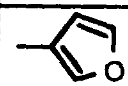 | cis | p-$CH_3O$-$C_6H_4$- | mp=74.5-75°C |
| 1BF | $C_6H_5$-$(CH_2)_3$- | H | 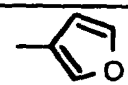 | trans | p-$CH_3O$-$C_6H_4$- | mp=79.5-80.5°C |
| 1BG | $C_6H_5$-$(CH_2)_3$- | H | 4-$CF_3$-$C_6H_4$- | trans | p-$CH_3O$-$C_6H_4$- | mp=94.5-96.0°C |
| 1BH | $C_6H_5$-$(CH_2)_3$- | H | 4-$CF_3$-$C_6H_4$- | cis | p-$CH_3O$-$C_6H_4$- | mp=114.0-115.5°C |

| | | | | | | |
|---|---|---|---|---|---|---|
| 1BI | $C_6H_5-(CH_2)_3-$ | H | 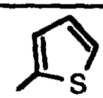 | cis | $p-CH_3O-C_6H_4-$ | mp=117-120°C |
| 1BJ | $C_6H_5-(CH_2)_3-$ | H | 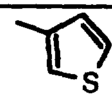 | cis | $p-CH_3O-C_6H_4-$ | mp=88.5-90.5°C |
| 1BK | $C_6H_5-(CH_2)_3-$ | H | $3-CF_3O-C_6H_4-$ | cis | $p-CH_3O-C_6H_4-$ | mp=66.0-68.0°C |
| 1BL | $C_6H_5-(CH_2)_3-$ | H | $3-CF_3O-C_6H_4-$ | trans | $p-CH_3O-C_6H_4-$ | CI MS (M+1)=456.2 |
| 1BM | $C_6H_5-(CH_2)_3-$ | H | $4-CH_3-C_6H_4-$ | cis | $p-CH_3O-C_6H_4-$ | mp=124-125°C |
| 1BN | $C_6H_5-(CH_2)_3-$ | H | $4-(C_6H_5-CH_2-O)-C_6H_4-$ | cis | $p-CH_3O-C_6H_4-$ | mp=131-132°C |
| 1BO | $C_6H_5-(CH_2)_3-$ | H | $CH_3CH_2-O-C_6H_4-$ | cis | $p-CH_3O-C_6H_4-$ | mp=84-85°C |
| 1BP | $C_6H_5-(CH_2)_3-$ | H | 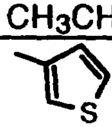 | trans | $p-CH_3O-C_6H_4-$ | mp=68-70°C |
| 1BQ | $C_6H_5-(CH_2)_3-$ | H | 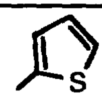 | trans | $p-CH_3O-C_6H_4-$ | mp=50-52°C |
| 1BR | $C_6H_5-(CH_2)_3-$ | H | $CH_3(CH_2)_3-O-C_6H_4-$ | cis | $p-CH_3O-C_6H_4-$ | mp=71.0-72.5°C |
| 1BS | $C_6H_5-(CH_2)_3-$ | H | $4-(C_6H_5-O)-C_6H_4-$ | cis | $p-CH_3O-C_6H_4-$ | mp=108-109°C |
| 1BT | $C_6H_5-(CH_2)_3-$ | H | $4-(C_6H_5)-C_6H_4-$ | cis | $p-CH_3O-C_6H_4-$ | mp=127.5-128.5°C |
| 1BU | $C_6H_5-(CH_2)_3-$ | H | $4-(CH_2=CH-CH_2-O)-C_6H_4-$ | cis | $p-CH_3O-C_6H_4-$ | mp=77.0-77.5°C |

| | | | | | | |
|---|---|---|---|---|---|---|
| 1BV | $C_6H_5$-$(CH_2)_3$- | H | 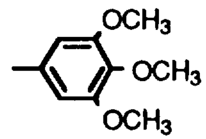 | cis | p-$CH_3O$-$C_6H_4$- | mp=116-117°C |
| 1BW | $C_6H_5$-$(CH_2)_3$- | H | 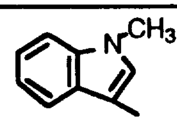 | cis | p-$CH_3O$-$C_6H_4$- | mp=129.5-130.5°C |
| 1BX | $C_6H_5$-$(CH_2)_3$- | H | 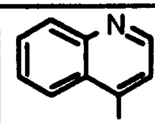 | cis | p-$CH_3O$-$C_6H_4$- | mp=170.0-170.5°C |
| 1BY | $C_6H_5$-$(CH_2)_3$- | H | 4-$(CH_3CH_2$-O)-$C_6H_4$- | cis | $C_6H_5$- | mp=89.5-90.0°C |
| 1BZ | $C_6H_5$-$(CH_2)_3$- | H | 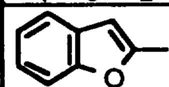 | cis | p-$CH_3O$-$C_6H_4$- | mp=100-104°C |
| 1CA | $C_6H_5$-$(CH_2)_3$- | H | 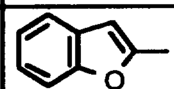 | trans | 4-$CH_3O$-$C_6H_4$- | mp=91-92°C |
| 1CB | $C_6H_5$-$(CH_2)_3$- | H | 4-$CH_3O$-$C_6H_4$- | cis | $(CH_3)_3CSi(CH_3)_2$-O-$C_6H_4$- | mp=93-94°C |
| 1CC | $C_6H_5$-$(CH_2)_2$- | H | 4-$CH_3O$-$C_6H_4$- | cis | 4-$CH_3O$-$C_6H_4$- | mp=128-129°C |
| 1CD | $C_6H_5$-O-$(CH_2)_2$- | H | 4-$CH_3O$-$C_6H_4$- | cis | 4-$CH_3O$-$C_6H_4$- | mp=120-121°C |
| 1CE | $C_6H_5$-$(CH_2)_6$- | H | 4-$CH_3O$-$C_6H_4$- | cis | 4-$CH_3O$-$C_6H_4$- | mp=80-81°C |
| 1CF | 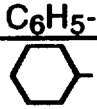 $(CH_2)_3$– | H | 4-$CH_3O$-$C_6H_4$- | cis | 4-$CH_3O$-$C_6H_4$- | mp=101-102°C |

| | | | | | | |
|---|---|---|---|---|---|---|
| 1CG | 4-F-$C_6H_4$-O-$(CH_2)_2$- | H | 4-$CH_3O$-$C_6H_4$- | cis | 4-$CH_3O$-$C_6H_4$- | elemental analysis: calc'd C=71.25, H=5.74, N=3.32; found C=71.34, H=5.73, N=3.43 |
| 1CH | 4-F-$C_6H_4$-O-$(CH_2)_2$- | H | 4-$CH_3O$-$C_6H_4$- | cis | $C_6H_5$- | elemental analysis: calc'd C=73.64, H=5.67, N=3. ; found C=73.39, H=5.51, N=3.54 |
| 1CI | $C_6H_5$-$(CH_2)_4$- | H | 4-$CH_3O$-$C_6H_4$- | cis | 4-$CH_3O$-$C_6H_4$- | elemental analysis: calc'd C=78.04, H=7.03, N=3.37; found C=77.69, H=6.98, N=3.60 |
| 1CM | $C_6H_5$-$(CH_2)_3$- | H | 4-$CH_3S$-$C_6H_4$- | trans | 4-$CH_3O$-$C_6H_4$- | mp=104-105.5°C |
| 1CN | $C_6H_5$-$(CH_2)_3$- | H | 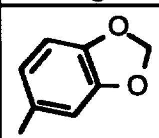 | cis | 4-$CH_3O$-$C_6H_4$- | mp=144.0-144.5°C |
| 1CO | $C_6H_5$-$(CH_2)_3$- | H | 4-($(CH_3)_2N$-$(CH_2)_3$-O)-$C_6H_4$- | cis | 4-$CH_3O$-$C_6H_4$- | mp=92-94°C |

| | | | | | | |
|---|---|---|---|---|---|---|
| 1CP | $C_6H_5-(CH_2)_3-$ | H | $4-CH_3O-C_6H_4-$ | cis | $C_6H_5-$ | elemental analysis: calc'd C=80.83, H=6.78, N=3.77; found C=80.69, H=6.78, N=3.90 |

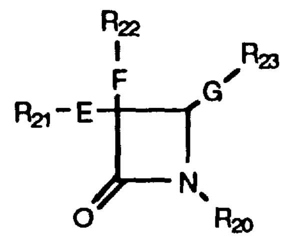

| Ex. | $R_{21}$-E- | $R_{22}$-F- | $R_{23}$-G- | $R_{20}$ | Rel. Stereo-chemistry | Data |
|---|---|---|---|---|---|---|
| 1S | $C_{10}H_{21}-$ | H | $C_6H_5-$ | p-MeO-$C_6H_4-$ | 3R,4R | mp=176.0-177.0°C |
| 1T | $C_{10}H_{21}-$ | H | $C_6H_5-$ | p-MeO-$C_6H_4-$ | 3S,4R | mp=83.5-84.0°C |
| 1U | $C_{10}H_{21}-$ | H | $C_6H_5-$ | 2,4,6-tri-MeO-$C_6H_4-$ | 3R,4R 3.6:1 cis/trans | |
| 1V | H | $C_6H_5-$ | $C_6H_5-$ | p-MeO-$C_6H_4-$ | 3R,4S | mp=158.5-159.0°C |
| 1W | H | i-Pr | $C_6H_5-$ | p-MeO-$C_6H_4-$ | 3R,4R | mp=162-164°C |
| 1X | $C_6H_5-CH_2-$ | $C_6H_5-$ | $C_6H_5-$ | p-MeO-$C_6H_4-$ | 3S,4S | mp=185.0-186.0°C |
| 1Y | $C_6H_5-CH_2-$ | $C_6H_5-$ | $C_6H_5-$ | p-MeO-$C_6H_4-$ | 3R,4S | mp=123.0-124.5°C |

| | | | | | | |
|---|---|---|---|---|---|---|
| 1Z | $CH_3CH_2$- | $C_6H_5$- | $C_6H_5$- | p-MeO-$C_6H_4$- | 3S,4S | mp=101.0-102.5ºC |
| 1AA | $CH_3CH_2$- | $C_6H_5$- | $C_6H_5$- | p-MeO-$C_6H_4$- | 3R,4S | mp=73.5-75.0ºC |
| 1AB | H | $CH_3CH_2$- | $C_6H_5$- | p-MeO-$C_6H_4$- | 3R,4R | mp=134.0-135.5ºC |
| 1AC | cyc-$C_5H_{11}$ | $C_6H_5$- | $C_6H_5$- | p-MeO-$C_6H_4$- | 8:5 mix cis/trans phenyl | mp=57.5-62.5ºC |
| 1AD | H | $CH_3CH_2$- | $C_6H_5$- | p-MeO-$C_6H_4$- | 3R,4S | mp=107-109ºC |
| 1AE | H | $CH_3CH_2$- | $C_6H_5$- | 2,4,6-tri-MeO-$C_6H_4$- | 3R,4R | CI MS: (M+1)342 |
| 1AF | H | $CH_3CH_2$- | $C_6H_5$-CH=CH- | p-MeO-$C_6H_4$- | 3R,4S | FAB MS:(M+1)307.9 |
| 1AG | H | $CH_3CH_2$- | $C_6H_5$-CH=CH- | p-MeO-$C_6H_4$- | 3S,4S | mp=84-86ºC |
| 1AH | H | $CH_3$- | $C_6H_5$- | p-MeO-$C_6H_4$- | 3S,4S | mp=122-123ºC |
| 1AI | H | $CH_3$- | $C_6H_5$- | p-MeO-$C_6H_4$- | 3S,4R | mp=93-94ºC |
| 1AJ | H | $CH_3CH_2$- | p-$NO_2$-$C_6H_4$- | p-MeO-$C_6H_4$- | 3S,4R | mp=115-116ºC |
| 1AK | H | $CH_3CH_2$- | 4-$NO_2$-$C_6H_4$- | 4-MeO-$C_6H_4$- | 3R,4R | mp=101-102ºC |
| 1AL | H | $CH_3CH_2$- | 4-MeO-$C_6H_4$- | 4-MeO-$C_6H_4$- | 3S,4S | mp=96-103ºC |

| | | | | | | |
|---|---|---|---|---|---|---|
| 1CJ | $(CH_3)_2$-N- | H | 4-MeO-$C_6H_4$- | $C_6H_5$- | cis | mp=115-117°C |
| 1CK | $C_6H_5$- | $CH_3CH_2$- | 4-MeO-$C_6H_4$- | 4-MeO-$C_6H_4$- | trans | mp=56-58°C |
| 1CL | $C_{10}H_{21}$- | H | 4-MeO-$C_6H_4$- | 4-MeO-$C_6H_4$- | cis | elem. anal.: calc'd C=76.56, H=8.80, N=3.31; found C=76.60, H=8.77, N=3.48 |

EXAMPLE 2

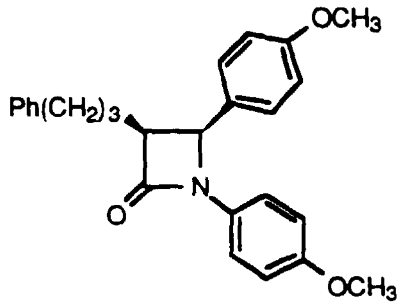

STEP 1: Add 39.1 mL (117.2 mmol) of ethyl magnesium bromide to a 0°C solution of 31.00g (97.6 mmol) of (-)-10-(diisopropylsulfonamido)-isoborneol (See Oppolzer, et al, Tet. Lett., 25 (1984), p. 5885) in 370mL dry THF. Stir the mixture for 0.5h at 0°C, then at room temperature for 0.5h. Add 37.74mL (39.67g, 117.2mmol) of 5-phenylvaleric acid anhydride to this mixture at 0°C and stir overnight at room temperature. Pour the mixture into 1 liter of half-saturated $NaHCO_3$ and extract with two-800mL portions of hexane. Dry the combined hexane layers with $Na_2SO_4$ and concentrate in vacuo. Chromatograph the residue (57.35g) in three portions over -800g $SiO_2$ eluting with 2% ethyl acetate-$CH_2Cl_2$ to obtain the desired ester (42.07g, 90.2%), FAB MS : (M+1) 479.

STEP 2: Using a procedure similar to Example 1, treat the ester of Step 1, washing the ether layer with 200mL 1N aqueous HCl, drying with $MgSO_4$ and concentrating in vacuo. Chromatograph the residue over 1 kg $SiO_2$, eluting with 20% ethyl acetate-hexane to give 24.19g (79%) of the recovered alcohol and 25.66 (66%) of the title enantiomerically enriched β-lactam. This lactam can be further enriched by chromatography over a Chiralcel™ OD column (Daicel Chemical Industries, Ltd., Fort Lee, NJ), eluting with 10% isopropanol-hexanes. Crystallize the resulting enantiomerically pure (3S,4S) compound from ether-hexane to obtain a white solid, mp = 84 - 85°C, $[\alpha]_D^{25}$ = -98.0° (MeOH).

Using a similar procedure, the following enantiomerically pure (3R,4R) compound (EXAMPLE 2A) can also be prepared:

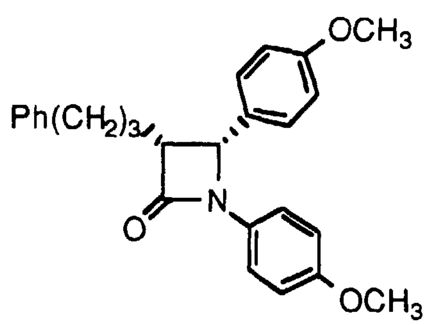

(m.p. = 84-85°C; FAB MS: (M+1) 402.2; $[\alpha]_D^{25}$ +98.0° (MeOH)).

EXAMPLE 3

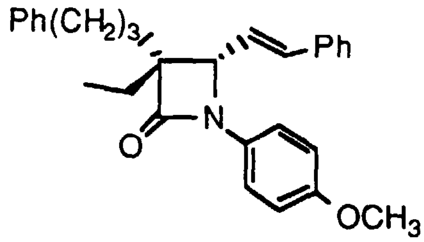

Freshly prepare a solution of lithium isopropylcyclohexyl amide (LICA) by dissolving 0.68mL (0.58g, 4.11 mmol) isopropylcyclohexyl amine in 20mL THF at -78°C under nitrogen. Add 2.58mL (4.03mmol) *n*-butyl lithium (1.6M, from Aldrich, Milwaukee, WI) and stir at -78°C for 1h. Add 1.04g (2.61 mmol) of the compound of Example 1K in 5mL dry THF to the solution. After 2h at -78°C, add 2.8mL (2.89g, 16mmol) of hexamethylphosphoric triamide followed by 0.33mL (641mg, 4.11 mmol) of ethyl iodide at -78°C. Stir the mixture overnight at room temperature. Quench the reaction with 40mL 1N aqueous HCl and extract with two-50mL portions of $CH_2Cl_2$. Combine the $CH_2Cl_2$ layers and wash sequentially with 50mL 1N aqueous HCl and 50mL $Na_2SO_3$. Dry over $MgSO_4$ and concentrate in vacuo. Chromatograph the residue over 40g $SiO_2$, eluting with 20% ethyl acetate-hexane to give 0.95g (83%) of the title compound as a colorless oil, FAB MS : (M+1) 426.4

Using a similar procedure, the following compounds shown in Tables 5 and 5Acan also be prepared:

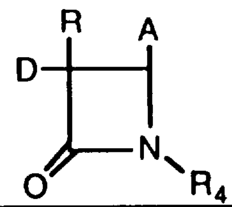

| Ex. | D | R | A | Relative Stereo-chemistry | $R_4$ | Data |
|---|---|---|---|---|---|---|
| 3A | $C_6H_5-(CH_2)_3-$ | $CH_3CH_2-$ | $C_6H_5-$ | 3S,4S | p-MeO-$C_6H_4$- | mp=103.0-103.5°C |
| 3B | $C_6H_5-(CH_2)_3-$ | $C_6H_5-$ | $C_6H_5-$ | 3R,4S | p-MeO-$C_6H_4$- | FAB MS: (M+1) 448 |
| 3C | $C_6H_5-(CH_2)_3-$ | $C_{10}H_{21}-$ | $C_6H_5-$ | 3S,4R | p-MeO-$C_6H_4$- | CI MS: (M+1) 512 |
| 3D | $C_6H_5-(CH_2)_3-$ | $CH_3CH_2-$ | $C_6H_5-$ | 3S,4S | 2,4,6-tri-MeO-$C_6H_4$- | mp=145.5-147.0°C |
| 3E | $C_6H_5-(CH_2)_3-$ | $CH_3CH_2-$ | $C_6H_5-$ | 3R,4S | p-MeO-$C_6H_4$- | mp=82.5-83.5°C |
| 3F | $C_6H_5-(CH_2)_3-$ | $CH_3CH_2-$ | $C_6H_5-$ | 3R,4S | 2,4,6-tri-MeO-$C_6H_4$- | mp=132-134°C |
| 3G | $C_6H_5-(CH_2)_3-$ | $CH_3CH_2-$ | $C_6H_5-CH=CH-$ | 3S,4S | p-MeO-$C_6H_4$- | mp=98-99°C |
| 3H | $C_6H_5-(CH_2)_2-$ | $CH_3CH_2-$ | $C_6H_5-$ | 3R,4S | p-MeO-$C_6H_4$- | mp=100.0-100.5°C |
| 3I | $C_6H_5-(CH_2)_2-$ | $CH_3-$ | $C_6H_5-$ | 3R,4S | p-MeO-$C_6H_4$- | mp=130-131°C |

| Ex. | D | R | A | Relative Stereo-chemistry | $R_4$ | Data |
|---|---|---|---|---|---|---|
| 3J | $C_6H_5$-$(CH_2)_3$- | $CH_3$- | $C_6H_5$- | 3S,4S | p-MeO-$C_6H_4$- | mp=88-89°C |
| 3K | $C_6H_5$-$(CH_2)_4$- | $CH_3$- | $C_6H_5$- | 3S,4S | p-MeO-$C_6H_4$- | mp=109-110°C |
| 3L | $C_6H_5$-$(CH_2)_4$- | $CH_3CH_2$- | $C_6H_5$- | 3S,4S | p-MeO-$C_6H_4$- | mp=95-96°C |
| 3M | $C_6H_5$-$(CH_2)_3$- | $CH_3CH_2$- | p-MeO-$C_6H_4$- | 3R,4S | p-MeO-$C_6H_4$- | FAB MS: (M+1) 430.4 |
| 3N | $C_6H_5$-$(CH_2)_3$- | $C_6H_4$-$(CH_2)_3$- | $C_6H_5$-CH=CH- | racemic | p-MeO-$C_6H_4$- | FAB MS: (M+1) 516.4 |
| 3O | $C_6H_5$-$(CH_2)_3$- | $CH_3CH_2$- | p-MeO-$C_6H_4$- | 3S,4S | p-MeO-$C_6H_4$- | FAB MS: (M+1) 430.4 |

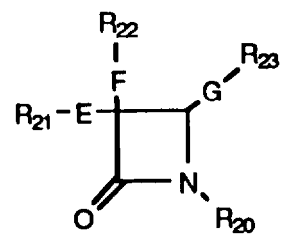

| Ex. | $R_{21}$-E- | $R_{22}$-F- | $R_{23}$-G- | Rel. Stereo-chemistry | $R_{20}$ | Data |
|---|---|---|---|---|---|---|
| 3Q | $C_{10}H_{21}$- | $CH_3CH_2$- | $C_6H_5$- | 3S,4S | p-MeO-$C_6H_4$- | mp=47.5-48.5°C |
| 3R | $C_{10}H_{21}$- | $C_6H_5$- | $C_6H_5$- | 3R,4S | p-MeO-$C_6H_4$- | mp=103.0-103.5°C |
| 3T | $C_{10}H_{21}$- | $CH_3CH_2$- | $C_6H_5$- | 3R,4R | p-MeO-$C_6H_4$- | mp=48.0-49.5°C |
| 3U | $C_{10}H_{21}$- | $C_6H_5$-$(CH_2)_3$- | $C_6H_5$- | 3R,4R | p-MeO-$C_6H_4$- | CI MS: (M+1) 512.6 |
| 3V | $C_{10}H_{21}$- | $CH_3CH_2$- | $C_6H_5$- | 3S,4R | 2,4,6-tri-MeO-$C_6H_4$- | CI MS: (M+1) 482 |
| 3W | $C_{10}H_{21}$- | $CH_3CH_2$- | $C_6H_5$- | 3R.4R | 2,4,6-tri-MeO-$C_6H_4$- | mp=96-98°C |
| 3X | $C_{10}H_{21}$- | $CH_3CH_2$- | $C_6H_5$-CH=CH- | 3S,4S | p-MeO-$C_6H_4$- | EI MS: $(M^+)$ 425 |
| 3Y | $C_{10}H_{21}$- | $CH_3$- | $C_6H_5$- | 3S,4S | p-MeO-$C_6H_4$- | mp=96-97°C |

EXAMPLE 4

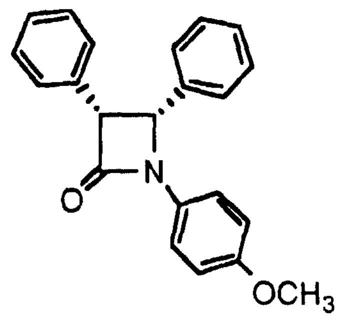

Add 100mg (0.30mmol) of the trans β-lactam of Example 1V in 2mL dry THF to a solution of 1.22mL (0.32mmol) LDA (1.5M, from Aldrich, Milwaukee, WI) in 0.5mL dry THF at -78°C under nitrogen. Stir for 5min at -78°C, then quench at low temperature with ~0.3mL acetic acid. Partition the mixture between 30mL ethyl acetate and 20mL water. Wash the organic layer with 20 mL 10% aqueous $NaHCO_3$ solution, dry over $MgSO_4$ and concentrate in vacuo to give 98mg of an oily white solid. Chromatograph the residue over $SiO_2$, eluting with 20% ethyl acetate-hexane to obtain 26 mg of the title cis β-lactam as a white solid, mp = 141.3 - 142.3°C.

EXAMPLE 5

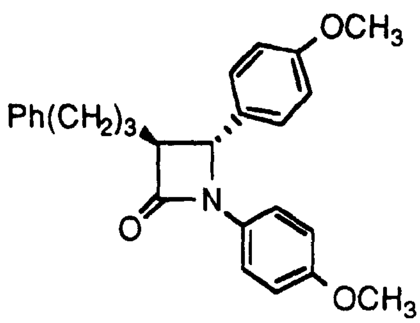

Dissolve 32.05g (79.8mmol) of the racemic cis β-lactam of Example 1 in 500mL of THF. Add 1.79g (16.0mmol) of potassium t-butoxide and stir at 0°C for 1.5h. Partition the reaction mixture between 600mL 1N aqueous HCl and 1.2liters of ether. Extract the aqueous layer with 400mL of ether. Combine the ether layers, dry over $MgSO_4$ and concentrate in vacuo to give 32.0g of a mixture of cis and trans β-lactams (2.7:1). Isolate the pure trans β-lactam via silica gel HPLC chromatography, eluting with 10% ethyl acetate-hexane. Crystallize from ethyl acetate-hexane to give white crystals, mp = 96.0 - 97.5°.

Using a procedure similar to that of Example 5, the following compounds shown in Tables 6 and 6A can be prepared:

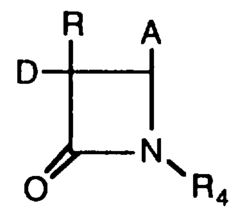

| Ex. | D | R | A | Relative Stereo-chemistry | $R_4$ | Data |
|---|---|---|---|---|---|---|
| 5B | $C_6H_5$-$(CH_2)_3$- | H | 4-$CH_3O$-$C_6H_4$- | 3S,4R single enantiomer | 4-$CH_3O$-$C_6H_4$- | FAB MS (M+1)=402.2 |
| 5C | $C_6H_5$-$(CH_2)_3$- | H | 3-$CH_3O$-$C_6H_4$- | 3R,4S | 4-$CH_3O$-$C_6H_4$- | mp=109.5-110.0°C |
| 5D | $C_6H_5$-$(CH_2)_3$- | H | 4-$CH_3O$-$C_6H_4$- | trans | 4-F-$C_6H_4$- | CIMS (M+1)=390 |
| 5E | $C_6H_5$-$(CH_2)_3$- | H | 4-$CH_3O$-$C_6H_4$- | trans | 3-$CH_3O$-$C_6H_4$- | elemental analysis: calc'd C=77.78, H=6.78, N=3.49; found C=77.75, H=6.70, N=3.60 |
| 5G | $C_6H_5$-$(CH_2)_3$- | H | 4-$CH_3O$-$C_6H_4$- | trans | 4-Cl-$C_6H_4$- | CIMS (M+1)=406 |
| 5H | $C_6H_5$-$(CH_2)_3$- | H | 4-$CH_3O$-$C_6H_4$- | trans | 4-($CH_3CH_2$-O)-$C_6H_4$- | elemental analysis: calc'd C=78.04, H=7.04, N=3.37; found C=78.01, H=7.03, N=3.47 |

| | | | | | | |
|---|---|---|---|---|---|---|
| 5I | $C_6H_5-(CH_2)_3-$ | H | $4-CH_3O-C_6H_4-$ | trans | $4-CH_3S-C_6H_4-$ | elemental analysis: calc'd C=74.79, H=6.52, N=3.35; found C=74.74, H=6.44, N=3.48 |
| 5J | $C_6H_5-(CH_2)_3-$ | H | $4-CH_3O-C_6H_4-$ | trans | $4-CH_3-C_6H_4-$ | elemental analysis: calc'd C=81.01, H=7.06, N=3.63; found C=81.25, H=7.09, N=3.75 |
| 5K | $C_6H_5-(CH_2)_3-$ | H | $4-CH_3O-C_6H_4-$ | trans | $4-(C_6H_5-O)-C_6H_4-$ | elemental analysis: calc'd C=80.32, H=6.31, N=3.02; found C=80.36, H=6.29, N=3.16 |
| 5L | 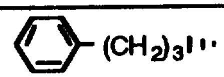 | H | 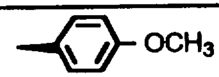 | trans optically pure | $C_6H_5-$ | mp=74-75°C $[\alpha]_D^{25}$ = -7.6° |
| 5M | 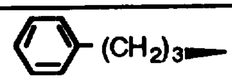 | H | 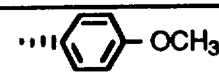 | trans optically pure | $C_6H_5-$ | mp=74-75°C $[\alpha]_D^{25}$ = -7.5° |
| 5P | $C_6H_5-(CH_2)_3-$ | H | 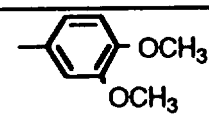 | trans | $4-CH_3O-C_6H_4-$ | mp=70.5-71.5°C |
| 5Q | $C_6H_5-(CH_2)_3-$ | H | 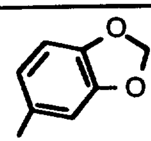 | trans | $4-CH_3O-C_6H_4-$ | mp=79.5-81.0°C |

| | | | | | | |
|---|---|---|---|---|---|---|
| 5R | $C_6H_5-(CH_2)_3-$ | H | $4-CH_3-C_6H_4-$ | trans | $4-CH_3O-C_6H_4-$ | mp=74.5-77.0°C |
| 5S | $C_6H_5-(CH_2)_3-$ | H | $4-(CH_3CH_2-O)-C_6H_4-$ | trans | $4-CH_3O-C_6H_4-$ | mp=85-87°C |
| 5T | $C_6H_5-(CH_2)_3-$ | H | $4-(C_6H_5-CH_2-O)-C_6H_4-$ | trans | $4-CH_3O-C_6H_4-$ | mp=111-112°C |
| 5U | $C_6H_5-(CH_2)_3-$ | H | $4-(CH_2=CH-CH_2-O)-C_6H_4-$ | trans | $4-CH_3O-C_6H_4-$ | mp=75.5-77.0°C |
| 5V | $C_6H_5-(CH_2)_3-$ | H | $4-(C_6H_5-O)-C_6H_4-$ | trans | $4-CH_3O-C_6H_4-$ | mp=103.5-105.5°C |
| 5W | $C_6H_5-(CH_2)_2-$ | H | $4-CH_3O-C_6H_4-$ | trans | $4-CH_3O-C_6H_4-$ | mp=75-77°C |
| 5X | $C_6H_5-O-(CH_2)_2-$ | H | $4-CH_3O-C_6H_4-$ | trans | $4-CH_3O-C_6H_4-$ | mp=122-123°C |
| 5Y | $C_6H_5-(CH_2)_6-$ | H | $4-CH_3O-C_6H_4-$ | trans | $4-CH_3O-C_6H_4-$ | elemental analysis: calc'd C=78.52, H=7.50, N=3.16; found C=78.32, H=7.37, N=3.25 |
| 5Z | $C_6H_5-(CH_2)_2-O-$ | H | $4-CH_3O-C_6H_4-$ | trans | $4-CH_3O-C_6H_4-$ | elemental analysis: calc'd C=74.42, H=6.25, N=3.47; found C=74.57, H=6.29, N=3.51 |
| 5AA | $4-F-C_6H_4-O-(CH_2)_2-$ | H | $4-CH_3O-C_6H_4-$ | trans | $4-MeO-C_6H_4-$ | elemental analysis: calc'd C=71.25, H=5.74, N=3.32; found C=71.34, H=5.73, N=3.43 |

| | | | | | | |
|---|---|---|---|---|---|---|
| 5AB | $C_6H_5-(CH_2)_4-$ | H | $4-CH_3O-C_6H_4-$ | trans | $4-MeO-C_6H_4-$ | elemental analysis: calc'd C=78.04, H=7.03, N=3.37; found C=77.90, H=6.94, N=3.51 |

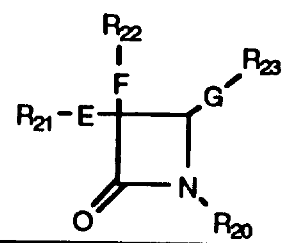

| Ex. | $R_{21}$-E- | $R_{22}$-F- | $R_{23}$-G- | Rel. Stereo-chemistry | $R_{20}$ | Data |
|---|---|---|---|---|---|---|
| 5A | $(CH_3)_2CH-$ | H | $C_6H_5-$ | 3S,4R | $4-MeO-C_6H_4-$ | mp=113-115°C |
| 5N | $C_6H_5-(CH_2)_2-N(CH_3)-$ | H | $4-CH_3O-C_6H_4-$ | trans | $C_6H_5-$ | mp=122-123°C |

EXAMPLE 6

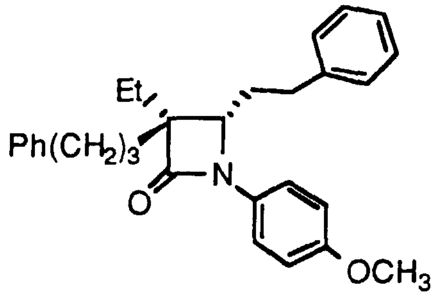

Dissolve 200mg (0.47mmol) of the β-lactam of Example 3G in 4mL EtOAc and add ~10mg 10% Pd/C. Hydrogenate at 1 atm for 4h. Filter the mixture through Celite and concentrate. Chromatograph the residue over 40g $SiO_2$, eluting with 20% EtOAc-hexane to obtain 188mg (94%) of the title β-lactam as a colorless oil, CI MS : (M+1) 428.1.

EXAMPLE 7

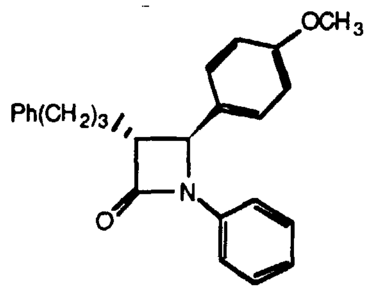

To a refluxing solution of 4.33 grams (.0205 moles) N-(4-methoxybenzylidine)-aniline and 7.6 grams (0.0410 moles) tributylamine in 40 mL heptane add,in portions, a solution of 4.03 grams (0.0205 moles) 5-phenylvaleroyl chloride in 15 mL heptane over about 2 hours, then reflux the solution for an additional 4 hours. Evaporate the solvent and take up the residue in 150 mL EtOAc. Wash with 1N HCl (2 x 30 mL), saturated $NaHCO_3$ ( 1 x 30 mL) and brine (1 x 30 mL), then dry over $MgSO_4$ and evaporate to give 7.69 grams of a semisolid. Recrystallization from 15% EtOAc in hexane to obtain 3.08 grams of the title compound, mp 75-76°C. An additional 2.31 g is obtained from the mother liquors by chromatography (silica gel, 5% ethyl acetate in hexane) and recrystallization.

Using a procedure similar to that of Example 7, the following compounds shown in Tables 7 and 7Acan be prepared:

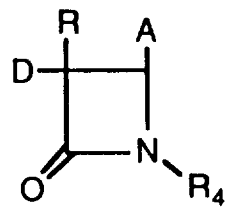

| Ex. | D | R | A | Relative Stereo-chemistry | $R_4$ | Data |
|---|---|---|---|---|---|---|
| 7A | $C_6H_5-(CH_2)_3-$ | H | $4-CH_3O-C_6H_4-$ | trans | $4-(CH_3CH_2OC(O))-C_6H_4-$ | mp=75-77°C |
| 7B | $C_6H_5-(CH_2)_3-$ | H | $4-CH_3O-C_6H_4-$ | trans | $3-NO_2-C_6H_4-$ | elemental analysis: calc'd C=72.10, H=5.81, N=6.73; found C=72.46, H=5.91, N=6.54 |
| 7C | $C_6H_5-(CH_2)_3-$ | H | $4-CH_3O-C_6H_4-$ | trans | $4-((CH_3CH_2)_2-N)-C_6H_4-$ | mp=91-93°C |
| 7D | $C_6H_5-(CH_2)_3-$ | H | $4-CH_3O-C_6H_4-$ | trans | $4-NC-C_6H_4-$ | mp=79-81°C |
| 7E | $C_6H_5-(CH_2)_3-$ | H | $4-CH_3O-C_6H_4-$ | trans | 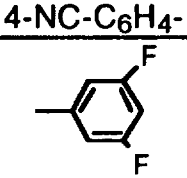 | elemental analysis: calc'd C=73.69, H=5.69, N=3.44; found C=73.36, H=5.77, N=3.48 |

| | | | | | | |
|---|---|---|---|---|---|---|
| 7F | $C_6H_5$-$(CH_2)_3$- | H | 4-$CH_3O$-$C_6H_4$- | trans | 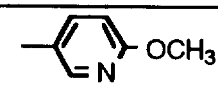 | mp=103-104°C |
| 7G | $C_6H_5$-$(CH_2)_3$- | H | 4-$CH_3O$-$C_6H_4$- | trans | 4-($CH_3C(O)$)-$C_6H_4$- | elemental analysis: calc'd C=78.42, H=6.58, N=3.39; found C=78.08, H=6.51, N=3.40 |
| 7H | $C_6H_5$-$(CH_2)_3$- | H | 4-$CH_3O$-$C_6H_4$- | trans | 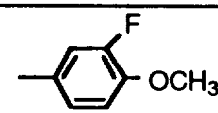 | mp=90-92°C |
| 7I | $C_6H_5$-$(CH_2)_3$- | H | 4-$CH_3O$-$C_6H_4$- | trans | 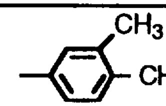 | mp=129-130°C |
| 7M | $C_6H_5$-$(CH_2)_3$- | H | 4-(($CH_3)_2CH$-O)-$C_6H_4$- | trans | 4-$CH_3O$-$C_6H_4$- | mp=96.5-97.5°C |
| 7O | $C_6H_5$-$(CH_2)_3$- | H | 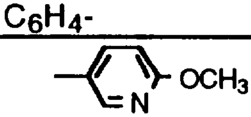 | trans | $C_6H_5$- | mp=89-91°C |
| 7P | $C_6H_5$-$(CH_2)_3$- | H | 4-($CH_3(CH_2)_2$-O)-$C_6H_4$- | trans | 4-$CH_3O$-$C_6H_4$- | mp=92.5-93.5°C |
| 7Q | $C_6H_5$-$(CH_2)_3$- | H | 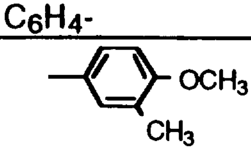 | trans | 4-$CH_3O$-$C_6H_4$- | EIMS (M)=415 |
| 7R | $C_6H_5$-$(CH_2)_3$- | H | 4-($CH_3OC(O)$)-$C_6H_4$- | trans | $C_6H_5$- | mp=84-85°C |

| | | | | | | |
|---|---|---|---|---|---|---|
| 7S | $C_6H_5$-$(CH_2)_3$- | H | 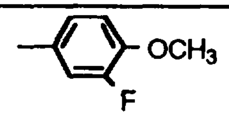 | trans | 4-$CH_3O$-$C_6H_4$- | EIMS (M)=420 |
| 7T | $C_6H_5$-$(CH_2)_3$- | H | 4-($CH_3OCH_2O$)-$C_6H_4$- | trans | $C_6H_5$- | CIMS (M+1)=402 |
| 7U | $C_6H_5$-$(CH_2)_3$- | H | 4-($(CH_3)_2N$)-$C_6H_4$- | trans | 4-$CH_3O$-$C_6H_4$- | mp=96.5-97.5°C |
| 7V | $C_6H_5$-$(CH_2)_3$- | H | 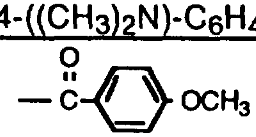 | trans | 4-$CH_3O$-$C_6H_4$- | EIMS (M)=429 |
| 7W | $C_6H_5$-$(CH_2)_3$- | H | 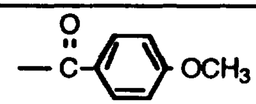 | cis | 4-$CH_3O$-$C_6H_4$- | EIMS (M)=429 |
| 7X | $C_6H_5$-$(CH_2)_3$- | H | 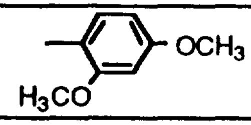 | trans | 4-$CH_3O$-$C_6H_4$- | EIMS (M)=431 |
| 7Y | $C_6H_5$-$(CH_2)_3$- | H | 4-($CH_3CH_2$-O)-$C_6H_4$- | trans | $C_6H_5$- | mp=95.0-95.5°C |
| 7Z | 4-$O_2N$-$C_6H_4$-$(CH_2)_3$- | H | 4-$CH_3O$-$C_6H_4$- | trans | 4-$CH_3O$-$C_6H_4$- | mp=136.5-137.0°C |
| 7AA | $C_6H_5$-$(CH_2)_3$- | H | 4-$CH_3O$-$C_6H_4$- | trans | 4-I-$C_6H_4$- | mp=96.5-97.5°C |
| 7AB | $C_6H_5$-$(CH_2)_3$- | H | 4-($CH_3(CH_2)_3$-O)-$C_6H_4$- | trans | 4-$CH_3O$-$C_6H_4$- | mp=79-82°C |
| 7AE | $C_6H_5$-$(CH_2)_2$-O- | H | 4-$CH_3O$-$C_6H_4$- | cis | 4-$CH_3O$-$C_6H_4$- | mp=149-150°C |

| | | | | | | |
|---|---|---|---|---|---|---|
| 7AI | 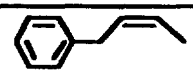 | H | 4-$CH_3O$-$C_6H_4$- | trans | 4-$CH_3O$-$C_6H_4$- | elemental analysis: calc'd C=78.17, H=6.31, N=3.51; found C=78.22, H=6.36, N=3.59 |
| 7AJ | 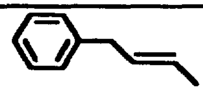 | H | 4-$CH_3O$-$C_6H_4$- | trans | 4-$CH_3O$-$C_6H_4$- | FAB mass spec 400(100) |
| 7AK | 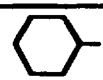 $(CH_2)_3$– | H | 4-$CH_3O$-$C_6H_4$- | trans | 4-$CH_3O$-$C_6H_4$- | mp=105-106°C |
| 7AM | $C_6H_5$-$(CH_2)_5$- | H | 4-$CH_3O$-$C_6H_4$- | trans | 4-$CH_3O$-$C_6H_4$- | elemental analysis: calc'd C=78.29, H=7.27, N=3.26; found C=78.37, H=7.26, N=3.41 |
| 7AN | 4-$O_2N$-$C_6H_4$-O-$(CH_2)_2$- | H | 4-$CH_3O$-$C_6H_4$- | trans | $C_6H_5$- | mp=54-56°C |
| 7AO | 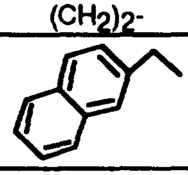 | H | 4-$CH_3O$-$C_6H_4$- | trans | $C_6H_5$- | mp=55-57°C |
| 7AP | 4-$(CH_3O)$-$C_6H_4$-O-$(CH_2)_2$- | H | 4-$CH_3O$-$C_6H_4$- | trans | $C_6H_5$- | elemental analysis: calc'd C=74.42, H=6.25, N=3.47; found C=74.13, H=6.03, N=3.26 |

| | | | | | | |
|---|---|---|---|---|---|---|
| 7AQ | 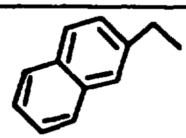 | H | 4-$CH_3O$-$C_6H_4$- | cis | $C_6H_5$- | $^1$H NMR ($CDCl_3$) 7.97-7.78 (m,2H); 7.69 (d, J=8Hz,1H); 7.45-7.69 (m,10H); 6.90 (d, J=8Hz,2H); 6.63 (d,J= 8Hz, 1H); 5.17(d,J=6Hz,1H); 4.15-4.0 (m,1H); 3.82 (s,2H); 3.5 (dd,J=6,14Hz,1H); 2.96(dd, J=9,14Hz,1H) |
| 7AR | $C_6H_5$-$(CH_2)_3$- | H | 4-$CH_3O$-$C_6H_4$- | trans | 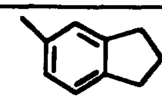 | mp=111-112°C |
| 7AS | $C_6H_5$-$(CH_2)_3$- | H | 4-$CH_3O$-$C_6H_4$- | trans | 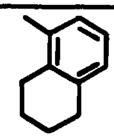 | MS Cl+ (M+1)=426 |
| 7AT | $C_6H_5$-$(CH_2)_3$- | H | 4-$CH_3O$-$C_6H_4$- | trans | 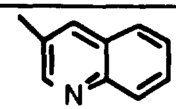 | mp=122-124°C |
| 7AU | $C_6H_5$-$(CH_2)_3$- | H | 4-($C_6H_5$-$CH_2O$)-$C_6H_4$- | trans | 4-($C_6H_5$-$CH_2O$)-$C_6H_4$- | mp=105-106°C |

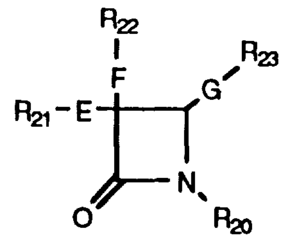

| Ex. | $R_{21}$-E- | $R_{22}$-F- | $R_{23}$-G- | Rel. Stereo-chemistry | $R_{20}$ | Data |
|---|---|---|---|---|---|---|
| 7J | $CH_3O$-C(O)-CH=C($CH_3$)-NH- | H | 4-$CH_3O$-$C_6H_4$- | cis | $C_6H_5$- | mp=186-187°C |
| 7K | $C_6H_5$-$(CH_2)_2$-N($CH_3$)- | H | 4-$CH_3O$-$C_6H_4$- | cis | $C_6H_5$- | mp=120-123°C |
| 7L | 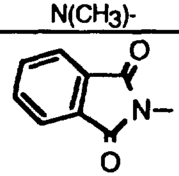 | H | 4-$CH_3O$-$C_6H_4$- | cis | $C_6H_5$- | mp=243-245.5°C |
| 7N | $C_6H_5$-$(CH_2)_3$- | H | -C(O)$OCH_2CH_3$ | cis | 4-$CH_3O$-$C_6H_4$- | mp=97-98.5°C |
| 7AC | $C_6H_5$- | $C_6H_5$- | 4-$CH_3O$-$C_6H_4$- | --- | 4-$CH_3O$-$C_6H_4$- | mp=78-81°C |
| 7AD | cyclo-$C_5H_9$- | $C_6H_5$- | 4-$CH_3O$-$C_6H_4$- | trans | 4-$CH_3O$-$C_6H_4$- | mp=60-63°C |
| 7AF | $C_6H_5$- | $C_6H_5$-$CH_2$- | 4-$CH_3O$-$C_6H_4$- | cis | 4-$CH_3O$-$C_6H_4$- | mp=108-110°C |

| | | | | | | |
|---|---|---|---|---|---|---|
| 7AG | $C_{10}H_{21}$- | H | 4-$CH_3O$-$C_6H_4$- | trans | 4-$CH_3O$-$C_6H_4$- | elemental analysis: calc'd C=76.56, H=8.80, N=3.31; found C=76.37, H=8.90, N=3.46 |
| 7AH | $C_6H_5$-$CH_2$- | $C_6H_5$- | 4-$CH_3O$-$C_6H_4$- | cis | 4-$CH_3O$-$C_6H_4$- | mp=114-117°C |
| 7AL | 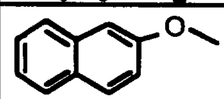 | H | 4-$CH_3O$-$C_6H_4$- | cis | 4-$CH_3O$-$C_6H_4$- | elemental analysis: calc'd C=76.22, H=5.45, N=3.29; found C=76.00, H=5.46, N=3.39 |

EXAMPLE 8

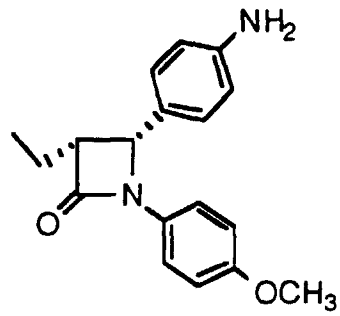

Stir a mixture of 981mg (3.01 mmol) of the cis β-lactam of Example 1AK, above, and 20mg 10% palladium on carbon in 10 mL ethyl acetate under 1atm hydrogen gas at room temperature for 24h. Filter the mixture through Celite and concentrate in vacuo. Chromatograph the residue over 50g $SiO_2$, eluting with 40% ethyl acetate-hexane to obtain 818mg (92%) of the title compound as a yellowish solid, mp = 142.0 - 142.5°C.

Using a similar procedure, the following compounds shown in Tables 8 and 8A can be prepared, and using conventional procedures, the amino group of compound 8A and the amino group of compound 8B can be protected with a t-butoxycarbonyl (BOC) group to obtain compounds 8G and 8H, respectively:

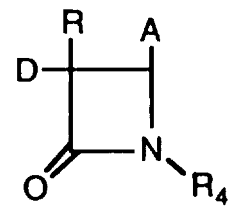

| Ex. | D | R | A | Relative Stereo-chemistry | $R_4$ | Data |
|---|---|---|---|---|---|---|
| 8B | $C_6H_5$-$(CH_2)_3$- | H | 4-$NH_2$-$C_6H_4$- | cis | 4-$CH_3O$-$C_6H_4$- | mp=138.5-139.5°C |
| 8C | $C_6H_5$-$(CH_2)_3$- | H | 4-$NH_2$-$C_6H_4$- | trans | 4-$CH_3O$-$C_6H_4$- | FAB MS (M+1)=387.1 |
| 8D | $C_6H_5$-$(CH_2)_3$- | H | 4-$CH_3O$-$C_6H_4$- | trans | 3-$NH_2$-$C_6H_4$- | mp=46-48°C |
| 8E | 4-$NH_2$-$C_6H_4$-$(CH_2)_3$- | H | 4-$CH_3O$-$C_6H_4$- | trans | 4-$CH_3O$-$C_6H_4$- | mp=129.0-131.0° C |
| 8F | $C_6H_5$-$(CH_2)_3$- | H | 4-OH-$C_6H_4$- | trans | 4-$CH_3O$-$C_6H_4$- | mp=171.0-171.5°C |
| 8H | $C_6H_5$-$(CH_2)_3$- | H | 4($(CH_3)_3$C-O-C(O)-NH)-$C_6H_4$- | cis | 4-$CH_3O$-$C_6H_4$- | CI MS:(M+1)=487 |

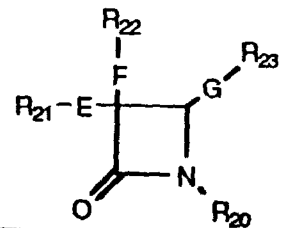

| Ex. | $R_{21}$-E- | $R_{22}$-F- | $R_{23}$-G- | Rel. Stereo-chemistry | $R_{20}$ | Data |
|---|---|---|---|---|---|---|
| 8A | $CH_3CH_2$- | H | 4-$NH_2$-$C_6H_4$- | 3S,4R | 4-$CH_3O$-$C_6H_4$- | mp=136-137°C |
| 8G | $CH_3CH_2$- | H | 4(($CH_3$)$_3$C-O-C(O)-NH)-$C_6H_4$- | 3S,4R | 4-$CH_3O$-$C_6H_4$- | CI MS:(M+1)=397 |

EXAMPLE 9

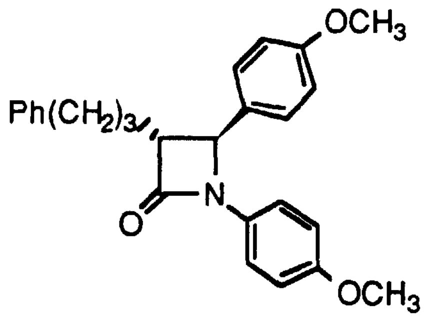

STEP 1: Combine 5-phenylvaleric acid (89.9 g, 0.504 mol) and thionyl chloride (89.3 mL, 1.225 mol), heat to 70°C and reflux for 1 h. Vacuum distill (50 - 100 mm Hg) the excess thionyl chloride and add 200 mL of dry toluene to the resultant residue. Vacuum distill again, then add 188 mL of dry THF to the crude 5-phenylvaleroyl chloride and use the resulting solution directly in the next step.

STEP 2: Combine 76 g (0.4289 mol) of R-(+)-4-benzyloxazolidinone and 1.3 L of dry THF under dry nitrogen atmosphere. Cool the resulting solution to -78°C and add 278 mL of a 1.6 M solution of n-butyllithium in hexane over 30-40 minutes. Stir for an additional 30 minutes, then add the solution of Step 1 over a period of 45 min. Allow the mixture to warm to 0°C and stir for 1 h. Quench the reaction mixture by adding 673.6 mL of $K_2CO_3$ (1 M aqueous solution) and stir for 1 h. Distill off the THF under vacuum at 30-35°C. Dilute the residue with 1 L of water and extract with 3x800 mL of $CH_2Cl_2$. Combine the organic extracts and wash with 800 mL of water, then with 800 mL of brine. Dry the organic extracts over $MgSO_4$, filter, then concentrate *in vacuo* to an oil. Dissolve the oil in 200 mL of hexane, then distill off the hexane under vacuum. Repeat the hexane treatment two more times, then dissolve the oil in 1.7 mL of $CH_2Cl_2$. The resulting solution is used directly in the next step.

STEP 3: Cool the solution of Step 2 to -5°C to 0°C under dry nitrogen atmosphere. Add 129.8 mL of di-n-butylboron triflate, maintaining the temperature of the reaction mixture at -6°C to -3°C. Following the addition, stir the mixture for 10 min., then add 97.12 mL of diisopropylethylamine, again maintaining the temperature at -6°C to -3°C. Following the addition, stir the mixture at 0°C for 30 min., then cool the mixture to -78°C and stir for 30 min.. Add 57.4 mL of p-anisaldehyde and stir the mixture at -78°C for 30 min., then at 0°C for 1 h. While maintaining the temperature at 0°C to 5°C, quench the mixture by adding 688.2 mL of a pH 7 buffer solution (68 g $KH_2PO_4$, 12 g NaOH and 800 mL of water), then add 473 mL of 30% $H_2O_2$ and stir the resulting mixture at 0°C for 1 h. Extract the mixture with 3x600 mL hexane:ethyl actetate (1:1), combine the organic extracts and wash with 800 mL of saturated $NaHCO_3$ (aqueous), then with 800 mL of brine. Dry the organic extracts over $NaSO_4$, filter, and evaporate to an oil. Crystallize the oil from hexane/ethyl acetate (1:1) to give 176 g of the product as a white solid.

STEP 4: Combine the product of Step 3 (170 g, 0.36 Mol), 1595 mL of THF and 400 mL of water, stir the mixture and cool to about 3°C. Add 226 mL (2.156 Mol) of 30% $H_2O_2$ to the mixture over 15 min., then add a solution of LiOH (36.2 g, 0.862 Mol) in 400 mL of water over a period of 30 min. Stir the reaction mixture at 0°C to 5°C for 3 h. Add a solution of 272 g of sodium sulfite in 850 mL of water over 70 min., keeping the temperature under 27°C. Concentrate the solvent under vacuum and add 7 L of water. Extract with 4x1.7 L of toluene. Acidify the aqueous layers to pH 2.4 with 3 N HCl. Extract with one 2.6 L portion and two 1.7 L portions of ethyl acetate. Combine the ethyl acetate extracts, wash with brine, dry over $NaSO_4$, filter, then evaporate to give the product as a white solid, 112 g.

STEP 5: Combine the product of Step 4 (19.47 g, 62 mmol), 400 mL of acetonitrile, 9.49 g (62 mmol) of 1-hydroxybenzotriazole (HOBT), 22.91 g (186 mmol) of p-anisidine and 14.05 g (68.2 mmol) of dicyclohexylcarbodiimide (DCC). Stir the reaction mixture at 40°C for 4 h and confirm the consumption of starting material by TLC (6:4 hexane/ ethyl acetate). Concentrate the mixture to 1/3 its volume and partition between 300 mL of water and 300 mL of ethyl acetate. Filter the organic layer, then wash with 200 mL of 1 N HCl, then with two 100 mL portions of saturated $NaHCO_3$, and two 100 mL portions of brine. Dry the organic layer over $NaSO_4$ and concentrate to give the product as a brown solid, 24 g.

STEP 6: Combine the product of Step 5 (115 g, 0.2745 Mol) and 2.3 L of THF under dry nitrogen atmosphere and cool to -70°C. Stir the mixture while adding a solution of 137 mL (0.551 Mol) of tri-n-butylphosphine in 113 mL THF, and 163 mL (1.03 Mol) of diethylazodicarboxylate (DEAD) over 2 h. Allow the mixture to warm to room temperature and stir overnight. Remove the solvent under vacuum. Filter the residue through a plug of silica gel using $CH_2Cl_2$/hexane/ ethyl acetate (70:24:6) as the eluant. Evaporate the solvent and purify the residue by preparative HPLC (silica gel, 15% ethyl actetate/ hexane) to give 88 g of the (3R,4S) enantiomerically pure title compound,

$[\alpha]_D^{25}$ = 19.3° (MeOH).

Example 10

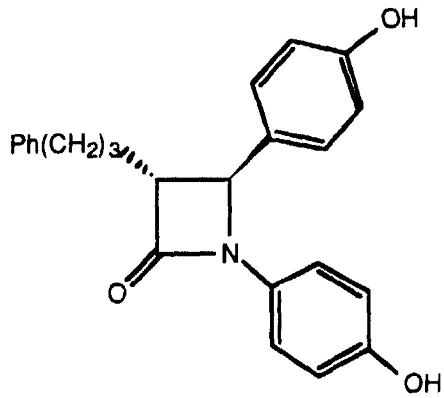

The compound of Example 7AU (0.686 g, 0.0012 moles) dissolved in 1:1 ethanol:ethyl acetate is hydrogenated over 0.70 grams 10% Pd on carbon at 50 psi for 16 hours. The resulting product is chromatographed (silica gel, 80: 20 hexane:ethyl acetate) to give 0.432 grams of the title compound, mp 160-161°C.

Using substantially the same procedure the following compound can be prepared:

Example 10A

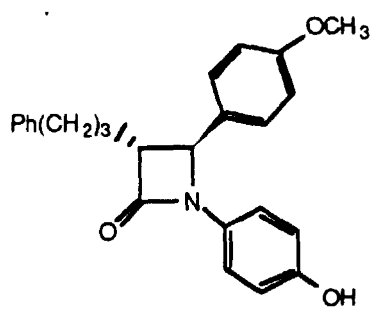

Elemental Analysis

Calculated for $C_{25}H_{25}NO_3$

C, 77.49; H, 6.50; N, 3.61

Found: C, 77.47; H, 6.46; N, 3.74

Example 11

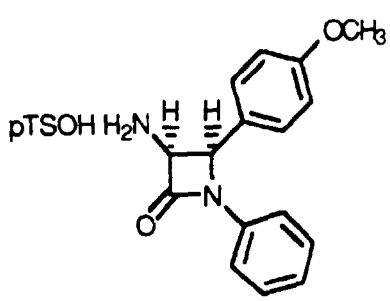

A flask is charged with the compound of Example 7J (27 g, 73.6 mmole), acetone (0.6 1), water (1.3 ml, 73 mmole), and p-toluenesulfonic acid-monohydrate (15.4 g, 81.1 mmole). The solution is stirred at 22°C for 5.5 h and the product collected by filtration (34.1 g). Recrystallization from methanol affords the title compound,26.0 g (79% yield), mp 200-202°C.

Example 12

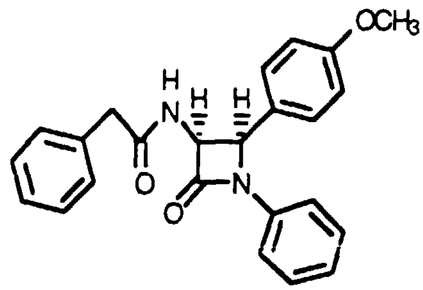

To a solution of the product of Example 11 (0.60 g, 1.36 mmole), triethylamine (0.14 g, 1.4 mmole) in $CH_2Cl_2$ (2 ml) was added phenylacetylchloride (0.310 g, 2.0 mmole) dropwise over 0.5 h. After 5 h the reaction mixture was concentrated to dryness, and the residue purified by chromatography (silica, 2:1 hexane: ethyl acetate) to afford 0.27 g (51% yield) of the title compound, mp 217-218°C.

Using substantially the same procedure, the following compounds can be prepared:

| Example 12A | Example 12B |
|---|---|
| 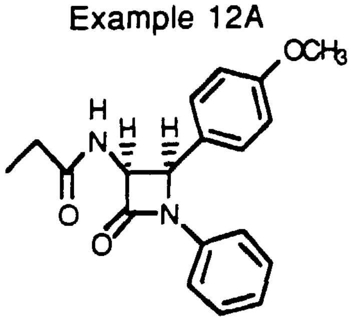 | 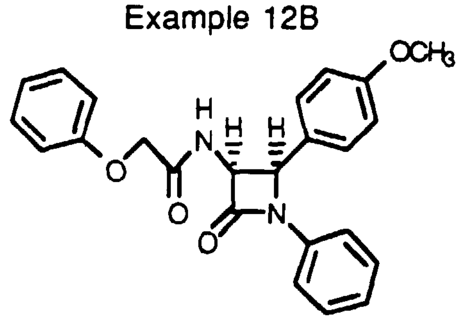 |
| mp 167-168°C | mp 192-193°C |

| Example 12C | Example 12D |
|---|---|
| 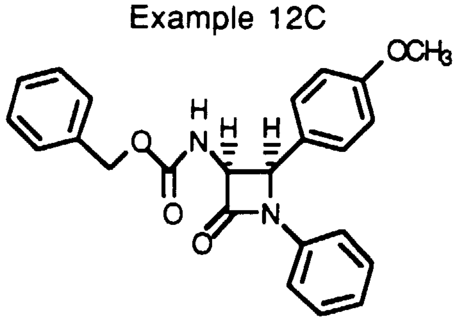 | 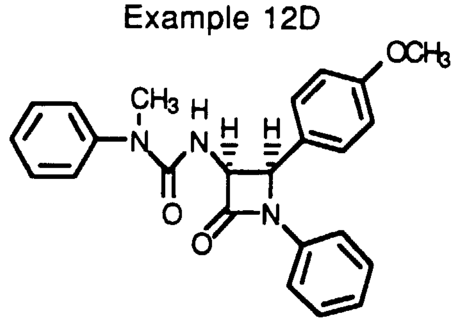 |
| 194-195°C | mp 161.5-163°C |

Example 13

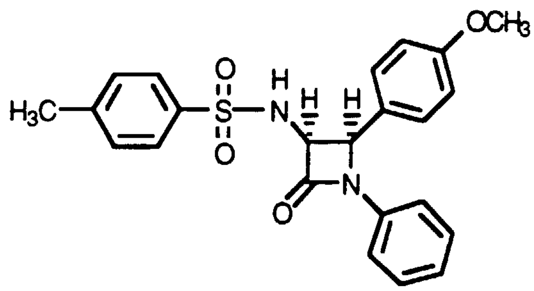

To a biphasic mixture of the product of Example 11 (0.80 g, 1.8 mmole), $CH_2Cl_2$ (15 ml) and 30% aqueous tetrabutylammonium hydroxide (2.36 ml, 3.64 mmole) was added p-toluenesulfonylchloride (0.515 g, 2.7 mmole). After 2 h the reaction mixture was washed with 1 X 1 N HCl, 2X $H_2O$ and the organic layer dried over $MgSO_4$. Concentration followed by recrystillaztion from ethyl acetate afforded 0.38 g (50% yield) of the title compound, mp 204-205°C.

Example 14

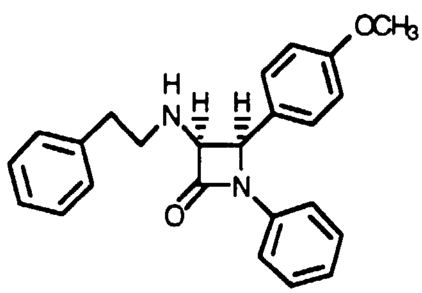

To a solution of the product of Example 11 (0.8 g, 1.8 mmole), methanol (20 ml) and triethylamine (0.19 g, 1.8 mmole) was added phenylacetaldehyde (0.44 g, 3.6 mmole). After 0.25 h , $NaBH_3CN$ (0.172 g, 2.7 mmole) and $ZnCl_2$ (0.174 g, 1.3 mmole) were added to the reaction. After 3.5 h, the reaction was quenched with ammonium chloride, concentrated partially in vacuo, and dissolved in $CH_2Cl_2$. The organic layer was washed 2X with water, dried and concentrated to afford crude product. Recrystallization from $CH_2Cl_2$ afforded 0.44 g (65% yield) of the title compound, mp 138-139.5°C.

Using substantially the same procedure, the following compounds can be prepared:

| | |
|---|---|
| Example 14A<br>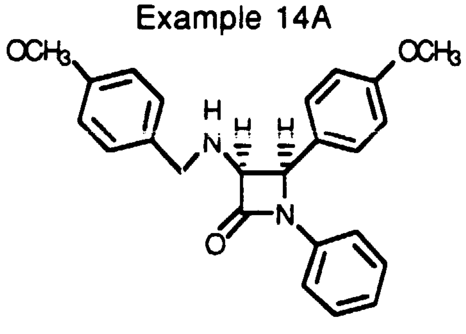<br>mp 148-149°C | Example 14B<br>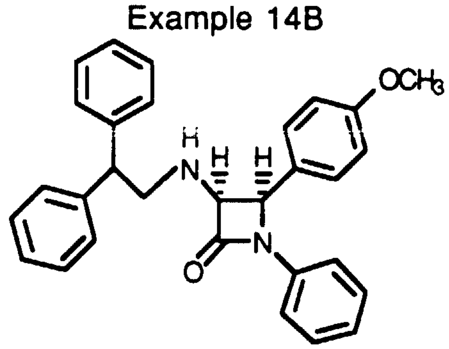<br>mp 179-180°C |
| Example 14C<br>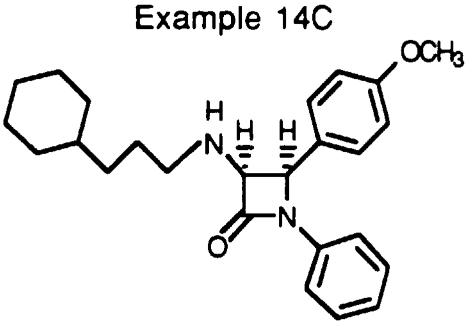<br>mp 100-101°C | |

Example 15

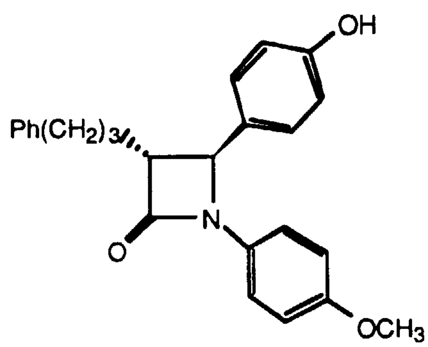

Add 49 mL (49 mmol) of 1M tetrabutylammonium fluoride 49 mL, 49 mmol, 1M in THF)to a room temperature solution of the compound of Example 1 AS (5.1 g, 9.8 mmol) in THF (25 mL). Allow the mixture to stir overnight. TLC (50% EtOAc/hexanes) indicates consumption of starting material. Transfer the reaction mixture to a separatory funnel, partion between saturated ammonium chloride and ether, extract with ether. Combine the etheral extracts, wash with water and brine, dry over anhydrous $Na_2SO_4$, filter and concentrate to a residue. Chromatograph ($SIO_2$, 30-40% EtOAc/hexane) affords 3.31g (84%) of the title compound, mp 91-92°C.

The following compounds can be prepared via substantialy the same procedure:

Example 15A

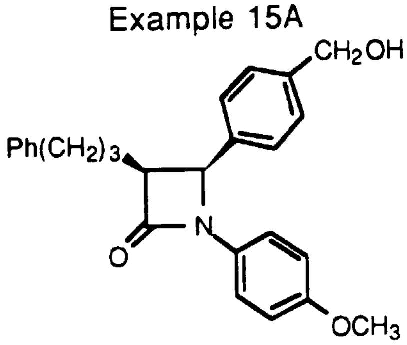

mp 112-113°C

Example 15B

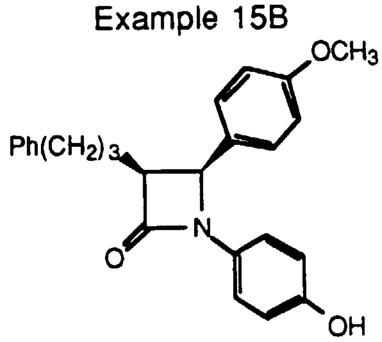

mp 170-171°C

Example 16

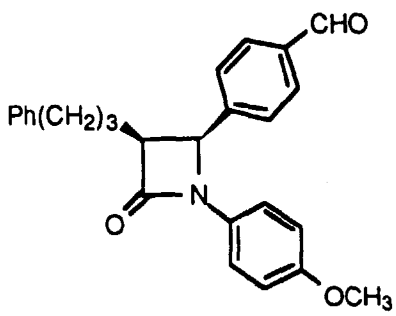

Add $MnO_2$ (0.64g, 7.4 mmol) to a solution of the product of Example 15A (0.20g, 0.5 mmol) in $CH_2Cl_2$ (50 mL) at room temperature. Allow the mixture to stir overnight. TLC (20% EtOAc/hexanes) indicates consumption of starting material. Filter the mixture through celite washing the filtercake well with $CH_2Cl_2$. Concentrate the filtrate to obtain 0.19g (95%) of the title compound, mp 129-130 °C.

Using substantially the same procedure, the following compound can be prepared:

Example 16A

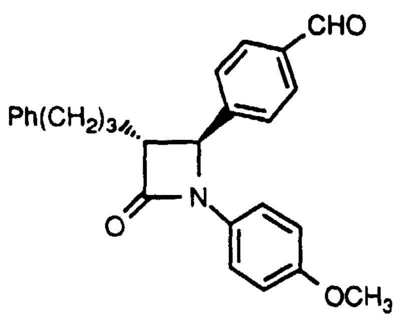

mp 100-101°C

Example 17

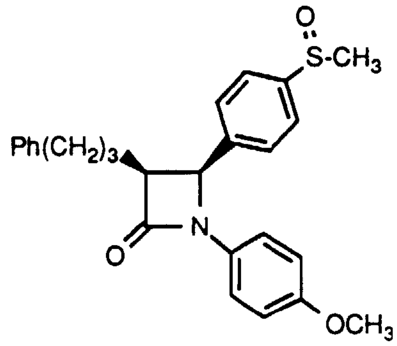

Add m-chloroperbenzoic acid (.16g, .092 mmol) to a solution of the compound of Example 1AU (0.31 g, 0.74 mmol) in $CH_2Cl_2$ (4 mL) at room temperature. Monitor the reaction by TLC (50% EtOAc/hexanes). Upon consumption of starting material (~1h), add $Ca(OH)_2$ (0.1g, 1.3 mmol) Stir the mixture for an additional 15 min.. Filter the mixture through celite washing the filtercake well with $CH_2Cl_2$. Concentrate the filtrate onto silica gel, using enough silica gel until a free flowing powder is obtained. Load the resulting powder onto a chromatography column loaded with silica gel and 5% $MeOH/CH_2Cl_2$. Elute with 5% $MeOH/CH_2Cl_2$ to obtain 0.26 g of the title sulfoxide (Example 17), mp 134-135°C and 0.50 g of the analogous sulfone:

Example 17A

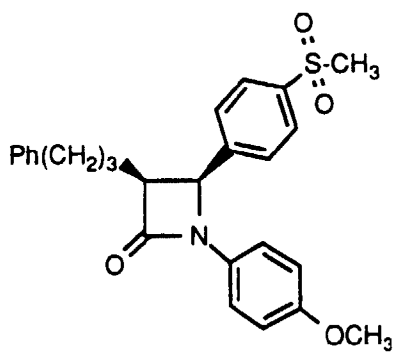

mp 116-117°C

The following compounds can be prepared via substantially the same procedure:

Example 17B

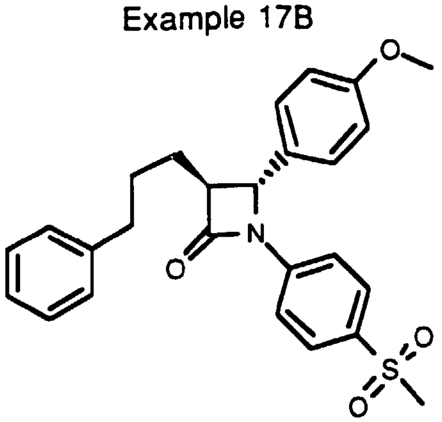

mp 56-58°C

Example 17C

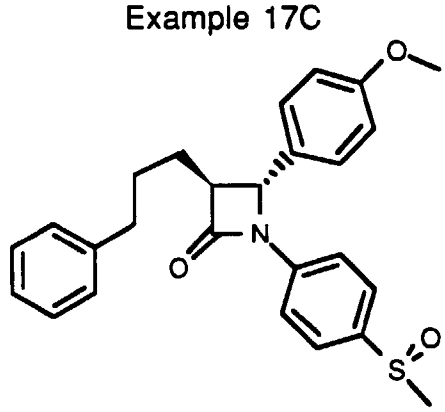

mp 52-53°C

Example 18

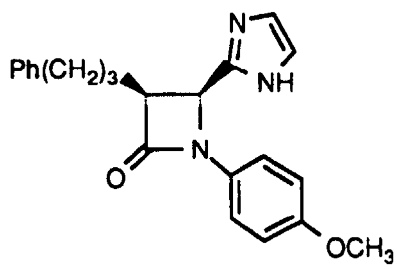

Reflux a mixture of the compound of Example 1 AW (1.72 g, 3.49 mmol), 20 mL of THF and 3N HCl (20 mL) overnight. Monitor the reaction by TLC (50% EtOAc/hexanes). Upon consumption of starting material cool the mixture to room temperature, neutralize with saturated $NaHCO_3$ and extract with ethyl acetate. Combine the extracts, wash with water and brine, dry over anhydrous $Na_2SO_4$, filter and concentrate to a solid. Recrystallize from $CH_2Cl_2$/MeOH to obtain 0.322g of the title compound, mp 190.5-191.5 °C.

Using substantially the same procedure the following compound can be prepared:

Example 18A

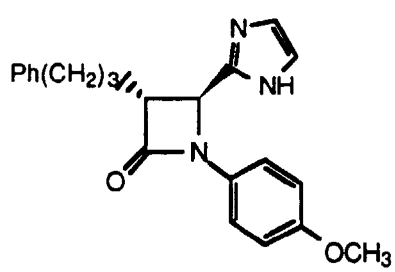

mp 189-191°C

Example 19

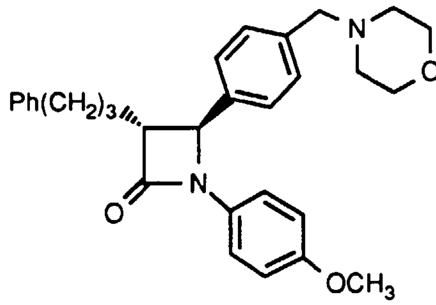

Add a solution of $NaBH_3CN$ (0.19g, 3.0 mmol) and $ZnCl_2$ (0.21g, 1.5 mmol) in methanol to a room temperature solution of the product of Example 16A (1.0g, 2.5 mmol) and morpholine (0.44 mL, 5.0 mmol) in THF. Monitor the reaction by TLC (50% EtOAc/hexanes). Upon consumption of starting material the mixture was diluted with 0.1 N NaOH (120 mL) and remove the organic solvents on a rotary evaporator. Extract the resulting solution with ethyl acetate. Combine the extracts, wash with water and brine, dry over anhydrous $Na_2SO_4$, filter and concentrate to a solid. Recrystallize from $Et_2O$/hexanes to obtain 0.81g (69%) of the title compound, mp 100-101 °C.

The following compounds can be prepared by substantially the same method:

| | |
|---|---|
| Example 19A<br>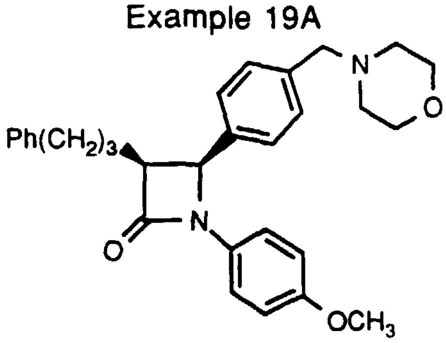<br>mp 97-98°C | Example 19B<br>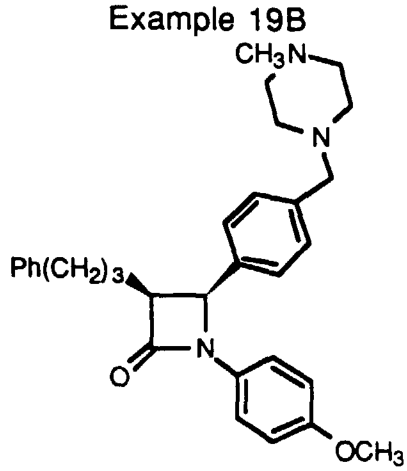<br>mp 141-142°C |
| Example 19C<br>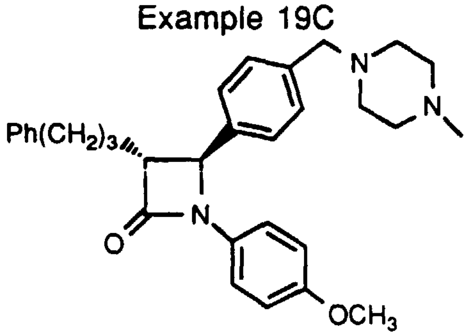<br>mp 103.5-104.5°C | Example 19D<br>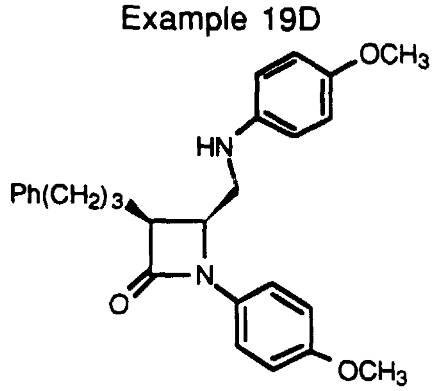<br>mp 94-95°C |
| Example 19E<br>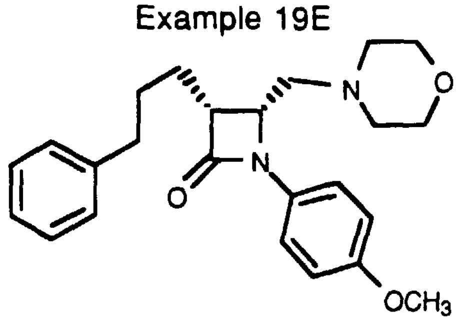<br>EI MS (M) = 394 | |

Example 20

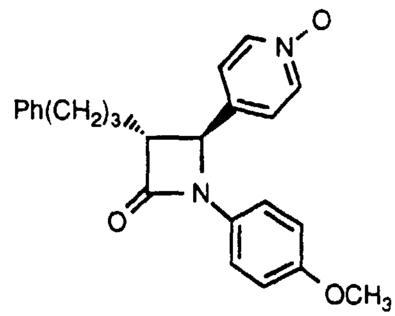

Heat a solution of the compound of Example 1 AR (1.10g, 2.95 mmol), 15% $H_2O_2$ (0.45 mL) in acetic acid (3 mL) to a 100 °C for 3.5h with TLC monitoring (5% $MeOH/CH_2Cl_2$). Upon consumption of the starting compound, cool to room temperature, neutralize with sat. $Na_2CO_3$ and dilute with ethyl acetate. Filter the mixture and wash the filtrate with water and brine, dry over anhydrous $Na_2SO_4$, filter and concentrate to a residue. Chromatograph the residue (silica gel, 10% $MeOH/CH_2Cl_2$) to give the title compound, FAB MS (M+1) = 389.

Example 21

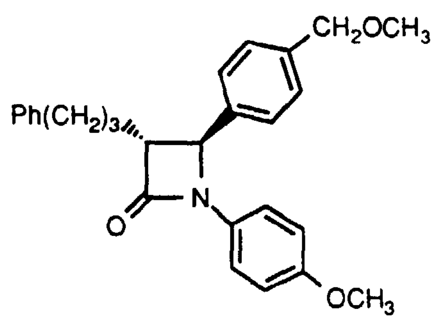

Add freshly prepared AgO (0.40 g, 1.73 mmol) to a solution of the product of Example 15A (0.46g, 1.15 mmol) and methyl iodide (0.21 mL, 3.45 mmol)in dry DMF (5 mL). Heat the mixture to 40-45 °C.with TLC monitoring (50% EtOAc/hexanes). Add proportional amounts of AgO and methyl iodide until TLC indicates consumption of starting compound. Cool to room temperature, partion between water and ethyl acetate, extract with ethyl acetate. Combine ethyl acetate extracts, wash with water and brine, dry over anhydrous $Na_2SO_4$, filter and concentrate to a residue. Chromatograph the residue (silica gel, 30% EtOAc/hexanes) to give the title compound, FAB MS (M+1) = 416.

The following compound can be prepared by substantially the same procedure:

Example 21A

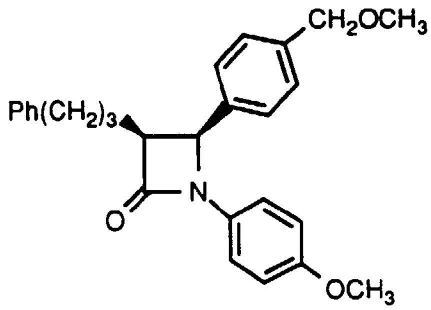

mp 84-85°C

Example 22

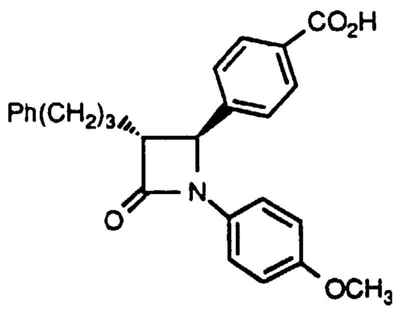

Add freshly prepared Jone's reagent (8 mL) dropwise to a solution of the product of Example 16 (2.76, 6.94 mmol) in acetone (80 mL) maintaining the temperature of the reaction mixture between 15-20 °C. Monitor by TLC (5% MeOH/ $CH_2Cl_2$). Upon consumption of starting compound, quench the reaction with methanol. Concentrate to a residue and partion the residue between $CH_2Cl_2$ and water. Extract with $CH_2Cl_2$, combine the extracts, wash with water, 10% $Na_2SO_3$ (aq) and brine, then dry over anhydrous $Na_2SO_4$. Filter and concentrate to afford 2.90g of the title compound, mp 64-65 °C.

The following compound can be prepared by substantially the same procedure.

Example 22A

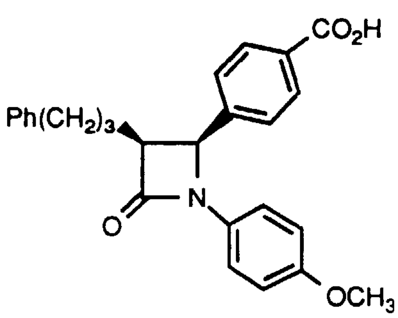

mp 158-159°C

Example 23

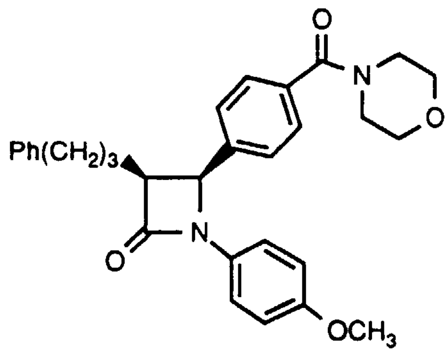

Add EDCI (0.2g, 1.03 mmoL) to a room temperature solution of the product of Example 22A (0.30, 0.72 mmol), N-methylmorpholine (0.11 mL, 0.94 mmol), morpholine (0.13 mL, 0.42mmol) and HOBT (0.12g, 0.87 mmol) in $CH_2Cl_2$ (8 mL). Stir the mixture overnight. Monitor by TLC (5% $MeOH/CH_2Cl_2$). Upon consumption of the starting compound, dilute with $CH_2Cl_2$, wash with 1M HCl, water, dry over anhydrous $Na_2SO_4$, filter and concentrate to a residue. Chromatogaph the residue ($SiO_2$, 3% $MeOH/CH_2Cl_2$) to afford 0.3g (86% yield) of the title compound, mp 61-62 °C.

Example 24

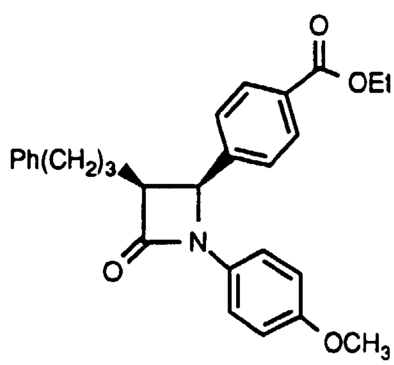

Add EDCI (0.2g, 1.03 mmoL) to a room temperature solution of the product of Example 22A (0.30, 0.72 mmol), N-methylmorpholine (0.11 mL, 0.94 mmol), ethanol (0.1 mL, 1.44 mmol) and HOBT (0.12g, 0.87 mmol) in $CH_2Cl_2$ (8 mL). Stir the mixture overnight. Monitor by TLC (5% $MeOH/CH_2Cl_2$). Upon consumption of starting compound, dilute with $CH_2Cl_2$, wash with 1M HCl, water, dry over anhydrous $Na_2SO_4$, filter and concentrate to a residue. Chromatogaph the residue ($SiO_2$, 3% $MeOH/CH_2Cl_2$) to afford 0.33g (100% yield) of the title compound, mp 76-77 °C.

The following compound can be prepared by substantially the same procedure:

Example 24A

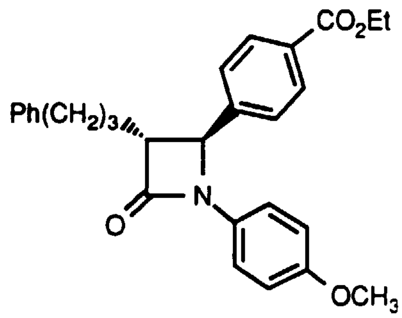

mp 74-75°C

Example 25

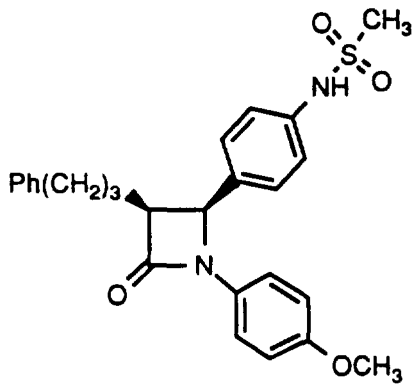

Add methanesulfonyl chloride (0.05 mL, 0.67 mmoL) to a 0°C solution of the product of Example 8B (0.26, 0.67 mmol), pyridine(3 drops) in $CH_2Cl_2$ (5 mL). Stir the mixture overnight. Monitor by TLC (50% EtOAc/hexanes). Upon consumption of starting material, dilute with $CH_2Cl_2$, wash with 0.5M HCl, 5% $NaHCO_3$, water and brine, dry over anhydrous sodium sulfate, filter and concentrate to a residue. Chromatogaph the residue ($SiO_2$, 50% EtOAc/hexanes) to afford 0.18g (58% yield) of the title compound, FAB MS (M+1) = 465.

By substantially the same procedure, the following compounds can be prepared:

| Example 25A | Example 25B |
|---|---|
| 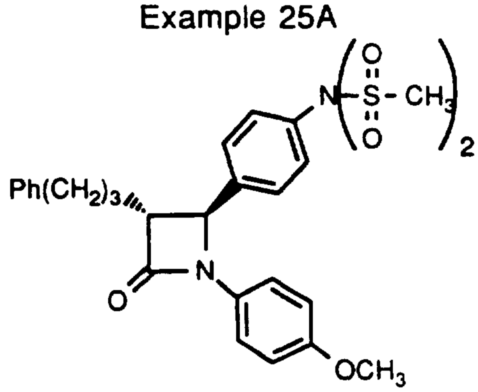 | 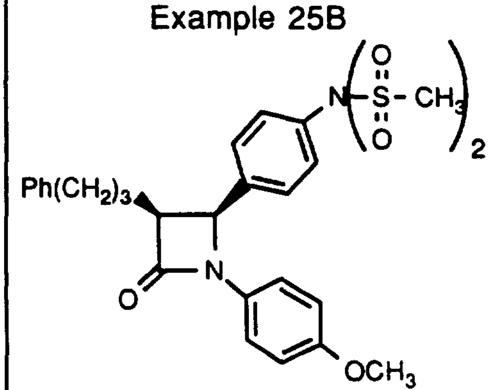 |
| mp 170.5-171.5°C | mp 175-176°C |

**Example 25C**

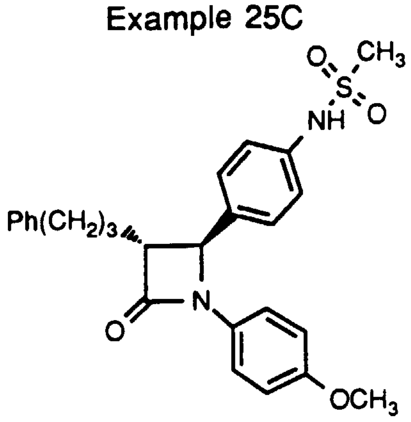

EI MS (M) = 542

Example 26

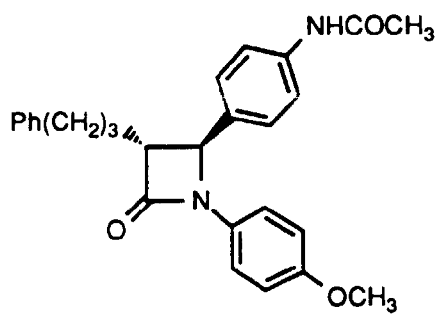

Add acetyl chloride (0.04 mL, 0.57 mmoL) to a solution of the product of Example 8C (0.20, 0.52 mmol) and triethylamine(0.11 mL, 0.79) in $CH_2Cl_2$ (3 mL) at room temperature . Monitor by TLC (50% EtOAc/hexanes). Upon consumption of starting material (~3h), dilute with $CH_2Cl_2$, wash with 5% $NaHCO_3$, water and brine, dry over anhydrous $Na_2SO_4$, filter and concentrate to afford 0.19g, (85% yield) of the title compound, mp 153-154 °C.

Example 27

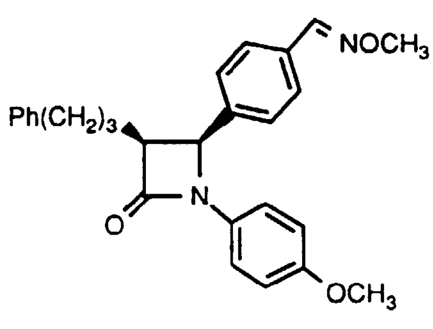

Reflux a suspension of the product of Example 16 (0.6g, 1.5 mmol), hydroxylamine hydrochloride (0.4g, 4.5 mmoL), sodium acetate (0.4g 4.5 mmol), methanol (12mL) and water (5mL) overnight. Monitor by TLC (50% EtOAc/hexanes). Upon consumption of starting material, evaporate to dryness, partition the residue between water and ethyl acetate, extract with EtOAc, combine the extracts, wash with water and brine, dry over anhydrous $Na_2SO_4$, filter and concentrate to a residue. Chromatograph the residue (silica gel, 20% EtOAc/hexanes) to provide the title compound, mp 98-99 °C; and Example 27A:

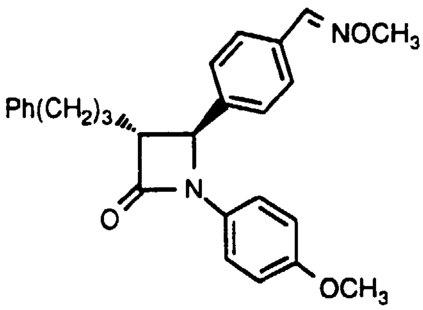

mp 63-64° C

Example 28

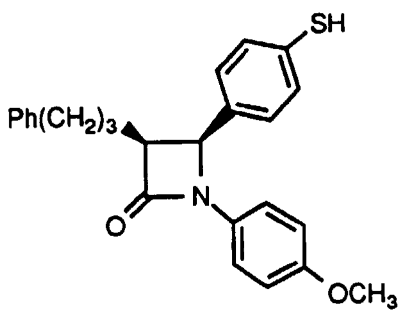

Reflux a solution of the product of Example 17 (3.2g, 7.38 mmol), $CH_2Cl_2$ (15 mL) trifluoracetic anhydride (15 mL) for 15 min. Monitor the reaction by TLC (100% EtOAc). Upon consumption of starting material, distill to remove most of solvent, cool to room temperature, and evaporate to a residue. Dissolve the residue in 50% tnethylamine/methanol solution (30 mL) stir to: 15 min., evaporate to dryness on a rotovap. Redissolve in $CH_2Cl_2$, wash with $NH_4Cl$ (sat.), dry over anhydrous $Na_2SO_4$, filter and concentrate onto enough silica gel (1g $SiO_2$/ mmol substrate) that a free flowing powder results. Load the powder onto a chromatography column packed with silica gel and 30 % EtOAc/hexanes. Elute with 30-60% EtOAc/hexanes to provide 2.65g (89%) of a residue. Recrystallize from $Et_2O/CH_2Cl_2$ to give the title compound, mp 134-135°C.

Example 29

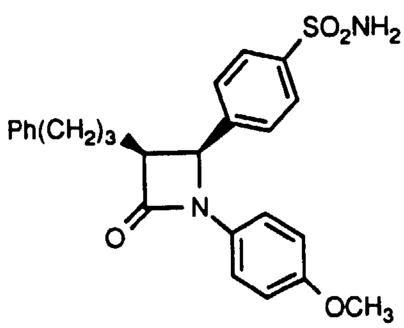

Dissolve the product of Example 28 ( 1.81g, 4.49 mmol) in THF (25 mL) cool to 0 °C. Add 1N NaOH (5.39 mL, 5.39 mmol), and a solution of aqueous ammonia (16.8 mL, 5.39 mmol), followed by cholorox (6.7 mL, 5.39 mmol). Monitor the reaction by TLC (50% EtOAc/hexane). Upon consumption of starting material, dilute with EtOAc, wash with water and brine, dry over anhydrous $Na_2SO_4$ and concentrate to provide the sulfinamine (1.88g).

Dissolve the sulfinamine (1.88g, 4.49 mmol) in $CH_2Cl_2$ (50 mL), add m-chloroperbenzoic acid (1.70g, 9.88 mmol).

Monitor the reaction by TLC (50% EtOAc/hexane). Upon consumption of starting material, quench with solid $Ca(OH)_2$ (1.78g, 24.0 mmol), stir for 20 min., filter and concentrate onto enough silica gel (1g $SiO_2$/ mmol substrate) that a free flowing powder results. Load the powder onto a chromatography column packed with silica gel and 50 % EtOAc/ hexanes. Elute with 50% EtOAc/hexanes to provide 0.49g (24%) of the title compound, mp 174-176°C.

Example 30

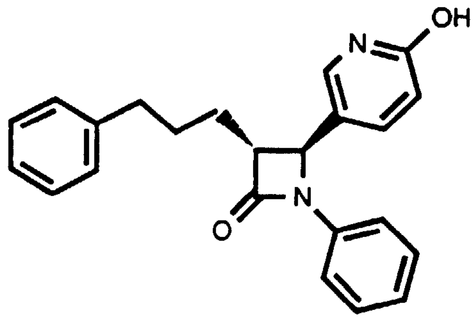

Add $BBr_3$ (5.15 mL, 5.15 mmol, 1M in $CH_2Cl_2$) to a 0 °C solution of the compound of Example 70 (0.768g, 2.06 mmol) in $CH_2Cl_2$ (25 mL). Monitor the reaction by TLC (30% EtOAc). Upon consumption of starting material, quench with $NaHCO_3$ (sat.) and methanol, stir for 30 min., wash with $NaHCO_3$ (sat.) and water, dry over anhydrous $Na_2SO_4$, filter and concentrate onto enough silica gel (1g SiO2/ mmol substrate) such that a free flowing powder results. Load the powder onto a chromatography column packed with silica gel and 100% EtOAc. Elute with 100% EtOAc followed by 5% MeOH/EtOAc to provide 0.234g (32%) of the title compound, CI MS (M+1) = 359.

Example 31

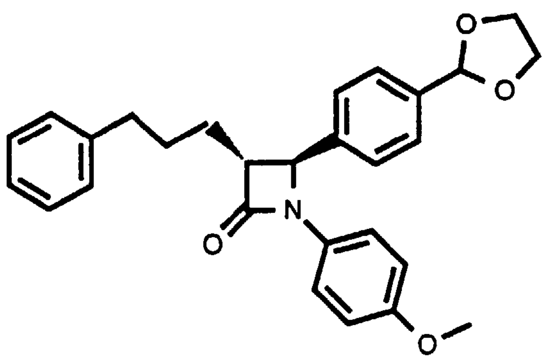

Reflux a mixture of the product of Example 16 (0.23g, 0.6 mmol) ethylene glycol (0.2mL, 3.6 mmol), p-toluene sulfonic acid and toluene (5 mL) overnight. Monitor by 1H NMR and TLC (50% EtOAc/hexanes). Cool to room temperature, wash with $NaHCO_3$(sat.), water and brine, dried over anhydrous $Na_2SO_4$. and concentrate to give 0.27g (100%) of the title compound, EI MS (M) = 443.

The following compound can be prepared by substantially the same procedure:

Example 31A

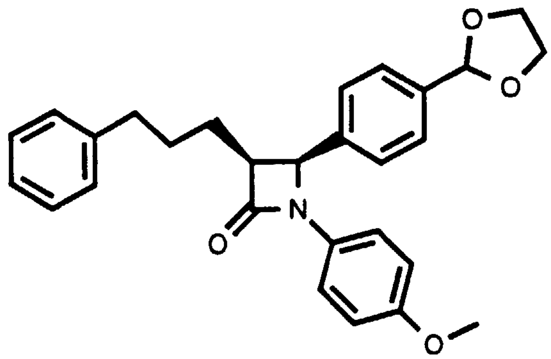

EI MS (M) = 443

Example 32

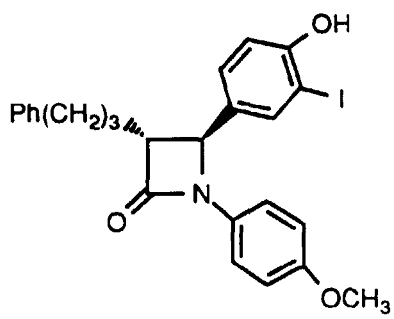

Stir a solution of 388mg (1.00 mmol) of the compound of Example 8F, and 180mg (1.20 mmol) chloramine-T NaI in 4.6 mL DMF. Add 377mg (1.2mmol) and stir for 18h at room temperature. Partition the reaction between 30 mL 1N HCl and 30 mL ethyl acetate. Wash the organic layer with 10% aqueous $Na_2SO_3$ and dry the organic solution with $MgSO_4$. Concentrate in vacuo and chromatograph the residue over (silica gel, 40% ethyl acetate-hexane) to give 170mg of the title compound, CI MS (M+1) = 514, and 41mg of the diodo analog (Example 32A):

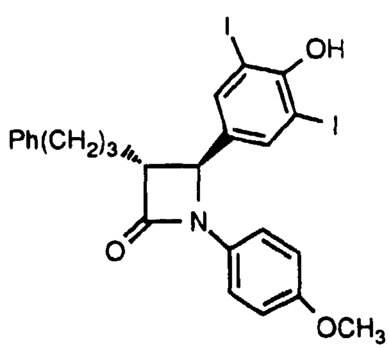

CI MS (M+1) = 640

Example 33

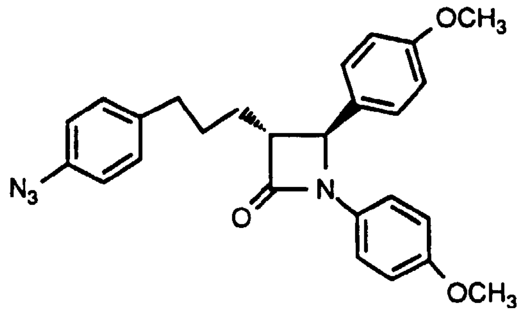

Stir 400 mg (0.96 mmol) of the compound of Example 8E, in 25 mL acetic acid and 5mL water at 0°C and add 133 mg (1.92 mmol) $NaNO_2$ in 4.7 mL water. Stir for 20 min. at 0°C add 212 mg (3.26 mmol) $NaN_3$ in 9 mL water and stir for 3h while warming to room temperature. Dilute with ethyl acetate and neutralize with $NH_4OH$. Dry the ethyl acetate layer with $MgSO_4$ and concentrate to a residue. Recrystallize the residue from ether-hexanes to give the titled compound, mp = 80-84°C.

Example 34

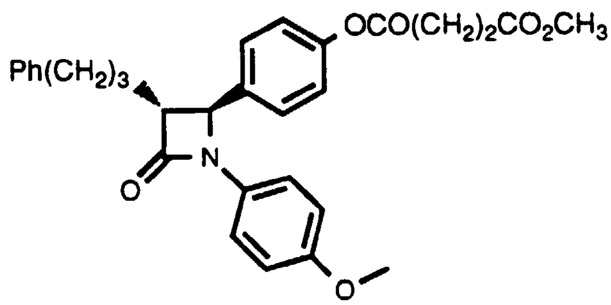

Dissolve 300 mg (0.77 mmol) of the compound of Example 8F, in 3 mL $CH_2Cl_2$ and add 98 µL (120 mg, 0.80 mmol) 3-carbomethoxypropionyl chloride and 146 µL (109 mg, 0.84 mmol) Hünig's base. Stir at room temperature for 1.5h. Add another full portion of acid chloride and Hünig's base and stir for an additional hour. Partition the reaction between ethyl acetate and 1N HCl. Dry the ethyl acetate layer with $MgSO_4$ and concentrate to a residue. Chromatograh the residue ($SiO_2$, 40% ethyl acetate-hexane) and filter through grade I basic alumina eluting with 50%ethyl acetate-hexane to give 334 mg (86%) of the title compound, EI MS (M+) = 501.25.

Example 35

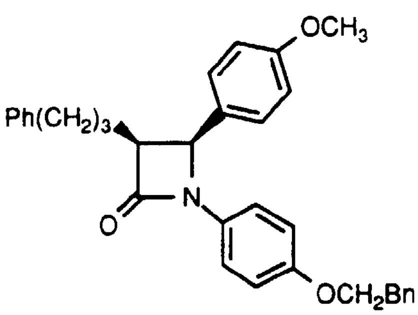

Benzyl bromide (0.44 g) was added to a solution of the compound of Example 15B (1.0 g) in acetone (15 mL)

containing $K_2CO_3$ ( 0.715 g). The reaction was heated at reflux for 26 h. The reaction mixture was then poured into water and the product extracted with ethyl acetate. The crude product was recrystallized from ethyl acetate/hexane to give (0.908 g, 74% yield) of the desired product, mp 115-116°C.

The following compounds were prepared via substantially the same procedure:

Example 35A

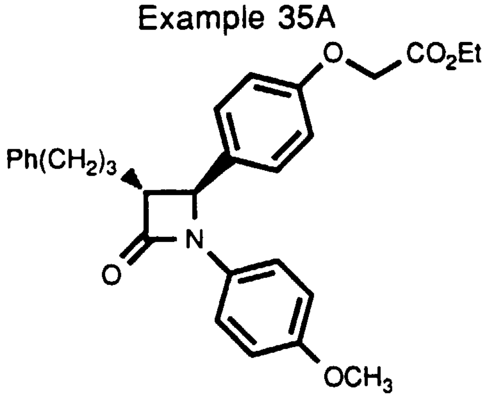

CI MS (M+1) = 474

Example 35B

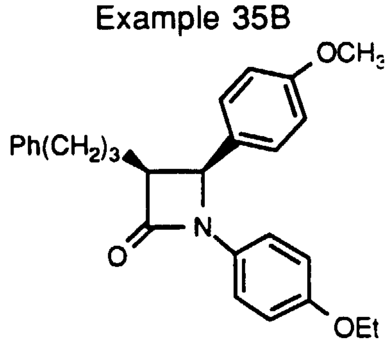

Elemental analysis:
calculated for $C_{27}H_{29}NO_3$:
C, 78,04; H, 7.03; N 3.37
Found: C, 78.00; H, 7.02; N, 3.55

Example 36

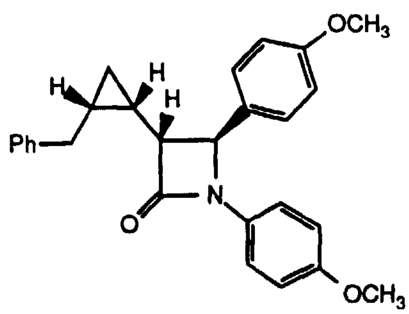

The compound of Example 7AI (0.19 g) was cooled to - 20°C. To this ws added a solution of diethyl zinc in toluene ( 4.3 mL of a 1.1 M solution) followed by diiodomethane (2.56 g). The reaction mixture was slowly allowed to warm to ambient temperature over three hours. Then the reaction mixture was warmed to 45°C for 5 min. After cooling the reaction mixture was treated with aqueous $NH_4Cl$ and product extracted with diethyl ether. The organic layer was washed with water, brine and concentrated to a residue. The residue was purified by chromatography ($SIO_2$, ethyl acetate/hexane (3:7)) to give the title compound (0.17 g, 88% yield),

| Elemental analysis: | | | |
|---|---|---|---|
| calculated - | C, 78.42; | H, 6.58; | N, 3.39 |
| found- | C, 78.32; | H, 6.48; | N, 3.61. |

Example 37

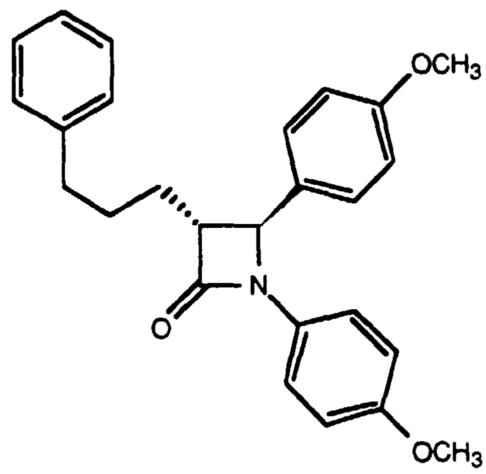

step (a)

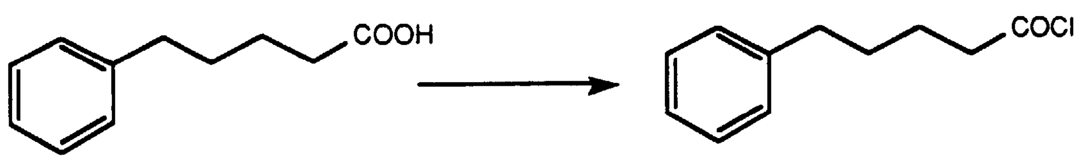

Combine 5-phenylvaleric acid (89.9 g, 0.504 mol) and $SOCl_2$ (89.3 mL, 1.225 mol) in a 500 mL round bottom flask equipped with a condenser and drying tube. Heat the flask to 70°C and maintain the reaction at reflux for 1h. Vacuum distill (50 - 100 mm Hg) the excess $SOCl_2$ and add 200 mL of dry toluene to the remaining mixture. Vacuum distill a second time to remove the toluene and any residual $SOCl_2$. Add 188 mL of dry THF to the crude acid chloride remaining in the reaction vessel and use the resulting solution directly in the next step.

step (b)

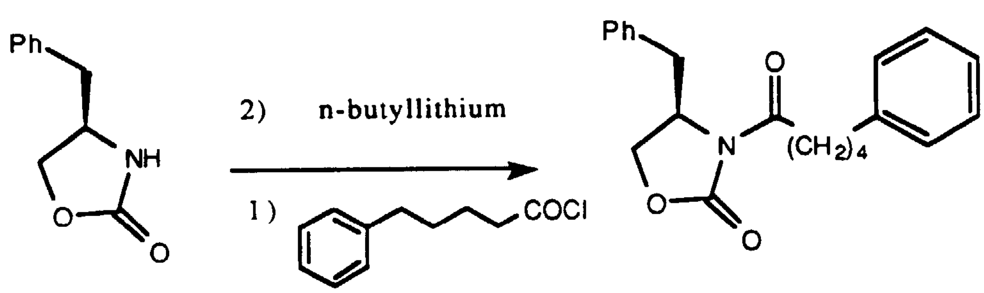


Combine 76 g (0.4289 mol) of R-(+)-4-benzyloxazolidinone and 1.3 L of dry THF under dry nitrogen atmosphere. Cool the resulting solution to -78°C and add 278 mL of a 1.6 M solution of n-butyllithium in hexane over a period of 30-40 minutes. Stir the mixture for an additional 30 minutes following the addition. Add the solution of 5-phenylvaleroyl chloride from step (a) over a period of 45 minutes. Allow the mixture to warm to 0°C and stir for 1 h. Quench the reaction mixture by adding 673.6 mL of $K_2CO_3$ (1 M aqueous solution) and stir for 1 h. Distill off the THF under vacuum at 30-35°C. Dilute the residue with 1 L of water and extract with three 800 mL portions of $CH_2Cl_2$. Combine the organic extracts and wash with 800 mL of water, then with 800 mL of brine. Dry the organic extracts over $MgSO_4$, filter, then concentrate *in vacuo* to an oil. Dissolve the oil in 200 mL of hexane, then distill off the hexane under vacuum. Repeat

the hexane treatment two more times, then dissolve the oil in 1.7 mL of $CH_2Cl_2$. The resulting solution is used directly in the next step.

step (c)

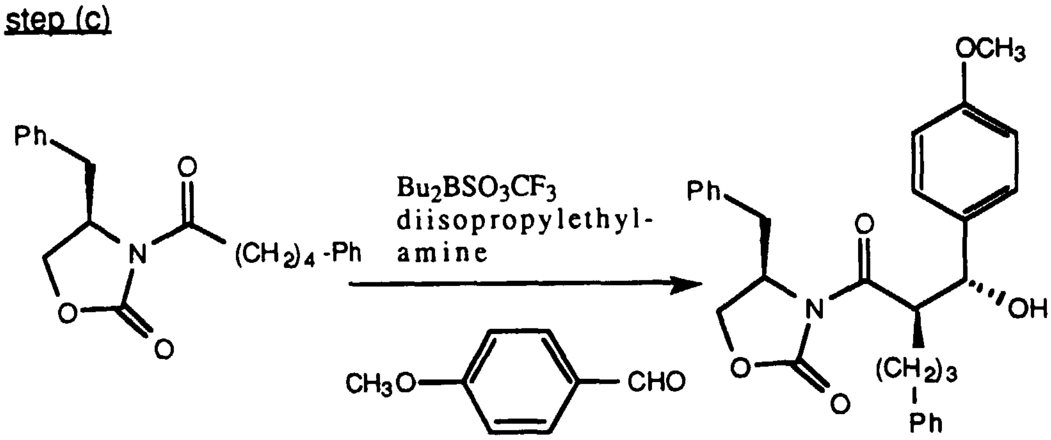


Cool the solution of the product from step (b) to -5°C to 0°C, under dry nitrogen atmosphere. Add 129.8 mL of di-n-butylboron triflate at a rate that maintains the temperature of the reaction mixture at -6°C to -3°C. Following the addition, stir the mixture for 10 minutes, then add 97.12 mL of diisopropylethylamine at a rate that maintains the temperature of the reaction mixture at -6°C to -3°C. Following the addition, stir the mixture at 0°C for 30 minutes, then cool the mixture to -78°C and stir for 30 minutes. Add 57.4 mL of p-anisaldehyde and stir the mixture at -78°C for 30 minutes, then at 0°C for 1 h. While maintaining the temperature at 0°C to 5°C, quench the mixture by adding 688.2 mL of a pH 7 buffer solution (68 g $KH_2PO_4$, 12 g NaOH and 800 mL of water), then add 473 mL of 30% H202 and stir the resulting mixture at 0°C for 1 h. Extract the mixture with three 600 mL portions of hexane:ethyl actetate (1:1). Combine the organic extracts and wash with 800 mL of saturated $NaHCO_3$ (aqueous), then with 800 mL of brine. Dry the organic extracts over $NaSO_4$, filter, and evaporate to an oil. Crystallize the oil from hexane/ethyl acetate (1:1) to give 176 g of the product as a white solid.

step (d)

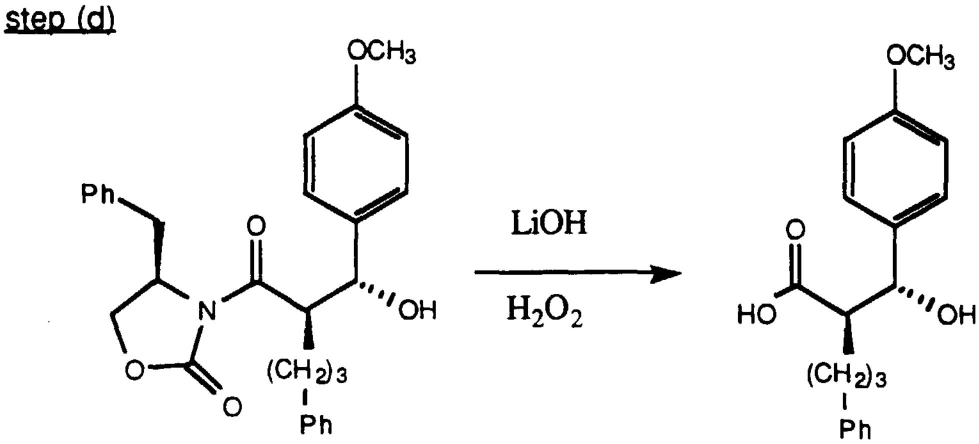


Combine the product of step (c) (170 g, 0.36 Mol), 1595 mL of THF and 400 mL of water, stir the mixture and cool to about 3°C. Add 226 mL (2.156 Mol) of 30% $H_2O_2$ to the mixture over 15 minutes, then add a solution of LiOH (36.2 g, 0.862 Mol) in 400 mL of water over a period of 30 minutes. Stir the reaction mixture at 0°C to 5°C for 3 h. Add a solution of 272 g of $Na_2SO_3$ in 850 mL of water over 70 minutes while keeping the temperature under 27°C. Distill off the bulk of the solvent under vacuum and add 7 L of water. Extract with four 1.7 L portions of toluene. Acidify the aqueous layers to pH 2.4 with 3 N HCl. Extract with one 2.6 L portion and two 1.7 L portions of ethyl acetate. Combine the ethyl acetate extracts, wash with brine, dry over $NaSO_4$, filter, then evaporate to give the product as a white solid, 112 g.

step (e)

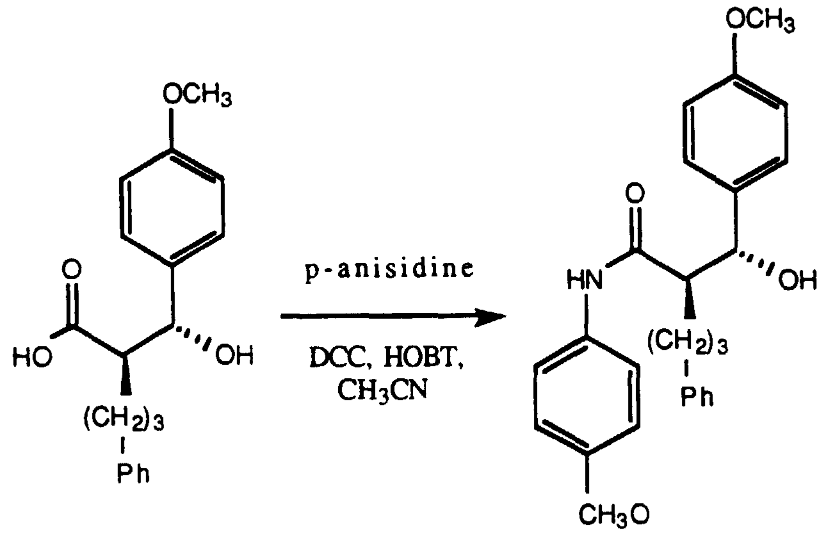


Combine the product of step (d) (19.47 g, 62 mmol), 400 mL of acetonitrile, 9.49 g (62 mmol) of 1-hydroxybenzotriazole (HOBT), 22.91 g (186 mmol) of p-anisidine and 14.05 g (68.2 mmol) of dicyclohexylcarbodiimide (DCC). Stir the reaction mixture at 40°C for 4 h and confirm the consumption of starting material by TLC (6:4 hexane/ ethyl acetate). Concentrate the mixture to 1/3 its volume and partition between 300 mL of water and 300 mL of ethyl acetate. Filter the organic layer, then wash with 200 mL of 1 N HCl, then with two 100 mL portions of saturated $NaHCO_3$, and two 100 mL portions of brine. Dry the organic layer over $NaSO_4$ and concentrate to give the product as a solid, 24 g.

step (f)

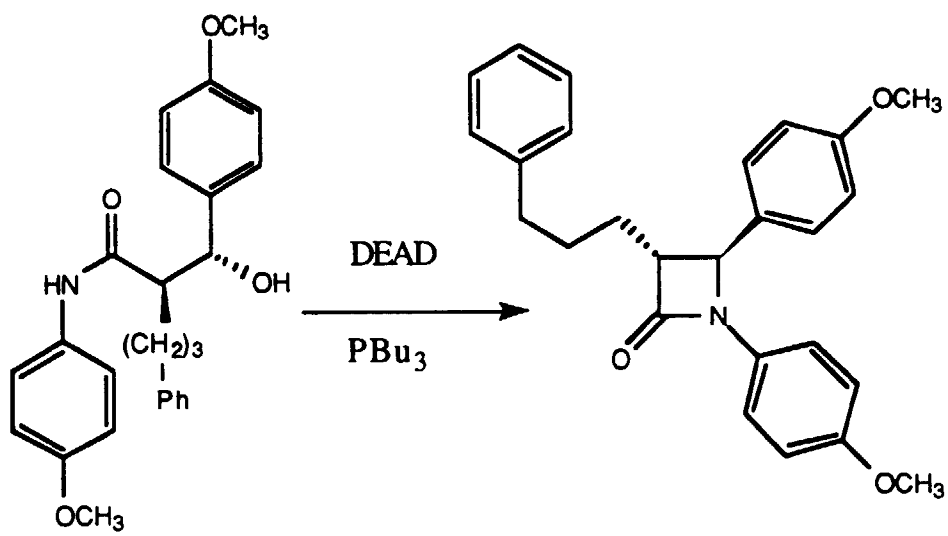


Combine the product of step (e) (115 g, 0.2745 Mol) and 2.3 L of THF under dry nitrogen atmosphere and cool to -70°C. Stir the mixture and simultaneously add a solution of 137 mL (0.551 Mol) of tri-n-butylphosphine in 113 mL THF, and 163 mL (1.03 Mol) of diethylazodicarboxylate (DEAD) over 2 h. Allow the mixture to warm to room temperature and stir overnight. Remove the solvent under vacuum. Filter the residue through a plug of silica gel using $CH_2Cl_2$/ hexane/ ethyl acetate (70:24:6) as the eluant. Evaporate the solvent and purify the residue by preparative HPLC (silica gel, 15% ethyl actetate/ hexane) to give 88 g (80% yield) of the β-lactam product.

Example 38

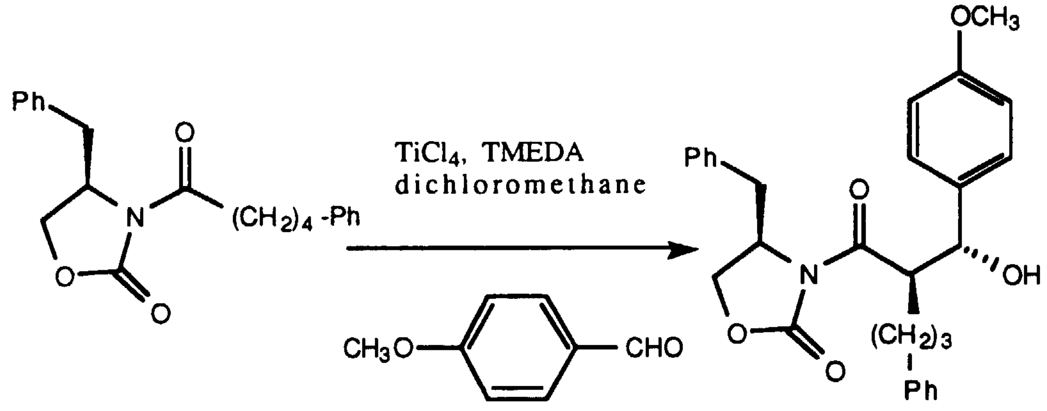


Cool a solution of 33.7 g (0.1 mol) of the product of Example 37, step (b), in 200 mL of $CH_2Cl_2$ to -20°C. Stir the cold solution and add 11 mL (0.1 mol) of $TiCl_4$. Stir the mixture for 10 min. at -20°C, then slowly add 30 mL (2 equiv.) of tetramethylethylenediamine (TMEDA) over a period of 10 min., while keeping the temperature below -10°C. Stir the mixture at -15° to -10°C for 70 min., then add 24 mL (2 equiv.) of p-anisaldehyde. Stir at -15° to -10°C for 1 hour, then allow the mixture to warm to 10°C while stirring for 40 min. Quench the reaction by adding 600 mL of 10% aqueous tartaric acid, then add 600 mL of ethyl acetate. Agitate well, then separate the layers, extracting the aqueous layer with another 200 mL of ethyl acetate. Combine the organic extracts and wash successively with water, saturated $NaHCO_3$ (aqueous) and brine. Dry the organic solution over anhydrous $Na_2SO_4$, filter, then concentrate to a residue. Crystallize the residue from a mixture of 100 mL of ethyl acetate + 210 mL of hexane to give 36.8 g of the desired compound, which can be used in step (d) of Example 37.

Example 39

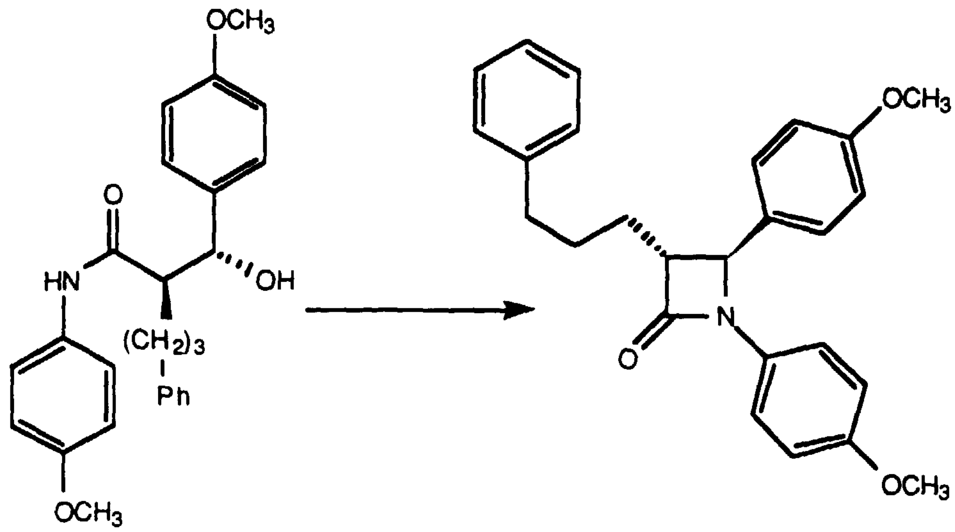

Step (a)

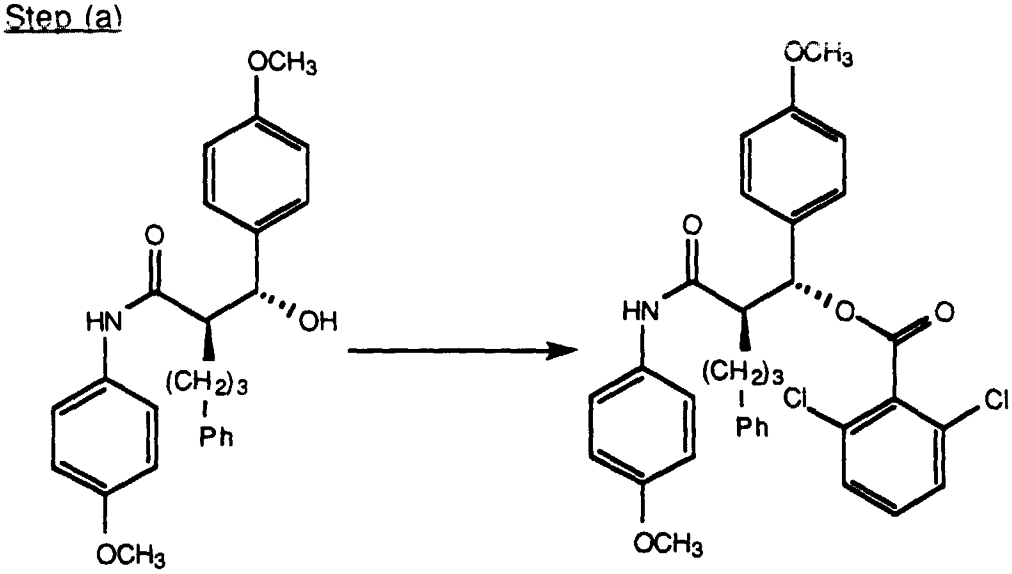

Dissolve 500 g (0.85 mol) of the product of Example 37, step(e), in 1700 mL of $CH_2Cl_2$, then add 4.0 g (12 mmol) of tera-n-butyl-ammonium hydrogen sulfate. Stir the mixture while cooling to 10° to 20°C and add 50% aqueous NaOH (200 g). Slowly add 60 g (285 mmol) of 2,6-dichlorobenzoyl chloride to the stirred mixture over a period of 30 min. Continue stirring at 15° to 20°C for 3 h., then pour the mixture into 2000 mL of cold water. Separate the layers and wash the organic layer with water until neutral pH is attained. Distill the methylene chloride solution to reduce the volume to 800 mL. Heat the solution to reflux and add 800 mL of heptane. Cool the hot solution to 0°C to crystallize. Collect the product by filtration to give 116 g of the dichlorobenzoate product.

Step (b)

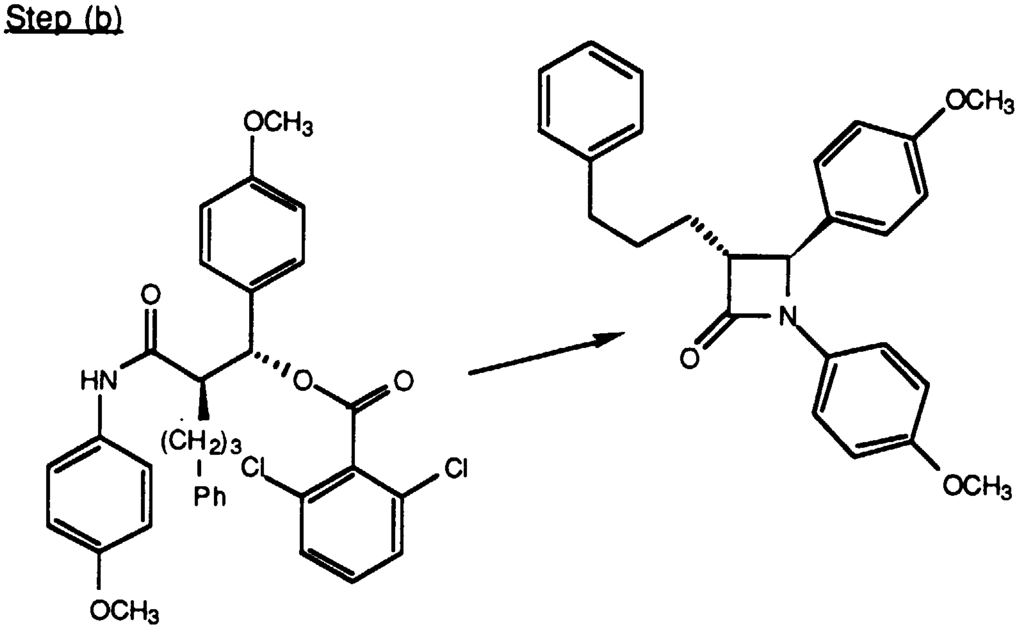

Combine 500 g (0.85 mol) of the product of step (a) with 250 g (1.1 mol) of benzyltriethylammonium chloride, 2000 mL of $CH_2Cl_2$ and 8000 mL of methyl t-butyl ether. Stir the mixture while cooling to 15° to 20°C, then add 1000 mL of 50% aqueous NaOH over a period of 10 min. Stir the mixture for 4 h., then pour into 5000 mL of water and 4 kg of ice. Separate the layers and wash the organic layer with water until the pH is neutral. Distill the solvent to reduce the volume to 2000 mL, then filter. Evaporate the filtrate to a residue and purify the residue by chromatography on silica gel to obtain the crude product. Crystallize the product from 6 volumes of a 1:2 mixture of methyl t-butyl ether and heptane at 0°C to give the product (240 g).

Example 40

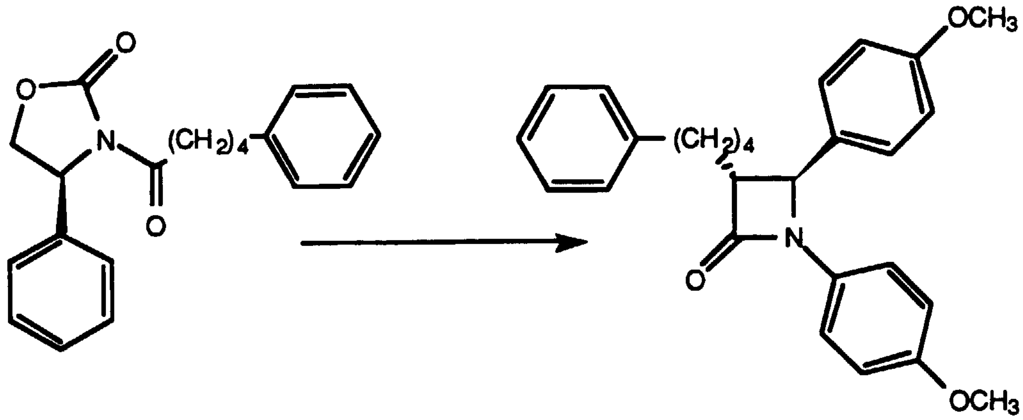

Step (a):

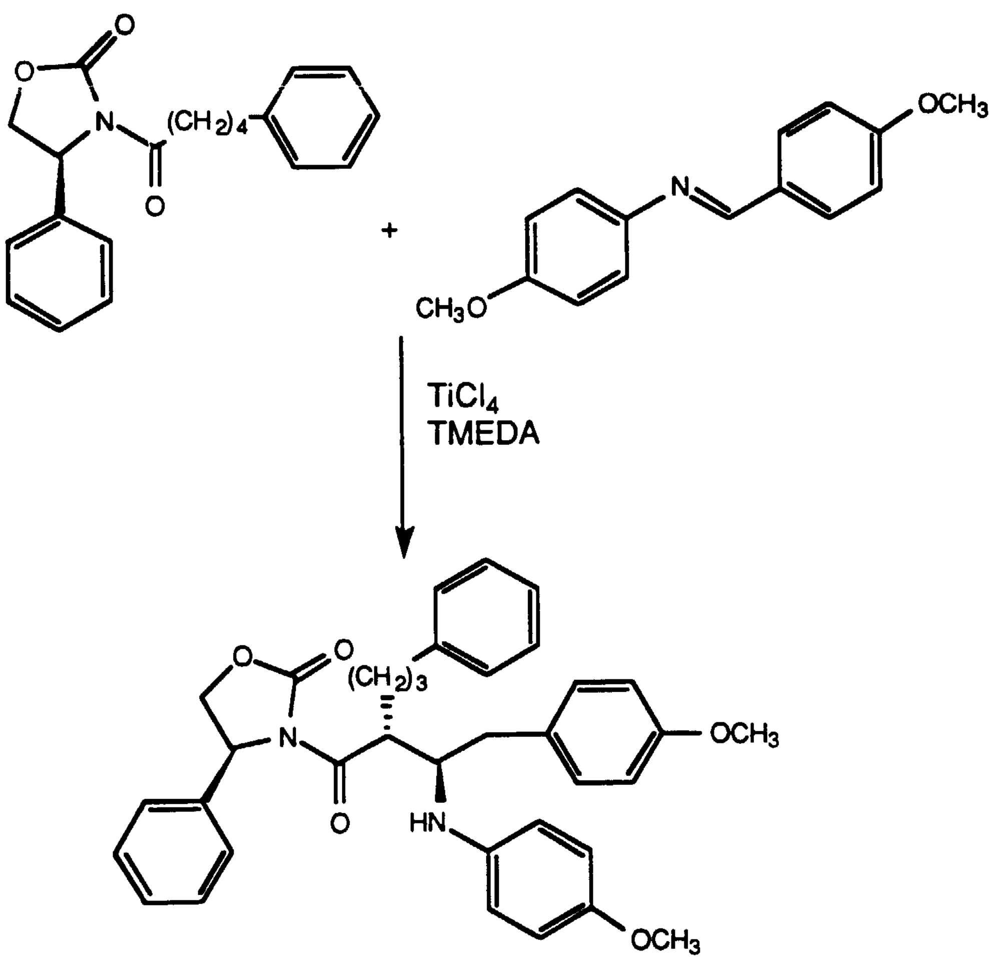


Dissolve 3.23 g (10 mmol) of the product of Example 37, step (b), in 50 mL of $CH_2Cl_2$, then stir under nitrogen atmosphere while cooling to -20°C. Add 10 mL (10 mmol) of a 1M solution of $TiCl_4$ in $CH_2Cl_2$, stir the mixture for 5 min., then add 1.5 mL (10 mmol) of TMEDA. Stir the mixture at -25° to -20°C for 1 h., then slowly add 4.8 g (20 mmol) of the Schiff's base derived from anisaldehyde and p-anisidine as a solution in 50 mL of $CH_2Cl_2$ over a period of 30

min. Stir the mixture at -20°C for 30 min, then gradually warm to 0°C. The reaction is monitored by HPLC (Zorbax® Sil column, 1:4 ethyl acetate/hexane), while stirring at 0°C, until complete. Quench the mixture by pouring into 50 mL of 10% aqueous tartaric acid. Extract with ethyl acetate, then wash the organic extract successively with saturated $NaHCO_3$ (aqueous) and brine. Dry the organic solution over anhydrous $Na_2SO_4$, filter, then concentrate to give the crude product. Crystallize from ethyl acetate/hexane to give the purified product.

**Step (b):**

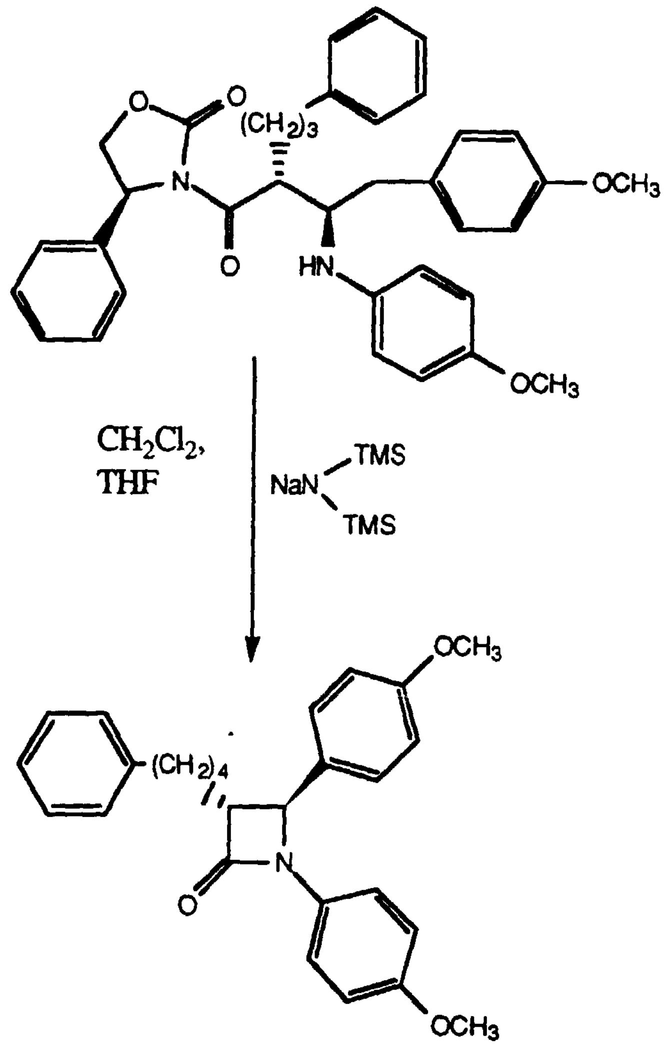


A solution of 0.505 g (0.89 mmol) of the product of step (a) in 25 mL of $CH_2Cl_2$ is stirred at 0°C, then treated with 1.77 mL (1.77 mmol) of a 1M solution of sodium bistrimethylsilylamide in THF. Stir the mixture while warming to room temperature, then continue stirring until the starting material is gone as determined by HPLC (typically 1 to 1 1/2 h.) Quench the mixture into 10% tartaric acid (aqueous). Wash the organic layer successively with saturated $NaHCO_3$ (aqueous) and brine, then dry over anhydrous $Na_2SO_4$. Filter and concentrate to give the title compound.

The following formulations exemplify some of the dosage forms of this invention. In each the term "active compound" designates a compound of formula I or II, preferably (3R,4S)-1,4-bis-(4-methoxyphenyl)-3-(3-phenylpropyl)-2-azetidinone. However, this compound may be replaced by an equally effective amount of other compounds of formula I or II.

EXAMPLE A

| Tablets | | | |
|---|---|---|---|
| No. | Ingredient | mg/tablet | mg/tablet |
| 1 | Active Compound | 100 | 500 |
| 2 | Lactose USP | 122 | 113 |
| 3 | Corn Starch, Food Grade, as a 10% paste in Purified Water | 30 | 40 |
| 4 | Corn Starch, Food Grade | 45 | 40 |
| 5 | Magnesium Stearate | 3 | 7 |
| | Total | 300 | 700 |

Method of Manufacture

Mix Item Nos. 1 and 2 in suitable mixer for 10-15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weight on a suitable tablet machine.

EXAMPLE B

| Capsules | | | |
|---|---|---|---|
| No. | Ingredient | mg/tablet | mg/tablet |
| 1 | Active Compound | 100 | 500 |
| 2 | Lactose USP | 106 | 123 |
| 3 | Corn Starch, Food Grade | 40 | 70 |
| 4 | Magnesium Stearate NF | 4 | 7 |
| | Total | 250 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

Using the test procedures described above, the following in vitro and in vivo data were obtained for the preferred compounds, which are referred to in the following table by the corresponding example numbers. For the in vitro ACAT data, negative percent inhibition denotes apparent stimulation, while positive numbers denote inhibition. For the in vivo results, data is reported as percent change versus control, therefore, negative numbers indicate a positive lipid-lowering effect.

| | ACAT (in vitro) | | | % Reduction (in vivo) | | |
|---|---|---|---|---|---|---|
| Ex. | IC50 (mM) | % Inhib. | Conc. (mM) | Serum Cholest. | Cholest. Esters | Dose mg/kg |
| 1 | --- | 38 | 10 | -45 | -95 | 50 |
| | | | | -17 | -55 | 10 |
| | | | | 0 | 0 | 5 |
| 1A | 7.5 | 83 | 25 | -10 | -26 | 100 |
| 1B | --- | 28 | 10 | 0 | 0 | 50 |
| 1C | --- | 22 | 10 | -6 | -15 | 50 |
| 1D | --- | 39 | 10 | 0 | 0 | 50 |
| 1E | --- | 3 | 10 | --- | --- | --- |
| 1F | --- | 61 | 10 | 0 | 20 | 50 |
| 1G | --- | 21 | 10 | -11 | 0 | 50 |
| 1H | --- | 57 | 10 | -21 | -51 | 50 |
| 1I | 4.5 | 81 | 10 | --- | --- | --- |
| 1J | 3.0 | 86 | 10 | --- | --- | --- |
| 1K | --- | -70 | 10 | 0 | 0 | 50 |
| 1L | --- | 20 | 10 | -31 | -75 | 50 |
| | | | | -22 | -34 | 10 |
| | | | | -25 | -30 | 5 |
| 1M | 7.0 | 80 | 10 | -11 | -31 | 25 |
| 1P | --- | 39 | 10 | -19 | -54 | 50 |
| 1S | --- | -19 | 10 | 0 | -32 | 85 |
| 1T | --- | -11 | 10 | 0 | 0 | 50 |
| 1U | 19 | 58 | 10 | 0 | 0 | 50 |
| 1V | --- | 64 | 10 | --- | --- | --- |
| 1W | --- | 9 | 10 | --- | --- | --- |
| 1X | --- | 9 | 10 | -12 | 0 | 50 |
| 1Y | --- | 50 | 10 | --- | --- | --- |
| 1Z | --- | -15 | 10 | -15 | -39 | 50 |
| 1AA | -- | -36 | 10 | --- | --- | --- |
| 1AB | --- | 17 | 10 | --- | --- | --- |
| 1AC | --- | 40 | 10 | -16 | -33 | 50 |

| | | | | | | |
|---|---|---|---|---|---|---|
| 1AD | --- | -5 | 10 | --- | --- | --- |
| 1AE | --- | -7 | 10 | --- | --- | --- |
| 1AF | --- | 0 | 10 | --- | --- | --- |
| 1AG | --- | -3 | 10 | -16 | -29 | 50 |
| 1AH | --- | 4 | 10 | --- | --- | --- |
| 1AI | --- | 27 | 10 | --- | --- | --- |
| 1AJ | --- | -17 | 10 | 0 | 0 | 50 |
| 1AK | --- | -33 | 10 | --- | --- | --- |
| 1AL | --- | -43 | 10 | 0 | 0 | 50 |
| 1AM | --- | --- | --- | -12 | -29 | 50 |
| 1AN | --- | --- | --- | -34 | -85 | 50 |
| 1AO | --- | --- | --- | -33 | -78 | 50 |
| 1AP | --- | --- | --- | -51 | -95 | 50 |
| 1AQ | --- | --- | --- | -20 | -22 | 50 |
| 1AR | --- | -41 | 10 | -22 | -25 | 50 |
| 1AS | 1.0 | 83 | 10 | 0 | -19 | 50 |
| 1AT | 5.0 | 70 | 10 | 0 | -28 | 50 |
| 1AU | --- | 44 | 10 | 0 | -21 | 50 |
| 1AV | --- | 7 | 10 | 15 | 0 | 50 |
| 1AW | --- | 68 | 10 | -14 | -16 | 50 |
| 1AX | --- | --- | --- | -16 | -52 | 50 |
| 1AY | --- | --- | --- | -28 | -78 | 50 |
| 1AZ | --- | --- | --- | -37 | -91 | 50 |
| 1BA | --- | --- | --- | 0 | -0 | 50 |
| 1BB | --- | -16 | 10 | 0 | 0 | 50 |
| 1BC | --- | -2 | 10 | 0 | 0 | 50 |
| 1BD | --- | 17 | 10 | 0 | -25 | 50 |
| 1BE | --- | 30 | 10 | -10 | -21 | 50 |
| 1BF | --- | -17 | 10 | 0 | -23 | 50 |
| 1BG | --- | 3 | 10 | 12 | 0 | 50 |
| 1BH | --- | 50 | 10 | 0- | 0 | 50 |
| 1BI | --- | 59 | 10 | -17 | -45 | 50 |
| 1BJ | --- | 56 | 10 | 0 | 0 | 50 |
| 1BK | --- | 55 | 10 | 0 | 0 | 50 |

| | | | | | | |
|---|---|---|---|---|---|---|
| 1BL | --- | 42 | 10 | -10 | 0 | 50 |
| 1BM | --- | 38 | 10 | 0 | -21 | 50 |
| 1BN | 7.0 | --- | --- | -16 | 0 | 50 |
| 1BO | 5.4 | 79 | 10 | -48 | -93 | 50 |
| 1BP | --- | 58 | 10 | -9 | 0 | 50 |
| 1BQ | --- | 35 | 10 | 0 | -17 | 50 |
| 1BR | --- | --- | --- | -15 | -70 | 50 |
| 1BS | --- | --- | --- | 0 | -43 | 50 |
| 1BT | --- | --- | --- | 0 | 0 | 50 |
| 1BU | --- | --- | --- | -39 | -95 | 50 |
| 1BV | --- | --- | --- | -11 | 0 | 50 |
| 1BW | --- | --- | --- | -9 | -29 | 50 |
| 1BX | --- | --- | --- | 0 | 0 | 50 |
| 1BY | --- | --- | --- | -18 | -72 | 50 |
| 1BZ | --- | --- | --- | -9 | 0 | 50 |
| 1CA | --- | --- | --- | -12 | 0 | 50 |
| 1CB | --- | 33 | 10 | 0 | 0 | 50 |
| 1CC | --- | 46 | 10 | 0 | 0 | 50 |
| 1CD | 7.0 | 84 | 10 | 0 | -23 | 50 |
| 1CE | --- | 15 | 10 | -19 | -17 | 50 |
| 1CF | --- | 53 | 10 | -23 | -47 | 50 |
| 1CG | --- | 8 | 10 | -30 | -61 | 50 |
| 1CH | --- | --- | --- | -49 | -95 | 50 |
| | | | | -41 | -90 | 10 |
| | | | | -24 | -80 | 3 |
| 1CI | --- | 26 | 10 | -8 | -23 | 50 |
| 1CJ | --- | --- | --- | 0 | 0 | 50 |
| 1CK | --- | -12 | 10 | 0 | -28 | 50 |
| 1CL | --- | 89 | 10 | 0 | -18 | 50 |
| 1CM | | | | 0 | 0 | 50 |
| 1CN | --- | -28 | 10 | 0 | 0 | 50 |
| 2 | 6.0 | 82 | 10 | -15 | -37 | 5 |
| | | | | -28 | -69 | 10 |
| 2A | 25 | --- | --- | 0 | 0 | 50 |

| | | | | | | |
|---|---|---|---|---|---|---|
| 3 | --- | 56 | 10 | 0 | 0 | 50 |
| 3A | --- | 33 | 10 | -12 | -29 | 50 |
| 3B | 6.0 | 84 | 10 | -11 | -16 | 50 |
| 3C | --- | -18 | 10 | --- | --- | --- |
| 3D | 12 | 42 | 10 | 0 | 0 | 50 |
| 3E | --- | -24 | 10 | 0 | 0 | 50 |
| 3F | 1.7 | 82 | 10 | 0 | -31 | 50 |
| 3G | 5.0 | 74 | 10 | 9 | 0 | 50 |
| 3H | --- | 66 | 10 | -10 | 31 | 50 |
| 3I | --- | 43 | 10 | -9 | 0 | 50 |
| 3J | --- | 45 | 10 | 0 | -16 | 50 |
| 3K | --- | 19 | 10 | 0 | 0 | 50 |
| 3L | --- | 53 | 10 | 0 | 0 | 25 |
| 3M | --- | 13 | 10 | 0 | 0 | 50 |
| 3N | --- | 62 | 10 | 0 | 0 | 50 |
| 3O | --- | -28 | 10 | 0 | 0 | 50 |
| 3Q | --- | 3 | 10 | -6 | 0 | 50 |
| 3R | --- | -8 | 10 | 0 | 0 | 50 |
| 3T | --- | -9 | 10 | --- | --- | --- |
| 3U | --- | 46 | 10 | 0 | 0 | 50 |
| 3V | 6.5 | 60 | 10- | 0 | 0 | 50 |
| 3W | --- | 56 | 10 | 0 | 0 | 50 |
| 3X | --- | 3 | 10 | --- | --- | --- |
| 3Y | --- | -33 | 10 | 0 | 0 | 50 |
| 4 | --- | 16 | 10 | --- | --- | --- |
| 5 | 18 | 33 | 10 | -29 | -77.5 | 50 |
| | | | | -20 | -72 | 25 |
| | | | | -21 | -60 | 10 |
| 5A | --- | -70 | 10 | --- | --- | --- |
| 5C | --- | 21 | 10 | -17 | -24 | 50 |
| 5D | --- | --- | --- | -38 | -95 | 50 |
| | | | | -41 | -94 | 30 |
| | | | | -24 | -77 | 10 |
| 5E | --- | --- | --- | -38 | -91 | 50 |

| | | | | | | |
|---|---|---|---|---|---|---|
| 5F | --- | --- | --- | -44 | -98 | 50 |
| | | | | -15 | -59 | 30 |
| | | | | -13 | -25 | 10 |
| 5G | --- | --- | --- | -44 | -93 | 50 |
| 5H | --- | --- | --- | -57 | -96 | 50 |
| 5I | --- | --- | --- | -26 | -70 | 50 |
| 5J | --- | --- | --- | -38 | -90 | 50 |
| 5K | --- | --- | --- | -28 | -49 | 50 |
| 5L | --- | --- | --- | -50 | -95 | 10 |
| | | | | -39 | -84 | 3 |
| | | | | -54 | -64 | 1 |
| 5M | --- | --- | --- | -8 | -36 | 10 |
| | | | | -12 | 0 | 3 |
| | | | | -20 | -50 | 50 |
| 5N | --- | -23 | 10 | -17 | -55 | 50 |
| 5P | --- | 53 | 10 | -11 | -33 | 50 |
| 5Q | --- | -36 | 10 | 0 | -19 | 50 |
| 5R | --- | 18 | 10 | -20 | -21 | 50 |
| 5S | --- | 28 | 10 | -52 | -98 | 50 |
| 5T | --- | --- | --- | -15 | -48 | 50 |
| 5U | --- | --- | --- | -48 | -86 | 50 |
| 5V | --- | --- | --- | -37 | -77 | 50 |
| 5W | --- | -16 | 10 | 0 | -19 | 50 |
| 5X | --- | 38 | 10 | 0 | -39 | 50 |
| 5Y | --- | --- | --- | -29 | 0 | 50 |
| 5Z | --- | --- | --- | -16 | 0 | 50 |
| 5AA | --- | --- | --- | -53 | -93 | 50 |
| 5AB | --- | 30 | 10 | -28 | -72 | 50 |
| 6 | 5 | 59 | 10 | 14 | 0 | 50 |
| 7 | --- | --- | --- | -49 | -95 | 40 |
| | | | | -41 | -89.5 | 10 |
| | | | | -30 | -50 | 3 |
| 7A | --- | --- | --- | -22 | -54 | 50 |
| 7B | --- | --- | --- | -13 | -45 | 50 |

| | | | | | | |
|---|---|---|---|---|---|---|
| 7C | --- | --- | --- | -51 | -95 | 50 |
| 7D | --- | --- | --- | -35 | -74 | 50 |
| 7E | --- | --- | --- | -52 | -93 | 50 |
| 7F | --- | --- | --- | 0 | -26 | 50 |
| 7J | --- | --- | --- | -21 | -27 | 50 |
| 7K | --- | --- | --- | 0 | -32 | 50 |
| 7L | -- | --- | --- | 0 | 0 | 50 |
| 7M | --- | --- | --- | -38 | -94 | 50 |
| 7N | --- | --- | --- | -11 | 0 | 50 |
| 7O | --- | --- | --- | -14 | -20 | 50 |
| 7P | --- | --- | --- | -40 | -95 | 50 |
| 7Q | --- | --- | --- | -16 | 0 | 50 |
| 7R | --- | --- | --- | -35 | -80 | 50 |
| 7S | --- | --- | --- | -27 | -82 | 50 |
| 7T | --- | --- | --- | -49 | -93 | 50 |
| 7U | --- | --- | --- | -29 | -60 | 50 |
| 7V | --- | --- | --- | 0 | 0 | 50 |
| 7W | --- | --- | --- | 12 | 0 | 50 |
| 7X | --- | --- | --- | -26 | -78 | 50 |
| 7Y | --- | --- | --- | -53 | -94 | 50 |
| | | | | -27 | -79 | 10 |
| | | | | -22 | -55 | 3 |
| 7Z | --- | --- | --- | -21 | -39 | 50 |
| 7AA | --- | --- | --- | -10 | -42 | 50 |
| 7AC | --- | 31 | 10 | 0 | 0 | 50 |
| 7AD | --- | -22 | 10 | -12 | -12 | 50 |
| 7AE | --- | -95 | 10 | -17 | -10 | 50 |
| 7AF | --- | 53 | 10 | 23 | -15 | 50 |
| 7AG | --- | --- | --- | 0 | -15 | 50 |
| 7AH | --- | -5 | 10 | 16 | 16 | 50 |
| 7AI | --- | --- | --- | -63 | -95 | 50 |
| | | | | -20 | -73 | 10 |
| | | | | -11 | -46 | 3 |
| 7AJ | --- | --- | --- | 0 | -18 | 50 |

| | | | | | | |
|---|---|---|---|---|---|---|
| 7AK | --- | --- | --- | 12 | -28 | 50 |
| 7AL | --- | --- | --- | -13 | 0 | 50 |
| 7AM | --- | --- | --- | -24 | -70 | 50 |
| 7AN | --- | --- | --- | -12 | -42 | 50 |
| 7AO | --- | --- | --- | -41 | -90 | 50 |
| 7AP | --- | --- | --- | -24 | -82 | 50 |
| 7AQ | --- | --- | --- | -52 | -91 | 50 |
| 7AR | --- | --- | --- | -32 | -88 | 50 |
| 7AS | --- | --- | --- | 0 | -20 | 50 |
| 7AT | --- | --- | --- | -10 | -32 | 50 |
| 8 | --- | -12 | 10 | --- | --- | --- |
| 8A | --- | -17 | 10 | 0 | 0 | 50 |
| 8B | --- | -5 | 10 | -9 | -35 | 50 |
| 8C | --- | 63 | 10 | 0 | 0 | 50 |
| 8E | --- | --- | --- | 0 | -32 | 50 |
| 8F | --- | 8 | 10 | -16 | -48 | 50 |
| | | | | -20 | -45 | 25 |
| 9 | 26 | 30 | 20 | -43 | -93 | 10 |
| | | | | -21.5 | -66 | 5 |
| | | | | -25 | -68 | 3 |
| 10A | --- | -37 | 10 | -19 | -58 | 50 |
| 11 | --- | --- | --- | -8 | 0 | 50 |
| 12 | --- | --- | --- | -12 | 0 | 50 |
| 12A | --- | --- | --- | -14 | -39 | 50 |
| 12B | --- | --- | --- | 0 | -26 | 50 |
| 12C | --- | --- | --- | 0 | 0 | 50 |
| 12D | --- | --- | --- | 0 | 0 | 50 |
| 13 | --- | --- | --- | -18 | 0 | 50 |
| 14 | --- | --- | --- | 30 | -87 | 50 |
| 14A | --- | --- | --- | 31 | -78 | 50 |
| 14B | --- | --- | --- | 0 | 0 | 50 |
| 14C | --- | --- | --- | 0 | 0 | 50 |
| 15 | --- | -6 | 10 | -21 | -46 | 50 |
| 15A | --- | 2 | 10 | 0 | 0 | 50 |

| | | | | | | |
|---|---|---|---|---|---|---|
| 15B | --- | -55 | 10 | 0 | -18 | 50 |
| 16 | --- | 42 | 10 | -25 | -29 | 50 |
| 16A | --- | 55 | 10 | -30 | -53 | 50 |
| 17 | --- | 25 | 10 | -17 | -52 | 50 |
| 17A | --- | 38 | 10 | -11 | -25 | 50 |
| 17B | --- | --- | --- | 0 | -39 | 50 |
| 18 | --- | --- | --- | 0 | 0 | 50 |
| 19 | --- | 24 | 10 | -20 | -51 | 50 |
| 19A | --- | 64 | 10 | -20 | -34 | 50 |
| 19B | --- | 53 | 10 | -26 | -31 | 50 |
| 19C | --- | --- | --- | -9 | -46 | 50 |
| 19D | --- | --- | --- | -15 | -18 | 50 |
| 19E | --- | --- | --- | -19 | -30 | 50 |
| 20 | --- | --- | --- | 12 | 0 | 50 |
| 21 | --- | --- | --- | -27 | -56 | 50 |
| 21A | --- | --- | --- | 0 | -25 | 50 |
| 22 | --- | --- | --- | -17 | -33 | 50 |
| 22A | --- | --- | --- | -16 | -22 | 50 |
| 23 | --- | --- | --- | -22 | -40 | 50 |
| 24 | --- | --- | --- | -17 | 0 | 50 |
| 24A | --- | --- | --- | -21 | -76 | 50 |
| 25 | --- | --- | --- | -8 | -34 | 50 |
| 25A | --- | --- | --- | -13 | 0 | 50 |
| 25B | --- | --- | --- | 0 | 0 | 50 |
| 25C | --- | --- | --- | -8 | -46 | 50 |
| 26 | --- | --- | --- | -4 | -26 | 50 |
| 27 | --- | --- | --- | -12 | -36 | 50 |
| 27A | --- | --- | --- | -15 | -42 | 50 |
| 28 | --- | --- | --- | -19 | 0 | 50 |
| 29 | --- | --- | --- | -22 | -24 | 50 |
| 31 | --- | --- | --- | -19 | -52 | 50 |
| 31A | --- | --- | --- | 0 | 0 | 50 |
| 32 | --- | --- | --- | 0 | 0 | 50 |
| 32A | --- | --- | --- | 0 | 0 | 50 |

| | | | | | | |
|---|---|---|---|---|---|---|
| 33 | --- | --- | --- | 0 | 0 | 50 |
| 34 | --- | --- | --- | -39 | -94 | 50 |
| 35 | --- | 18 | 10 | -15 | -32 | 50 |
| 35A | --- | --- | --- | -34 | -84 | 50 |
| 35B | --- | 65 | 10 | -53 | -99 | 50 |
| 36 | --- | --- | --- | -53 | -94 | 50 |

## Claims

**Claims for the following Contracting States : AT, BE, CH, LI, DE, DK, FR, GB, IT, LU, MC, NL, SE, PT**

1. A compound having the structural formula

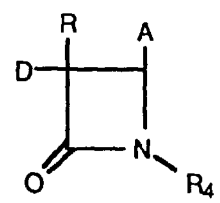

wherein

A is

$-CH=CH-B$;

$-C\equiv C-B$;

$-(CH_2)_p-X-B$, wherein p is 0, 1 or 2 and X is a bond, -NH- or $-S(O)_{0-2}-$;

heteroaryl, benzofused heteroaryl, W-substituted heteroaryl or W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, and wherein W is 1-3 substituents on the ring carbon atoms selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, $-CF_3$, $-OCF_3$, benzyl, $R_{14}$ benzyl, benzyloxy, $R_{14}$-benyloy, phenoxy, $R_{14}$-phenoxy, dioxolanyl, $NO_2$, $-NR_{10}R_{11}$, $NR_{10}R_{11}$ ($C_{1-6}$ alkyl)-, $NR_{10}R_{11}$ ($C_{1-6}$ alkoxy)-, OH, halogeno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldimethylsilyloxymethyl, $-C(O)R_{12}$ and

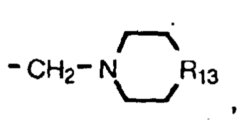

and wherein the substituents on the substituted heteroaryl ring nitrogen atoms, when present, are selected from the group consisting of ($C_{1-6}$ alkyl, $C_{1-6}$ alkoxy,$-C(O)OR_5$, $-C(O)R_5$, OH, $NR_{10}R_{11}$(lower alkyl)-, $NR_{10}R_{11}$ ($C_{1-6}$ alkoxy)-, $-S(O)_2NH_2$ and 2-(trimethylsilyl)ethoxymethyl;

$-C(O)-B$; or

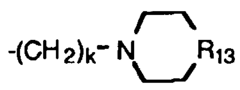,

wherein k is 1 or 2;

D is

B'-$(CH_2)_m$C(O)-, wherein m is 1, 2, 3, 4 or 5;
B'-$(CH_2)_q$-, wherein q is 2, 3, 4, 5 or 6;
B'-$(CH_2)_e$-Z-$(CH_2)_r$-, wherein Z is -O-, -C(O)-, phenylene, -$NR_8$- or -$S(O)_{0-2}$-, e is 0, 1, 2, 3, 4 or 5 and r is 1, 2, 3, 4 or 5, provided that the sum of e and r is 1, 2, 3, 4, 5 or 6;
B'-($C_2$-$C_6$ alkenylene)-; B'-($C_4$-$C_6$ alkadienylene)-;
B'-$(CH_2)_t$-Z-($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6;
B'-$(CH_2)_f$-V-$(CH_2)_g$-, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6;
B'-$(CH_2)_t$-V-($C_2$-$C_6$ alkenylene)- or B'-($C_2$-$C_6$ alkenylene). V-$(CH_2)_t$-, wherein V and t are as defined above, provided that the sum of t and the number of carbon atoms in the alkenylene chain is 2, 3, 4, 5 or 6;
B'-$(CH_2)_a$-Z-$(CH_2)_b$-V-$(CH_2)_d$-, wherein Z and V are as defined above and a ,b and d are independently 0, 1, 2, 3, 4, 5 or 6, provided that the sum of a, b and d is 0, 1, 2, 3, 4, 5 or 6;
T-$(CH_2)_s$-, wherein T is cycloalkyl of 3-6 carbon atoms and s is 1, 2, 3, 4, 5 or 6; or
naphthylmethyl, heteroarylmethyl, or W-substituted heteroarylmethyl, wherein heteroaryl and W are as defined above;

B is

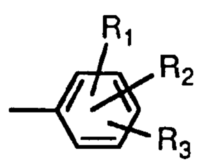

B' is naphthyl, heteroaryl or W-substituted heteroaryl, wherein heteroaryl is as defined above, or

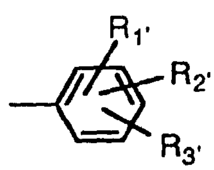

R is hydrogen, fluoro, $C_1$-$C_{15}$ alkyl, $C_2$-$C_{15}$ alkenyl, $C_2$-$C_{15}$ alkynyl, or B-$(CH_2)_h$-, wherein h is 0, 1, 2, or 3;
$R_1$, $R_2$ and $R_3$ are independently selected from the group consisting of H, $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoximino)-$C_{1-6}$ alkyl, $C_{3-6}$ alkanedioyl, $C_{1-6}$ alkyl-$C_{3-6}$ alkanedioyl, allyloxy, -$CF_3$, -$OCF_3$, benzyl, $R_{14}$ benzyl, benzyloxy, $R_{14}$-benyloxy, phenoxy, $R_{14}$-phenoxy, dioxolanyl, $NO_2$, -$NR_{10}R_{11}$, $NR_{10}R_{11}$ ($C_{1-6}$ alkyl)-, $NR_{10}R_{11}$ ($C_{1-6}$ alkoxy)-, OH, o-halogeno, m-halogeno, -NHC (O) $OR_5$, -NHC(O)$R_5$, $R_6O_2$SNH-, $(R_6O_2S)_2$N-, -$S(O)_2NH_2$, -$S(O)_{0-2}R_{10}$, tert-butyldimethylsilyloxymethyl, -C(O)$R_{12}$,

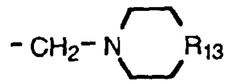

and

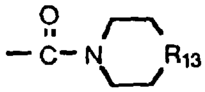

,

or $R_1$ is hydrogen and $R_2$ and $R_3$, together with adjacent carbon atoms to which they are attached, form a dioxolanyl ring;

$R_{1'}$, $R_{2'}$ and $R_{3'}$ are independently selected from the group consisting of H, $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoximino)-$C_{1-6}$ alkyl, $C_{3-6}$ alkanedioyl, $C_{1-6}$ alkyl-$C_{3-6}$ alkanedioyl, allyloxy, $-CF_3$, $-OCF_3$, benzyl, $R_{14}$-benzyl, benzyloxy, $R_{14}$-benyloxy, phenoxy, $R_{14}$-phenoxy, dioxolanyl, $NO_2$, $-NR_{10}R_{11}$, $NR_{10}R_{11}$ ($C_{1-6}$ alkyl)-, $NR_{10}R_{11}$ ($C_{1-6}$ alkoxy)-, OH, halogeno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldimethylsiloxymethtyl, $-C(O)R_{12}$,

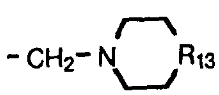 and 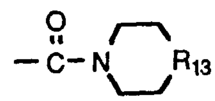

,

or $R_{1'}$ is hydrogen and $R_{2'}$ and $R_{3'}$, together with adjacent carbon atoms to which they are attached, form a dioxolanyl ring;

$R_4$ is

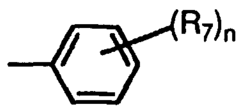

,

wherein n is 0, 1, 2 or 3, indanyl, benzofuranyl, benzodioxolyl, tetrahydronaphthyl, pyridyl, pyrazinyl, pyrimidinyl or quinolyl;

$R_5$ is $C_{1-6}$ alkyl, phenyl, $R_{14}$-Phenyl, benzyl or $R_{14}$-benzyl;

$R_6$ is OH, $C_{1-6}$ alkyl, phenyl, benzyl, $R_{14}$-Phenyl or $R_{14}$-benzyl;

$R_7$ is $C_{1-6}$ alkyl, $C_{1-6}$ alkoxy, OH, halogeno, $-NR_{10}R_{11}$, $-NHC(O)OR_5$, $-NHC(O)R_5$, $NO_2$, -CN, $-N_3$, -SH, $-S(O)_{0-2}$-($C_{1-6}$ alkyl), $-COOR_9$, $-CONR_{10}R_{11}$, $-COR_{12}$, phenoxy, benzyloxy, $-OCF_3$, or tert-butyldimethylsilyloxy, and where n is 2 or 3, the $R_7$ groups can be the same or different;

$R_8$ is H, $C_{1-6}$ alkyl, phenyl $C_{1-6}$ alkyl, or $-C(O)R_9$;

$R_9$ is H, $C_{1-6}$ alkyl, phenyl or phenyl $C_{1-6}$ alkyl;

$R_{10}$ and $R_{11}$ are independently selected from H and $C_{1-6}$ alkyl;

$R_{12}$ is H, OH, $C_{1-6}$ alkoxy, phenoxy, benzyloxy,

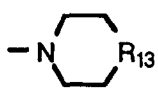

,

$-NR_{10}R_{11}$, $C_{1-6}$ alkyl, phenyl or $R_{14}$-phenyl;

$R_{13}$ is -O-, $-CH_2-$, -NH- or -N($C_{1-6}$ alkyl)-; and

$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;

or a pharmaceutically acceptable salt thereof.

**2.** A compound of claim 1 wherein R is hydrogen.

**3.** A compound of claim 1 or 2 wherein D is B'-$(CH_2)_q$-, B'-$(CH_2)_e$-Z-$(CH_2)_r$-, B'-($C_2$-$C_6$ alkenylene)- or B'-$(CH_2)_f$-V-$(CH_2)_g$-, wherein B', Z, V, q, e, r, f, and g are as defined in claim 1.

**4.** A compound of claim 3 wherein D is B'-$(CH_2)$q- wherein B' is phenyl and q is 3 or 4; B'-$(CH_2)_e$-Z-$(CH_2)_r$ wherein B' is p-fluorophenyl or p-methoxyphenyl, e is zero, Z is -O-, and r is 2; B'-($C_2$-$C_6$ alkenylene)- is 3-phenyl-1-propenyl; or B'-$(CH_2)_f$-V-$(CH_2)_g$- wherein B' is phenyl, f is 1, V is cyclopropylene, and g is zero.

**5.** A compound of claim 1, 2, 3 or 4 wherein A is -$(CH_2)_p$-X-B wherein wherein X, B and p are as defined in claim 1.

**6.** A compound of claim 5 wherein p is zero and X is a bond.

**7.** A compound of claim 6 wherein $R_1$, $R_2$ and $R_3$ are selected from the group consisting of H, OH, $C_{1-6}$ alkoxy, $NO_2$, $C_{1-6}$-alkoxy $C_{1-6}$ alkoxy, m-halogeno, $C_{1-6}$ alkyl $C_{3-6}$ alkandioyl, $NR_{10}R_{11}$($C_{1-6}$ alkoxy)-, allyloxy, phenoxy, $C_{1-6}$ alkoxycarbonyl $C_{1-6}$ alkoxy and -C(O)$R_{12}$.

**8.** A compound of claim 1, 2, 3, 4, 5 or 6 wherein $R_4$ is phenyl, $R_7$-substituted phenyl or indanyl.

**9.** A compound of claim 8 wherein $R_7$ is selected from the group consisting of $C_{1-6}$ alkyl, $C_{1-6}$ alkoxy, halogeno, -$OCF_3$, $C_{1-6}$ alkylthio, -$NR_{10}R_{11}$, -CN, OH, and -$COR_{12}$.

**10.** Use of a pharmaceutical composition for the manufacture of a medicament for lowering serum cholesterol, which composition comprises a compound having the structure

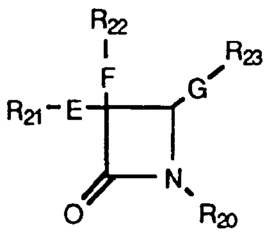

wherein

$R_{20}$ is phenyl, W-substituted phenyl, naphthyl, W-substituted naphthyl, benzodioxolyl, heteroaryl, W-substituted heteroaryl, benzofused heteroaryl and W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof;
$R_{21}$, $R_{22}$ and $R_{23}$ are independently selected from H or $R_{20}$;
W is 1 to 3 substituents independently selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, -$CF_3$, -$OCF_3$, benzyl, $R_{14}$ benzyl, benzyloxy, $R_{14}$-benyloxy, phenoxy, $R_{14}$-phenoxy, dioxolanyl, $NO_2$, -$NR_{10}R_{11}$, $NR_{10}R_{11}$ ($C_{1-6}$ alkyl)-, $NR_{10}R_{11}$ ($C_{1-6}$ alkoxy)-, OH, halogeno, -NHC(O)$OR_5$, -NHC(O)$R_5$, $R_6O_2$SNH-, $(R_6O_2S)_2$N-, -$S(O)_2NH_2$, -$S(O)_{0-2}R_{10}$, tert-butyldimethylsilyloxymethyl, -C(O)$R_{12}$,

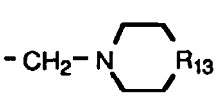

and

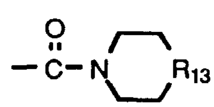

;

E, F and G are independently a bond; $C_3$-$C_6$ cycloalkylene; $C_1$-$C_{10}$ alkylene; $C_2$-$C_{10}$ alkenylene; $C_2$-$C_{10}$ alkynylene; an alkylene, alkenylene or alkynylene chain as defined substituted by one or more substituents independently selected from the group consisting of phenyl, W-substituted phenyl, heteroaryl and W-substituted

heteroaryl, wherein heteroaryl is as defined above; an alkylene, alkenylene or alkynylene chain as defined interrupted by one or more groups independently selected from the group consisting of -O-, -S-, -SO-, $-SO_2-$, $-NR_8$, -C(O)-, $C_3$-$C_6$ cycloalkylene, phenylene, W-substituted phenylene, heteroarylene and W-substituted heteroarylene; or an interrupted alkylene, alkenylene or alkynylene chain as defined substituted by one or more substituents independently selected from the group consisting of phenyl, W-substituted phenyl, heteroaryl and W-substituted heteroaryl; or one of $R_{21}$-E and $R_{22}$-F is selected from the group consisting of halogeno, OH, $C_{1-6}$ alkoxy, $-OC(O)R_5$, $-NR_{10}R_{11}$, -SH or -S($C_{1-6}$ alkyl);

$R_5$ is $C_{1-6}$ alkyl, phenyl, $R_{14}$-phenyl, benzyl or $R_{14}$-benzyl;

$R_6$ is OH, $C_{1-6}$ alkyl, phenyl, benzyl, $R_{14}$-phenyl or $R_{14}$-benzyl;

$R_8$ is H, $C_{1-6}$ alkyl, phenyl $C_{1-6}$ alkyl or $-C(O)R_9$;

$R_9$ is H, $C_{1-6}$ alkyl, phenyl or phenyl $C_{1-6}$ alkyl;

$R_{10}$ and $R_{11}$ are independently selected from H and $C_{1-6}$ alkyl;

$R_{12}$ is H, OH, $C_{1-6}$ alkoxy, phenoxy, benzyloxy,

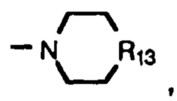

$-NR_{10}R_{11}$, $C_{1-6}$ alkyl, phenyl or $R_{14}$-phenyl;

$R_{13}$ is -O-, $-CH_2-$, -NH- or -N($C_{1-6}$ alkyl)-;

$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;

provided that when G is a bond, $R_{23}$ is not H, and provided that when $R_{23}$ is W-substituted phenyl, W is not p-halogeno;

or a pharmaceutically acceptable salt thereof;

in a pharmaceutically acceptable carrier.

**11.** A compound of claim 1 selected from the group consisting of compounds of the formula

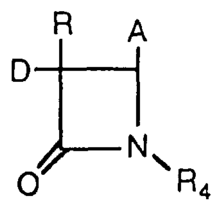

wherein D,R, A and $R_4$ and relative stereochemistry between D and A are as defined in the following table:

| D | R | A | Relative Stereo-chemistry | $R_4$ |
|---|---|---|---|---|
| $C_6H_5-(CH_2)_3-$ | H | $p-CH_3O-C_6H_4-$ | 3S,4S | $p-CH_3O-C_6H_4-$ |
| $C_6H_5-(CH_2)_3-$ | H | $p-NO_2-C_6H_4-$ | 3R,4R | $p-CH_3O-C_6H_4-$ |
| $C_6H_5-(CH_2)_3-$ | H | $p-OH-C_6H_4-$ | 3S,4S | $p-CH_3O-C_6H_4-$ |
| $C_6H_5-(CH_2)_3-$ | H | $3-CH_3O-C_6H_4-$ | 3S,4S | $p-CH_3O-C_6H_4-$ |
| $C_6H_5-(CH_2)_3-$ | H | $p-CH_3O-C_6H_4-$ | trans | $p-CF_3O-C_6H_4-$ |
| $C_6H_5-(CH_2)_3-$ | H | $4-CH_3O-C_6H_4-$ | cis | $p-Cl-C_6H_4-$ |
| $C_6H_5-(CH_2)_3-$ | H | $4-CH_3O-C_6H_4-$ | cis | $p-CH_3-C_6H_4-$ |
| $C_6H_5-(CH_2)_3-$ | H | 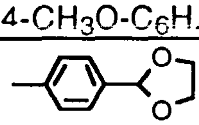 | trans | $p-CH_3O-C_6H_4-$ |
| $C_6H_5-(CH_2)_3-$ | H | $4-((CH_3)_2CH-O)-C_6H_4-$ | cis | $p-CH_3O-C_6H_4-$ |
| $C_6H_5-(CH_2)_3-$ | H | $4-(CH_3(CH)_2-O)-C_6H_4-$ | cis | $p-CH_3O-C_6H_4-$ |
| $C_6H_5-(CH_2)_3-$ | H | 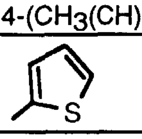 | cis | $p-CH_3O-C_6H_4-$ |
| $C_6H_5-(CH_2)_3-$ | H | $CH_3CH_2-O-C_6H_4-$ | cis | $p-CH_3O-C_6H_4-$ |
| $C_6H_5-(CH_2)_3-$ | H | $CH_3(CH_2)_3-O-C_6H_4-$ | cis | $p-CH_3O-C_6H_4-$ |
| $C_6H_5-(CH_2)_3-$ | H | $4-(C_6H_5-O)-C_6H_4-$ | cis | $p-CH_3O-C_6H_4-$ |
| $C_6H_5-(CH_2)_3-$ | H | $4-(CH_2=CH-CH_2-O)-C_6H_4-$ | cis | $p-CH_3O-C_6H_4-$ |
| $C_6H_5-(CH_2)_3-$ | H | $4-(CH_3CH_2-O)-C_6H_4-$ | cis | $C_6H_5-$ |
| 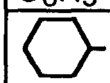 $(CH_2)_3-$ | H | $4-CH_3O-C_6H_4-$ | cis | $4-CH_3O-C_6H_4-$ |
| $4-F-C_6H_4-O-(CH_2)_2-$ | H | $4-CH_3O-C_6H_4-$ | cis | $4-CH_3O-C_6H_4-$ |
| $4-F-C_6H_4-O-(CH_2)_2-$ | H | $4-CH_3O-C_6H_4-$ | cis | $C_6H_5-$ |
| $C_6H_5-(CH_2)_3-$ | H | $4-((CH_3)_2N-(CH_2)_3-O)-C_6H_4-$ | cis | $4-CH_3O-C_6H_4-$ |

| | | | | |
|---|---|---|---|---|
| $C_6H_5-(CH_2)_3-$ | H | $4-CH_3O-C_6H_4-$ | cis | $C_6H_5-$ |
| $C_6H_5-(CH_2)_3-$ | H | $4-CH_3O-C_6H_4-$ | 3S,4S | $p-CH_3O-C_6H_4-$ |
| $C_6H_5-(CH_2)_3-$ | H | $p-CH_3O-C_6H_4-$ | 3S,4R | $p-CH_3O-C_6H_4-$ |
| $C_6H_5-(CH_2)_3-$ | H | $4-CH_3O-C_6H_4-$ | trans | $4-F-C_6H_4-$ |
| $C_6H_5-(CH_2)_3-$ | H | $4-CH_3O-C_6H_4-$ | trans | $3-CH_3O-C_6H_4-$ |
| $C_6H_5-(CH_2)_3-$ | H | $4-CH_3O-C_6H_4-$ | trans | $4-Cl-C_6H_4-$ |
| $C_6H_5-(CH_2)_3-$ | H | $4-CH_3O-C_6H_4-$ | trans | $4-(CH_3CH_2-O)-C_6H_4-$ |
| $C_6H_5-(CH_2)_3-$ | H | $4-CH_3O-C_6H_4-$ | trans | $4-CH_3S-C_6H_4-$ |
| $C_6H_5-(CH_2)_3-$ | H | $4-CH_3O-C_6H_4-$ | trans | $4-CH_3-C_6H_4-$ |
| $C_6H_5-(CH_2)_3-$ | H | $4-CH_3O-C_6H_4-$ | trans | $4-(C_6H_5-O)-C_6H_4-$ |
| 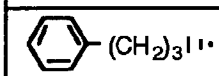 | H | 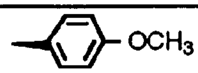 | trans optically pure | $C_6H_5-$ |
| 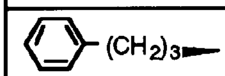 | H | 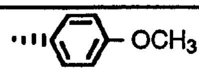 | trans optically pure | $C_6H_5-$ |
| $C_6H_5-(CH_2)_3-$ | H | $4-(CH_3CH_2-O)-C_6H_4-$ | trans | $4-CH_3O-C_6H_4-$ |
| $C_6H_5-(CH_2)_3-$ | H | $4-(C_6H_5-CH_2-O)-C_6H_4-$ | trans | $4-CH_3O-C_6H_4-$ |
| $C_6H_5-(CH_2)_3-$ | H | $4-(CH_2=CH-CH_2-O)-C_6H_4-$ | trans | $4-CH_3O-C_6H_4-$ |
| $C_6H_5-(CH_2)_3-$ | H | $4-(C_6H_5-O)-C_6H_4-$ | trans | $4-CH_3O-C_6H_4-$ |
| $C_6H_5-O-(CH_2)_2-$ | H | $4-CH_3O-C_6H_4-$ | trans | $4-CH_3O-C_6H_4-$ |
| $4-F-C_6H_4-O-(CH_2)_2-$ | H | $4-CH_3O-C_6H_4-$ | trans | $4-CH_3O-C_6H_4-$ |
| $C_6H_5-(CH_2)_4-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $C_6H_5-$ |
| $C_6H_5-(CH_2)_3-$ | H | $4-CH_3O-C_6H_4-$ | trans | $4-(CH_3CH_2OC(O))-C_6H_4-$ |
| $C_6H_5-(CH_2)_3-$ | H | $4-CH_3O-C_6H_4-$ | trans | $3-NO_2-C_6H_4-$ |
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 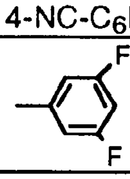 |
| $C_6H_5-(CH_2)_3-$ | H | $4-CH_3O-C_6H_4-$ | trans | $4-(CH_3C(O))-C_6H_4-$ |

| | | | | |
|---|---|---|---|---|
| $C_6H_5-(CH_2)_3-$ | H | $4-CH_3O-C_6H_4-$ | trans | 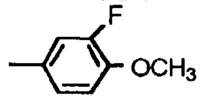 |
| $C_6H_5-(CH_2)_3-$ | H | $4-CH_3O-C_6H_4-$ | trans | 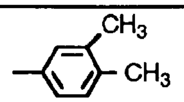 |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 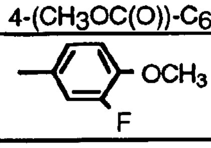 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | $4-((CH_3)_2N)-C_6H_4-$ | trans | $4-CH_3O-C_6H_4-$ |
| $C_6H_5-(CH_2)_3-$ | H | 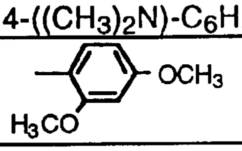 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $4-O_2N-C_6H_4-(CH_2)_3-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $4-I-C_6H_4-$ |
| $C_6H_5-(CH_2)_3-$ | H | $4-(CH_3(CH_2)_3-O)-C_6H_4-$ | trans | $4-CH_3O-C_6H_4-$ |
| 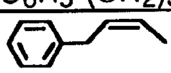 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 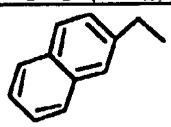 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 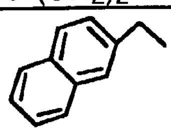 | H | $4-CH_3O-C_6H_4-$ | cis | $C_6H_5-$ |
| $C_6H_5-(CH_2)_3-$ | H | $4-CH_3O-C_6H_4-$ | trans | 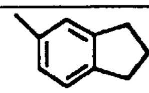 |
| $C_6H_5-(CH_2)_3-$ | H | $4-NH_2-C_6H_4-$ | cis | $4-CH_3O-C_6H_4-$ |
| $C_6H_5-(CH_2)_3-$ | H | $4-CH_3O-C_6H_4-$ | trans | $3-NH_2-C_6H_4-$ |
| $C_6H_5-(CH_2)_3-$ | H | $4-OH-C_6H_4-$ | trans | $4-CH_3O-C_6H_4-$ |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-CH_3O-C_6H_4-$ | 3R,4S | $4-OH-C_6H_4-$ |

| | | | | |
|---|---|---|---|---|
| $C_6H_5-(CH_2)_3-$ | H | 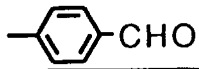 | trans | $4-CH_3O-C_6H_4-$ |
| $C_6H_5-(CH_2)_3-$ | H | $4-CH_3O-C_6H_4-$ | trans | $4-(CH_3SO_2)-C_6H_4-$ |
| $C_6H_5-(CH_2)_3-$ | H | 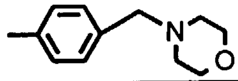 | trans | $4-CH_3O-C_6H_4-$ |
| $C_6H_5-(CH_2)_3-$ | H | 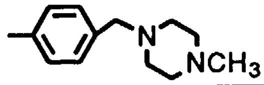 | trans | $4-CH_3O-C_6H_4-$ |
| $C_6H_5-(CH_2)_3-$ | H | $4-(CH_3OCH_2)-C_6H_4-$ | trans | $4-CH_3O-C_6H_4-$ |
| $C_6H_5-(CH_2)_3-$ | H | 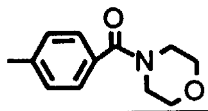 | cis | $4-CH_3O-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 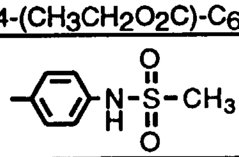 | trans | $4-CH_3O-C_6H_4-$ |
| $C_6H_5-(CH_2)_3-$ | H | 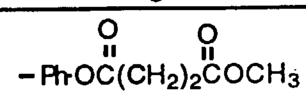 Wherein Ph is 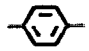 | trans | $4-CH_3O-C_6H_4-$ |
| $C_6H_5-(CH_2)_3-$ | H | $-Ph-OCH_2CO_2Et$ Wherein Ph is 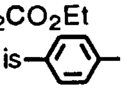 and Et is $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 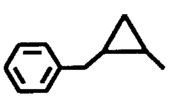 | H | $4-CH_3O-C_6H_4-$ | cis | $4-CH_3O-C_6H_4-$ |

**12.** A compound of claim 1, or 11 which is (3R,4S)-1,4-bis-(4-methoxyphenyl)-3-(3-phenylpropyl)-2-azetidinone.

**13.** Use of claim 10 wherein the compound is selected from the group consisting of compounds of the formula

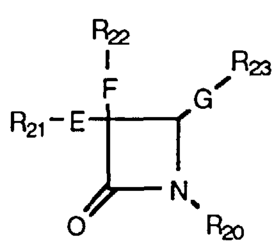

wherein $R_{21}$-E-, $R_{22}$-F-, $R_{23}$-G-, $R_{20}$ and relative stereochemistry between $R_{21}$-E- and $R_{23}$-G- are as defined in

the following table:

| $R_{21}$-E- | $R_{22}$-F- | $R_{23}$-G- | Rel. Stereo-chem. | $R_{20}$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| 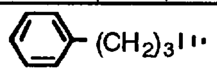 | H | 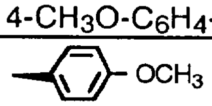 | trans optically pure | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |

| | | | | |
|---|---|---|---|---|
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 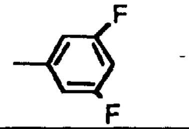 |
| $C_6H_5-(CH_2)_3-$ | H | $4-CH_3O-C_6H_4-$ | trans | $4-(CH_3C(O))-C_6H_4-$ |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 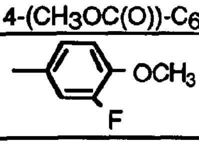 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | 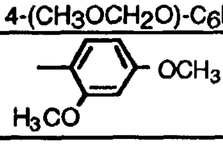 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $C_6H_5-(CH_2)_3-$ | H | $4-CH_3O-C_6H_4-$ | trans | $4-I-C_6H_4-$ |
| 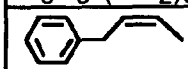 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 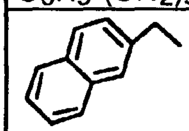 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 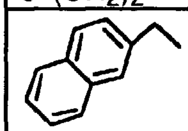 | H | $4-CH_3O-C_6H_4-$ | cis | $C_6H_5-$ |
| $C_6H_5-(CH_2)_3-$ | H | $4-CH_3O-C_6H_4-$ | trans | 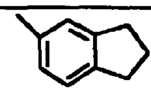 |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 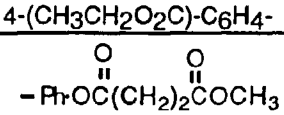 Wherein Ph is 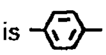 | trans | $4-CH_3O-C_6H_4-$ |

| | | | | |
|---|---|---|---|---|
| $C_6H_5-(CH_2)_3-$ | H | $-Ph-OCH_2CO_2Et$ Wherein Ph is 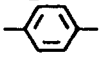 and Et is $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 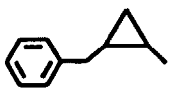 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 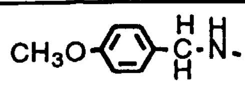 | H | $4-CH_3O-C_6H_4-$ | cis | $C_6H_5-$ |

**14.** The use of a compound of claim 1 for the manufacture of a medicament for lowering serum cholesterol.

**15.** The use of a compound of claim 1 for the manufacture of a medicament for inhibiting the enzyme acyl CoA:cholesterol acyl transferase.

**16.** A process for the preparation of a pharmaceutical composition as defined in claim 10 which comprises admixing a compound as defined in claim 10 with a pharmaceutically acceptable carrier.

**17.** Use of a composition as defined in claim 10 for the manufacture of a medicament for inhibiting the enzyme acyl CoA:cholesterol acyl transferase.

**18.** A process for producing a compound of claim 1, wherein R is H, and D and A have trans relative stereochemistry, comprising cyclizing a hydroxyamide of the formula

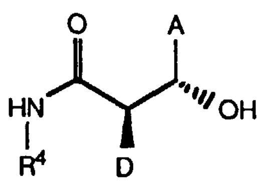 or 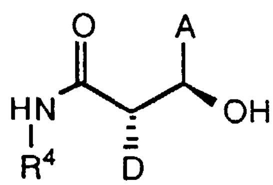

wherein D, A and $R^4$ are as defined in claim 1, by treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride.

**19.** A process for producing a compound of claim 1 having the formula

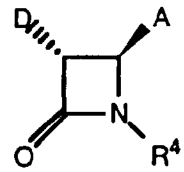
or
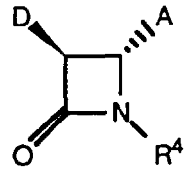

wherein D, A and $R^4$ are as defined in claim 1, comprising:

(a) reacting a carboxylic acid of the formula $DCH_2$. COOH, wherein D is as defined above, with a chlorinating agent;
(b) deprotonating a chiral oxazolidinone of the formula

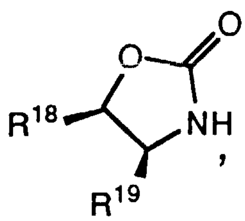
,
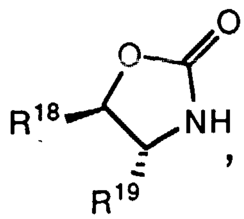
,
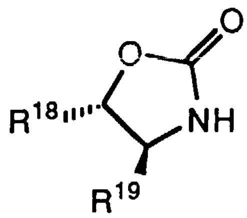
or
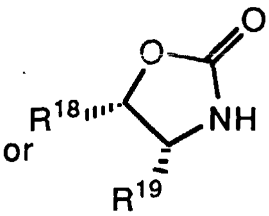

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; with a strong base and treating the resulting anion with the product of step (a);
(c) enolizing the product of step (b) with either:

(i) a dialkylboron triflate and a tertiary amine base;
(ii) $TiCl_4$ and tetramethylethylenediamine (TMEDA) or a mixture of TMEDA and triethylamine;

then condensing with an aldehyde of the formula A-CHO, wherein A is as defined above;
(d) hydrolyzing the product of step (c) with a base and hydrogen peroxide;
(e) condensing the product of step (d) with an amine of the formula $R^4NH_2$, wherein $R^4$ is as defined above, by treating with a dehydrative coupling agent, optionally adding an activating agent; and
(f) cyclizing the product of step (e) by treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride.

**20.** A process for producing a compound of claim 1, wherein R is hydrogen, and D and A have trans relative stereochemistry, comprising:

(a) enolizing a compound of the formula

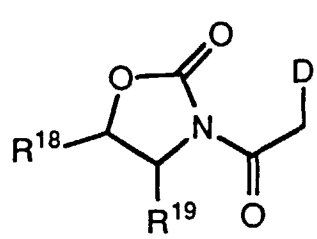

with $TiCl_4$ and tetramethylethylenediamine (TMEDA), then condensing with an imine of the formula A-CH=N-$R^4$, wherein A and $R^4$ are as defined above, and wherein the group

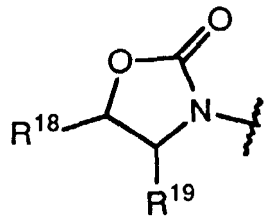

represents an chiral auxiliary of the formula

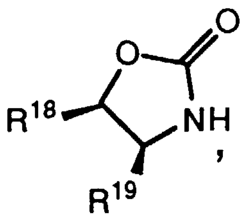
,
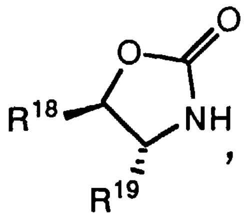
,
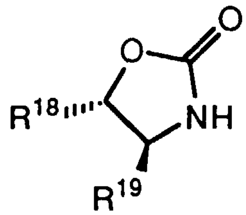
or
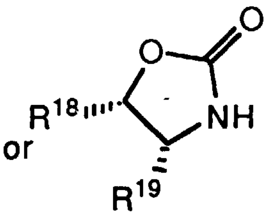

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; and
(b) cyclizing the product of step (a) by treating with a strong non-nucleophilic base.

**21.** A process for preparing compounds of claim 1 comprising:

(a) treating an ester of the formula D-CH(R)-C(O)O$R^{17}$, wherein $R^{17}$ is $C_{1-6}$ alkyl, menthyl, or 10-(siisopropylsulfonamido)isobornyi, with a strong base, and then with an imine of the formula A-CH=N-$R^4$, wherein A and $R^4$ are as defined in claim 1; or
(b) for preparing a compound of claim 1 wherein R is not hydrogen, reacting a compound of claim 1, wherein R is hydrogen, with a strong base and an alkylating agent or an acylating agent; or
(c) for preparing a compound of claim 1, wherein R is hydrogen, and D and A have cis relative stereochemistry, reacting a compound of claim 1, wherein R is hydrogen, and D and A have trans relative stereochemistry, with a strong base at -80° to -40°C, and then with a proton source; or
(d) for preparing a compound of claim 1, wherein R is hydrogen, and D and A have trans relative stereochemistry, reacting a compound of claim 1, wherein R is hydrogen, and D and A have cis relative stereochemistry, with an alkali metal t-butoxide, then quenching with acid; or
(e) reacting a carboxylic acid derivative of the formula D-CH(R)-C(O)-Z, wherein D and R are as defined in claim 1, and Z is Cl,

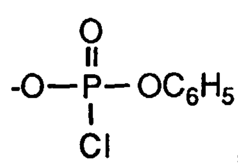
, or
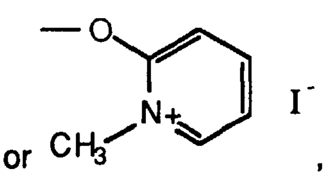
,

with an imine of the formula A-CH=N-$R^4$, wherein A and $R^4$ are as defined in claim 1, in the presence of a tertiary amine base.

**22.** A process for preparing a compound of formula II

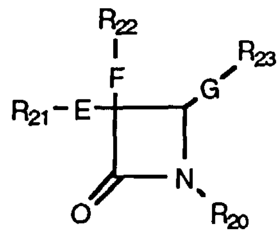

II

wherein

$R_{20}$ is phenyl, W-substituted phenyl, naphthyl, W-substituted naphthyl, benzodioxolyl, heteroaryl, W-substituted heteroaryl, benzofused heteroaryl and W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof;

$R_{21}$, $R_{22}$ and $R_{23}$ are independently selected from H or $R_{20}$;

W is 1 to 3 substituents independently selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $-NR_{10}R_{11}$, $NR_{10}R_{11}$($C_{1-6}$ alkyl)-, $NR_{10}R_{11}$($C_{1-6}$ alkoxy)-, OH, halogeno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldimethyl-silyloxymethyl, $-C(O)R_{12}$,

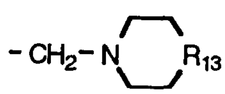
and
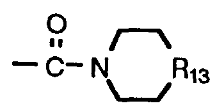
;

E, F and G are independently a bond; $C_3$-$C_6$ cycloalkylene; $C_1$-$C_{10}$ alkylene; $C_2$-$C_{10}$ alkenylene; $C_2$-$C_{10}$ alkynylene; an alkylene, alkenylene or alkynylene chain as defined substituted by one or more substituents independently selected from the group consisting of phenyl, W-substituted phenyl, heteroaryl and W-substituted heteroaryl, wherein heteroaryl is as defined above; an alkylene, alkenylene or alkynylene chain as defined interrupted by one or more groups independently selected from the group consisting of -O-, -S-, -SO-, $-SO_2-$, $-NR_8$, -C(O)-, $C_3$-$C_6$ cycloalkylene, phenylene, W-substituted phenylene, heteroarylene and W-substituted heteroarylene; or an interrupted alkylene, alkenylene or alkynylene chain as defined substituted by one or more substituents independently selected from the group consisting of phenyl, W-substituted phenyl, heteroaryl and W-substituted heteroaryl; or one of $R_{21}$-E and $R_{22}$-F is selected from the group consisting of halogeno, OH, lower alkoxy, $-OC(O)R_5$, $-NR_{10}R_{11}$, -SH or -S($C_{1-6}$ alkyl);

$R_5$ is $C_{1-6}$ alkyl, phenyl, $R_{14}$-phenyl, benzyl or $R_{14}$-benzyl;

$R_6$ is OH, $C_{1-6}$ alkyl, phenyl, benzyl, $R_{14}$-phenyl or $R_{14}$-benzyl;

$R_8$ is H, $C_{1-6}$ alkyl, phenyl $C_{1-6}$ alkyl or $-C(O)R_9$;

$R_9$ is H, $C_{1-6}$ alkyl, phenyl or phenyl $C_{1-6}$ alkyl;

$R_{10}$ and $R_{11}$ are independently selected from H and $C_{1-6}$ alkyl;

$R_{12}$ is H, OH, $C_{1-6}$ alkoxy, phenoxy, benzyloxy,

, 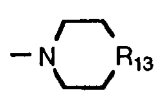 ,

$-NR_{10}R_{11}$, $C_{1-6}$ alkyl, phenyl or $R_{14}$-phenyl;

$R_{13}$ is -O-, $-CH_2-$, -NH- or -N($C_{1-6}$ alkyl)-;

$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;

provided that when G is a bond, $R_{23}$ is not H, and provided that when $R_{23}$ is W-substituted phenyl, W is not

p-halogeno;
or a pharmaceutically acceptable salt thereof;
wherein $R_{22}$-F is H, and $R_{21}$-E- and $R_{23}$-G- have trans relative stereochemistry, comprising
Process C: cyclizing a hydroxyamide of the formula

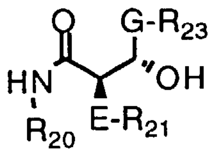 or 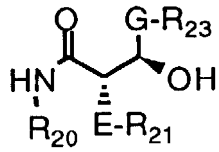

wherein $R_{21}$-E-, $R_{23}$-G- and $R_{20}$ are as defined above, by treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride; or

Process D:

(a) reacting a carboxylic acid of the formula $R_{21}$-E-$CH_2$-COOH, wherein $R_{21}$-E- is as defined above, with a chlorinating agent;
(b) deprotonating a chiral oxazolidinone of the formula

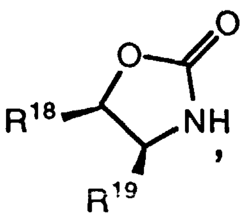, 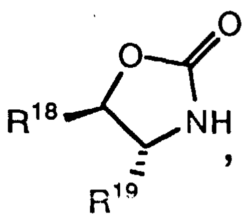, 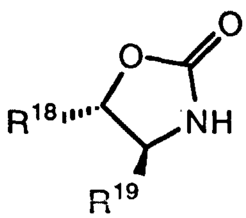 or 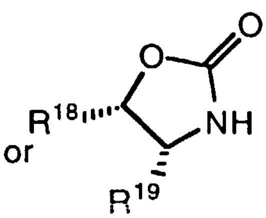

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; with a strong base and treating the resulting anion with the product of step (a);
(c) enolizing the product of step (b) with either:

(i) a dialkylboron triflate and a tertiary amine base;
(ii) $TiCl_4$ and tetramethylethylenediamine (TMEDA) or a mixture of TMEDA and triethylamine;

then condensing with an aldehyde of the formula $R_{23}$-G-CHO, wherein $R_{23}$-G- is as defined above;
(d) hydrolyzing the product of step (c) with a base and hydrogen peroxide;
(e) condensing the product of step (d) with an amine of the formula $R_{20}NH_2$, wherein $R_{20}$ is as defined above, by treating with a dehydrative coupling agent, optionally adding an activating agent; and
(f) cyclizing the product of step (e) as described in Process C.

**23.** A process for producing a compound of formula II as defined in claim 22, wherein $R_{22}$-F- is hydrogen, and $R_{21}$-E- and $R_{23}$-G- have trans relative stereochemistry, comprising:

(a) enolizing a compound of the formula

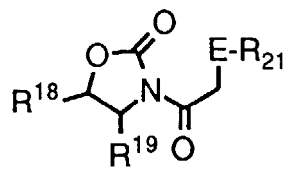

with $TiCl_4$ and tetramethylethylenediamine (TMEDA), then condensing with an imine of the formula $R_{23}$-G-CH=N-$R_{20}$, wherein $R_{23}$-G- and $R_{20}$ are as defined in claim 22, and wherein the group

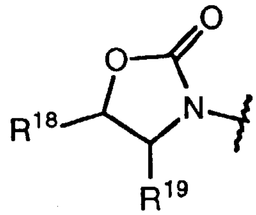

represents an chiral auxiliary of the formula

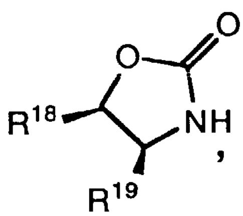 , 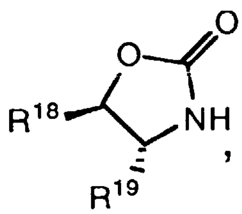 , 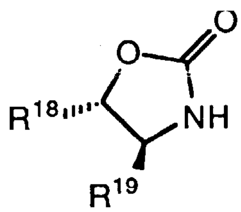 or 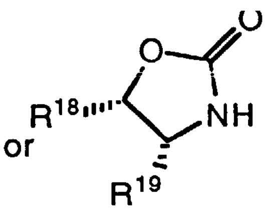

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; and

(b) cyclizing the product of step (a) by treating with a strong non-nucleophilic base.

**24.** A process for preparing a compound of formula II as defined in claim 22, comprising

(a) treating an ester of the formula $R_{21}$-E-CH(F-$R_{22}$)-C(O)$OR_{17}$, wherein $R^{17}$ is $C_{1-6}$ alkyl, menthyl, or 10-(diisopropylsulfonamido)isobornyl, with a strong base, and then with an imine of the formula $R_{23}$-G-CH=N-$R_{20}$, wherein $R_{23}$-G- and $R_{20}$ are as defined in claim 22, or

(b) for preparing a compound of formula II as defined in claim 22, wherein $R_{22}$-F- is not hydrogen, reacting a compound of formula II as defined in claim 2, wherein $R_{22}$-F- is hydrogen, with a strong base and an alkylating agent or an acylating agent; or

(c) for preparing a compound of formula II as defined in claim 22, wherein $R_{22}$-F- is hydrogen, and $R_{21}$-E- and $R_{23}$-G- have cis relative stereochemistry, reacting a compound of formula II as defined in claim 22, wherein $R_{22}$-F- is hydrogen, and $R_{21}$-E- and $R_{23}$-G- have trans relative stereo-chemistry, with a strong base at -80° to -40°C, and then with a proton source; or

(d) for preparing a compound of formula II as defined in claim 22, wherein $R_{22}$-F- is hydrogen, and $R_{21}$-E- and $R_{23}$-G- have trans relative stereochemistry, reacting a compound of formula II as defined in claim 22, wherein $R_{22}$-F- is hydrogen, and $R_{21}$-E- and $R_{23}$-G- have cis relative stereochemistry, with an alkali metal t-butoxide, then quenching with acid; or

(e) reacting a carboxylic acid derivative of the formula $R_{21}$-E-CH(F-$R_{22}$)-C(O)-Z, wherein $R_{21}$-E- and $R_{22}$-F- are as defined in claim 22, and Z is Cl,

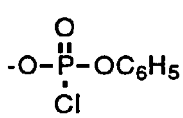 , or 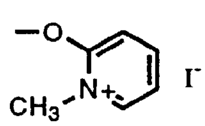 ,

with an imine of the formula $R_{23}$-G-CH=N-$R_{20}$, wherein $R_{23}$-G- and $R_{20}$ are as defined in claim 22, in the presence of a tertiary amine base.

**Claims for the following Contracting State : GR**

**1.** A compound having the structural formula

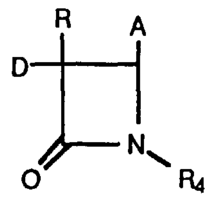

wherein

A is

-CH=CH-B;
-C≡C-B;
-$(CH_2)_p$-X-B, wherein p is 0, 1 or 2 and X is a bond, -NH- or -$S(O)_{0\text{-}2}$-;
heteroaryl, benzofused heteroaryl, W-substituted heteroaryl or W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, and wherein W is 1-3 substituents on the ring carbon atoms selected from the group consisting of $C_{1\text{-}6}$ alkyl, hydroxy $C_{1\text{-}6}$ alkyl, $C_{1\text{-}6}$ alkoxy, $C_{1\text{-}6}$ alkoxy-$C_{1\text{-}6}$ alkyl, $C_{1\text{-}6}$ alkoxy-$C_{1\text{-}6}$ alkoxy, $C_{1\text{-}6}$ alkoxycarbonyl-$C_{1\text{-}6}$ alkoxy, ($C_{1\text{-}6}$ alkoxyimino)-$C_{1\text{-}6}$ alkyl, $C_{3\text{-}6}$ alkanedioyl, $C_{1\text{-}6}$ alkyl-$C_{3\text{-}6}$ alkanedioyl, allyloxy, -$CF_3$, -$OCF_3$, benzyl, $R_{14}$ benzyl, benyloxy, $R_{14}$-benyloxy, phenoxy, $R_{14}$-phenoxy, dioxolanyl, $NO_2$, -$NR_{10}R_{11}$, $NR_{10}R_{11}$ ($C_{1\text{-}6}$ alkyl)-, $NR_{10}R_{11}$ ($C_{1\text{-}6}$ alkoxy)-, OH, halogeno, -NHC(O)$OR_5$, -NHC(O)$R_5$, $R_6O_2SNH$-, $(R_6O_2S)_2N$-, -$S(O)_2NH_2$, -$S(O)_{0\text{-}2}R_{10}$, tert-butyldi methylsilyloxymethyl, -C(O)$R_{12}$ and

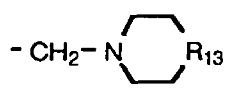,

and wherein the substituents on the substituted heteroaryl ring nitrogen atoms, when present, are selected from the group consisting of $C_{1\text{-}6}$ alkyl, $C_{1\text{-}6}$ alkoxy, -C(O)$OR_5$, -C(O)$R_5$, OH, $NR_{10}R_{11}$ (lower alkyl)-, $NR_{10}R_{11}$ ($C_{1\text{-}6}$ alkoxy)-, -$S(O)_2NH_2$ and 2-(trimethylsilyl)ethoxymethyl;
-C(O)-B; or

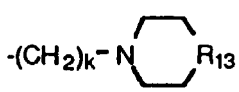,

wherein k is 1 or 2;

D is

B'-$(CH_2)_m$C(O)-, wherein m is 1, 2, 3, 4 or 5;
B'-$(CH_2)_q$-, wherein q is 2, 3, 4, 5 or 6;
B'-$(CH_2)_e$-Z-$(CH_2)_r$-, wherein Z is -O-, -C(O)-, phenylene, -$NR_8$- or -$S(O)_{0\text{-}2}$-, e is 0, 1, 2, 3, 4 or 5 and r is

1, 2, 3, 4 or 5, provided that the sum of e and r is 1,2,3,4,5 or 6;
B'-($C_{2}$-$C_6$ alkenylene)-; B'-($C_4$-$C_6$ alkadienylene)-;
B'-$(CH_2)_t$-Z-($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6;
B'$(CH_2)_f$-V-$(CH_2)_g$-, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6;
B'-$(CH_2)_t$-V-($C_2$-$C_6$ alkenylene)- or B'-($C_2$-$C_6$ alkenylene)-V-$(CH_2)_t$-, wherein V and t are as defined above, provided that the sum of t and the number of carbon atoms in the alkenylene chain is 2, 3, 4, 5 or 6;
B'-$(CH_2)_a$-Z-$(CH_2)_b$-V-$(CH_2)_d$-, wherein Z and V are as defined above and a ,b and d are independently 0, 1, 2, 3, 4, 5 or 6, provided that the sum of a, b and d is 0, 1, 2, 3, 4, 5 or 6;
T-$(CH_2)_s$-, wherein T is cycloalkyl of 3-6 carbon atoms and s is 1, 2, 3, 4, 5 or 6; or
naphthylmethyl, heteroarylmethyl, or W-substituted heteroarylmethyl, wherein heteroaryl and W are as defined above;

B is

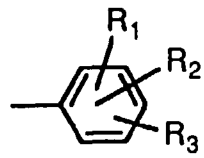

B' is naphthyl, heteroaryl or W-substituted heteroaryl, wherein heteroaryl is as defined above, or

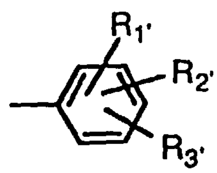

R is hydrogen, fluoro, $C_1$-$C_{15}$ alkyl, $C_2$-$C_{15}$ alkenyl, $C_2$-$C_{15}$ alkynyl, or B-$(CH_2)_h$ -, wherein h is 0, 1, 2, or 3;
$R_1$, $R_2$ and $R_3$ are independently selected from the group consisting of H, $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, -$CF_3$, -$OCF_3$, benzyl, $R_{14}$ benzyl, benzyloxy, $R_{14}$-benyloxy, phenoxy, $R_{14}$-phenoxy, dioxolanyl, $NO_2$, -$NR_{10}R_{11}$, $NR_{10}R_{11}$ ($C_{1-6}$ alkyl)-, $NR_{10}R_{11}$ ($C_{1-6}$ alkoxy)-, OH, o-halogeno, m-halogeno, -NHC(O)$OR_5$, -NHC(O)$R_5$, $R_6O_2SNH$-, $(R_6O_2S)_2N$-, -$S(O)_2NH_2$, -S$(O)_{0-2}R_{10}$, tert-butyldimethylsilyloxymethyl, -C(O)$R_{12}$,

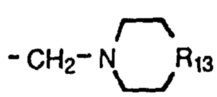

and

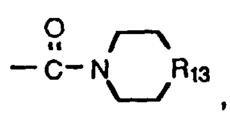

or $R_1$ is hydrogen and $R_2$ and $R_3$, together with adjacent carbon atoms to which they are attached, form a dioxolanyl ring;
$R_{1'}$, $R_{2'}$ and $R_{3'}$ are independently selected. from the group consisting of H, $C_{1-6}$ alkyl, hydroxy $C_{1-6}$ alkyl, $C_{1-6}$

alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, $-CF_3$, $-OCF_3$, benzyl, $R_{14}$-benzyl, benzyloxy, $R_{14}$-benyloxy, phenoxy, $R_{14}$-Phenoxy, dioxolanyl, $NO_2$, $-NR_{10}R_{11}$, $NR_{10}R_{11}$ ($C_{1-6}$ alkyl)-, $NR_{10}R_{11}$ ($C_{1-6}$ alkoxy)-, OH, halogeno, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-butyldimethylsilyloxymethyl, $-C(O)R_{12}$,

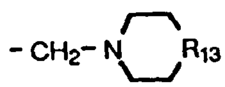 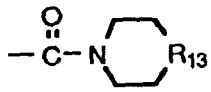 ,

or $R_{1'}$ is hydrogen and $R_{2'}$ and $R_{3'}$, together with adjacent carbon atoms to which they are attached, form a dioxolanyl ring;

$R_4$ is

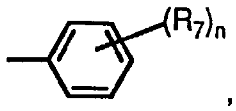 ,

wherein n is 0, 1, 2 or 3, indanyl, benzofuranyl, benzodioxolyl, tetrahydronaphthyl, pyridyl, pyrazinyl, pyrimidinyl or quinolyl;

$R_5$ is $C_{1-6}$ alkyl, phenyl, $R_{14}$-Phenyl, benzyl or $R_{14}$-benzyl;

$R_6$ is OH, $C_{1-6}$ alkyl, phenyl, benzyl, $R_{14}$-Phenyl or $R_{14}$-benzyl;

$R_7$ is $C_{1-6}$ alkyl, $C_{1-6}$ alkoxy, OH, halogeno, $-NR_{10}R_{11}$, $-NHC(O)OR_5$, $-NHC(O)R_5$, $NO_2$, -CN, $-N_3$, -SH, $-S(O)_{0-2}$-($C_{1-6}$ alkyl), $-COOR_9$, $-CONR_{10}R_{11}$, $-COR_{12}$, phenoxy, benzyloxy, $-OCF_3$, or tert-butyldimethylsilyloxy, and where n is 2 or 3, the $R_7$ groups can be the same or different;

$R_8$ is H, $C_{1-6}$ alkyl, phenyl $C_{1-6}$ alkyl, or $-C(O)R_9$;

$R_9$ is H, $C_{1-6}$ alkyl, phenyl or phenyl $C_{1-6}$ alkyl;

$R_{10}$ and $R_{11}$ are independently selected from H and $C_{1-6}$ alkyl;

$R_{12}$ is H, OH, $C_{1-6}$ alkoxy, phenoxy, benzyloxy,

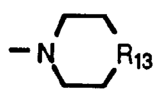 ,

$-NR_{10}R_{11}$, $C_{1-6}$ alkyl, phenyl or $R_{14}$-phenyl;

$R_{13}$ is -O-, $-CH_2$-, -NH- or -N($C_{1-6}$ alkyl)-; and

$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;

or a pharmaceutically acceptable salt thereof.

2. A compound of claim 1 wherein R is hydrogen.

3. A compound of claim 1 or 2 wherein D is B'-$(CH_2)_q$-, B'-$(CH_2)_e$-Z-$(CH_2)_r$-, B'-($C_2$-$C_6$ alkenylene)- or B'-$(CH_2)_f$-V-$(CH_2)_g$-, wherein B', Z, V, q, e, r, f, and g are as defined in claim 1.

4. A compound of claim 3 wherein D is B'-$(CH_2)_q$- wherein B' is phenyl and q is 3 or 4; B'-$(CH_2)_e$-Z-$(CH_2)_r$- wherein B' is p-fluorophenyl or p-methoxyphenyl, e is zero, Z is -O-, and r is 2; B'-($C_2$-$C_6$ alkenylene)- is 3-phenyl-1-propenyl; or B'-$(CH_2)_f$-V-$(CH_2)$g- wherein B' is phenyl, f is 1, V is cyclopropylene, and g is zero.

5. A compound of claim 1, 2, 3 or 4 wherein A is -$(CH_2)_p$-X-B wherein wherein X, B and p are as defined in claim 1.

6. A compound of claim 5 wherein p is zero and X is a bond.

7. A compound of claim 6 wherein $R_1$, $R_2$ and $R_3$ are selected from the group consisting of H, OH, $C_{1-6}$ alkoxy, $NO_2$, $C_{1-6}$-alkoxy $C_{1-6}$ alkoxy, m-halogeno, $C_{1-6}$ alkyl $C_{3-6}$ alkandioyl, $NR_{10}R_{11}$ ($C_{1-6}$ alkoxy)-, allyloxy, phenoxy, $C_{1-6}$ alkoxycarbonyl $C_{1-6}$ alkoxy and -C(O)$R_{12}$.

8. A compound of claim 1, 2, 3, 4, 5 or 6 wherein $R_4$ is phenyl, $R_7$-substituted phenyl or indanyl.

9. A compound of claim 8 wherein $R_7$ is selected from the group consisting of $C_{1-6}$ alkyl, $C_{1-6}$ alkoxy, halogeno, -$OCF_3$, $C_{1-6}$ alkylthio, -$NR_{10}R_{11}$, -CN, OH, and -$COR_{12}$.

10. A process for preparing a pharmaceutical composition for use in the manufacture of a medicament for lowering serum cholesterol, which process comprises admixing a pharmaceutical acceptable carrier with a compound having the structure

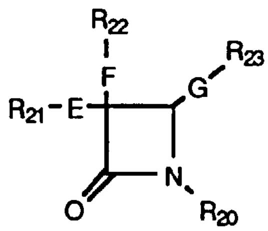

wherein

$R_{20}$ is phenyl, W-substituted phenyl, naphthyl, W-substituted naphthyl, benzodioxolyl, heteroaryl, W-substituted heteroaryl, benzofused heteroaryl and W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroarvls. the N-oxides thereof:
$R_{21}$, $R_{22}$ and $R_{23}$ are independently selected from H or $R_{20}$;
W is 1 to 3 substituents independently selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{1-6}$ alkanedioyl, allyloxy, -$CF_3$, -$OCF_3$, benzyl, $R_{14}$ benzyl, benzyloxy, $R_{14}$-benyloxy, phenoxy, $R_{14}$-phenoxy, dioxolanyl, $NO_2$, -$NR_{10}R_{11}$, $NR_{10}R_{11}$ ($C_{1-6}$ alkyl)-, $NR_{10}R_{11}$ ($C_{1-6}$ alkoxy)-, OH, halogeno, -NHC(O)$OR_5$, -NHC(O)$R_5$, $R_6O_2SNH$-, $(R_6O_2S)_2N$-, -$S(O)_2NH_2$, -$S(O)_{0-2}R_{10}$, tert-butyldimethylsilyloxymethyl, -C(O)$R_{12}$,

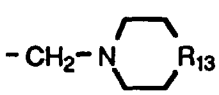 and 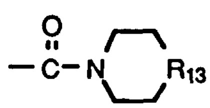 ;

E, F and G are independently a bond; $C_3$-$C_6$ cycloalkylene; $C_1$-$C_{10}$ alkylene; $C_2$-$C_{10}$ alkenylene; $C_2$-$C_{10}$ alkynylene; an alkylene, alkenylene or alkynylene chain as defined substituted by one or more substituents independently selected from the group consisting of phenyl, W-substituted phenyl, heteroaryl and W-substituted heteroaryl, wherein heteroaryl is as defined above; an alkylene, alkenylene or alkynylene chain as defined interrupted by one or more groups independently selected from the group consisting of -O-, -S-, -SO-, -$SO_2$-, -$NR_8$, -C(O)-, $C_3$-$C_6$ cycloalkylene, phenylene, W-substituted phenylene, heteroarylene and W-substituted heteroarylene; or an interrupted alkylene, alkenylene or alkynylene chain as defined substituted by one or more substituents independently selected from the group consisting of phenyl, W-substituted phenyl, heteroaryl and W-substituted heteroaryl; or one of $R_{21}$-E and $R_{22}$-F is selected from the group consisting of halogeno, OH, $C_{1-6}$alkoxy, -OC(O)$R_5$, -$NR_{10}R_{11}$, -SH or -S($C_{1-6}$ alkyl);
$R_5$ is $C_{1-6}$ alkyl, phenyl, $R_{14}$-phenyl, benzyl or $R_{14}$-benzyl;
$R_6$ is OH, $C_{1-6}$alkyl, phenyl, benzyl, $R_{14}$-Phenyl or $R_{14}$-benzyl;
$R_8$ is H, $C_{1-6}$ alkyl, phenyl $C_{1-6}$ alkyl or -C(O)$R_9$;
$R_9$ is H, $C_{1-6}$ alkyl, phenyl or phenyl $C_{1-6}$ alkyl;
$R_{10}$ and $R_{11}$ are independently selected from H and $C_{1-6}$ alkyl;
$R_{12}$ is H, OH, $C_{1-6}$ alkoxy, phenoxy, benzyloxy,

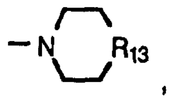

$-NR_{10}R_{11}$, $C_{1-6}$ alkyl, phenyl or $R_{14}$-phenyl;
$R_{13}$ is -O-, $-CH_2-$, -NH- or $-N(C_{1-6}$ alkyl)-;
$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;
provided that when G is a bond, $R_{23}$ is not H, and provided that when $R_{23}$ is W-substituted phenyl, W is not p-halogeno;
or a pharmaceutically acceptable salt thereof,

**11.** A compound of claim 1 selected from the group consisting of compounds of the formula

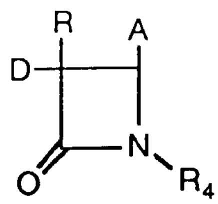

wherein D,R, A and $R_4$ and relative stereochemistry between D and A are as defined in the following table:

| D | R | A | Relative Stereo-chemistry | $R_4$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-$NO_2$-$C_6H_4$- | 3R,4R | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | 3-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 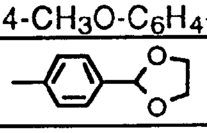 | trans | p-$CH_3O$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | 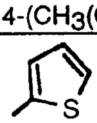 | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($C_6H_5$-O)-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 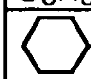 $(CH_2)_3$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-($(CH_3)_2N$-$(CH_2)_3$-O)-$C_6H_4$- | cis | 4-$CH_3O$-$C_6H_4$- |

| | | | | |
|---|---|---|---|---|
| $C_6H_5-(CH_2)_3-$ | H | $4-CH_3O-C_6H_4-$ | cis | $C_6H_5-$ |
| $C_6H_5-(CH_2)_3-$ | H | $4-CH_3O-C_6H_4-$ | 3S,4S | $p-CH_3O-C_6H_4-$ |
| $C_6H_5-(CH_2)_3-$ | H | $p-CH_3O-C_6H_4-$ | 3S,4R | $p-CH_3O-C_6H_4-$ |
| $C_6H_5-(CH_2)_3-$ | H | $4-CH_3O-C_6H_4-$ | trans | $4-F-C_6H_4-$ |
| $C_6H_5-(CH_2)_3-$ | H | $4-CH_3O-C_6H_4-$ | trans | $3-CH_3O-C_6H_4-$ |
| $C_6H_5-(CH_2)_3-$ | H | $4-CH_3O-C_6H_4-$ | trans | $4-Cl-C_6H_4-$ |
| $C_6H_5-(CH_2)_3-$ | H | $4-CH_3O-C_6H_4-$ | trans | $4-(CH_3CH_2-O)-C_6H_4-$ |
| $C_6H_5-(CH_2)_3-$ | H | $4-CH_3O-C_6H_4-$ | trans | $4-CH_3S-C_6H_4-$ |
| $C_6H_5-(CH_2)_3-$ | H | $4-CH_3O-C_6H_4-$ | trans | $4-CH_3-C_6H_4-$ |
| $C_6H_5-(CH_2)_3-$ | H | $4-CH_3O-C_6H_4-$ | trans | $4-(C_6H_5-O)-C_6H_4-$ |
| 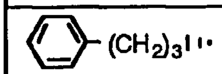 | H | 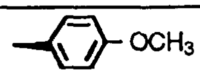 | trans optically pure | $C_6H_5-$ |
| 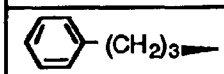 | H | 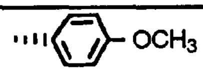 | trans optically pure | $C_6H_5-$ |
| $C_6H_5-(CH_2)_3-$ | H | $4-(CH_3CH_2-O)-C_6H_4-$ | trans | $4-CH_3O-C_6H_4-$ |
| $C_6H_5-(CH_2)_3-$ | H | $4-(C_6H_5-CH_2-O)-C_6H_4-$ | trans | $4-CH_3O-C_6H_4-$ |
| $C_6H_5-(CH_2)_3-$ | H | $4-(CH_2=CH-CH_2-O)-C_6H_4-$ | trans | $4-CH_3O-C_6H_4-$ |
| $C_6H_5-(CH_2)_3-$ | H | $4-(C_6H_5-O)-C_6H_4-$ | trans | $4-CH_3O-C_6H_4-$ |
| $C_6H_5-O-(CH_2)_2-$ | H | $4-CH_3O-C_6H_4-$ | trans | $4-CH_3O-C_6H_4-$ |
| $4-F-C_6H_4-O-(CH_2)_2-$ | H | $4-CH_3O-C_6H_4-$ | trans | $4-CH_3O-C_6H_4-$ |
| $C_6H_5-(CH_2)_4-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $C_6H_5-$ |
| $C_6H_5-(CH_2)_3-$ | H | $4-CH_3O-C_6H_4-$ | trans | $4-(CH_3CH_2OC(O))-C_6H_4-$ |
| $C_6H_5-(CH_2)_3-$ | H | $4-CH_3O-C_6H_4-$ | trans | $3-NO_2-C_6H_4-$ |
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 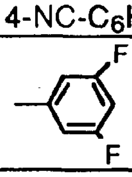 |
| $C_6H_5-(CH_2)_3-$ | H | $4-CH_3O-C_6H_4-$ | trans | $4-(CH_3C(O))-C_6H_4-$ |

| | | | | |
|---|---|---|---|---|
| $C_6H_5-(CH_2)_3-$ | H | $4-CH_3O-C_6H_4-$ | trans | 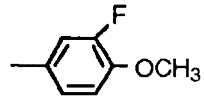 |
| $C_6H_5-(CH_2)_3-$ | H | $4-CH_3O-C_6H_4-$ | trans | 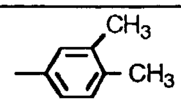 |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 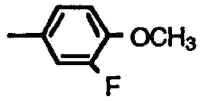 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | $4-((CH_3)_2N)-C_6H_4-$ | trans | $4-CH_3O-C_6H_4-$ |
| $C_6H_5-(CH_2)_3-$ | H | 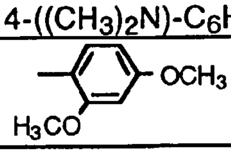 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $4-O_2N-C_6H_4-(CH_2)_3-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $4-I-C_6H_4-$ |
| $C_6H_5-(CH_2)_3-$ | H | $4-(CH_3(CH_2)_3-O)-C_6H_4-$ | trans | $4-CH_3O-C_6H_4-$ |
| 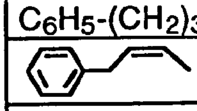 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 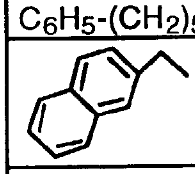 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 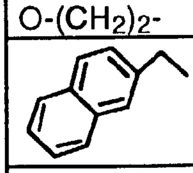 | H | $4-CH_3O-C_6H_4-$ | cis | $C_6H_5-$ |
| $C_6H_5-(CH_2)_3-$ | H | $4-CH_3O-C_6H_4-$ | trans | 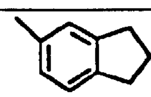 |
| $C_6H_5-(CH_2)_3-$ | H | $4-NH_2-C_6H_4-$ | cis | $4-CH_3O-C_6H_4-$ |
| $C_6H_5-(CH_2)_3-$ | H | $4-CH_3O-C_6H_4-$ | trans | $3-NH_2-C_6H_4-$ |
| $C_6H_5-(CH_2)_3-$ | H | $4-OH-C_6H_4-$ | trans | $4-CH_3O-C_6H_4-$ |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-CH_3O-C_6H_4-$ | 3R,4S | $4-OH-C_6H_4-$ |

| | | | | |
|---|---|---|---|---|
| $C_6H_5-(CH_2)_3-$ | H | 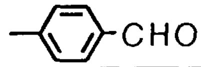 | trans | $4-CH_3O-C_6H_4-$ |
| $C_6H_5-(CH_2)_3-$ | H | $4-CH_3O-C_6H_4-$ | trans | $4-(CH_3SO_2)-C_6H_4-$ |
| $C_6H_5-(CH_2)_3-$ | H | 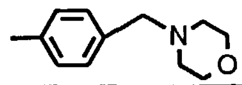 | trans | $4-CH_3O-C_6H_4-$ |
| $C_6H_5-(CH_2)_3-$ | H | 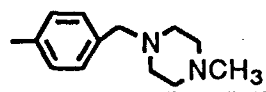 | trans | $4-CH_3O-C_6H_4-$ |
| $C_6H_5-(CH_2)_3-$ | H | $4-(CH_3OCH_2)-C_6H_4-$ | trans | $4-CH_3O-C_6H_4-$ |
| $C_6H_5-(CH_2)_3-$ | H | 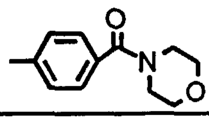 | cis | $4-CH_3O-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 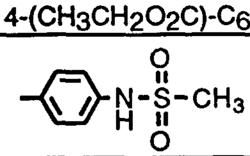 | trans | $4-CH_3O-C_6H_4-$ |
| $C_6H_5-(CH_2)_3-$ | H | $-PhO\overset{O}{\overset{\|}{C}}(CH_2)_2\overset{O}{\overset{\|}{C}}OCH_3$ Wherein Ph is 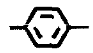 | trans | $4-CH_3O-C_6H_4-$ |
| $C_6H_5-(CH_2)_3-$ | H | $-Ph-OCH_2CO_2Et$ Wherein Ph is 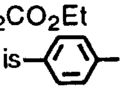 and Et is $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 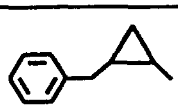 | H | $4-CH_3O-C_6H_4-$ | cis | $4-CH_3O-C_6H_4-$ |

**12.** A compound of claim 1, or 11 which is (3R,4S)-1,4-bis-(4-methoxyphenyl)-3-(3-phenylpropyl)-2-azetidinone.

**13.** Use of claim 10 wherein the compound is selected from the group consisting of compounds of the formula

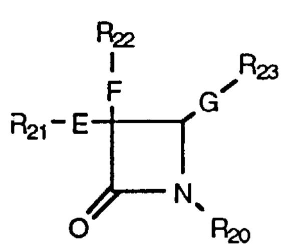

wherein $R_{21}$-E-, $R_{22}$-F-, $R_{23}$-G-, $R_{20}$ and relative stereochemistry between $R_{21}$-E- and $R_{23}$-G- are as defined in

the following table:

| $R_{21}$-E- | $R_{22}$-F- | $R_{23}$-G- | Rel. Stereo-chem. | $R_{20}$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| 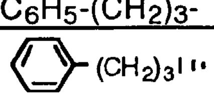 | H | 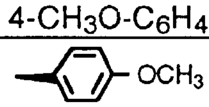 | trans optically pure | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |

| | | | | |
|---|---|---|---|---|
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 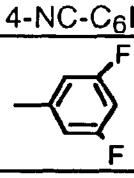 |
| $C_6H_5-(CH_2)_3-$ | H | $4-CH_3O-C_6H_4-$ | trans | $4-(CH_3C(O))-C_6H_4-$ |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 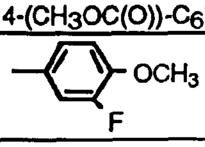 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | 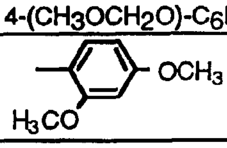 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $C_6H_5-(CH_2)_3-$ | H | $4-CH_3O-C_6H_4-$ | trans | $4-I-C_6H_4-$ |
| 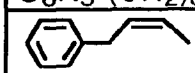 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 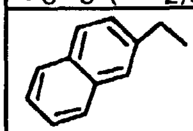 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 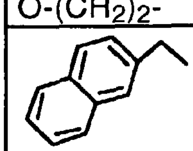 | H | $4-CH_3O-C_6H_4-$ | cis | $C_6H_5-$ |
| $C_6H_5-(CH_2)_3-$ | H | $4-CH_3O-C_6H_4-$ | trans | 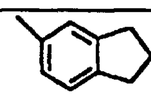 |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 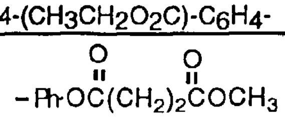 Wherein Ph is 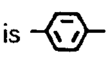 | trans | $4-CH_3O-C_6H_4-$ |

| | | | | |
|---|---|---|---|---|
| $C_6H_5-(CH_2)_3-$ | H | $-Ph-OCH_2CO_2Et$ Wherein Ph is 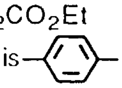 and Et is $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 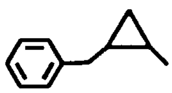 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 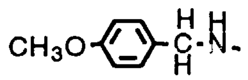 | H | $4-CH_3O-C_6H_4-$ | cis | $C_6H_5-$ |

**14.** The use of a compound of claim I for the manufacture of a medicament for lowering serum cholesterol.

**15.** The use of a compound of claim 1 for the manufacture of a medicament for inhibiting the enzyme acyl CoA:cholesterol acyl transferase.

**16.** Use of a composition as defined in claim 10 for the manufacture of a medicament for lowering serum cholesterol levels in a mammal in need of such treatment.

**17.** Use of a composition as defined in claim 10 for the manufacture of a medicament for inhibiting the enzyme acyl CoA:cholesterol acyl transferase.

**18.** A process for producing a compound of claim 1, wherein R is H, and D and A have trans relative stereochemistry, comprising cyclizing a hydroxyamide of the formula

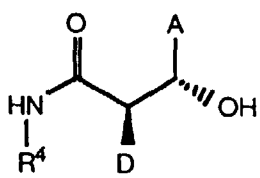
or
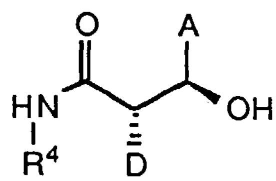

wherein D, A and $R^4$ are as defined in claim 1, by-treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride.

**19.** A process for producing a compound of claim 1 having the formula

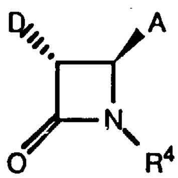 or 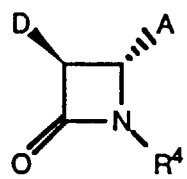

wherein D, A and $R^4$ are as defined in claim 1, comprising:

(a) reacting a carboxylic acid of the formula $DCH_{2-}$ COOH, wherein D is as defined above, with a chlorinating agent;
(b) deprotonating a chiral oxazolidinone of the formula

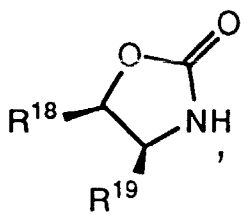 , 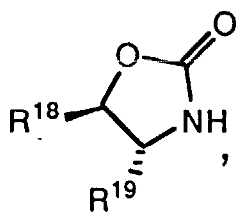 , 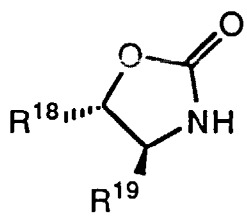 or 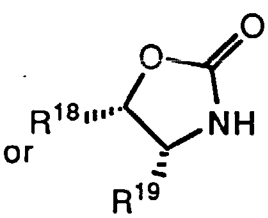

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; with a strong base and treating the resulting anion with the product of step (a);

(c) enolizing the product of step (b) with either:

(i) a dialkylboron triflate and a tertiary amine base;
(ii) $TiCl_4$ and tetramethylethylenediamine (TMEDA) or a mixture of TMEDA and triethylamine;

then condensing with an aldehyde of the formula A-CHO, wherein A is as defined above;
(d) hydrolyzing the product of step (c) with a base and hydrogen peroxide;
(e) condensing the product of step (d) with an amine of the formula $R^4NH_2$, wherein $R^4$ is as defined above, by treating with a dehydrative coupling agent, optionally adding an activating agent; and
(f) cyclizing the product of step (e) by treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride.

**20.** A process for producing a compound of claim 1, wherein R is hydrogen, and D and A have trans relative stereochemistry, comprising:

(a) enolizing a compound of the formula

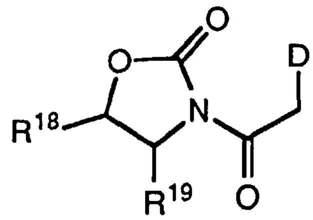

with $TiCl_4$ and tetramethylethylenediamine (TMEDA), then condensing with an imine of the formula A-CH=N-$R^4$, wherein A and $R^4$ are as defined above, and wherein the group

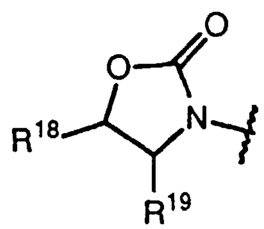

represents an chiral auxiliary of the formula

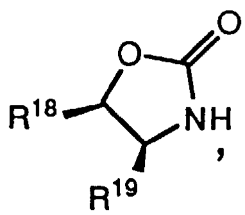 , 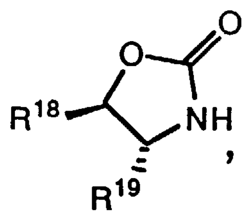 , 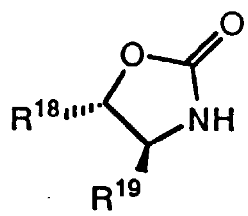 or 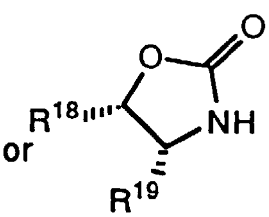

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; and

(b) cyclizing the product of step (a) by treating with a strong non-nucleophilic base.

**21.** A process for preparing compounds of claim 1 comprising:

(a) treating an ester of the formula D-CH(R)-C(O)O$R^{17}$, wherein $R^{17}$ is $C_{1-6}$ alkyl, menthyl, or 10-(siisopropylsulfonamido)isobornyl, with a strong base, and then with an imine of the formula A-CH=N-$R^4$, wherein A and $R^4$ are as defined in claim 1; or

(b) for preparing a compound of claim 1 wherein R is not hydrogen, reacting a compound of claim 1, wherein R is hydrogen, with a strong base and an alkylating agent or an acylating agent; or

(c) for preparing a compound of claim 1, wherein R is hydrogen, and D and A have cis relative stereochemistry, reacting a compound of claim 1, wherein R is hydrogen, and D and A have trans relative stereochemistry, with a strong base at -80° to -40°C, and then with a proton source; or

(d) for preparing a compound of claim 1, wherein R is hydrogen, and D and A have trans relative stereochemistry, reacting a compound of claim 1, wherein R is hydrogen, and D and A have cis relative stereochemistry, with an alkali metal t-butoxide, then quenching with acid; or

(e) reacting a carboxylic acid derivative of the formula D-CH(R)-C(O)-Z, wherein D and R are as defined in claim 1, and Z is Cl,

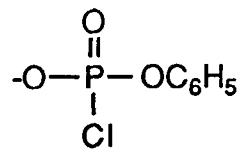, or 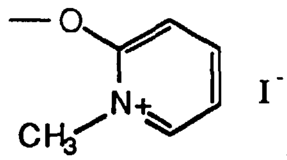 ,

with an imine of the formula A-CH=N-$R^4$, wherein A and $R^4$ are as defined in claim 1, in the presence of a tertiary amine base.

**22.** A process for preparing a compound of formula II

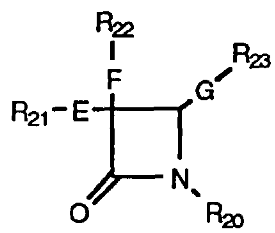

II

wherein

$R_{20}$ is phenyl, W-substituted phenyl, naphthyl, W-substituted naphthyl, benzodioxolyl, heteroaryl, W-substituted heteroaryl, benzofused heteroaryl and W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof;

$R_{21}$, $R_{22}$ and $R_{23}$ are independently selected from H or $R_{20}$; W is 1 to 3 substituents independently selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $-NR_{10}R_{11}$, $NR_{10}R_{11}(C_{1-6}$ alkyl)-, $NR_{10}R_{11}(C_{1-6}$ alkoxy)-, OH, halogeno, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-butyldimethyl-silyloxymethyl, $-C(O)R_{12}$,

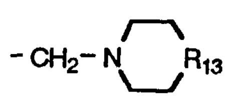 and 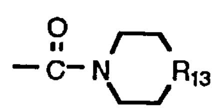 ;

E, F and G are independently a bond; $C_3$-$C_6$ cycloalkylene; $C_1$-$C_{10}$ alkylene; $C_2$-$C_{10}$ alkenylene; $C_2$-$C_{10}$ alkynylene; an alkylene, alkenylene or alkynylene chain as defined substituted by one or more substituents independently selected from the group consisting of phenyl, W-substituted phenyl, heteroaryl and W-substituted heteroaryl, wherein heteroaryl is as defined above; an alkylene, alkenylene or alkynylene chain as defined interrupted by one or more groups independently selected from the group consisting of -O-, -S-, -SO-, $-SO_2$-, $-NR_8$, -C(O)-, $C_3$-$C_6$ cycloalkylene, phenylene, W-substituted phenylene, heteroarylene and W-substituted heteroarylene; or an interrupted alkylene, alkenylene or alkynylene chain as defined substituted by one or more substituents independently selected from the group consisting of phenyl, W-substituted phenyl, heteroaryl and W-substituted heteroaryl; or one of $R_{21}$-E and $R_{22}$-F is selected from the group consisting of halogeno, OH, lower alkoxy, $-OC(O)R_5$, $-NR_{10}R_{11}$, -SH or $-S(C_{1-6}$ alkyl);

$R_5$ is $C_{1-6}$ alkyl, phenyl, $R_{14}$-phenyl, benzyl or $R_{14}$-benzyl;

$R_6$ is OH, $C_{1-6}$ alkyl, phenyl, benzyl, $R_{14}$-phenyl or $R_{14}$-benzyl;

$R_8$ is H, $C_{1-6}$ alkyl, phenyl $C_{1-6}$ alkyl or $-C(O)R_9$;

$R_9$ is H, $C_{1-6}$ alkyl, phenyl or phenyl $C_{1-6}$ alkyl;

$R_{10}$ and $R_{11}$ are independently selected from H and $C_{1-6}$ alkyl;
$R_{12}$ is H, OH, $C_{1-6}$ alkoxy, phenoxy, benzyloxy,

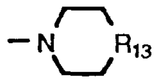,

$-NR_{10}R_{11}$, $C_{1-6}$ alkyl, phenyl or $R_{14}$-phenyl;
$R_{13}$ is -O-, $-CH_2-$, -NH- or $-N(C_{1-6}$ alkyl)-;
$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;
provided that when G is a bond, $R_{23}$ is not H, and provided that when $R_{23}$ is W-substituted phenyl, W is not p-halogeno;
or a pharmaceutically acceptable salt thereof;
wherein $R_{22}$-F is H, and $R_{21}$-E- and $R_{23}$-G- have trans relative stereochemistry, comprising
Process C: cyclizing a hydroxyamide of the formula

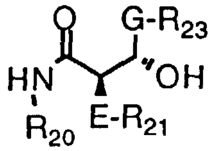 or 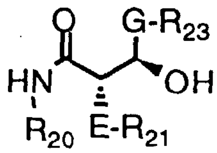

wherein $R_{21}$-E-, $R_{23}$-G- and $R_{20}$ are as defined above, by treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride; or

Process D:

(a) reacting a carboxylic acid of the formula $R_{21}-E-CH_2-COOH$, wherein $R_{21}$-E- is as defined above, with a chlorinating agent;
(b) deprotonating a chiral oxazolidinone of the formula

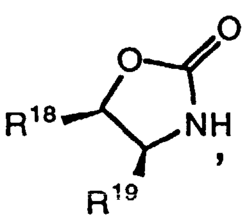, 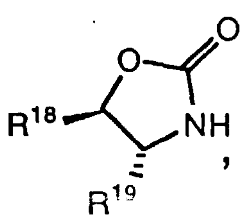, 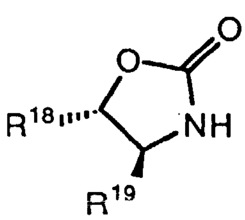 or 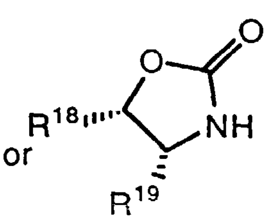

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; with a strong base and treating the resulting anion with the product of step (a);
(c) enolizing the product of step (b) with either:

(i) a dialkylboron triflate and a tertiary amine base;
(ii) $TiCl_4$ and tetramethylethylenediamine (TMEDA) or a mixture of TMEDA and triethylamine;

then condensing with an aldehyde of the formula $R_{23}$-G-CHO, wherein $R_{23}$-G- is as defined above;
(d) hydrolyzing the product of step (c) with a base and hydrogen peroxide;
(e) condensing the product of step (d) with an amine of the formula $R_{20}NH_2$, wherein $R_{20}$ is as defined above, by treating with a dehydrative coupling agent, optionally adding an activating agent; and
(f) cyclizing the product of step (e) as described in Process C.

**23.** A process for producing a compound of formula II as defined in claim 22, wherein $R_{22}$-F- is hydrogen, and $R_{21}$-E- and $R_{23}$-G- have trans relative stereochemistry, comprising:

(a) enolizing a compound of the formula

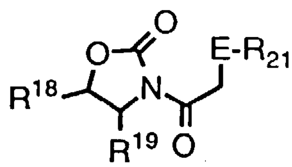

with $TiCl_4$ and tetramethylethylenediamine (TMEDA), then condensing with an imine of the formula $R_{23}$-G-CH=N-$R_2$O, wherein $R_{23}$-G- and $R_{20}$ are as defined in claim 2 2, and wherein the group

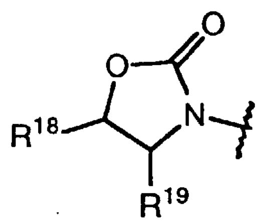

represents an chiral auxiliary of the formula

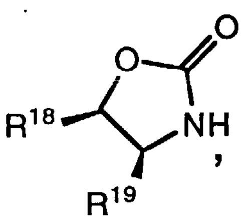 , 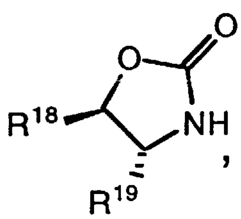 , 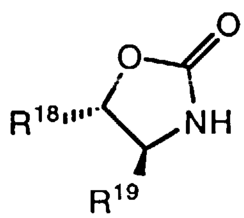 or 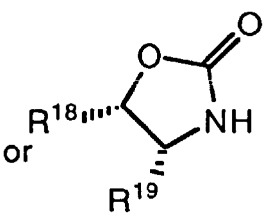

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; and
(b) cyclizing the product of step (a) by treating with a strong non-nucleophilic base.

**24.** A process for preparing a compound of formula II as defined in claim 22, comprising

(a) treating an ester of the formula $R_{21}$-E-CH(F-$R_{22}$)-C(O)O$R^{17}$, wherein $R^{17}$ is $C_{1-6}$ alkyl, menthyl, or 10-(diisopropylsulfonamido)isobornyl, with a strong base, and then with an imine of the formula $R_{23}$-G-CH=N-$R_{20}$, wherein $R_{23}$-G- and $R_{20}$ are as defined in claim 22, or
(b) for preparing a compound of formula II as defined in claim 22, wherein $R_{22}$-F- is not hydrogen, reacting a compound of formula II as defined in claim 22, wherein $R_{22}$-F- is hydrogen, with a strong base and an alkylating agent or an acylating agent; or
(c) for preparing a compound of formula II as defined in claim 22, wherein $R_{22}$-F- is hydrogen, and $R_{21}$-E- and $R_{23}$-G- have cis relative stereochemistry, reacting a compound of formula II as defined in claim 22, wherein $R_{22}$-F- is hydrogen, and $R_{21}$-E- and $R_{23}$-G- have trans relative stereo-chemistry, with a strong base at -80° to -40°C, and then with a proton source; or
(d) for preparing a compound of formula II as defined in claim 22, wherein $R_{22}$-F- is hydrogen, and $R_{21}$-E- and $R_{23}$-G- have trans relative stereochemistry, reacting a compound of formula II as defined in claim 22 wherein

$R_{22}$-F- is hydrogen, and $R_{21}$-E- and $R_{23}$-G- have cis relative stereochemistry, with an alkali metal t-butoxide, then quenching with acid; or

(e) reacting a carboxylic acid derivative of the formula $R_{21}$-E-CH(F-$R_{22}$)-C(O)-Z, wherein $R_{21}$-E- and $R_{22}$-F- are as defined in claim 22, and Z is Cl,

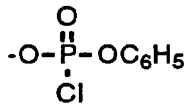 , or 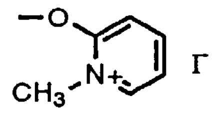 ,

with an imine of the formula $R_{23}$-G-CH=N-$R_{20}$, wherein $R_{23}$-G- and $R_{20}$ are as defined in claim 22, in the presence of a tertiary amine base.

**Claims for the following Contracting State : ES**

**1.** A process for producing a compound having the structural formula I

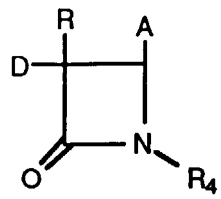 I

wherein

A is

-CH=CH-B;
-C≡C-B;
-$(CH_2)_p$-X-B, wherein p is 0, 1 or 2 and X is a bond, -NH- or -$S(O)_{0-2}$-;

heteroaryl, benzofused heteroaryl, W-substituted heteroaryl or W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, and wherein W is 1-3 substituents on the ring carbon atoms selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, -$CF_3$, -$OCF_3$, benzyl, $R_{14}$-benzyl, benzyloxy, $R_{14}$-benzyloxy, phenoxy, $R_{14}$-phenoxy, dioxolanyl, $NO_2$, -$NR_{10}R_{11}$, $NR_{10}R_{11}$($C_{1-6}$ alkyl)-, $NR_{10}R_{11}$($C_{1-6}$ alkoxy)-, OH, halogeno, -NHC(O)$OR_5$, -NHC(O)$R_5$, $R_6O_2SNH$-, $(R_6O_2S)_2N$-, -$S(O)_2NH_2$, -$S(O)_{0-2}R_{10}$, tert-butyldimethylsilyloxymethyl, -C(O)$R_{12}$ and

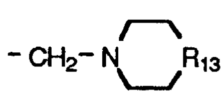 ,

and wherein the substituents on the substituted heteroaryl ring nitrogen atoms, when present, are selected from the group consisting of $C_{1-6}$ alkyl, $C_{1-6}$ alkoxy, -C(O)$OR_5$, -C(O)$R_5$, OH, $NR_{10}R_{11}$ ($C_{1-6}$ alkyl)-, $NR_{10}R_{11}$($C_{1-6}$ alkoxy)-, -$S(O)_2NH_2$ and 2-(trimethylsilyl)ethoxymethyl;
-C(O)-B; or

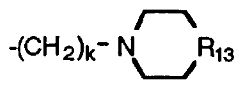,

wherein k is 1 or 2;
D is

B'-$(CH_2)_m$C(O)-, wherein m is 1, 2, 3, 4 or 5;
B'-$(CH_2)_q$-, wherein q is 2, 3, 4, 5 or 6;
B'-$(CH_2)$e-Z-$(CH_2)_r$, wherein Z is -O-, -C(O)-, phenylene, -$NR_8$- or -$S(O)_{0\text{-}2}$-, e is 0, 1, 2, 3, 4 or 5 and r is 1, 2, 3, 4 or 5, provided that the sum of e and r is 1, 2, 3, 4, 5 or 6;
B'-($C_2$-$C_6$ alkenylene)-; B'-($C_4$-$C_6$ alkadienylene)-;
B'-$(CH_2)_t$-Z-($C_2$-$C_6$ alkenylene),wherein Z is as defined above, and wherein t is 0, 1, 2 or 3, provided that the sum of t and the number of carbon atoms in the alkenylene chain is 2, 3, 4, 5 or 6;
B'-$(CH_2)_f$-V-$(CH_2)_g$-, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6;
B'-$(CH_2)_t$-V-($C_2$-$C_6$ alkenylene)- or B'-($C_2$-$C_6$ alkenylene)-V-$(CH_2)_t$-, wherein V and t are as defined above, provided that the sum of t and the number of carbon atoms in the alkenylene chain is 2, 3, 4, 5 or 6;
B'-$(CH_2)_a$-Z-$(CH_2)_b$-V-$(CH_2)_d$-, wherein Z and V are as defined above and a ,b and d are independently 0, 1, 2, 3, 4, 5 or 6, provided that the sum of a, b and d is 0, 1, 2, 3, 4, 5 or 6;
T-$(CH_2)_s$-, wherein T is cycloalkyl of 3-6 carbon atoms and s is 1, 2, 3, 4, 5 or 6; or
naphthylmethyl, heteroarylmethyl, or W-substituted heteroarylmethyl, wherein heteroaryl and W are as defined above;

B is

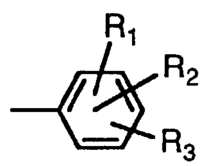

B' is naphthyl, heteroaryl or W-substituted heteroaryl, wherein heteroaryl is as defined above, or

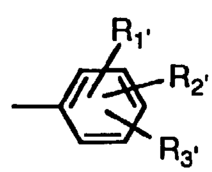

R is hydrogen, fluoro, $C_1$-$C_{15}$ alkyl, $C_1$-$C_{15}$ alkenyl, $C_1$-$C_{15}$ alkynyl, or B-$(CH_2)_h$ -, wherein h is 0, 1, 2, or 3; $R_1$, $R_2$ and $R_3$ are independently selected from the group consisting of H, $C_{1\text{-}6}$ alkyl, hydroxy $C_{1\text{-}6}$ alkyl, $C_{1\text{-}6}$ alkoxy, $C_{1\text{-}6}$ alkoxy-$C_{1\text{-}6}$ alkyl, $C_{1\text{-}6}$ alkoxy-$C_{1\text{-}6}$ alkoxy, $C_{1\text{-}6}$ alkoxycarbonyl-$C_{1\text{-}6}$ alkoxy, ($C_{1\text{-}6}$ alkoxyimino)-$C_{1\text{-}6}$ alkyl, $C_{3\text{-}6}$ alkanedioyl, $C_{1\text{-}6}$ alkyl-$C_{3\text{-}6}$ alkanedioyl, allyloxy, -$CF_3$, -$OCF_3$, benzyl, $R_{14}$-benzyl, benzyloxy, $R_{14}$-benzyloxy, phenoxy, $R_{14}$-phenoxy, dioxolanyl, $NO_2$, -$NR_{10}R_{11}$, $NR_{10}R_{11}$($C_{1\text{-}6}$ alkyl)-, $NR_{10}R_{11}$ ($C_{1\text{-}6}$ alkoxy)-, OH, o-halogeno, m-halogeno, -NHC(O)$OR_5$, -NHC(O) $R_5$, $R_6O_2$SNH-, $(R_6O_2S)_2$N-, -$S(O)_2NH_2$, -$S(O)_{0\text{-}2}R_{10}$, tert-butyldimethylsilyloxymethyl, -C(O)$R_{12}$,

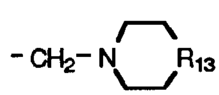

and

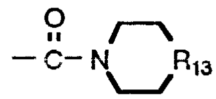

,

or $R_1$ is hydrogen and $R_2$ and $R_3$, together with adjacent carbon atoms to which they are attached, form a dioxolanyl ring; $R_{1'}$, $R_{2'}$ and $R_{3'}$ are independently selected from the group consisting of H, $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkane-dioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $-NR_{10}R_{11}$, $NR_{10}R_{11}$($C_{1-6}$ alkyl)-, $NR_{10}R_{11}$($C_{1-6}$ alkoxy)-, OH, halogeno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldimethyl-silyloxymethyl, $-C(O)R_{12}$,

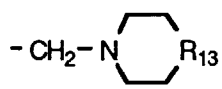

and

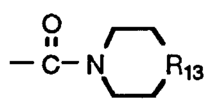

,

or $R_{1'}$ is hydrogen and $R_{2'}$ and $R_{3'}$, together with adjacent carbon atoms to which they are attached, form a dioxolanyl ring;

$R_4$ is

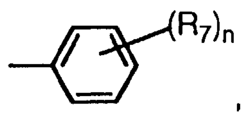

,

wherein n is 0, 1, 2 or 3, indanyl, benzofuranyl, benzodioxolyl, tetrahydronaphthyl, pyridyl, pyrazinyl, pyrimidinyl or quinolyl;

$R_5$ is $C_{1-6}$ alkyl, phenyl, $R_{14}$-phenyl, benzyl or $R_{14}$-benzyl;

$R_6$ is OH, $C_{1-6}$ alkyl, phenyl, benzyl, $R_{14}$-phenyl or $R_{14}$-benzyl;

$R_7$ is $C_{1-6}$ alkyl, $C_{1-6}$ alkoxy, OH, halogeno, $-NR_{10}R_{11}$, $-NHC(O)OR_5$, $-NHC(O)R_5$, $NO_2$, -CN, $-N_3$, -SH, $-S(O)_{0-2}$-($C_{1-6}$ alkyl), $-COOR_9$, $-CONR_{10}R_{11}$, $-COR_{12}$, phenoxy, benzyloxy, $-OCF_3$, or tert-butyldimethylsilyloxy, and where n is 2 or 3, the $R_7$ groups can be the same or different;

$R_8$ is H, $C_{1-6}$ alkyl, phenyl $C_{1-6}$ alkyl, or $-C(O)R_9$;

$R_9$ is H, $C_{1-6}$ alkyl, phenyl or phenyl $C_{1-6}$ alkyl;

$R_{10}$ and $R_{11}$ are independently selected from H and $C_{1-6}$ alkyl;

$R_{12}$ is H, OH, $C_{1-6}$, alkoxy, phenoxy, benzyloxy

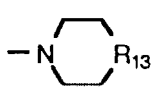

,

$-NR_{10}R_{11}$, $C_{1-6}$ alkyl, phenyl or $R_{14}$-phenyl;

$R_{13}$ is -O-, $-CH_2-$, -NH- or -N($C_{1-6}$ alkyl)-; and

$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;

or a pharmaceutically acceptable salt thereof, wherein R is H, and D and A have trans relative stereochemistry, comprising Process A: cyclizing a hydroxyamide of the formula

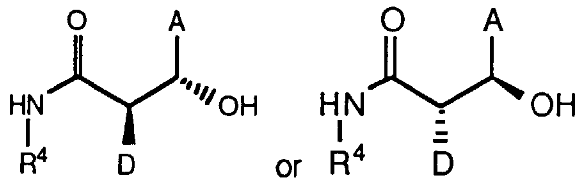

wherein D, A and $R^4$ are as defined above, by treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride; or

Process B:

(a) reacting a carboxylic acid of the formula $DCH_2$-COOH, wherein D is as defined above, with a chlorinating agent;
(b) deprotonating a chiral oxazolidinone of the formula

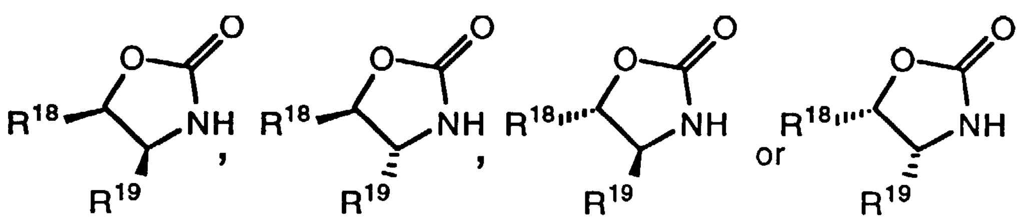

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; with a strong base and treating the resulting anion with the product of step (a);
(c) enolizing the product of step (b) with either:

(i) a dialkylboron triflate and a tertiary amine base;
(ii) $TiCl_4$ and tetramethylethylenediamine (TMEDA) or a mixture of TMEDA and triethylamine;

then condensing with an aldehyde of the formula A-CHO, wherein A is as defined above;
(d) hydrolyzing the product of step (c) with a base and hydrogen peroxide;
(e) condensing the product of step (d) with an amine of the formula $R^4NH_2$, wherein $R^4$ is as defined above, by treating with a dehydrative coupling agent, optionally adding an activating agent; and
(f) cyclizing the product as described in Process A, above.

**2.** A process for producing a compound of formula I as defined in claim 1 , wherein R is hydrogen, and D and A have trans relative stereochemistry, comprising:

(a) enolizing a compound of the formula

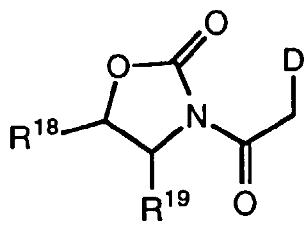

with $TCl_4$ and tetramethylethylenediamine (TMEDA), then condensing with an imine of the formula A-CH=N-$R^4$, wherein A and $R^4$ are as defined above, and wherein the group

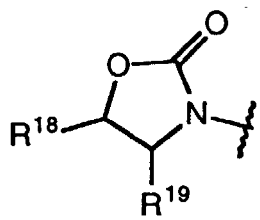

represents an chiral auxiliary of the formula

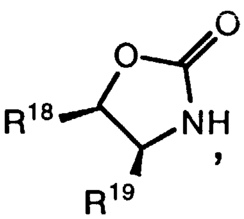 , 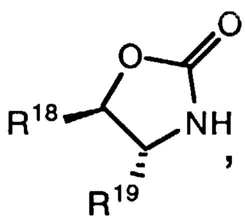 , 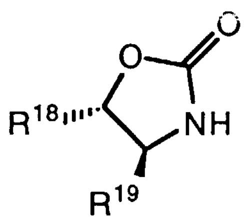 or 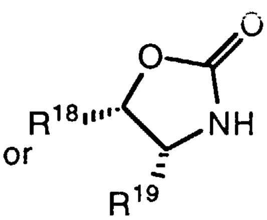

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; and
(b) cyclizing the product of step (a) by treating with a strong non-nucleophilic base.

**3.** A process for preparing compounds of formula I as defined in claim 1 comprising:

(a) treating an ester of the formula D-CH(R)-C(O)O$R^{17}$, wherein $R^{17}$ is $C_{1-6}$ alkyl, menthyl, or 10-(diisopropylsulfonamido)isobornyl, with a strong base, and then with an imine of the formula A-CH=N-$R^4$, wherein A and $R^4$ are as defined in claim 1, or
(b) for preparing a compound of formula I as defined in claim 1 wherein R is not hydrogen, reacting a compound of formula I as defined in claim 1, wherein R is hydrogen, with a strong base and an alkylating agent or an acylating agent; or
(c) for preparing a compound of formula I as defined in claim 1 wherein R is hydrogen, and D and A have cis relative stereochemistry, reacting a compound of formula I of claim 1, wherein R is hydrogen, and D and A have trans relative stereochemistry, with a strong base at -80° to -40°C, and then with a proton source; or
(d) for preparing a compound of formula I as defined in claim 1, wherein R is hydrogen, and D and A have trans relative stereochemistry, reacting a compound of formula I as defined in claim 1, wherein R is hydrogen, and D and A have cis relative stereochemistry, with an alkali metal t-butoxide, then quenching with acid; or
(e) reacting a carboxylic acid derivative of the formula D-CH(R)-C(O)-Z, wherein D and R are as defined in claim 1, and Z is Cl,

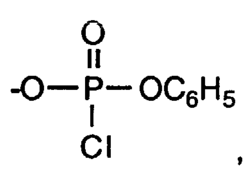 , or 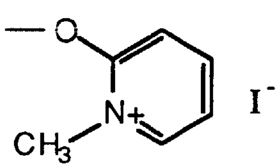 ,

with an imine of the formula A-CH=N-$R^4$, wherein A and $R^4$ are as defined in claim 1, in the presence of a tertiary amine base.

**4.** A process for preparing a compound of formula II

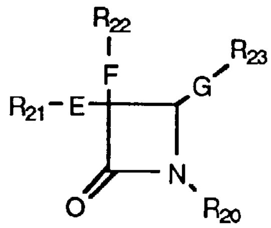

II

wherein

$R_{20}$ is phenyl, W-substituted phenyl, naphthyl, W-substituted naphthyl, benzodioxolyl, heteroaryl, W-substituted heteroaryl, benzofused heteroaryl and W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof;
$R_{21}$, $R_{22}$ and $R_{23}$ are independently selected from H or $R_{20}$;
W is 1 to 3 substituents independently selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, $-CF_3$, $-OCF_3$, benzyl, $R_{14}$-benzyl, benzyloxy, $R_{14}$-benzyloxy, phenoxy, $R_{14}$-phenoxy, dioxolanyl, $NO_2$, $NR_{10}R_{11}$, $NR_{10}R_{11}$ ($C_{1-6}$ alkyl)-, $NR_{10}R_{11}$($C_{1-6}$ alkoxy)-, OH, halogeno, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-butyldimethyl-silyloxymethyl, $-C(O)R_{12}$,

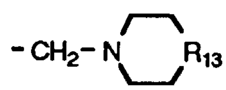
and
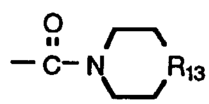
;

E, F and G are independently a bond; $C_3$-$C_6$ cycloalkylene; $C_1$-$C_{10}$ alkylene; $C_2$-$C_{10}$ alkenylene; $C_2$-$C_{10}$ alkynylene; an alkylene, alkenylene or alkynylene chain as defined substituted by one or more substituents independently selected from the group consisting of phenyl, W-substituted phenyl, heteroaryl and W-substituted heteroaryl, wherein heteroaryl is as defined above; an alkylene, alkenylene or alkynylene chain as defined interrupted by one or more groups independently selected from the group consisting of -O-, -S-, -SO-, $-SO_2$-, $-NR_8$, -C(O)-, $C_3$-$C_6$ cycloalkylene, phenylene, W-substituted phenylene, heteroarylene and W-substituted heteroarylene; or an interrupted alkylene, alkenylene or alkynylene chain as defined substituted by one or more substituents independently selected from the group consisting of phenyl, W-substituted phenyl, heteroaryl and W-substituted heteroaryl; or one of $R_{21}$-E and $R_{22}$-F is selected from the group consisting of halogeno, OH, lower alkoxy, $-OC(O)R_5$, $-NR_{10}R_{11}$, -SH or -S($C_{1-6}$ alkyl);
$R_5$ is $C_{1-6}$ alkyl, phenyl, $R_{14}$-phenyl, benzyl or $R_{14}$-benzyl;
$R_6$ is OH, $C_{1-6}$ alkyl, phenyl, benzyl, $R_{14}$-phenyl or $R_{14}$-benzyl;
$R_8$ is H, $C_{1-6}$ alkyl, phenyl $C_{1-6}$ alkyl or $-C(O)R_9$;
$R_9$ is H, $C_{1-6}$ alkyl, phenyl or phenyl $C_{1-6}$ alkyl;
$R_{10}$ and $R_{11}$ are independently selected from H and $C_{1-6}$ alkyl;
$R_{12}$ is H, OH, $C_{1-6}$ alkoxy, phenoxy, benzyloxy,

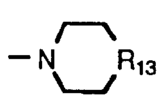
,

$-NR_{10}R_{11}$, $C_{1-6}$ alkyl, phenyl or $R_{14}$-phenyl;
$R_{13}$ is -O-, $-CH_2$-, -NH- or -N($C_{1-6}$ alkyl)-;
$R_{14}$ is 1-3 groups independently selected from the group consisting of $C_{1-6}$ alkyl, $C_{1-6}$ alkoxy, -COOH, $NO_2$,

$-NR_{10}R_{11}$, OH or halogeno;
provided that when G is a bond, $R_{23}$ is not H, and provided that when $R_{23}$ is W-substituted phenyl, W is not p-halogeno;
or a pharmaceutically acceptable salt thereof;
wherein $R_{22}$-F is H, and $R_{21}$-E- and $R_{23}$-G- have trans relative stereochemistry, comprising
Process C: cyclizing a hydroxyamide of the formula

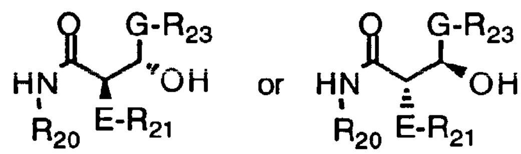

wherein $R_{21}$-E-, $R_{23}$-G- and $R_{20}$ are as defined above, by treating with:

(i) a dialkylazodicarboxylate and a trialkylphosphine; or
(ii) a di- or tri-chlorobenzoyl chloride, an aqueous base and a phase transfer catalyst, then treating the resulting di- or tri-chlorobenzoate with an aqueous base and a phase transfer catalyst; or
(iii) a dialkylchlorophosphate, an aqueous base and a phase transfer catalyst; or
(iv) a di- or tri-chlorobenzoyl chloride and an alkali metal hydride; or

Process D:

(a) reacting a carboxylic acid of the formula $R_{21}$-E-$CH_2$-COOH, wherein $R_{21}$-E- is as defined above, with a chlorinating agent;
(b) deprotonating a chiral oxazolidinone of the formula

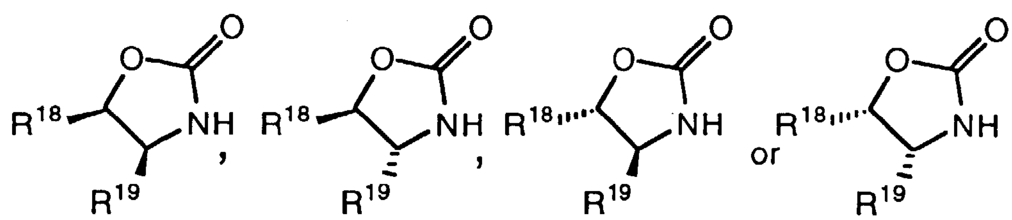
, , or

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; with a strong base and treating the resulting anion with the product of step (a);
(c) enolizing the product of step (b) with either:

(i) a dialkylboron triflate and a tertiary amine base;
(ii) $TiCl_4$ and tetramethylethylenediamine (TMEDA) or a mixture of TMEDA and triethylamine;

then condensing with an aldehyde of the formula $R_{23}$-G-CHO, wherein $R_{23}$-G- is as defined above;
(d) hydrolyzing the product of step (c) with a base and hydrogen peroxide;
(e) condensing the product of step (d) with an amine of the formula $R_{20}NH_2$, wherein $R_{20}$ is as defined above, by treating with a dehydrative coupling agent, optionally adding an activating agent; and
(f) cyclizing the product of step (e) as described in Process C.

**5.** A process for producing a compound of formula II as defined in claim 4, wherein $R_{22}$-F- is hydrogen, and $R_{21}$-E- and $R_{23}$-G- have trans relative stereochemistry, comprising:

(a) enolizing a compound of the formula

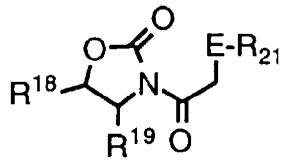

with $TiCl_4$ and tetramethylethylenediamine (TMEDA), then condensing with an imine of the formula $R_{23}$-G-CH=N-$R_2O$, wherein $R_{23}$-G- and R20 are as defined in claim 4 , and wherein the group

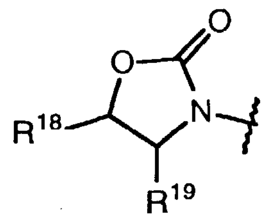

represents an chiral auxiliary of the formula

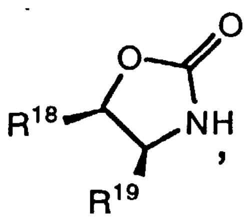 , 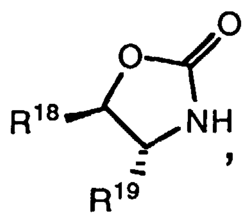 , 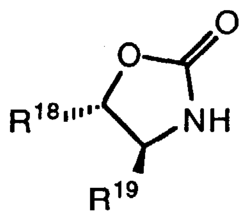 or 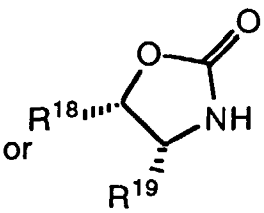

wherein $R^{18}$ and $R^{19}$ are independently selected from the group consisting of: hydrogen, $C_1$-$C_6$ alkyl, phenyl, naphthyl, substituted phenyl, substituted naphthyl, and benzyl; and

(b) cyclizing the product of step (a) by treating with a strong non-nucleophilic base.

**6.** A process for preparing a compound of formula II as defined in claim 4 , comprising

(a) treating an ester of the formula $R_{21}$-E-CH(F-$R_{22}$)-C(O)O$R^{17}$, wherein $R^{17}$ is $C_{1-6}$ alkyl, menthyl, or 10-(diisopropylsulfonamido)-isobornyl, with a strong base, and then with an imine of the formula $R_{23}$-G-CH=N-$R_{20}$, wherein $R_{23}$-G- and $R_{20}$ are as defined in claim 4 ; or

(b) for preparing a compound of formula II as defined in claim 4, wherein $R_{22}$-F- is not hydrogen, reacting a compound of formula II as defined in claim 4 , wherein $R_{22}$-F- is hydrogen, with a strong base and an alkylating agent or an acylating agent; or

(c) for preparing a compound of formula II as defined in claim 4 wherein $R_{22}$-F- is hydrogen, and $R_{21}$-E- and $R_{23}$-G- have cis relative stereochemistry, reacting a compound of formula II as defined in claim 4 , wherein $R_{22}$-F- is hydrogen, and $R_{21}$-E- and $R_{23}$-G- have trans relative stereo-chemistry, with a strong base at -80° to -40°C, and then with a proton source; or

(d) for preparing a compound of formula II as defined in claim 4 , wherein $R_{22}$-F- is hydrogen, and $R_{21}$-E- and $R_{23}$-G- have trans relative stereochemistry, reacting a compound of formula II as defined in claim 4, wherein $R_{22}$-F- is hydrogen, and $R_{21}$-E- and $R_{23}$-G- have cis relative stereochemistry, with an alkali metal t-butoxide, then quenching with acid; or

(e) reacting a carboxylic acid derivative of the formula $R_{21}$-E-CH(F-$R_{22}$)-C(O)-Z, wherein $R_{21}$-E- and $R_{22}$-F- are as defined in claim 4, and Z is Cl,

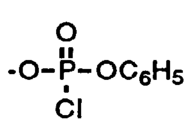 , or 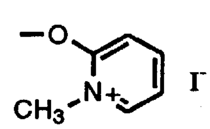 ,

with an imine of the formula $R_{23}$-G-CH=N-$R_{20}$, wherein $R_{23}$-G- and $R_{20}$ are as defined in claim 4, in the presence of a tertiary amine base.

**7.** A process of claim 1 wherein R is hydrogen.

**8.** A process of claim 1 or 7 wherein D is B'-$(CH_2)_q$-, B'-$(CH_2)_e$-Z-$(CH_2)_r$-, B'-($C_2$-$C_6$ alkenylene)- or B'-$(CH_2)_f$-V-$(CH_2)_g$-, wherein B', Z, V, q, e, r, f, and g are as defined in claim 1.

**9.** A process of claim 8 wherein D is B'-$(CH_2)_q$- wherein B' is phenyl and q is 3 or 4; B'-$(CH_2)_e$-Z-$(CH_2)_r$- wherein B' is p-fluorophenyl or p-methoxyphenyl, e is zero, Z is -O-, and r is 2; B'-($C_2$-$C_6$ alkenylene)- is 3-phenyl-1-propenyl; or B'-$(CH_2)_t$-V-$(CH_2)_g$- wherein B' is phenyl, f is 1, V is cyclopropylene, and g is zero.

**10.** A process of claim 1, 7, 8 or 9 wherein A is -$(CH_2)_p$-X-B wherein wherein X, B and p are as defined in claim 1.

**11.** A process of claim 10 wherein p is zero and X is a bond.

**12.** A process of claim 11 wherein $R_1$, $R_2$ and $R_3$ are selected from the group consisting of H, OH, $C_{1-6}$ alkoxy, $NO_2$, $C_{1-6}$-alkoxy $C_{1-6}$ alkoxy, m-halogeno, $C_{1-6}$ alkyl $C_{3-6}$ alkandioyl, $NR_{10}R_{11}$ ($C_{1-6}$- alkoxy)-, allyloxy, phenoxy, $C_{1-6}$ alkoxycarbonyl $C_{1-6}$ alkoxy and -C(O)$R_{12}$.

**13.** A process of claim 1, 7 ,8, 9, 10 or 11 wherein $R_4$ is phenyl, $R_7$-substituted phenyl or indanyl.

**14.** A process Of claim 13 wherein $R_7$ is selected from the group consisting of $C_{1-6}$ alkyl, $C_{1-6}$ alkoxy, halogeno, -$OCF_3$, $C_{1-6}$ alkylthio, -$NR_{10}R_{11}$, -CN, OH, and -$COR_{12}$.

**15.** A process for preparing a pharmaceutical composition for use in the manufacture of a medicament for lowering serum cholesterol, which process comprises admixing a pharmaceutical acceptable carrier with a compound having the structure

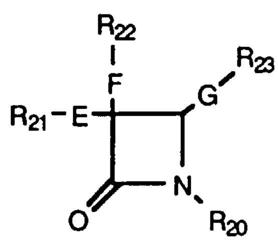

wherein

$R_{20}$ is phenyl, W-substituted phenyl, naphthyl, W-substituted naphthyl, benzodioxolyl, heteroaryl, W-substituted heteroaryl, benzofused heteroaryl and W-substituted benzofused heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof;
$R_{21}$, $R_{22}$ and $R_{23}$ are independently selected from H or $R_{20}$;
W is 1 to 3 substituents independently selected from the group consisting of $C_{1-6}$ alkyl, hydroxy $C_{1-6}$ alkyl, $C_{1-6}$ alkoxy, $C_{1-6}$ alkoxy-$C_{1-6}$ alkyl, $C_{1-6}$ alkoxy-$C_{1-6}$ alkoxy, $C_{1-6}$ alkoxycarbonyl-$C_{1-6}$ alkoxy, ($C_{1-6}$ alkoxyimino)-$C_{1-6}$ alkyl, $C_{3-6}$ alkanedioyl, $C_{1-6}$ alkyl-$C_{3-6}$ alkanedioyl, allyloxy, -$CF_3$, -$OCF_3$, benzyl, $R_{14}$ benzyl, benzyloxy, $R_{14}$-benyloxy, phenoxy, $R_{14}$-phenoxy, dioxolanyl, $NO_2$, -$NR_{10}R_{11}$, $NR_{10}R_{11}$ ($C_{1-6}$ alkyl)-, $NR_{10}R_{11}$ ($C_{1-6}$ alkoxy)-, OH, halogeno, -NHC(O)$OR_5$, -NHC(O)$R_5$, $R_6O_2$SNH-, $(R_6O_2S)_2$N-, -$S(O)_2NH_2$, -$S(O)_{0-2}R_{10}$, tert-butyldimethylsilyloxymethyl, -C(O)$R_{12}$,

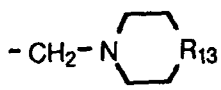
and
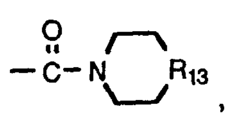
,

E, F and G are independently a bond; $C_3$-$C_6$ cycloalkylene; $C_1$-$C_{10}$ alkylene; $C_2$-$C_{10}$ alkenylene; $C_2$-$C_{10}$ alkynylene; an alkylene, alkenylene or alkynylene chain as defined substituted by one or more substituents independently selected from the group consisting of phenyl, W-substituted phenyl, heteroaryl and W-substituted heteroaryl, wherein heteroaryl is as defined above; an alkylene, alkenylene or alkynylene chain as defined interrupted by one or more groups independently selected from the group consisting of -O-, -S-, -SO-, $-SO_2-$, $-NR_8$, -C(O)-, $C_3$-$C_6$ cycloalkylene, phenylene, W-substituted phenylene, heteroarylene and W-substituted heteroarylene; or an interrupted alkylene, alkenylene or alkynylene chain as defined substituted by one or more substituents independently selected from the group consisting of phenyl, W-substituted phenyl, heteroaryl and W-substituted heteroaryl; or one of $R_{21}$-E and $R_{22}$-F is selected from the group consisting of halogeno, OH, $C_{1-6}$ alkoxy, $-OC(O)R_5$, $-NR_{10}R_{11}$, -SH or $-S(C_{1-6}$ alkyl);

$R_5$ is $C_{1-6}$ alkyl, phenyl, $R_{14}$-phenyl, benzyl or $R_{14}$-benzyl;

$R_6$ is OH, $C_{1-6}$ alkyl, phenyl, benzyl, $R_{14}$-phenyl or $R_{14}$-benzyl;

$R_8$ is H, $C_{1-6}$ alkyl, phenyl $C_{1-6}$ alkyl or $-C(O)R_9$;

$R_9$ is H, $C_{1-6}$ alkyl, phenyl or phenyl $C_{1-6}$ alkyl;

$R_{10}$ and $R_{11}$ are independently selected from H and $C_{1-6}$ alkyl;

$R_{12}$ is H, OH, $C_{1-6}$ alkoxy, phenoxy, benzyloxy,

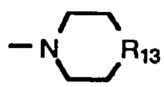

,

$-NR_{10}R_{11}$, $C_{1-6}$ alkyl, phenyl or $R_{14}$-phenyl;

$R_{13}$ is -O-, $-CH_2-$, -NH- or $-N(C_{1-6}$ alkyl)-;

$R_{14}$ is 1-3 groups independently selected from the group consisting of $C_{1-6}$ alkyl, $C_{1-6}$ alkoxy, -COOH, $NO_2$, $-NR_{10}R_{11}$, OH or halogeno;

provided that when G is a bond, $R_{23}$ is not H, and provided that when $R_{23}$ is W-substituted phenyl, W is not p-halogeno;

or a pharmaceutically acceptable salt thereof;

in a pharmaceutically acceptable carrier.

**16.** A process of claim 1 wherein the compounds prepared are selected from the group consisting of compounds of the formula

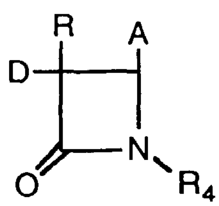

wherein D,R, A and $R_4$ and relative stereochemistry between D and A are as defined in the following table:

| D | R | A | Relative Stereo-chemistry | $R_4$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-$NO_2$-$C_6H_4$- | 3R,4R | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | 3-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 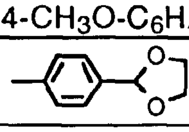 | trans | p-$CH_3O$-$C_6H_4$- |
| $C_6H_5$-$(CH_2)_3$- | H | 4-($(CH_3)_2$CH-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | 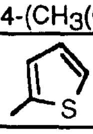 | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($C_6H_5$-O)-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 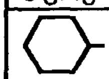 $(CH_2)_3$– | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-($(CH_3)_2$N-$(CH_2)_3$-O)-$C_6H_4$- | cis | 4-$CH_3O$-$C_6H_4$- |

| | | | | |
|---|---|---|---|---|
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| $C_6H_5$-$(CH_2)_3$- | H | 4-$CH_3O$-$C_6H_4$- | trans | 4-($C_6H_5$-O)-$C_6H_4$- |
| 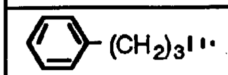 | H | 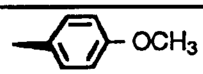 | trans optically pure | $C_6H_5$- |
| 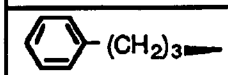 | H | 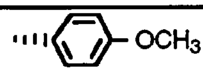 | trans optically pure | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($C_6H_5$-$CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| $C_6H_5$-O-$(CH_2)_2$- | H | 4-$CH_3O$-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |
| $C_6H_5$-$(CH_2)_3$- | H | 4-$CH_3O$-$C_6H_4$- | trans | 4-($CH_3CH_2OC(O)$)-$C_6H_4$- |
| $C_6H_5$-$(CH_2)_3$- | H | 4-$CH_3O$-$C_6H_4$- | trans | 3-$NO_2$-$C_6H_4$- |
| $C_6H_5$-$(CH_2)_3$- | H | 4-$CH_3O$-$C_6H_4$- | trans | 4-(($CH_3CH_2$)$_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 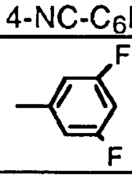 |
| $C_6H_5$-$(CH_2)_3$- | H | 4-$CH_3O$-$C_6H_4$- | trans | 4-($CH_3C(O)$)-$C_6H_4$- |

| | | | | |
|---|---|---|---|---|
| $C_6H_5$-$(CH_2)_3$- | H | 4-$CH_3O$-$C_6H_4$- | trans | 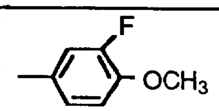 |
| $C_6H_5$-$(CH_2)_3$- | H | 4-$CH_3O$-$C_6H_4$- | trans | 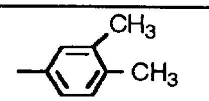 |
| $C_6H_5$-$(CH_2)_3$- | H | 4-(($CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC$(O))-$C_6H_4$- | trans | $C_6H_5$- |
| $C_6H_5$-$(CH_2)_3$- | H | 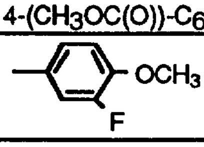 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 4-(($CH_3)_2N$)-$C_6H_4$- | trans | 4-$CH_3O$-$C_6H_4$- |
| $C_6H_5$-$(CH_2)_3$- | H | 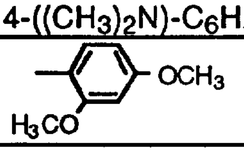 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| 4-$O_2N$-$C_6H_4$-$(CH_2)_3$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | 4-I-$C_6H_4$- |
| $C_6H_5$-$(CH_2)_3$- | H | 4-($CH_3(CH_2)_3$-O)-$C_6H_4$- | trans | 4-$CH_3O$-$C_6H_4$- |
| 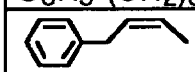 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 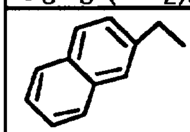 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 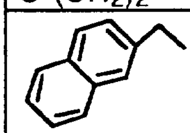 | H | 4-$CH_3O$-$C_6H_4$- | cis | $C_6H_5$- |
| $C_6H_5$-$(CH_2)_3$- | H | 4-$CH_3O$-$C_6H_4$- | trans | 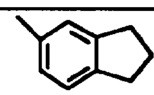 |
| $C_6H_5$-$(CH_2)_3$- | H | 4-$NH_2$-$C_6H_4$- | cis | 4-$CH_3O$-$C_6H_4$- |
| $C_6H_5$-$(CH_2)_3$- | H | 4-$CH_3O$-$C_6H_4$- | trans | 3-$NH_2$-$C_6H_4$- |
| $C_6H_5$-$(CH_2)_3$- | H | 4-OH-$C_6H_4$- | trans | 4-$CH_3O$-$C_6H_4$- |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-$CH_3O$-$C_6H_4$- | 3R,4S | 4-OH-$C_6H_4$- |

| | | | | |
|---|---|---|---|---|
| $C_6H_5-(CH_2)_3-$ | H | 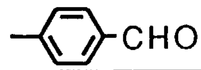 | trans | $4-CH_3O-C_6H_4-$ |
| $C_6H_5-(CH_2)_3-$ | H | $4-CH_3O-C_6H_4-$ | trans | $4-(CH_3SO_2)-C_6H_4-$ |
| $C_6H_5-(CH_2)_3-$ | H | 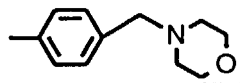 | trans | $4-CH_3O-C_6H_4-$ |
| $C_6H_5-(CH_2)_3-$ | H | 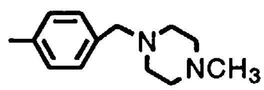 | trans | $4-CH_3O-C_6H_4-$ |
| $C_6H_5-(CH_2)_3-$ | H | $4-(CH_3OCH_2)-C_6H_4-$ | trans | $4-CH_3O-C_6H_4-$ |
| $C_6H_5-(CH_2)_3-$ | H | 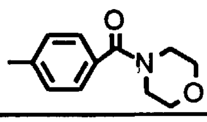 | cis | $4-CH_3O-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 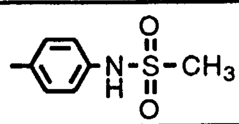 | trans | $4-CH_3O-C_6H_4-$ |
| $C_6H_5-(CH_2)_3-$ | H | 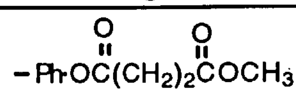 Wherein Ph is 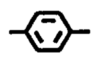 | trans | $4-CH_3O-C_6H_4-$ |
| $C_6H_5-(CH_2)_3-$ | H | $-Ph-OCH_2CO_2Et$ Wherein Ph is 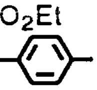 and Et is $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 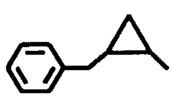 | H | $4-CH_3O-C_6H_4-$ | cis | $4-CH_3O-C_6H_4-$ |

**17.** A process of claim 1 wherein the compound prepared is (3R,4S)-1,4-bis-(4-methoxyphenyl)-3-(3-phenylpropyl)-2-azetidinone.

**18.** A process of claim 15, wherein the compound used is selected from the group consisting of compounds of the formula

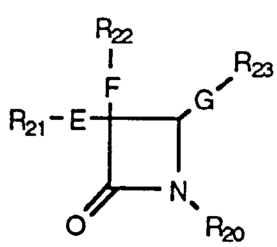

wherein $R_{21}$-E-, $R_{22}$-F-, $R_{23}$-G-, $R_{20}$ and relative stereochemistry between $R_{21}$-E- and $R_{23}$-G- are as defined in the following table:

| $R_{21}$-E- | $R_{22}$-F- | $R_{23}$-G- | Rel. Stereo-chem. | $R_{20}$ |
|---|---|---|---|---|
| $C_6H_5-(CH_2)_3-$ | H | $p-CH_3O-C_6H_4-$ | 3S,4S | $p-CH_3O-C_6H_4-$ |
| $C_6H_5-(CH_2)_3-$ | H | $p-OH-C_6H_4-$ | 3S,4S | $p-CH_3O-C_6H_4-$ |
| $C_6H_5-(CH_2)_3-$ | H | $p-CH_3O-C_6H_4-$ | trans | $p-CF_3O-C_6H_4-$ |
| $C_6H_5-(CH_2)_3-$ | H | $4-CH_3O-C_6H_4-$ | cis | $p-Cl-C_6H_4-$ |
| $C_6H_5-(CH_2)_3-$ | H | $4-CH_3O-C_6H_4-$ | cis | $p-CH_3-C_6H_4-$ |
| $C_6H_5-(CH_2)_3-$ | H | $4-((CH_3)_2CH-O)-C_6H_4-$ | cis | $p-CH_3O-C_6H_4-$ |
| $C_6H_5-(CH_2)_3-$ | H | $4-(CH_3(CH)_2-O)-C_6H_4-$ | cis | $p-CH_3O-C_6H_4-$ |
| $C_6H_5-(CH_2)_3-$ | H | $CH_3CH_2-O-C_6H_4-$ | cis | $p-CH_3O-C_6H_4-$ |
| $C_6H_5-(CH_2)_3-$ | H | $CH_3(CH_2)_3-O-C_6H_4-$ | cis | $p-CH_3O-C_6H_4-$ |
| $C_6H_5-(CH_2)_3-$ | H | $4-(CH_2=CH-CH_2-O)-C_6H_4-$ | cis | $p-CH_3O-C_6H_4-$ |
| $C_6H_5-(CH_2)_3-$ | H | $4-(CH_3CH_2-O)-C_6H_4-$ | cis | $C_6H_5-$ |
| $4-F-C_6H_4-O-(CH_2)_2-$ | H | $4-CH_3O-C_6H_4-$ | cis | $C_6H_5-$ |
| $C_6H_5-(CH_2)_3-$ | H | $4-CH_3O-C_6H_4-$ | cis | $C_6H_5-$ |
| $C_6H_5-(CH_2)_3-$ | H | $4-CH_3O-C_6H_4-$ | 3S,4S | $p-CH_3O-C_6H_4-$ |
| $C_6H_5-(CH_2)_3-$ | H | $p-CH_3O-C_6H_4-$ | 3S,4R | $p-CH_3O-C_6H_4-$ |
| $C_6H_5-(CH_2)_3-$ | H | $4-CH_3O-C_6H_4-$ | trans | $4-F-C_6H_4-$ |
| $C_6H_5-(CH_2)_3-$ | H | $4-CH_3O-C_6H_4-$ | trans | $3-CH_3O-C_6H_4-$ |
| $C_6H_5-(CH_2)_3-$ | H | $4-CH_3O-C_6H_4-$ | trans | $4-Cl-C_6H_4-$ |
| $C_6H_5-(CH_2)_3-$ | H | $4-CH_3O-C_6H_4-$ | trans | $4-(CH_3CH_2-O)-C_6H_4-$ |
| $C_6H_5-(CH_2)_3-$ | H | $4-CH_3O-C_6H_4-$ | trans | $4-CH_3S-C_6H_4-$ |
| $C_6H_5-(CH_2)_3-$ | H | $4-CH_3O-C_6H_4-$ | trans | $4-CH_3-C_6H_4-$ |
| 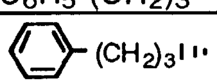 | H | 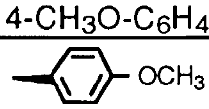 | trans optically pure | $C_6H_5-$ |
| $C_6H_5-(CH_2)_3-$ | H | $4-(CH_3CH_2-O)-C_6H_4-$ | trans | $4-CH_3O-C_6H_4-$ |
| $C_6H_5-(CH_2)_3-$ | H | $4-(CH_2=CH-CH_2-O)-C_6H_4-$ | trans | $4-CH_3O-C_6H_4-$ |
| $C_6H_5-(CH_2)_3-$ | H | $4-(C_6H_5-O)-C_6H_4-$ | trans | $4-CH_3O-C_6H_4-$ |
| $4-F-C_6H_4-O-(CH_2)_2-$ | H | $4-CH_3O-C_6H_4-$ | trans | $4-CH_3O-C_6H_4-$ |
| $C_6H_5-(CH_2)_4-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $C_6H_5-$ |

| | | | | |
|---|---|---|---|---|
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 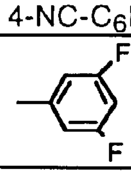 |
| $C_6H_5-(CH_2)_3-$ | H | $4-CH_3O-C_6H_4-$ | trans | $4-(CH_3C(O))-C_6H_4-$ |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 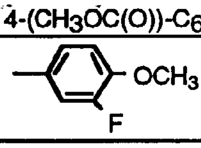 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | 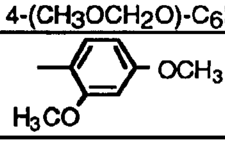 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $C_6H_5-(CH_2)_3-$ | H | $4-CH_3O-C_6H_4-$ | trans | $4-I-C_6H_4-$ |
| 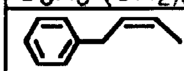 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 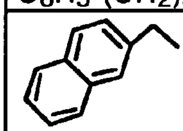 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 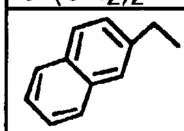 | H | $4-CH_3O-C_6H_4-$ | cis | $C_6H_5-$ |
| $C_6H_5-(CH_2)_3-$ | H | $4-CH_3O-C_6H_4-$ | trans | 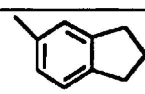 |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | $-Ph-O\overset{O}{\overset{\|}{C}}(CH_2)_2\overset{O}{\overset{\|}{C}}OCH_3$ Wherein Ph is 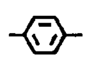 | trans | $4-CH_3O-C_6H_4-$ |

| | | | | |
|---|---|---|---|---|
| $C_6H_5-(CH_2)_3-$ | H | -Ph-$OCH_2CO_2Et$ Wherein Ph is 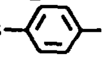 and Et is $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 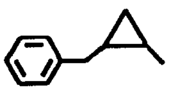 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 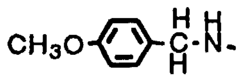 | H | $4-CH_3O-C_6H_4-$ | cis | $C_6H_5-$ |

**19.** The method of use of a compound defined in claim 1 for the manufacture of a medicament for lowering serum cholesterol.

**20.** The method of use of a compound defined in claim 1 for the manufacture of a medicament for inhibiting the enzyme acyl CoA: cholesterol acyl transferase.

**21.** The method of use of a composition defined in claim 15 for the manufacture of a medicament for lowering serum cholesterol levels in a mammal in need of such treatment.

**22.** The method of use of a composition defined in claim 15 for the manufacture of a medicament for inhibiting the enzyme acyl CoA:cholesterol acyl transferase.

**Patentansprüche**

**Patentansprüche für folgende Vertragsstaaten : AT, BE, CH, LI, DE, DK, FR, GB, IT, LU, MC, NL, SE, PT**

1. Verbindung mit der Strukturformel

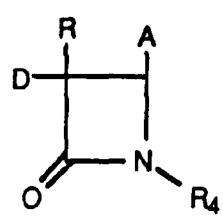

worin

A -CH=CH-B;
-C≡C-B;
$-(CH_2)_p-X-B$, worin p 0, 1 oder 2 ist und X eine Bindung, -NH- oder $-S(O)_{0-2}-$ ist;
Heteroaryl, benzokondensiertes Heteroaryl, W-substituiertes Heteroaryl oder W-substituiertes benzokondensiertes Heteroaryl, worin Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroaryl-Gruppen deren N-Oxide, und wobei W 1-3 Substituenten an den Ringkohlenstoffatomen ist, die aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino) -$C_{1-6}$-alkyl, $C_{3-6}$-Alkandioyl, $C_{1-6}$-Alkyl-$C_{3-6}$-alkandioyl, Allyloxy, -$CF_3$, -$OCF_3$, Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$ ($C_{1-6}$-Alkyl)-, $NR_{10}R_{11}$ ($C_{1-6}$-Alkoxy)-, OH, Halogen, -NHC(O)$OR_5$, -NHC(O)$R_5$, $R_6O_2$SNH-, $(R_6O_2S)_2$N-, -$S(O)_2NH_2$, -$S(O)_{0-2}R_{10}$, tert-Butyldimethylsilyloxymethyl, -C(O)$R_{12}$ und

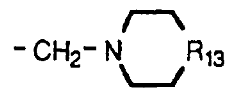

besteht und wobei,
sofern vorhanden, die Substituenten an den substituierten Heteroaryl-Stickstoffatomen aus der Gruppe ausgewählt sind,
die aus $C_{1-6}$-Alkyl, $C_{1-6}$-Alkoxy, -C(O)$OR_5$, -C(O)$R_5$, OH, $NR_{10}R_{11}$($C_{1-6}$)-, $NR_{10}R_{11}$ ($C_{1-6}$-Alkoxy)-, -$S(O)_2NH_2$ und 2-(Trimethylsilyl)ethoxymethyl besteht;
-C(O)-B oder

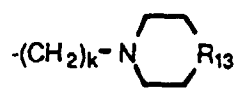

ist, worin k 1 oder 2 ist;

D

B'-$(CH_2)_m$C(O)-, worin m 1, 2, 3, 4 oder 5 ist;
B'-$(CH_2)_q$-, worin q 2, 3, 4, 5 oder 6 ist;
B'-$(CH_2)_e$-Z-$(CH_2)_r$-, t worin Z -O-, -C(O)-, Phenylen, -$NR_8$- oder -$S(O)_{0-2}$- ist, e 0, 1, 2, 3, 4 oder 5 ist und r 1, 2, 3, 4 oder 5 ist, vorausgesetzt, daß die Summe von e und r 1, 2, 3, 4, 5 oder 6 ist;
B'-($C_{2-6}$-Alkenylen)-, B'-($C_4$-$C_6$-Alkadienylen)-;
B'-$(CH_2)_t$-Z- ($C_2$-$C_6$-Alkenylen)-, worin Z wie vorstehend definiert ist und worin t 0, 1, 2 oder 3 ist, vorausgesetzt, daß die Summe von t und der Zahl der Kohlenstoffatome in der Alkylenkette 2, 3, 4, 5 oder 6 ist;
B'-$(CH_2)_f$-V- $(CH_2)_g$-, worin V $C_3$-$C_6$-Cycloalkylen ist, f 1, 2, 3, 4 oder 5 ist und g 0, 1, 2, 3, 4 oder 5 ist, vorausgesetzt, daß die Summe von f und g 1, 2, 3, 4, 5 oder 6 ist;
B'-$(CH_2)_t$-V-($C_2$-$C_6$-Alkenylen)- oder B'-($C_2$-$C_6$-Alkenylen)-V-$(CH_2)_t$-, worin V und t wie vorstehend definiert sind, vorausgesetzt, daß die Summe von t und der Zahl der Kohlenstoffatome in der Alkenylenkette 2, 3, 4, 5 oder 6 ist;
B'-$(CH_2)_a$-Z-$(CH_2)_b$-V-$(CH_2)_d$-, worin Z und V wie vorstehend definiert sind und a, b und d unabhängig 0, 1, 2, 3, 4, 5 oder 6 sind, vorausgesetzt, daß die Summe von a, b und d 0, 1, 2, 3, 4, 5 oder 6 ist;
T-$(CH_2)_s$-, worin T Cycloalkyl mit 3-6 Kohlenstoffatomen ist und s 1, 2, 3, 4, 5 oder 6 ist, oder
Naphthylmethyl, Heteroarylmethyl oder W-substituiertes Heteroarylmethyl ist, worin Heteroaryl und W wie vorstehend definiert sind;

B

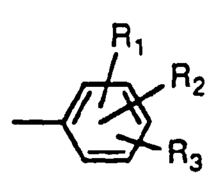

ist;

B' Naphthyl, Heteroaryl oder W-substituiertes Heteroaryl, worin Heteroaryl wie vorstehend definiert ist, oder

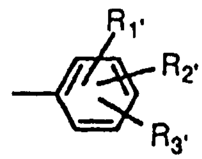

ist;

R Wasserstoff, Fluor, $C_1$-$C_{15}$-Alkyl, $C_2$-$C_{15}$-Alkenyl, $C_2$-$C_{15}$-Alkinyl oder B-$(CH_2)_h$- ist, worin h 0, 1, 2 oder 3 ist;

$R_1$, $R_2$ und $R_3$ unabhängig aus der Gruppe ausgewählt sind, die aus H, $C_{1\text{-}6}$-Alkyl, Hydroxy-$C_{1\text{-}6}$-alkyl, $C_{1\text{-}6}$-Alkoxy, $C_{1\text{-}6}$-Alkoxy-$C_{1\text{-}6}$-alkyl, $C_{1\text{-}6}$-Alkoxy-$C_{1\text{-}6}$-alkoxy, $C_{1\text{-}6}$-Alkoxycarbonyl-$C_{1\text{-}6}$-alkoxy, ($C_{1\text{-}6}$-Alkoxyimino) -$C_{1\text{-}6}$-alkyl, $C_{3\text{-}6}$-Alkandioyl, $C_{1\text{-}6}$-Alkyl-$C_{3\text{-}6}$-alkandioyl, Allyloxy, -$CF_3$, -$OCF_3$, Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$ ($C_{1\text{-}6}$-Alkyl)-, $NR_{10}R_{11}$ ($C_{1\text{-}6}$-Alkoxy)-, OH, o-Halogen, m-Halogen, -NHC(O)$OR_5$, -NHC(O)$R_5$, $R_6O_2SNH$-, $(R_6O_2S)_2N$-, -$S(O)_2NH_2$, -$S(O)_{0\text{-}2}R_{10}$, tert-Butyldimethylsilyloxymethyl, -C(O)$R_{12}$

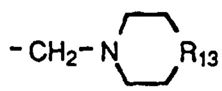 und 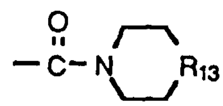

besteht,
oder $R_1$ Wasserstoff ist und $R_2$ und $R_3$ zusammen mit den benachbarten Kohlenstoffatomen, an die sie gebunden sind, einen Dioxolanylring bilden;

$R_{1'}$, $R_{2'}$ und $R_{3'}$ unabhängig aus der Gruppe ausgewählt sind, die aus H, $C_{1\text{-}6}$-Alkyl, Hydroxy-$C_{1\text{-}6}$-alkyl, $C_{1\text{-}6}$-Alkoxy, $C_{1\text{-}6}$-Alkoxy-$C_{1\text{-}6}$-alkyl, $C_{1\text{-}6}$-Alkoxy-$C_{1\text{-}6}$-alkoxy, $C_{1\text{-}6}$-Alkoxycarbonyl-$C_{1\text{-}6}$-alkoxy, ($C_{1\text{-}6}$-Alkoxyimino)-$C_{1\text{-}6}$-alkyl, $C_{3\text{-}6}$-Alkandioyl, $C_{1\text{-}6}$-Alkyl-$C_{3\text{-}6}$-alkandioyl, Allyloxy, -$CF_3$, -$OCF_3$, Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$ ($C_{1\text{-}6}$-Alkyl)-, $NR_{10}R_{11}$ ($C_{1\text{-}6}$-Alkoxy)-, OH, Halogen, -NHC(O)$OR_5$, -NHC(O)$R_5$, $R_6O_2SNH$-, $(R_6O_2S)_2N$-, -$S(O)_2NH_2$, -$S(O)_{0\text{-}2}R_{10}$, tert-Butyldimethylsilyloxymethyl, -C(O)$R_{12}$,

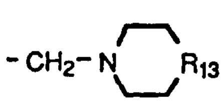 und 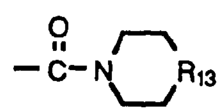

besteht, oder $R_{1'}$ Wasserstoff ist und $R_{2'}$ und $R_{3'}$ zusammen mit den benachbarten Kohlenstoffatomen, an die sie gebunden sind, einen Dioxolanylring bilden;

$R_4$ 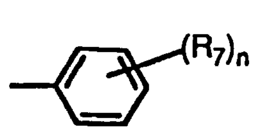 ,

worin n 0, 1, 2 oder 3 ist, Indanyl, Benzofuranyl, Benzodioxolyl, Tetrahydronaphthyl, Pyridyl, Pyrazinyl, Pyrimidinyl oder Chinolyl ist;

$R_5$ $C_{1-6}$-Alkyl, Phenyl, $R_{14}$-Phenyl, Benzyl oder $R_{14}$-Benzyl ist;

$R_6$ OH, $C_{1-6}$-Alkyl, Phenyl, Benzyl, $R_{14}$-Phenyl oder $R_{14}$-Benzyl ist;

$R_7$ $C_{1-6}$-Alkyl, $C_{1-6}$-Alkoxy, OH, Halogen, $-NR_{10}R_{11}$, $-NHC(O)OR_5$, $-NHC(O)R_5$, $NO_2$, -CN, $-N_3$, -SH, $-S(O)_{0-2}-(C_{1-6}$-Alkyl), $-COOR_9$, $-CONR_{10}R_{11}$, $-COR_{12}$, Phenoxy, Benzyloxy, $-OCF_3$ oder tert-Butyldimethylsilyloxy ist und wenn n 2 oder 3 ist, die Gruppen $R_7$ gleich oder verschieden sein können;

$R_8$ H, $C_{1-6}$-Alkyl, Phenyl-$C_{1-6}$-alkyl oder $-C(O)R_9$ ist;

$R_9$ H, $C_{1-6}$-Alkyl oder Phenyl-$C_{1-6}$-alkyl ist;

$R_{10}$ und $R_{11}$ unabhängig aus H und $C_{1-6}$-Alkyl ausgewählt sind;

$R_{12}$ H, OH, $C_{1-6}$-Alkoxy, Phenoxy, Benzyloxy,

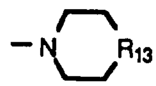

$-NR_{10}R_{11}$, $C_{1-6}$-Alkyl, Phenyl oder $R_{14}$-Phenyl ist;

$R_{13}$ -O-, $-CH_2-$, -NH- oder $-N(C_{1-6}$-Alkyl)- ist und

$R_{14}$ 1-3 Gruppen ist, die unabhängig aus der Gruppe ausgewählt sind, die aus $C_{1-6}$-Alkyl, $C_{1-6}$-Alkoxy, -COOH, $NO_2$, $-NR_{10}R_{11}$, OH oder Halogen besteht,

oder ein pharmazeutisch annehmbares Salz derselben.

**2.** Verbindung von Anspruch 1, wobei R Wasserstoff ist.

**3.** Verbindung von Anspruch 1 oder 2, wobei D $B'-(CH_2)_q-$, $B'-(CH_2)_e-Z-(CH_2)_r-$, $B'-(C_2-C_6$-Alkenylen) oder $B'-(CH_2)_f-V-(CH_2)_g-$ ist, worin B', Z, V, q, e, r, f und g wie in Anspruch 1 definiert sind.

**4.** Verbindung von Anspruch 3, wobei D $B'-(CH_2)_q-$, worin B' Phenyl ist und q 3 oder 4 ist; $B'-(CH_2)_e-Z-(CH_2)_r-$, worin B' p-Fluorphenyl oder p-Methoxyphenyl ist, e Null ist, Z -O- ist und r 2 ist; $B'-(C_2-C_6$-Alkenylen)- 3-Phenyl-1-propenyl ist oder $B'-(CH_2)_f-V-(CH_2)_g-$ ist, worin B' Phenyl ist, f 1 ist, V Cyclopropylen ist und g null ist.

**5.** Verbindung von Anspruch 1, 2, 3 oder 4, wobei A $-(CH_2)_p-X-B$ ist, worin X, B und p wie in Anspruch 1 definiert sind.

**6.** Verbindung von Anspruch 5, wobei p null ist und X eine Bindung ist.

**7.** Verbindung von Anspruch 6, wobei $R_1$, $R_2$ und $R_3$ aus der Gruppe ausgewählt sind, die aus H, OH, $C_{1-6}$-Alkoxy, $NO_2$, $C_{1-6}$-Alkoxy-$C_{1-6}$-alkoxy, m-Halogen, $C_{1-6}$-Alkyl-$C_{3-6}$-alkandioyl, $NR_{10}R_{11}(C_{1-6}$-Alkoxy)-, Allyloxy, Phenoxy, $C_{1-6}$-Alkoxycarbonyl-$C_{1-6}$-alkoxy und $-C(O)R_{12}$ besteht.

**8.** Verbindung von Anspruch 1, 2, 3, 4, 5 oder 6, wobei $R_4$ Phenyl, $R_7$-substituiertes Phenyl oder Indanyl ist.

**9.** Verbindung von Anspruch 8, wobei $R_7$ aus der Gruppe ausgewählt ist, die aus $C_{1-6}$-Alkyl, $C_{1-6}$-Alkoxy, Halogen, $-OCF_3$, $C_{1-6}$-Alkylthio, $-NR_{10}R_{11}$, -CN, OH und $-COR_{12}$ besteht.

**10.** Verwendung einer pharmazeutischen Zusammensetzung zur Herstellung eines Arzneimittels zum Erniedrigen von Serumcholesterin, wobei die Zusammensetzung eine Verbindung mit der Struktur

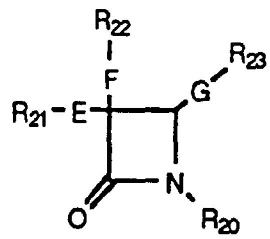

worin

$R_{20}$ Phenyl, W-substituiertes Phenyl, Naphthyl, W-substituiertes Naphthyl, Benzodioxolyl, Heteroaryl, W-substituiertes Heteroaryl, benzokondensiertes Heteroaryl und W-substituiertes benzokondensiertes Heteroaryl, wobei Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroaryl-Gruppen deren N-Oxid ist;

$R_{21}$, $R_{22}$ und $R_{23}$ unabhängig aus H oder $R_{20}$ ausgewählt sind;

W 1 bis 3 Substituenten ist, die unabhängig aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino) -$C_{1-6}$-alkyl, $C_{3-6}$-Alkandioyl, $C_{1-6}$-Alkyl-$C_{3-6}$-alkandioyl, Allyloxy, -$CF_3$, -$OCF_3$, Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$ ($C_{1-6}$-Alkyl)-, $NR_{10}R_{11}$ ($C_{1-6}$-Alkoxy)-, OH, Halogen, -NHC(O)$OR_5$, -NHC(O)$R_5$, $R_6O_2$SNH-, $(R_6O_2S)_2$N-, -S(O)$_2$NH$_2$, -S(O)$_{0-2}$R$_{10}$, tert-Butyldimethylsilyloxymethyl, -C(O)$R_{12}$,

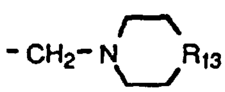

und

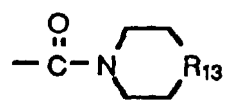

besteht;

E, F und G unabhängig eine Bindung, $C_{3-6}$-Cycloalkylen, $C_1$-$C_{10}$-Alkylen, $C_2$-$C_{10}$-Alkenylen, $C_2$-$C_{10}$-Alkinylen, eine definierte Alkylen-, Alkenylen- oder Alkinylenkette, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, wobei Heteroaryl wie vorstehend definiert ist, eine definierte Alkylen-, Alkenylen- oder Alkinylenkette, die durch eine oder mehr Gruppen unterbrochen ist, die unabhängig aus der Gruppe ausgewählt sind, die aus -O-, -S-, -SO-, -$SO_2$-, -$NR_8$, -C(O)-, $C_3$-$C_6$-Cycloalkylen, Phenylen, W-substituiertem Phenylen, Heteroarylen und W-substituiertem Heteroarylen besteht, oder eine definierte unterbrochene Alkylen-, Alkenylen- oder Alkinylengruppe sind, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, oder eines von $R_{21}$-E und $R_{22}$-F aus der Gruppe ausgewählt ist, die aus Halogen, OH, $C_{1-6}$-Alkoxy, -OC(O)$R_5$, -$NR_{10}R_{11}$, -SH oder -S($C_{1-6}$-Alkyl) besteht;

$R_5$ $C_{1-6}$-Alkyl, Phenyl, $R_{14}$-Phenyl, Benzyl oder $R_{14}$-Benzyl ist;

$R_6$ OH, $C_{1-6}$-Alkyl, Phenyl, Benzyl, $R_{14}$-Phenyl oder $R_{14}$-Benzyl ist;

$R_8$ H, $C_{1-6}$-Alkyl, Phenyl-$C_{1-6}$-alkyl oder -C(O)$R_9$ ist;

$R_9$ H, $C_{1-6}$-Alkyl, Phenyl oder Phenyl-$C_{1-6}$-alkyl ist;

$R_{10}$ und $R_{11}$ unabhängig aus H und $C_{1-6}$-Alkyl ausgewählt sind;

$R_{12}$ H, OH, $C_{1-6}$-Alkoxy, Phenoxy, Benzyloxy,

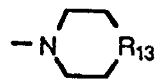,

$-NR_{10}R_{11}$, $C_{1-6}$-Alkyl, Phenyl oder $R_{14}$-Phenyl ist;

$R_{13}$ -O-, $-CH_2-$, -NH- oder -N($C_{1-6}$-Alkyl)- ist;

$R_{14}$ 1-3 Gruppen ist, die unabhängig aus der Gruppe ausgewählt sind, die aus $C_{1-6}$-Alkyl, $C_{1-6}$-Alkoxy, -COOH, $NO_2$, $-NR_{10}R_{11}$, OH oder Halogen besteht;
vorausgesetzt, daß wenn G eine Bindung ist, $R_{23}$ nicht H ist und vorausgesetzt, daß wenn $R_{23}$ W-substituiertes Phenyl ist, W nicht p-Halogen ist,
oder ein pharmazeutisch annehmbares Salz derselben in einem pharmazeutisch annehmbaren Träger umfaßt.

**11.** Verbindung von Anspruch 1, die aus der Gruppe ausgewählt ist, die aus Verbindungen der Formel

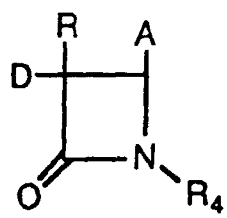

besteht, worin D, R, A und $R_4$ und die relative Stereochemie zwischen D und A wie in der folqenden Tabelle definiert sind:

| D | R | A | relative Stereochemie | $R_4$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-$NO_2$-$C_6H_4$- | 3R,4R | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | 3-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 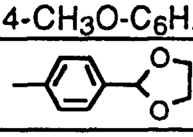 | trans | p-$CH_3O$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | 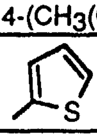 | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($C_6H_5$-O)-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 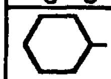 $(CH_2)_3$– | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-($(CH_3)_2N$-$(CH_2)_3$-O)-$C_6H_4$- | cis | 4-$CH_3O$-$C_6H_4$- |

| | | | | |
|---|---|---|---|---|
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| $C_6H_5$-$(CH_2)_3$- | H | 4-$CH_3O$-$C_6H_4$- | trans | 4-($C_6H_5$-O)-$C_6H_4$- |
| 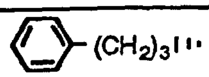 | H | 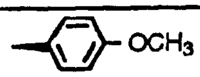 | trans optisch rein | $C_6H_5$- |
| 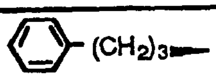 | H | 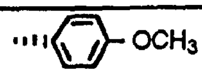 | trans optisch rein | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($C_6H_5$-$CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| $C_6H_5$-O-$(CH_2)_2$- | H | 4-$CH_3O$-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |
| $C_6H_5$-$(CH_2)_3$- | H | 4-$CH_3O$-$C_6H_4$- | trans | 4-($CH_3CH_2OC(O)$)-$C_6H_4$- |
| $C_6H_5$-$(CH_2)_3$- | H | 4-$CH_3O$-$C_6H_4$- | trans | 3-$NO_2$-$C_6H_4$- |
| $C_6H_5$-$(CH_2)_3$- | H | 4-$CH_3O$-$C_6H_4$- | trans | 4-($(CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 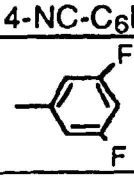 |
| $C_6H_5$-$(CH_2)_3$- | H | 4-$CH_3O$-$C_6H_4$- | trans | 4-($CH_3C(O)$)-$C_6H_4$- |

| | | | | |
|---|---|---|---|---|
| $C_6H_5-(CH_2)_3-$ | H | $4-CH_3O-C_6H_4-$ | trans | 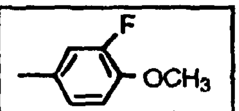 |
| $C_6H_5-(CH_2)_3-$ | H | $4-CH_3O-C_6H_4-$ | trans | 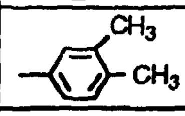 |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 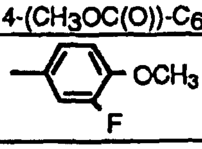 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | $4-((CH_3)_2N)-C_6H_4-$ | trans | $4-CH_3O-C_6H_4-$ |
| $C_6H_5-(CH_2)_3-$ | H | 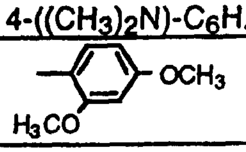 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $4-O_2N-C_6H_4-(CH_2)_3-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $4-I-C_6H_4-$ |
| $C_6H_5-(CH_2)_3-$ | H | $4-(CH_3(CH_2)_3-O)-C_6H_4-$ | trans | $4-CH_3O-C_6H_4-$ |
| 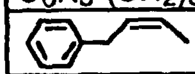 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 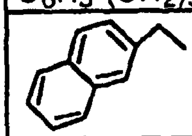 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 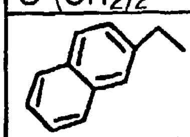 | H | $4-CH_3O-C_6H_4-$ | cis | $C_6H_5-$ |
| $C_6H_5-(CH_2)_3-$ | H | $4-CH_3O-C_6H_4-$ | trans | 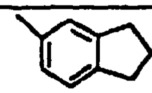 |
| $C_6H_5-(CH_2)_3-$ | H | $4-NH_2-C_6H_4-$ | cis | $4-CH_3O-C_6H_4-$ |
| $C_6H_5-(CH_2)_3-$ | H | $4-CH_3O-C_6H_4-$ | trans | $3-NH_2-C_6H_4-$ |
| $C_6H_5-(CH_2)_3-$ | H | $4-OH-C_6H_4-$ | trans | $4-CH_3O-C_6H_4-$ |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-CH_3O-C_6H_4-$ | 3R,4S | $4-OH-C_6H_4-$ |

| | | | | |
|---|---|---|---|---|
| $C_6H_5-(CH_2)_3-$ | H | 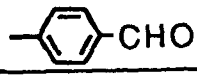 | trans | $4-CH_3O-C_6H_4-$ |
| $C_6H_5-(CH_2)_3-$ | H | $4-CH_3O-C_6H_4-$ | trans | $4-(CH_3SO_2)-C_6H_4-$ |
| $C_6H_5-(CH_2)_3-$ | H | 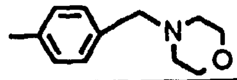 | trans | $4-CH_3O-C_6H_4-$ |
| $C_6H_5-(CH_2)_3-$ | H | 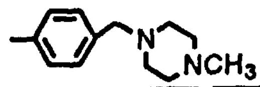 | trans | $4-CH_3O-C_6H_4-$ |
| $C_6H_5-(CH_2)_3-$ | H | $4-(CH_3OCH_2)-C_6H_4-$ | trans | $4-CH_3O-C_6H_4-$ |
| $C_6H_5-(CH_2)_3-$ | H | 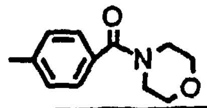 | cis | $4-CH_3O-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 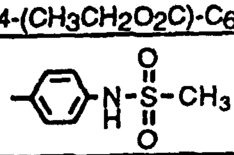 | trans | $4-CH_3O-C_6H_4-$ |
| $C_6H_5-(CH_2)_3-$ | H | 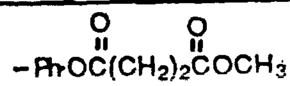 worin 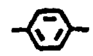 ist | trans | $4-CH_3O-C_6H_4-$ |
| $C_6H_5-(CH_2)_3-$ | H | $-Ph-OCH_2CO_2Et$ worin 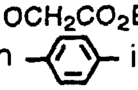 ist und Et $-CH_2CH_3$ ist | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 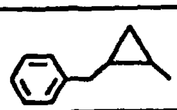 | H | $4-CH_3O-C_6H_4-$ | cis | $4-CH_3O-C_6H_4-$ |

**12.** Verbindung von Anspruch 1 oder 11, die (3R,4S)-1,4-Bis-(4-methoxyphenyl)-3-(3-(phenylpropyl)-2-azetidinon ist.

**13.** Verwendung von Anspruch 10, wobei die Verbindung aus der Gruppe ausgewählt ist, die aus Verbindungen der Formel

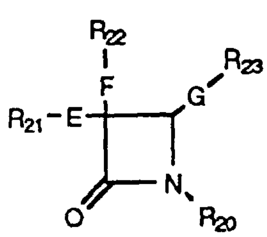

besteht, wobei $R_{21}$-E-, $R_{22}$-F-, $R_{23}$-G-, $R_{20}$ und die relative Stereochemie zwischen $R_{21}$-E- und $R_{23}$-G- wie in der folgenden Tabelle definiert sind:

| $R_{21}$-E- | $R_{22}$-F- | $R_{23}$-G- | rel. Stereo-chem. | $R_{20}$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| 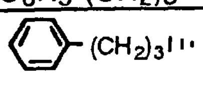 | H | 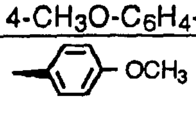 | trans optisch rein | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |

| | | | | |
|---|---|---|---|---|
| $C_6H_5$-$(CH_2)_3$- | H | 4-$CH_3O$-$C_6H_4$- | trans | 4-(($CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 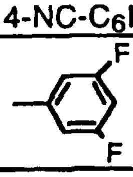 |
| $C_6H_5$-$(CH_2)_3$- | H | 4-$CH_3O$-$C_6H_4$- | trans | 4-($CH_3C(O)$)-$C_6H_4$- |
| $C_6H_5$-$(CH_2)_3$- | H | 4-(($CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 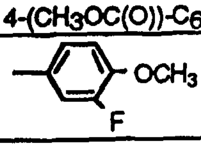 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 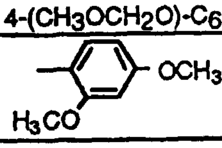 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| $C_6H_5$-$(CH_2)_3$- | H | 4-$CH_3O$-$C_6H_4$- | trans | 4-I-$C_6H_4$- |
| 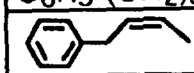 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 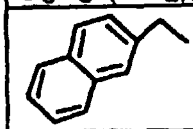 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 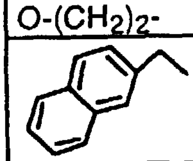 | H | 4-$CH_3O$-$C_6H_4$- | cis | $C_6H_5$- |
| $C_6H_5$-$(CH_2)_3$- | H | 4-$CH_3O$-$C_6H_4$- | trans | 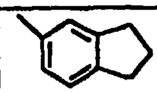 |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-($CH_3CH_2O_2C$)-$C_6H_4$- | trans | 4-$CH_3O$-$C_6H_4$- |
| $C_6H_5$-$(CH_2)_3$- | H | $-Ph-O\overset{O}{\overset{\|}{C}}(CH_2)_2\overset{O}{\overset{\|}{C}}OCH_3$ worin Ph 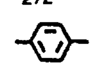 ist | trans | 4-$CH_3O$-$C_6H_4$- |

| | | | | |
|---|---|---|---|---|
| $C_6H_5-(CH_2)_3-$ | H | $-Ph-OCH_2CO_2Et$ worin Ph 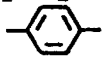 ist und Et $-CH_2CH_3$ ist | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 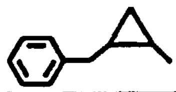 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 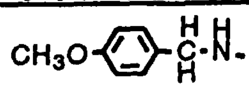 | H | $4-CH_3O-C_6H_4-$ | cis | $C_6H_5-$ |

**14.** Verwendung einer Verbindung von Anspruch 1 zur Herstellung eines Arzneimittels zum Erniedrigen des Serumcholesterins.

**15.** Verwendung einer Verbindung von Anspruch 1 zur Herstellung eines Arzneimittels zum Hemmen des Enzyms Acyl-CoA-Cholesterin-Acyltransferase.

**16.** Verfahren zur Herstellung einer in Anspruch 10 definierten pharmazeutischen Zusammensetzung, das das Vermischen einer in Anspruch 10 definierten Verbindung mit einem pharmazeutisch annehmbaren Träger umfaßt.

**17.** Verwendung einer in Anspruch 10 definierten Zusammensetzung zur Herstellung eines Arzneimittels zum Hemmen des Enzyms Acyl-CoA:Cholesterin-Acyltransferase.

**18.** Verfahren zum Herstellen einer Verbindung von Anspruch 1, wobei R H ist und D und A eine relative trans-Stereochemie besitzen, das das Cyclisieren eines Hydroxyamids der Formel

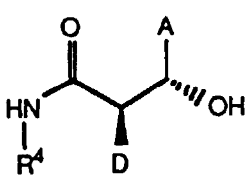 oder 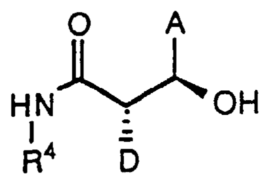

worin D, A und $R^4$ wie in Anspruch 1 definiert sind, durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder
(iii)einem Dialkylchlorphosphat, einer wäßrigen Base und einem Phasentransferkatalysator oder
(iv) einem Di- oder Trichlorbenzoylchlorid und einem Alkalimetallhydrid umfaßt.

**19.** Verfahren zum Herstellen einer Verbindung von Anspruch 1 mit der Formel

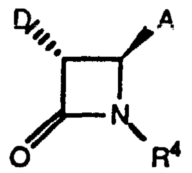 oder 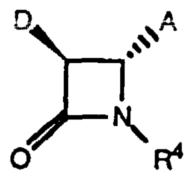

worin D, A und $R^4$ wie in Anspruch 1 definiert sind, umfassend:

(a) das Umsetzen einer Carbonsäure der Formel $DCH_2$-COOH, worin D wie vorstehend definiert ist, mit einem Chlorierungsmittel,

(b) Deprotonieren eines chiralen Oxazolidinons der Formel

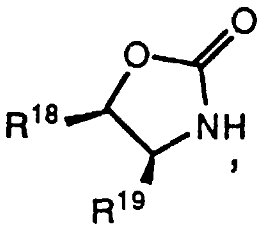 , 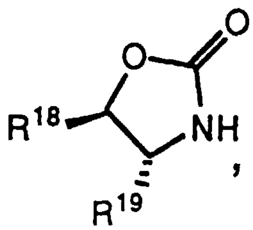 , 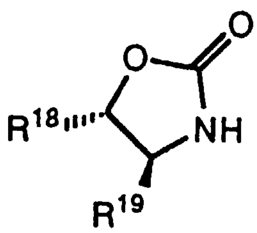 oder 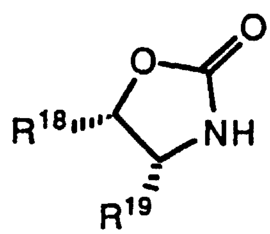

worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, mit einer starken Base und Behandeln des sich daraus ergebenden Anions mit dem Produkt aus Schritt (a),

(c) Enolisieren des Produkts aus Schritt (b) entweder mit:

(i) einem Dialkylbortriflat und einer tertiären Aminbase
(ii) $TiCl_4$ und Tetramethylethylendiamin (TMEDA) oder einem Gemisch aus TMEDA und Triethylamin,

anschließend Kondensieren mit einem Aldehyd der Formel A-CHO, worin A wie vorstehend definiert ist,

(d) Hydrolysieren des Produktes von Schritt (c) mit einer Base und Wasserstoffperoxid,

(e) Kondensieren des Produkts von Schritt (d) mit einem Amin der Formel $R^4NH_2$, worin $R^4$ wie vorstehend definiert ist, durch Behandeln mit einem dehydratisierenden Kupplungsmittel, gegebenenfalls Zusetzen eines Aktivierungsmittels und

(f) Cyclisieren des Produktes von Schritt (e) durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder
(iii)einem Dialkylchlorphosphat, einer wäßrigen Base und einem Phasentransferkatalysator oder
(iv) einem Di- oder Trichlorbenzoylchlorid und einem Alkalimetallhydrid.

**20.** Verfahren zum Herstellen einer Verbindung von Anspruch 1, wobei R Wasserstoff ist und D und A eine relative trans-Stereochemie besitzen, umfassend:

(a) das Enolisieren einer Verbindung der Formel

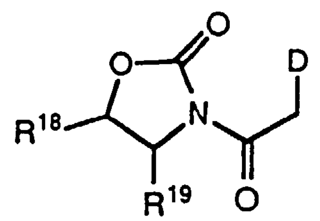

mit $TiCl_4$ und Tetramethylethylendiamin (TMEDA), anschließend Kondensieren mit einem Imin der Formel A-CH=N-$R^4$, wobei A und $R^4$ wie vorstehend definiert sind, und wobei die Gruppe

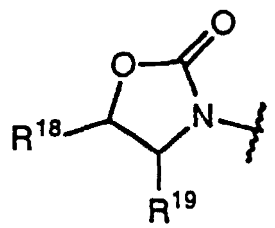

einen chiralen Hilfsstoff der Formel

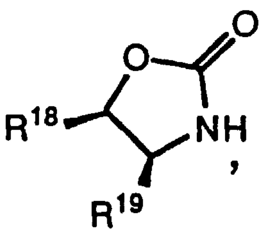 , 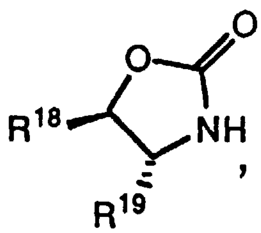 , 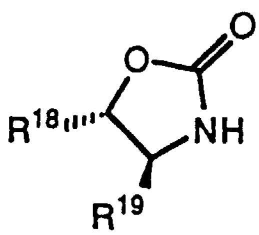 oder 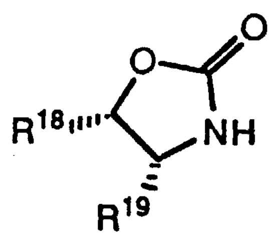

darstellt, worin $R^{18}$ und $R^{19}$ unabhängig aus der Gruppe ausgewählt sind, die aus Wasserstoff, $C_1$-$C_6$-Alkyl, Phenyl, Naphthyl, substituiertem Phenyl, substituiertem Naphthyl und Benzyl besteht, und

(b) Cyclisieren des Produkts aus Schritt (a) durch Behandeln mit einer starken nicht-nukleophilen Base.

**21.** Verfahren zum Herstellen von Verbindungen von Anspruch 1, umfassend:

(a) Behandeln eines Esters der Formel D-CH(R)-C(O)$OR^{17}$, worin $R^{17}$ $C_{1-6}$-Alkyl, Menthyl oder 10-(Diisopropylsulfonamido)isobornyl ist, mit einer starken Base und anschließend mit einem Imin der Formel A-CH=N-$R^4$, worin A und $R^4$ wie in Anspruch 1 definiert sind, oder

(b) zum Herstellen einer Verbindung von Anspruch 1, worin R nicht Wasserstoff ist, Umsetzen einer Verbindung von Anspruch 1, worin R Wasserstoff ist, mit einer starken Base und einem Alkylierungsmittel oder einem Acylierungsmittel oder

(c) zum Herstellen einer Verbindung von Anspruch 1, worin R Wasserstoff ist und D und A die relative cis-Stereochemie besitzen, Umsetzen einer Verbindung von Anspruch 1, worin R Wasserstoff ist und D und A die relative trans-Stereochemie besitzen, mit einer starken Base bei -80°C bis -40°C und anschließend mit einer Protonenquelle oder

(d) zum Herstellen einer Verbindung von Anspruch 1, wobei R Wasserstoff ist und D und A die relative trans-Stereochemie besitzen, Umsetzen einer Verbindung von Anspruch 1, worin R Wasserstoff ist und D und A die relative cis-Stereochemie besitzen, mit einem Alkalimetall-t-butoxid, anschließend Abbrechen mit Säure oder

(e) Umsetzen eines Carbonsäurederivates der Formel D-CH(R)-C(O)-Z, worin D und R wie in Anspruch 1 definiert sind und Z Cl,

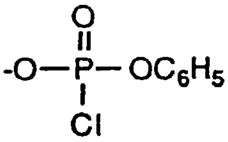

oder

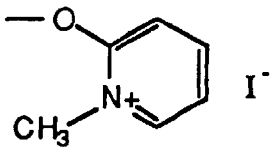

ist, mit einem Imin der Formel -A-CH=$NR^4$, worin A und $R^4$ wie in Anspruch 1 definiert sind, in Anwesenheit einer tertiären Aminbase.

**22.** Verfahren zum Herstellen einer Verbindung der Formel II

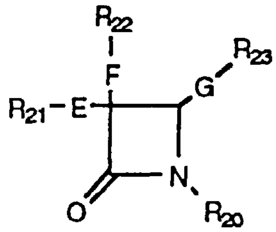

II

worin

$R_{20}$ Phenyl, W-substituiertes Phenyl, Naphthyl, W-substituiertes Naphthyl, Benzodioxolyl, Heteroaryl, W-substituiertes Heteroaryl, benzokondensiertes Heteroaryl und W-substituiertes benzokondensiertes Heteroaryl ist, wobei Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroarylgruppen das N-Oxid derselben ist;

$R_{21}$, $R_{22}$ und $R_{23}$ unabhängig aus H oder $R_{20}$ ausgewählt sind;

W 1 bis 3 Substituenten ist, die unabhängig aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino) -$C_{1-6}$-alkyl, $C_{3-6}$-Alkandioyl, $C_{1-6}$-Alkyl-$C_{3-6}$-alkandioyl, Allyloxy, -$CF_3$, -$OCF_3$ Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$($C_{1-6}$-Alkyl)-$NR_{10}R_{11}$($C_{1-6}$-Alkoxy)-, OH, Halogen, -NHC(O)$OR_5$, -NHC(O)$R_5$, $R_6O_2SNH$-, $(R_6O_2S)_2N$-, -$S(O)_2NH_2$, -S$(O)_{0-2}R_{10}$, tert-Butyldimethylsilyloxymethyl, -C(O)$R_{12}$,

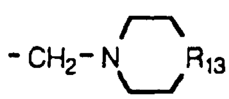

und

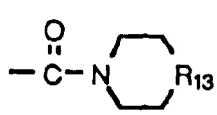

besteht;

E, F und G unabhängig eine Bindung, $C_{3-6}$-Cycloalkylen, $C_1$-$C_{10}$-Alkylen, $C_2$-$C_{10}$-Alkenylen, $C_2$-$C_{10}$-Alkinylen, eine definierte Alkylen-, Alkenylen- oder Alkinylenkette, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, wobei Heteroaryl wie vorstehend definiert ist, eine definierte Alkylen-, Alkenylen- oder Alkinylenkette, die durch eine oder mehr Gruppen unterbrochen ist, die unabhängig aus der Gruppe ausgewählt sind, die aus -O-, -S-, -SO-, $-SO_2-$, $-NR_8$, -C(O)-, $C_3$-$C_6$-Cycloalkylen, Phenylen, W-substituiertem Phenylen, Heteroarylen und W-substituiertem Heteroarylen besteht, oder eine definierte unterbrochene Alkylen-, Alkenylen- oder Alkinylengruppe sind, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, oder eines von $R_{21}$-E und $R_{22}$-F aus der Gruppe ausgewählt ist, die aus Halogen, OH, $C_{1-6}$-Alkoxy, $-OC(O)R_5$, $-NR_{10}R_{11}$, -SH oder -S($C_{1-6}$-Alkyl) besteht;

$R_5$ $C_{1-6}$-Alkyl, Phenyl, $R_{14}$-Phenyl, Benzyl oder $R_{14}$-Benzyl ist;

$R_6$ OH, $C_{1-6}$-Alkyl, Phenyl, Benzyl, $R_{14}$-Phenyl oder $R_{14}$-Benzyl ist;

$R_8$ H, $C_{1-6}$-Alkyl, Phenyl-$C_{1-6}$-alkyl oder $-C(O)R_9$ ist;

$R_9$ H, $C_{1-6}$-Alkyl, Phenyl oder Phenyl-$C_{1-6}$-alkyl ist;

$R_{10}$ und $R_{11}$ unabhängig aus H und $C_{1-6}$-Alkyl ausgewählt sind,

$R_{12}$ H, OH, $C_{1-6}$-Alkoxy, Phenoxy, Benzyloxy,

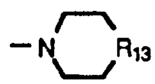 ,

$-NR_{10}R_{11}$, $C_{1-6}$-Alkyl, Phenyl oder $R_{14}$-Phenyl ist;

$R_{13}$ -O-, $-CH_2-$, -NH- oder -N($C_{1-6}$-Alkyl)- ist;

$R_{14}$ 1-3 Gruppen ist, die unabhängig aus der Gruppe ausgewählt sind, die aus $C_{1-6}$-Alkyl, $C_{1-6}$-Alkoxy, -COOH, $NO_2$, $-NR_{10}R_{11}$, OH oder Halogen besteht;
vorausgesetzt, daß wenn G eine Bindung ist, $R_{23}$ nicht H ist und vorausgesetzt, daß wenn $R_{23}$ W-substituiertes Phenyl ist, W nicht p-Halogen ist,
oder eines pharmazeutisch annehmbaren Salzes derselben, wobei $R_{22}$-F H ist und $R_{21}$-E- und $R_{23}$-G- die relative trans-Stereochemie besitzen, umfassend

Verfahren C: das Cyclisieren eines Hydroxyamids der Formel

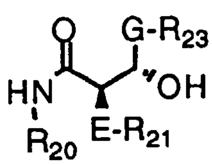 oder 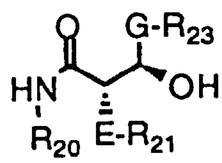

worin $R_{21}$-E-, $R_{23}$-G- und $R_{20}$ wie vorstehend definiert sind, durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder
(iii)einem Dialkylchlorphosphat, einer wäßrigen Base und einem Phasentransferkatalysator oder
(iv) einem Di- oder Trichlorbenzoylchlorid und einem Alkalimetallhydrid oder

Verfahren D:

(a) das Umsetzen einer Carbonsäure der Formel $R_{21}$-E-$CH_2$-COOH, wobei $R_{21}$-E- wie vorstehend definiert ist, mit einem Chlorierungsmittel,
(b) Deprotonieren eines chiralen Oxazolidinons der Formel

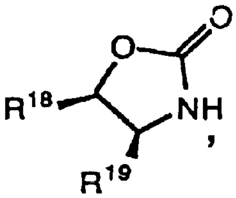 , 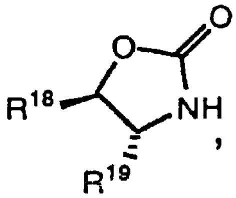 , 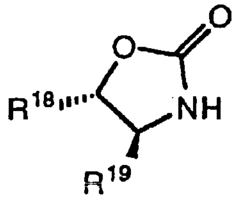 oder 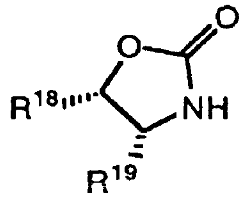

worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, mit einer starken Base und Behandeln des sich daraus ergebenden Anions mit dem Produkt aus Schritt (a),
(c) Enolisieren des Produkts aus Schritt (b) entweder mit:

(i) einem Dialkylbortriflat und einer tertiären Aminbase
(ii) $TiCl_4$ und Tetramethylethylendiamin (TMEDA) oder einem Gemisch aus TMEDA und Triethylamin,

anschließend Kondensieren mit einem Aldehyd der Formel $R_{23}$-G-CHO, worin $R_{23}$-G- wie vorstehend definiert ist,
(d) Hydrolysieren des Produktes von Schritt (c) mit einer Base und Wasserstoffperoxid,
(e) Kondensieren des Produkts von Schritt (d) mit einem Amin der Formel $R_{20}NH_2$, worin $R_{20}$ wie vorstehend definiert ist, durch Behandeln mit einem dehydratisierenden Kupplungsmittel, gegebenenfalls Zusetzen eines Aktivierungsmittels und
(f) Cyclisieren des Produktes von Schritt (e) wie in Verfahren C beschrieben.

**23.** Verfahren zum Herstellen einer Verbindung der in Anspruch 22 definierten Formel II, worin $R_{22}$-F- Wasserstoff ist und $R_{21}$-E- und $R_{23}$-G- die relative trans-Stereochemie besitzen, umfassend:

(a) Enolisieren einer Verbindung der Formel

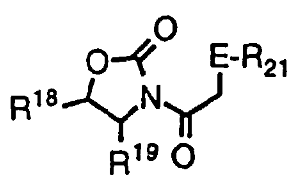

mit $TiCl_4$ und Tetramethylethylendiamin (TMEDA), anschließend Kondensieren mit einem Imin der Formel $R_{23}$-G-CH=N-$R_{20}$, worin $R_{23}$-G- und $R_{20}$ wie in Anspruch 22 definiert sind und wobei die Gruppe

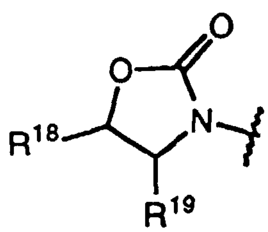

eine chirale Hilfsgruppe der Formel

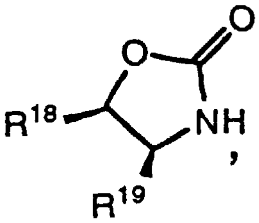,
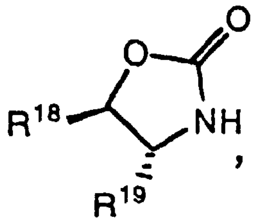,
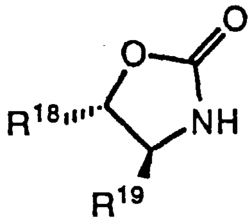 oder 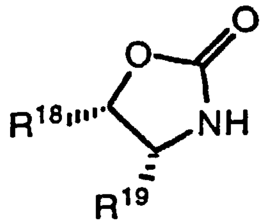

darstellt, worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, und

(b) Cyclisieren des Produktes aus Schritt (a) durch Behandeln mit einer starken nukleophilen Base.

**24.** Verfahren zum Herstellen einer Verbindung der in Anspruch 22 definierten Formel II, umfassend

(a) das Behandeln eines Esters der Formel 1 $R_{21}$-E-CH(F-$R_{22}$)-C(O)O$R^{17}$, worin $R^{17}$ $C_{1-6}$-Alkyl, Menthyl oder 10-(Diisopropylsulfonamido)isobornyl ist, mit einer starken Base und anschließend mit einem Imin der Formel $R_{23}$-G-CH=N-$R_{20}$, worin $R_{23}$-G- und $R_{20}$ wie in Anspruch 22 definiert sind, oder

(b) zum Herstellen einer Verbindung der in Anspruch 22 definierten Formel II, worin $R_{22}$-F- nicht Wasserstoff ist, Umsetzen einer Verbindung der in Anspruch 22 definierten Formel II, worin $R_{22}$-F- Wasserstoff ist, mit einer starken Base und einem Alkylierungsmittel oder einem Acylierungsmittel oder

(c) zum Herstellen einer Verbindung der in Anspruch 22 definierten Formel II, worin $R_{22}$-F- Wasserstoff ist und $R_{21}$-E- und $R_{23}$-G- die relative cis-Stereochemie besitzen, Umsetzen einer Verbindung der in Anspruch 22 definierten Formel II, worin $R_{22}$-F-Wasserstoff ist und $R_{21}$-E- und $R_{23}$-G- die relative trans-Stereochemie besitzen, mit einer starken Base bei -80°C bis -40°C und anschließend mit einer Protonenquelle oder

(d) zum Herstellen einer Verbindung der in Anspruch 22 definierten Formel II, wobei $R_{22}$-F- Wasserstoff ist und $R_{21}$-E- und $R_{23}$-G- die relative trans-Stereochemie besitzen, das Umsetzen einer Verbindung der in Anspruch 22 definierten Formel II, wobei $R_{22}$-F-Wasserstoff ist und $R_{21}$-E- und $R_{23}$-G- die relative cis-Stereochemie besitzen, mit einem Alkalimetall-t-butoxid, anschließend Abbrechen mit Säure oder

(e) Umsetzen eines Carbonsäurederivates der Formel $R_{21}$-E-CH(F-$R_{22}$)-C(O)-Z, worin $R_{21}$-E- und $R_{22}$-F- wie in Anspruch 22 definiert sind und Z Cl,

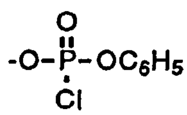 oder 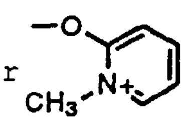 $I^-$

ist, mit einem Imin der Formel $R_{23}$-G-CH=N-$R_{20}$, worin $R_{23}$-G- und $R_{20}$ wie in Anspruch 22 definiert sind, in Anwesenheit einer tertiären Aminbase.

**Patentansprüche für folgenden Vertragsstaat : GR**

**1.** Verbindung mit der Strukturformel

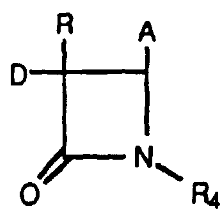

worin

A

-CH=CH-B;

-C≡C-B;

$-(CH_2)_p$-X-B, worin p 0, 1 oder 2 ist und X eine Bindung, -NH- oder $-S(O)_{0-2}$- ist;

Heteroaryl, benzokondensiertes Heteroaryl, W-substituiertes Heteroaryl oder W-substituiertes benzokondensiertes Heteroaryl, worin Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroaryl-Gruppen deren N-Oxide, und wobei W 1-3 Substituenten an den Ringkohlenstoffatomen ist, die aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino) -$C_{1-6}$-alkyl, $C_{3-6}$-Alkandioyl, $C_{1-6}$-Alkyl-$C_{3-6}$-alkandioyl, Allyloxy, $-CF_3$, $-OCF_3$, Benzyl, $R_{14}$-Benzyl, Benzyloxy, $R_{14}$-Benzyloxy, Phenoxy, $R_{14}$-Phenoxy, Dioxolanyl, $NO_2$, $-NR_{10}R_{11}$, $NR_{10}R_{11}$ ($C_{1-6}$-Alkyl)-, $NR_{10}R_{11}$ ($C_{1-6}$-Alkoxy)-, OH, Halogen, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-Butyldimethylsilyloxymethyl, $-C(O)R_{12}$ und

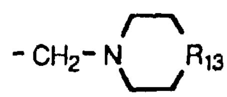

besteht und wobei,

sofern vorhanden, die Substituenten an den substituierten Heteroaryl-Stickstoffatomen aus der Gruppe ausgewählt sind, die aus $C_{1-6}$-Alkyl, $C_{1-6}$-Alkoxy, $-C(O)OR_5$, $-C(O)R_5$, OH, $NR_{10}R_{11}(C_{1-6})$-, $NR_{10}R_{11}$($C_{1-6}$-Alkoxy)-, $-S(O)_2NH_2$ und 2-(Trimethylsilyl)ethoxymethyl besteht;

-C(O)-B oder

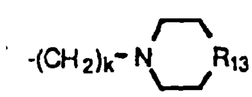

ist, worin k 1 oder 2 ist;

D

B'-$(CH_2)_m$C(O)-, worin m 1, 2, 3, 4 oder 5 ist;

B'-$(CH_2)_q$-, worin q 2, 3, 4, 5 oder 6 ist;

B'-$(CH_2)_e$-Z-$(CH_2)_r$-, E worin Z -O-, -C(O)-, Phenylen, $-NR_8$- oder $-S(O)_{0-2}$- ist, e 0, 1, 2, 3, 4 oder 5 ist und r 1, 2, 3, 4 oder 5 ist, vorausgesetzt, daß die Summe von e und r 1, 2, 3, 4, 5 oder 6 ist;

B'-($C_{2-6}$-Alkenylen)-, B'-($C_4$-$C_6$-Alkadienylen)-;

B'-$(CH_2)_t$-Z-($C_2$-$C_6$-Alkenylen)-, worin Z wie vorstehend definiert ist und worin t 0, 1, 2 oder 3 ist, vorausgesetzt, daß die Summe von t und der Zahl der Kohlenstoffatome in der Alkylenkette 2, 3, 4, 5 oder 6 ist;

B'- $(CH_2)_f$-V-$(CH_2)_g$-, worin V $C_3$-$C_6$-Cycloalkylen ist, f 1, 2, 3, 4 oder 5 ist und g 0, 1, 2, 3, 4 oder 5 ist, vorausgesetzt, daß die Summe von f und g 1, 2, 3, 4, 5 oder 6 ist;

B'- $(CH_2)_t$-V-($C_2$-$C_6$-Alkenylen) - oder B'-($C_2$-$C_6$-Alkenylen)-V-$(CH_2)_t$-, worin V und t wie vorstehend definiert sind, vorausgesetzt, daß die Summe von t und der Zahl der Kohlenstoffatome in der Alkenylenkette 2, 3, 4, 5 oder 6 ist;

B'-$(CH_2)_a$-Z-$(CH_2)_b$-V-$(CH_2)_d$-, worin Z und V wie vorstehend definiert sind und a, b und d unabhängig 0, 1, 2, 3, 4, 5 oder 6 sind, vorausgesetzt, daß die Summe von a, b und d 0, 1, 2, 3, 4, 5 oder 6 ist;

T-$(CH_2)_s$-, worin T Cycloalkyl mit 3-6 Kohlenstoffatomen ist und s 1, 2, 3, 4, 5 oder 6 ist, oder

Naphthylmethyl, Heteroarylmethyl oder W-substituiertes Heteroarylmethyl ist, worin Heteroaryl und W wie vorstehend definiert sind;

B

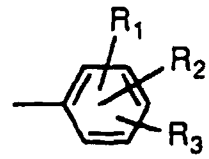

ist;

B' Naphthyl, Heteroaryl oder W-substituiertes Heteroaryl, worin Heteroaryl wie vorstehend definiert ist, oder

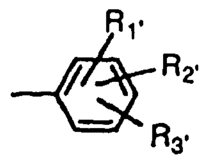

ist;

R Wasserstoff, Fluor, $C_1$-$C_{15}$-Alkyl, $C_2$-$C_{15}$-Alkenyl, $C_2$-$C_{15}$-Alkinyl oder B-$(CH_2)_h$- ist, worin h 0, 1, 2 oder 3 ist;

$R_1$, $R_2$ und $R_3$ unabhängig aus der Gruppe ausgewählt sind, die aus H, $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl; $C_{1-6}$-Alkyl-$C_{3-6}$-alkandioyl, Allyloxy, $-CF_3$, $-OCF_3$, Benzyl, $R_{14}$-Benzyl, Benzyloxy, $R_{14}$-Benzyloxy, Phenoxy, $R_{14}$-Phenoxy, Dioxolanyl, $NO_2$, $-NR_{10}R_{11}$, $NR_{10}R_{11}$ ($C_{1-6}$-Alkyl)-, $NR_{10}R_{11}$ ($C_{1-6}$-Alkoxy)-, OH, o-Halogen, m-Halogen, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-Butyldimethylsilyloxymethyl,

$-C(O)R_{12}$ 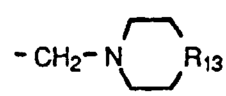 und 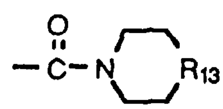

besteht,
oder $R_1$ Wasserstoff ist und $R_2$ und $R_3$ zusammen mit den benachbarten Kohlenstoffatomen, an die sie gebunden sind, einen Dioxolanylring bilden;

$R_{1'}$, $R_{2'}$ und $R_{3'}$ unabhängig aus der Gruppe ausgewählt sind, die aus H, $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl, $C_{1-6}$-Alkyl-$C_{3-6}$-alkandioyl, Allyloxy, $-CF_3$, $-OCF_3$, Benzyl, $R_{14}$-Benzyl, Benzyloxy, $R_{14}$-Benzyloxy, Phenoxy, $R_{14}$-Phenoxy, Dioxolanyl, $NO_2$, $-NR_{10}R_{11}$, $NR_{10}R_{11}$ ($C_{1-6}$-Alkyl)-, $NR_{10}R_{11}$ ($C_{1-6}$-Alkoxy)-, OH, Halogen, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-Butyldimethylsilyloxymethyl, $-C(O)R_{12}$,

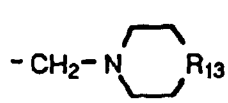 und 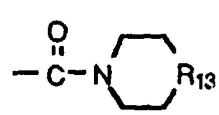

besteht, oder $R_{1'}$ Wasserstoff ist und $R_{2'}$ und $R_{3'}$ zusammen mit den benachbarten Kohlenstoffatomen, an die sie gebunden sind, einen Dioxolanylring bilden;

$R_4$

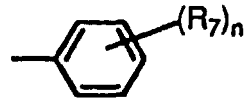

,

worin n 0, 1, 2 oder 3 ist, Indanyl, Benzofuranyl, Benzodioxolyl, Tetrahydronaphthyl, PyridyL, Pyrazinyl, Pyrimidinyl oder Chinolyl ist;

$R_5$ $C_{1-6}$-Alkyl, Phenyl, $R_{14}$-Phenyl, Benzyl oder $R_{14}$-Benzyl ist;

$R_6$ OH, $C_{1-6}$-Alkyl, Phenyl, Benzyl, $R_{14}$-Phenyl oder $R_{14}$-Benzyl ist;

$R_7$ $C_{1-6}$-Alkyl, $C_{1-6}$-Alkoxy, OH, Halogen, $-NR_{10}R_{11}$, $-NHC(O)OR_5$, $-NHC(O)R_5$, $NO_2$, -CN, $-N_3$, -SH, $-S(O)_{0-2}-(C_{1-6}$-Alkyl), $-COOR_9$, $-CONR_{10}R_{11}$, $-COR_{12}$, Phenoxy, Benzyloxy, $-OCF_3$ oder tert-Butyldimethylsilyloxy ist und wenn n 2 oder 3 ist, die Gruppen $R_7$ gleich oder verschieden sein können;

$R_8$ H, $C_{1-6}$-Alkyl, Phenyl-$C_{1-6}$-alkyl oder $-C(O)R_9$ ist;

$R_9$ H, $C_{1-6}$-Alkyl oder Phenyl-$C_{1-6}$-alkyl ist;

$R_{10}$ und $R_{11}$ unabhängig aus H und $C_{1-6}$-Alkyl ausgewählt sind;

$R_{12}$ H, OH, $C_{1-6}$-Alkoxy, Phenoxy, Benzyloxy,

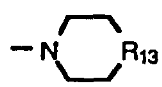

$-NR_{10}R_{11}$, $C_{1-6}$-Alkyl, Phenyl oder $R_{14}$-Phenyl ist;

$R_{13}$ -O-, $-CH_2-$, -NH- oder $-N(C_{1-6}$-Alkyl)- ist und

$R_{14}$ 1-3 Gruppen ist, die unabhängig aus der Gruppe ausgewählt sind, die aus $C_{1-6}$-Alkyl, $C_{1-6}$-Alkoxy, -COOH, $NO_2$, $-NR_{10}R_{11}$, OH oder Halogen besteht,

oder ein pharmazeutisch annehmbares Salz derselben.

**2.** Verbindung von Anspruch 1, wobei R Wasserstoff ist.

**3.** Verbindung von Anspruch 1 oder 2, wobei D $B'-(CH_2)_q-$, $B'-(CH_2)_e-Z-(CH_2)_r-$, $B'-(C_2-C_6$-Alkenylen)- oder $B'-(CH_2)_f-V-(CH_2)_g$-ist, worin B', Z, V, q, e, r, f und g wie in Anspruch 1 definiert sind.

**4.** Verbindung von Anspruch 3, wobei D $B'-(CH_2)_q-$, worin B' Phenyl ist und q 3 oder 4 ist; $B'-(CH_2)_e-Z-(CH_2)_r-$, e worin B' p-Fluorphenyl oder p-Methoxyphenyl ist, e Null ist, Z -O- ist und r 2 ist; $B'-(C_2-C_6$-Alkenylen)-3-Phenyl-1-propenyl ist oder $B'-(CH_2)_f-V-(CH_2)_g-$ ist, worin B' Phenyl ist, f 1 ist, V Cyclopropylen ist und g null ist.

**5.** Verbindung von Anspruch 1, 2, 3 oder 4, wobei $A-(CH_2)_p-X-B$ ist, worin X, B und p wie in Anspruch 1 definiert sind.

**6.** Verbindung von Anspruch 5, wobei p null ist und X eine Bindung ist.

**7.** Verbindung von Anspruch 6, wobei $R_1$, $R_2$ und $R_3$ aus der Gruppe ausgewählt sind, die aus H, OH, $C_{1-6}$-Alkoxy, $NO_2$, $C_{1-6}$-Alkoxy-$C_{1-6}$-alkoxy, m-Halogen, $C_{1-6}$-Alkyl-$C_{3-6}$-alkandioyl, $NR_{10}R_{11}(C_{1-6}$-Alkoxy)-, Allyloxy, Phenoxy, $C_{1-6}$-Alkoxycarbonyl-$C_{1-6}$-alkoxy und $-C(O)R_{12}$ besteht.

**8.** Verbindung von Anspruch 1, 2, 3, 4, 5 oder 6, wobei $R_4$ Phenyl, $R_7$-substituiertes Phenyl oder Indanyl ist.

**9.** Verbindung von Anspruch 8, wobei $R_7$ aus der Gruppe ausgewählt ist, die aus $C_{1-6}$-Alkyl, $C_{1-6}$-Alkoxy, Halogen, $-OCF_3$, $C_{1-6}$-Alkylthio, $-NR_{10}R_{11}$, -CN, OH und $-COR_{12}$ besteht.

**10.** Verfahren zum Herstellen einer pharmazeutischen Zusammensetzung zur Verwendung bei der Herstellung eines Arzneimittels zum Erniedrigen von Serumcholesterin, wobei das Verfahren das Vermischen eines pharmazeutisch annehmbaren Trägers mit einer Verbindung mit der Struktur

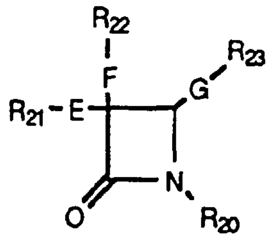

worin

$R_{20}$ Phenyl, W-substituiertes Phenyl, Naphthyl, W-substituiertes Naphthyl, Benzodioxolyl, Heteroaryl, W-substituiertes Heteroaryl, benzokondensiertes Heteroaryl und W-substituiertes benzokondensiertes Heteroaryl, wobei Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroaryl-Gruppen deren N-Oxid ist;

$R_{21}$, $R_{22}$ und $R_{23}$ unabhängig aus H oder $R_{20}$ ausgewählt sind;

W 1 bis 3 Substituenten ist, die unabhängig aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl, $C_{1-6}$-Alkyl-$C_{3-6}$-alkandioyl, Allyloxy, $-CF_3$, $-OCF_3$, Benzyl, $R_{14}$-Benzyl, Benzyloxy, $R_{14}$-Benzyloxy, Phenoxy, $R_{14}$-Phenoxy, Dioxolanyl, $NO_2$, $-NR_{10}R_{11}$, $NR_{10}R_{11}$ ($C_{1-6}$-Alkyl)-, $NR_{10}R_{11}$ ($C_{1-6}$-Alkoxy)-, OH, Halogen, $-NHC(O)OR_5$, $-NHC(O)R_5$, $R_6O_2SNH$-, $(R_6O_2S)_2N$-, $-S(O)_2NH_2$, -S $(O)_{0-2}R_{10}$, tert-Butyldimethylsilyloxymethyl, $-C(O)R_{12}$,

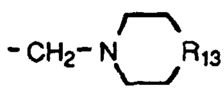

und

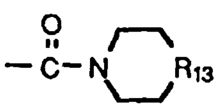

besteht;

E, F und G unabhängig eine Bindung, $C_{3-6}$-Cycloalkylen, $C_1$-$C_{10}$-Alkylen, $C_2$-$C_{10}$-Alkenylen, $C_2$-$C_{10}$-Alkinylen, eine definierte Alkylen-, Alkenylen- oder Alkinylenkette, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, wobei Heteroaryl wie vorstehend definiert ist, eine definierte Alkylen-, Alkenylen- oder Alkinylenkette, die durch eine oder mehr Gruppen unterbrochen ist, die unabhängig aus der Gruppe ausgewählt sind, die aus -O-, -S-, -SO-, $-SO_2$-, $-NR_8$, -C(O)-, $C_3$-$C_6$-Cycloalkylen, Phenylen, W-substituiertem Phenylen, Heteroarylen und W-substituiertem Heteroarylen besteht, oder eine definierte unterbrochene Alkylen-, Alkenylen- oder Alkinylengruppe sind, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, oder eines von $R_{21}$-E und $R_{22}$-F aus der Gruppe ausgewählt ist, die aus Halogen, OH, $C_{1-6}$-Alkoxy, $-OC(O)R_5$, $-NR_{10}R_{11}$, -SH oder -S($C_{1-6}$-Alkyl) besteht;

| | |
|---|---|
| $R_5$ | $C_{1-6}$-Alkyl, Phenyl, $R_{14}$-Phenyl, Benzyl oder $R_{14}$-Benzyl ist; |
| $R_6$ | OH, $C_{1-6}$-Alkyl, Phenyl, Benzyl, $R_{14}$-Phenyl oder $R_{14}$-Benzyl ist; |
| $R_8$ | H, $C_{1-6}$-Alkyl, Phenyl-$C_{1-6}$-alkyl oder -C(O)$R_9$ ist; |
| $R_9$ | H, $C_{1-6}$-Alkyl, Phenyl oder Phenyl-$C_{1-6}$-alkyl ist; |
| $R_{10}$ und $R_{11}$ | unabhängig aus H und $C_{1-6}$-Alkyl ausgewählt sind; |
| $R_{12}$ | H, OH, $C_{1-6}$-Alkoxy, Phenoxy, Benzyloxy, 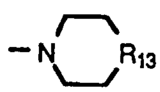 , -$NR_{10}R_{11}$, $C_{1-6}$-Alkyl, Phenyl oder $R_{14}$-Phenyl ist; |
| $R_{13}$ | -O-, -$CH_2$-, -NH- oder -N($C_{1-6}$-Alkyl)- ist; |
| $R_{14}$ | 1-3 Gruppen ist, die unabhängig aus der Gruppe ausgewählt sind, die aus $C_{1-6}$-Alkyl, $C_{1-6}$-Alkoxy, -COOH, $NO_2$, -$NR_{10}R_{11}$, OH oder Halogen besteht; |

vorausgesetzt, daß wenn G eine Bindung ist, $R_{23}$ nicht H ist und vorausgesetzt, daß wenn $R_{23}$ W-substituiertes Phenyl ist, W nicht p-Halogen ist,
oder einem pharmazeutisch annehmbaren Salz derselben in einem pharmazeutisch annehmbaren Träger umfaßt.

**11.** Verbindung von Anspruch 1, die aus der Gruppe ausgewählt ist, die aus Verbindungen der Formel

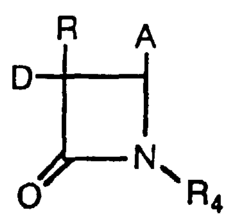

besteht, worin D, R, A und $R_4$ und die relative Stereochemie zwischen D und A wie in der folgenden Tabelle definiert sind:

| D | R | A | relative Stereochemie | $R_4$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-$NO_2$-$C_6H_4$- | 3R,4R | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | 3-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 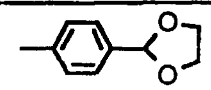 | trans | p-$CH_3O$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | 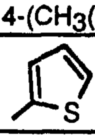 | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($C_6H_5$-O)-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 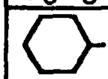 $(CH_2)_3$– | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-($(CH_3)_2N$-$(CH_2)_3$-O)-$C_6H_4$- | cis | 4-$CH_3O$-$C_6H_4$- |

| | | | | |
|---|---|---|---|---|
| $C_6H_5-(CH_2)_3-$ | H | $4-CH_3O-C_6H_4-$ | cis | $C_6H_5-$ |
| $C_6H_5-(CH_2)_3-$ | H | $4-CH_3O-C_6H_4-$ | 3S,4S | $p-CH_3O-C_6H_4-$ |
| $C_6H_5-(CH_2)_3-$ | H | $p-CH_3O-C_6H_4-$ | 3S,4R | $p-CH_3O-C_6H_4-$ |
| $C_6H_5-(CH_2)_3-$ | H | $4-CH_3O-C_6H_4-$ | trans | $4-F-C_6H_4-$ |
| $C_6H_5-(CH_2)_3-$ | H | $4-CH_3O-C_6H_4-$ | trans | $3-CH_3O-C_6H_4-$ |
| $C_6H_5-(CH_2)_3-$ | H | $4-CH_3O-C_6H_4-$ | trans | $4-Cl-C_6H_4-$ |
| $C_6H_5-(CH_2)_3-$ | H | $4-CH_3O-C_6H_4-$ | trans | $4-(CH_3CH_2-O)-C_6H_4-$ |
| $C_6H_5-(CH_2)_3-$ | H | $4-CH_3O-C_6H_4-$ | trans | $4-CH_3S-C_6H_4-$ |
| $C_6H_5-(CH_2)_3-$ | H | $4-CH_3O-C_6H_4-$ | trans | $4-CH_3-C_6H_4-$ |
| $C_6H_5-(CH_2)_3-$ | H | $4-CH_3O-C_6H_4-$ | trans | $4-(C_6H_5-O)-C_6H_4-$ |
| 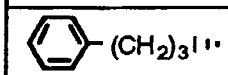 | H | 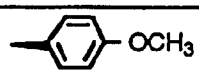 | trans optisch rein | $C_6H_5-$ |
| 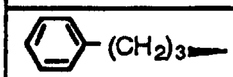 | H | 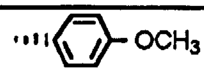 | trans optisch rein | $C_6H_5-$ |
| $C_6H_5-(CH_2)_3-$ | H | $4-(CH_3CH_2-O)-C_6H_4-$ | trans | $4-CH_3O-C_6H_4-$ |
| $C_6H_5-(CH_2)_3-$ | H | $4-(C_6H_5-CH_2-O)-C_6H_4-$ | trans | $4-CH_3O-C_6H_4-$ |
| $C_6H_5-(CH_2)_3-$ | H | $4-(CH_2{=}CH-CH_2-O)-C_6H_4-$ | trans | $4-CH_3O-C_6H_4-$ |
| $C_6H_5-(CH_2)_3-$ | H | $4-(C_6H_5-O)-C_6H_4-$ | trans | $4-CH_3O-C_6H_4-$ |
| $C_6H_5-O-(CH_2)_2-$ | H | $4-CH_3O-C_6H_4-$ | trans | $4-CH_3O-C_6H_4-$ |
| $4-F-C_6H_4-O-(CH_2)_2-$ | H | $4-CH_3O-C_6H_4-$ | trans | $4-CH_3O-C_6H_4-$ |
| $C_6H_5-(CH_2)_4-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $C_6H_5-$ |
| $C_6H_5-(CH_2)_3-$ | H | $4-CH_3O-C_6H_4-$ | trans | $4-(CH_3CH_2OC(O))-C_6H_4-$ |
| $C_6H_5-(CH_2)_3-$ | H | $4-CH_3O-C_6H_4-$ | trans | $3-NO_2-C_6H_4-$ |
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 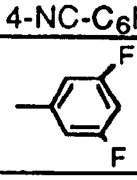 |
| $C_6H_5-(CH_2)_3-$ | H | $4-CH_3O-C_6H_4-$ | trans | $4-(CH_3C(O))-C_6H_4-$ |

| | | | | |
|---|---|---|---|---|
| $C_6H_5$-$(CH_2)_3$- | H | 4-$CH_3O$-$C_6H_4$- | trans | 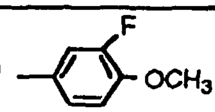 |
| $C_6H_5$-$(CH_2)_3$- | H | 4-$CH_3O$-$C_6H_4$- | trans | 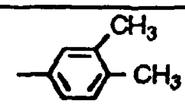 |
| $C_6H_5$-$(CH_2)_3$- | H | 4-($(CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 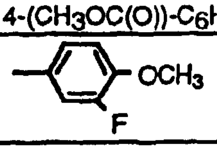 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 4-($(CH_3)_2N$)-$C_6H_4$- | trans | 4-$CH_3O$-$C_6H_4$- |
| $C_6H_5$-$(CH_2)_3$- | H | 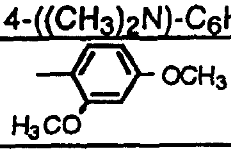 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| 4-$O_2N$-$C_6H_4$-$(CH_2)_3$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | 4-I-$C_6H_4$- |
| $C_6H_5$-$(CH_2)_3$- | H | 4-($CH_3(CH_2)_3$-O)-$C_6H_4$- | trans | 4-$CH_3O$-$C_6H_4$- |
| 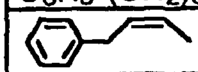 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 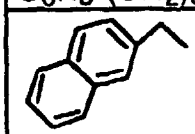 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 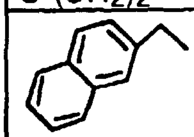 | H | 4-$CH_3O$-$C_6H_4$- | cis | $C_6H_5$- |
| $C_6H_5$-$(CH_2)_3$- | H | 4-$CH_3O$-$C_6H_4$- | trans | 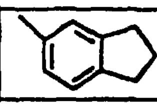 |
| $C_6H_5$-$(CH_2)_3$- | H | 4-$NH_2$-$C_6H_4$- | cis | 4-$CH_3O$-$C_6H_4$- |
| $C_6H_5$-$(CH_2)_3$- | H | 4-$CH_3O$-$C_6H_4$- | trans | 3-$NH_2$-$C_6H_4$- |
| $C_6H_5$-$(CH_2)_3$- | H | 4-OH-$C_6H_4$- | trans | 4-$CH_3O$-$C_6H_4$- |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-$CH_3O$-$C_6H_4$- | 3R,4S | 4-OH-$C_6H_4$- |

| | | | | |
|---|---|---|---|---|
| $C_6H_5-(CH_2)_3-$ | H | 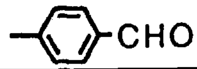 | trans | $4-CH_3O-C_6H_4-$ |
| $C_6H_5-(CH_2)_3-$ | H | $4-CH_3O-C_6H_4-$ | trans | $4-(CH_3SO_2)-C_6H_4-$ |
| $C_6H_5-(CH_2)_3-$ | H | 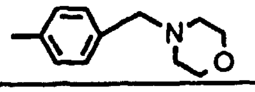 | trans | $4-CH_3O-C_6H_4-$ |
| $C_6H_5-(CH_2)_3-$ | H | 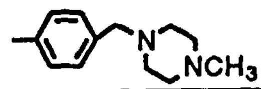 | trans | $4-CH_3O-C_6H_4-$ |
| $C_6H_5-(CH_2)_3-$ | H | $4-(CH_3OCH_2)-C_6H_4-$ | trans | $4-CH_3O-C_6H_4-$ |
| $C_6H_5-(CH_2)_3-$ | H | 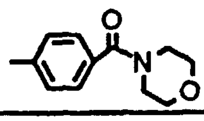 | cis | $4-CH_3O-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 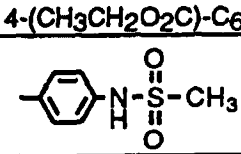 | trans | $4-CH_3O-C_6H_4-$ |
| $C_6H_5-(CH_2)_3-$ | H | $-Ph-OC(CH_2)_2COCH_3$ worin 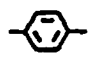 ist | trans | $4-CH_3O-C_6H_4-$ |
| $C_6H_5-(CH_2)_3-$ | H | $-Ph-OCH_2CO_2Et$ worin 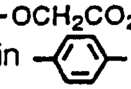 ist und Et $-CH_2CH_3$ ist | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 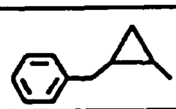 | H | $4-CH_3O-C_6H_4-$ | cis | $4-CH_3O-C_6H_4-$ |

**12.** Verbindung von Anspruch 1 oder 11, die (3R,4S)-1,4-Bis-(4-methoxyphenyl)-3-(3-(phenylpropyl)-2-azetidinon ist.

**13.** Verwendung von Anspruch 10, wobei die Verbindung aus der Gruppe ausgewählt ist, die aus Verbindungen der Formel

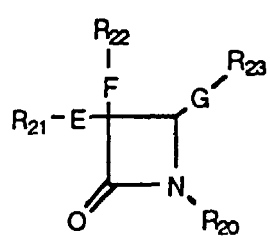

besteht, wobei $R_{21}$-E-, $R_{22}$-F-, $R_{23}$-G-, $R_{20}$ und die relative Stereochemie zwischen $R_{21}$-E- und $R_{23}$-G- wie in der folgenden Tabelle definiert sind:

| $R_{21}$-E- | $R_{22}$-F- | $R_{23}$-G- | rel. Stereo-chem. | $R_{20}$ |
|---|---|---|---|---|
| $C_6H_5-(CH_2)_3-$ | H | p-$CH_3O-C_6H_4-$ | 3S,4S | p-$CH_3O-C_6H_4-$ |
| $C_6H_5-(CH_2)_3-$ | H | p-$OH-C_6H_4-$ | 3S,4S | p-$CH_3O-C_6H_4-$ |
| $C_6H_5-(CH_2)_3-$ | H | p-$CH_3O-C_6H_4-$ | trans | p-$CF_3O-C_6H_4-$ |
| $C_6H_5-(CH_2)_3-$ | H | 4-$CH_3O-C_6H_4-$ | cis | p-$Cl-C_6H_4-$ |
| $C_6H_5-(CH_2)_3-$ | H | 4-$CH_3O-C_6H_4-$ | cis | p-$CH_3-C_6H_4-$ |
| $C_6H_5-(CH_2)_3-$ | H | 4-$((CH_3)_2CH-O)-C_6H_4-$ | cis | p-$CH_3O-C_6H_4-$ |
| $C_6H_5-(CH_2)_3-$ | H | 4-$(CH_3(CH)_2-O)-C_6H_4-$ | cis | p-$CH_3O-C_6H_4-$ |
| $C_6H_5-(CH_2)_3-$ | H | $CH_3CH_2-O-C_6H_4-$ | cis | p-$CH_3O-C_6H_4-$ |
| $C_6H_5-(CH_2)_3-$ | H | $CH_3(CH_2)_3-O-C_6H_4-$ | cis | p-$CH_3O-C_6H_4-$ |
| $C_6H_5-(CH_2)_3-$ | H | 4-$(CH_2=CH-CH_2-O)-C_6H_4-$ | cis | p-$CH_3O-C_6H_4-$ |
| $C_6H_5-(CH_2)_3-$ | H | 4-$(CH_3CH_2-O)-C_6H_4-$ | cis | $C_6H_5-$ |
| 4-$F-C_6H_4-O-(CH_2)_2-$ | H | 4-$CH_3O-C_6H_4-$ | cis | $C_6H_5-$ |
| $C_6H_5-(CH_2)_3-$ | H | 4-$CH_3O-C_6H_4-$ | cis | $C_6H_5-$ |
| $C_6H_5-(CH_2)_3-$ | H | 4-$CH_3O-C_6H_4-$ | 3S,4S | p-$CH_3O-C_6H_4-$ |
| $C_6H_5-(CH_2)_3-$ | H | p-$CH_3O-C_6H_4-$ | 3S,4R | p-$CH_3O-C_6H_4-$ |
| $C_6H_5-(CH_2)_3-$ | H | 4-$CH_3O-C_6H_4-$ | trans | 4-$F-C_6H_4-$ |
| $C_6H_5-(CH_2)_3-$ | H | 4-$CH_3O-C_6H_4-$ | trans | 3-$CH_3O-C_6H_4-$ |
| $C_6H_5-(CH_2)_3-$ | H | 4-$CH_3O-C_6H_4-$ | trans | 4-$Cl-C_6H_4-$ |
| $C_6H_5-(CH_2)_3-$ | H | 4-$CH_3O-C_6H_4-$ | trans | 4-$(CH_3CH_2-O)-C_6H_4-$ |
| $C_6H_5-(CH_2)_3-$ | H | 4-$CH_3O-C_6H_4-$ | trans | 4-$CH_3S-C_6H_4-$ |
| $C_6H_5-(CH_2)_3-$ | H | 4-$CH_3O-C_6H_4-$ | trans | 4-$CH_3-C_6H_4-$ |
| 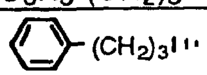 | H | 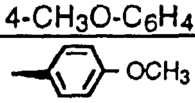 | trans optisch rein | $C_6H_5-$ |
| $C_6H_5-(CH_2)_3-$ | H | 4-$(CH_3CH_2-O)-C_6H_4-$ | trans | 4-$CH_3O-C_6H_4-$ |
| $C_6H_5-(CH_2)_3-$ | H | 4-$(CH_2=CH-CH_2-O)-C_6H_4-$ | trans | 4-$CH_3O-C_6H_4-$ |
| $C_6H_5-(CH_2)_3-$ | H | 4-$(C_6H_5-O)-C_6H_4-$ | trans | 4-$CH_3O-C_6H_4-$ |
| 4-$F-C_6H_4-O-(CH_2)_2-$ | H | 4-$CH_3O-C_6H_4-$ | trans | 4-$CH_3O-C_6H_4-$ |
| $C_6H_5-(CH_2)_4-$ | H | 4-$CH_3O-C_6H_4-$ | trans | 4-$CH_3O-C_6H_4-$ |
| $C_6H_5-(CH_2)_3-$ | H | 4-$CH_3O-C_6H_4-$ | trans | $C_6H_5-$ |

| | | | | |
|---|---|---|---|---|
| $C_6H_5$-$(CH_2)_3$- | H | 4-$CH_3O$-$C_6H_4$- | trans | 4-($(CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 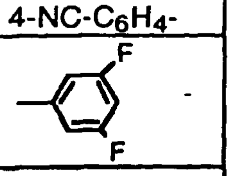 |
| $C_6H_5$-$(CH_2)_3$- | H | 4-$CH_3O$-$C_6H_4$- | trans | 4-($CH_3C(O)$)-$C_6H_4$- |
| $C_6H_5$-$(CH_2)_3$- | H | 4-($(CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 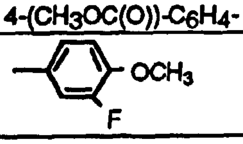 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 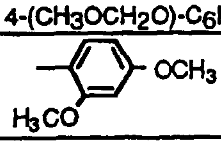 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| $C_6H_5$-$(CH_2)_3$- | H | 4-$CH_3O$-$C_6H_4$- | trans | 4-I-$C_6H_4$- |
| 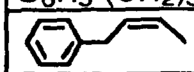 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 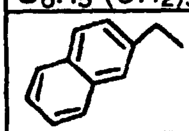 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 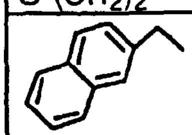 | H | 4-$CH_3O$-$C_6H_4$- | cis | $C_6H_5$- |
| $C_6H_5$-$(CH_2)_3$- | H | 4-$CH_3O$-$C_6H_4$- | trans | 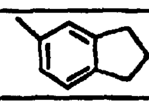 |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-($CH_3CH_2O_2C$)-$C_6H_4$- | trans | 4-$CH_3O$-$C_6H_4$- |
| $C_6H_5$-$(CH_2)_3$- | H | O O<br>–Ph-$OC(CH_2)_2COCH_3$<br>worin Ph 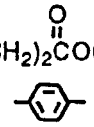 ist | trans | 4-$CH_3O$-$C_6H_4$- |

| | | | | |
|---|---|---|---|---|
| $C_6H_5-(CH_2)_3-$ | H | -Ph-$OCH_2CO_2Et$ worin Ph 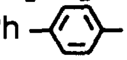 ist und Et $-CH_2CH_3$ ist | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 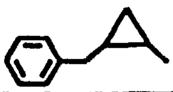 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 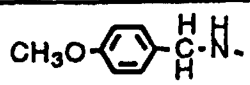 | H | $4-CH_3O-C_6H_4-$ | cis | $C_6H_5-$ |

**14.** Verwendung einer Verbindung von Anspruch 1 zur Herstellung eines Arzneimittels zum Erniedrigen des Serumcholesterins.

**15.** Verwendung einer Verbindung von Anspruch 1 zur Herstellung eines Arzneimittels zum Hemmen des Enzyms Acyl-CoA:Cholesterin-Acyltransferase.

**16.** Verwendung einer in Anspruch 10 definierten pharmazeutischen Zusammensetzung zur Herstellung eines Arzneimittels zum Erniedrigen der Serumcholesterinwerte in einem Säuger, der einer derartigen Behandlung bedarf.

**17.** Verwendung einer in Anspruch 10 definierten Zusammensetzung zur Herstellung eines Arzneimittels zum Hemmen des Enzyms Acyl-CoA:Cholesterin-Acyltransferase.

**18.** Verfahren zum Herstellen einer Verbindung von Anspruch 1, wobei R H ist und D und A eine relative trans-Stereochemie besitzen, das das Cyclisieren eines Hydroxyamids der Formel

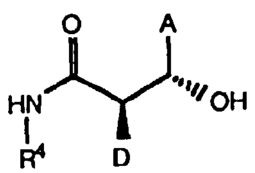 oder 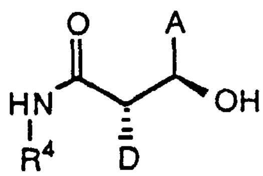

worin D, A und $R^4$ wie in Anspruch 1 definiert sind, durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder
(iii)einem Dialkylchlorphosphat, einer wäßrigen Base und einem Phasentransferkatalysator oder
(iv) einem Di- oder Trichlorbenzoylchlorid und einem Alkalimetallhydrid umfaßt.

**19.** Verfahren zum Herstellen einer Verbindung von Anspruch 1 mit der Formel

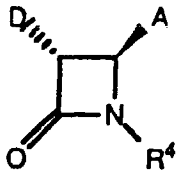

oder

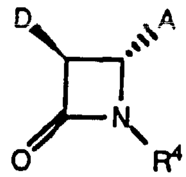

worin D, A und $R^4$ wie in Anspruch 1 definiert sind, umfassend:

(a) das Umsetzen einer Carbonsäure der Formel $DCH_2$-COOH, worin D wie vorstehend definiert ist, mit einem Chlorierungsmittel,
(b) Deprotonieren eines chiralen Oxazolidinons der Formel

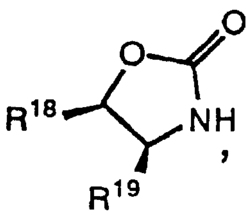

,

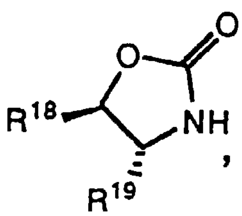

,

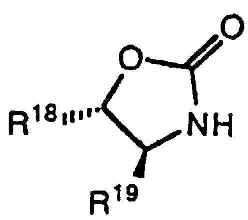

oder

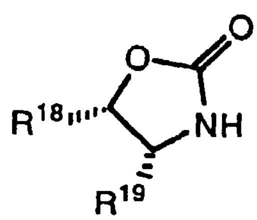

worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, mit einer starken Base und Behandeln des sich daraus ergebenden Anions mit dem Produkt aus Schritt (a),
(c) Enolisieren des Produkts aus Schritt (b) entweder mit:

(i) einem Dialkylbortriflat und einer tertiären Aminbase
(ii) $TiCl_4$ und Tetramethylethylendiamin (TMEDA) oder einem Gemisch aus TMEDA und Triethylamin,

anschließend Kondensieren mit einem Aldehyd der Formel A-CHO, worin A wie vorstehend definiert ist,
(d) Hydrolysieren des Produktes von Schritt (c) mit einer Base und Wasserstoffperoxid,
(e) Kondensieren des Produkts von Schritt (d) mit einem Amin der Formel $R^4NH_2$, worin $R^4$ wie vorstehend definiert ist, durch Behandeln mit einem dehydratisierenden Kupplungsmittel, gegebenenfalls Zusetzen eines Aktivierungsmittels und
(f) Cyclisieren des Produktes von Schritt (e) durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder
(iii)einem Dialkylchlorphosphat, einer wäßrigen Base und einem Phasentransferkatalysator oder
(iv) einem Di- oder Trichlorbenzoylchlorid und einem Alkalimetallhydrid.

**20.** Verfahren zum Herstellen einer Verbindung von Anspruch 1, wobei R Wasserstoff ist und D und A eine relative trans-Stereochemie besitzen, umfassend:

(a) das Enolisieren einer Verbindung der Formel

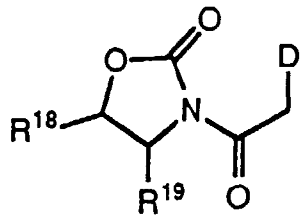

mit $TiCl_4$ und Tetramethylethylendiamin (TMEDA), anschließend Kondensieren mit einem Imin der Formel A-CH=N-$R^4$, wobei A und $R^4$ wie vorstehend definiert sind, und wobei die Gruppe

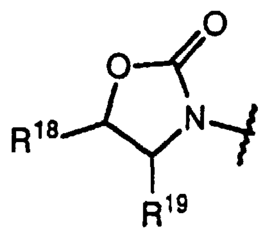

einen chiralen Hilfsstoff der Formel

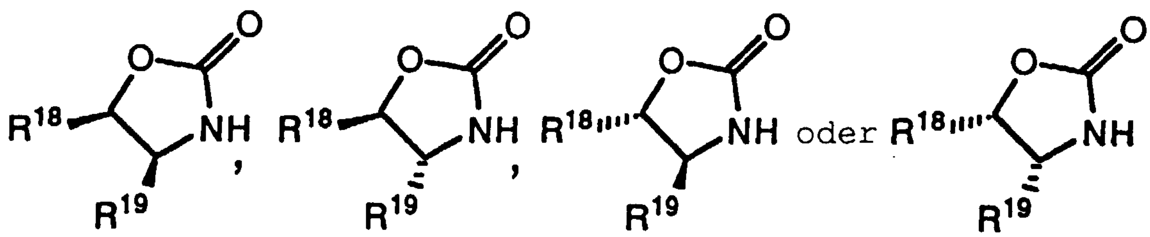

, , oder

darstellt, worin $R^{18}$ und $R^{19}$ unabhängig aus der Gruppe ausgewählt sind, die aus Wasserstoff, $C_1$-$C_6$-Alkyl; Phenyl, Naphthyl, substituiertem Phenyl, substituiertem Naphthyl und Benzyl besteht, und

(b) Cyclisieren des Produkts aus Schritt (a) durch Behandeln mit einer starken nicht-nukleophilen Base.

**21.** Verfahren zum Herstellen von Verbindungen von Anspruch 1, umfassend:

(a) Behandeln eines Esters der Formel D-CH(R)-C(O)$OR^{17}$, worin $R^{17}$ $C_{1-6}$-Alkyl, Menthyl oder 10-(Diisopropylsulfonamido)isobornyl ist, mit einer starken Base und anschließend mit einem Imin der Formel A-CH=N-$R^4$, worin A und $R^4$ wie in Anspruch 1 definiert sind, oder

(b) zum Herstellen einer Verbindung von Anspruch 1, worin R nicht Wasserstoff ist, Umsetzen einer Verbindung von Anspruch 1, worin R Wasserstoff ist, mit einer starken Base und einem Alkylierungsmittel oder einem Acylierungsmittel oder

(c) zum Herstellen einer Verbindung von Anspruch 1, worin R Wasserstoff ist und D und A die relative cis-Stereochemie besitzen, Umsetzen einer Verbindung von Anspruch 1, worin R Wasserstoff ist und D und A die relative trans-Stereochemie besitzen, mit einer starken Base bei -80°C bis -40°C und anschließend mit einer Protonenquelle oder

(d) zum Herstellen einer Verbindung von Anspruch 1, wobei R Wasserstoff ist und D und A die relative trans-Stereochemie besitzen, Umsetzen einer Verbindung von Anspruch 1, worin R Wasserstoff ist und D und A die relative cis-Stereochemie besitzen, mit einem Alkalimetall-t-butoxid, anschließend Abbrechen mit Säure oder

(e) Umsetzen eines Carbonsäurederivates der Formel D-CH(R)-C(O)-Z, worin D und R wie in Anspruch 1 definiert sind und Z Cl,

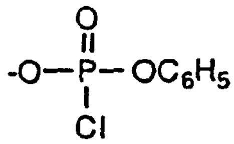

oder

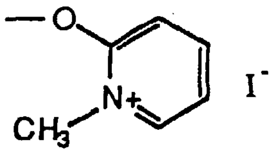

ist, mit einem Imin der Formel -A-CH=$NR^4$, worin A und $R^4$ wie in Anspruch 1 definiert sind, in Anwesenheit einer tertiären Aminbase.

**22.** Verfahren zum Herstellen einer Verbindung der Formel II

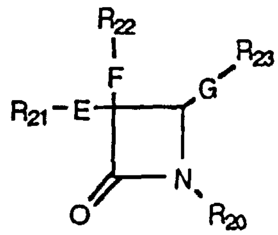

II

worin

| | |
|---|---|
| $R_{20}$ | Phenyl, W-substituiertes Phenyl, Naphthyl, W-substituiertes Naphthyl, Benzodioxolyl, Heteroaryl, W-substituiertes Heteroaryl, benzokondensiertes Heteroaryl und W-substituiertes benzokondensiertes Heteroaryl ist, wobei Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroarylgruppen das N-Oxid derselben ist; |
| $R_{21}$, $R_{22}$ und $R_{23}$ | unabhängig aus H oder $R_{20}$ ausgewählt sind; |
| W | 1 bis 3 Substituenten ist, die unabhängig aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl, $C_{1-6}$-Alkyl-$C_{3-6}$-alkandioyl, Allyloxy, -$CF_3$, -$OCF_3$, Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$($C_{1-6}$-Alkyl)-, $NR_{10}R_{11}$($C_{1-6}$-Alkoxy)-, OH, Halogen, -NHC(O)$OR_5$, -NHC(O)$R_5$, $R_6O_2SNH$-, $(R_6O_2S)_2N$-, -$S(O)_2NH_2$, -S$(O)_{0-2}R_{10}$, tert-Butyldimethylsilyloxymethyl, -C(O)$R_{12}$, 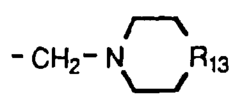 und 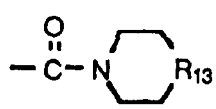 besteht; |
| E, F und G | unabhängig eine Bindung, $C_{3-6}$-Cycloalkylen, $C_1$-$C_{10}$-Alkylen, $C_2$-$C_{10}$-Alkenylen, $C_2$-$C_{10}$-Alkinylen, eine definierte Alkylen-, Alkenylen- oder Alkinylenkette, die durch einen oder meh- |

rere Substituenten substituiert ist, der unabhängig aus der Gruppe ausgewählt sind, die aus Phenyl, W-substituiertem Phenyl, Heteroaryl und W-substituiertem Heteroaryl besteht, wobei Heteroaryl wie vorstehend definiert ist, eine definierte Alkylen-, Alkenylen- oder Alkinylenkette, die durch eine oder mehr Gruppen unterbrochen ist, die unabhängig aus der Gruppe ausgewählt sind, die aus -O-, -S-, -SO-, $-SO_2-$, $-NR_8$, -C(O)-, $C_3$-$C_6$-Cycloalkylen, Phenylen, W-substituiertem Phenylen, Heteroarylen und W-substituiertem Heteroarylen besteht, oder eine definierte unterbrochene Alkylen-, Alkenylen- oder Alkinylengruppe sind, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, oder eines von $R_{21}$-E und $R_{22}$-F aus der Gruppe ausgewählt ist, die aus Halogen, OH, $C_{1-6}$-Alkoxy, $-OC(O)R_5$, $-NR_{10}R_{11}$, -SH oder -S ($C_{1-6}$-Alkyl) besteht;

$R_5$ $C_{1-6}$-Alkyl, Phenyl, $R_{14}$-Phenyl, Benzyl oder $R_{14}$-Benzyl ist;

$R_6$ OH, $C_{1-6}$-Alkyl, Phenyl, Benzyl, $R_{14}$-Phenyl oder $R_{14}$-Benzyl ist;

$R_8$ H, $C_{1-6}$-Alkyl, Phenyl-$C_{1-6}$-alkyl oder $-C(O)R_9$ ist;

$R_9$ H, $C_{1-6}$-Alkyl, Phenyl oder Phenyl-$C_{1-6}$-alkyl ist;

$R_{10}$ und $R_{11}$ unabhängig aus H und $C_{1-6}$-Alkyl ausgewählt sind,

$R_{12}$ H, OH, $C_{1-6}$-Alkoxy, Phenoxy, Benzyloxy,

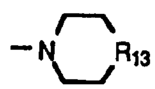 ,

$-NR_{10}R_{11}$, $C_{1-6}$-Alkyl, Phenyl oder $R_{14}$-Phenyl ist;

$R_{13}$ -O-, $-CH_2-$, -NH- oder -N($C_{1-6}$-Alkyl)- ist;

$R_{14}$ 1-3 Gruppen ist, die unabhängig aus der Gruppe ausgewählt sind, die aus $C_{1-6}$-Alkyl, $C_{1-6}$-Alkoxy, -COOH, $NO_2$, $-NR_{10}R_{11}$, OH oder Halogen besteht;
vorausgesetzt, daß wenn G eine Bindung ist, $R_{23}$ nicht H ist und vorausgesetzt, daß wenn $R_{23}$ W-substituiertes Phenyl ist, W nicht p-Halogen ist,
oder eines pharmazeutisch annehmbaren Salzes derselben, wobei $R_{22}$-F H ist und $R_{21}$-E- und $R_{23}$-G- die relative trans-Stereochemie besitzen, umfassend

Verfahren C: das Cyclisieren eines Hydroxyamids der Formel

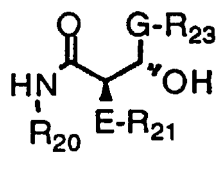 oder 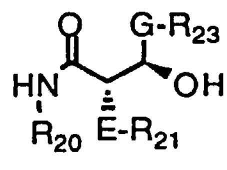

worin $R_{21}$-E-, $R_{23}$-G- und $R_{20}$ wie vorstehend definiert sind, durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder
(iii)einem Dialkylchlorphosphat, einer wäßrigen Base und einem Phasentransferkatalysator oder
(iv) einem Di- oder Trichlorbenzoylchlorid und einem Alkalimetallhydrid oder

Verfahren D:

(a) das Umsetzen einer Carbonsäure der Formel $R_{21}$-E-$CH_2$-COOH, wobei $R_{21}$-E- wie vorstehend definiert ist, mit einem Chlorierungsmittel,
(b) Deprotonieren eines chiralen Oxazolidinons der Formel

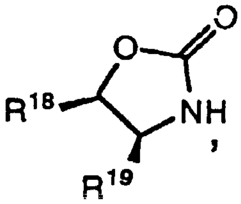
,
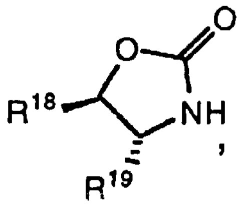
,
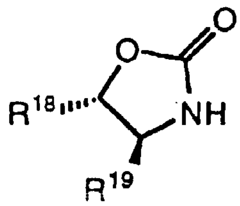
oder
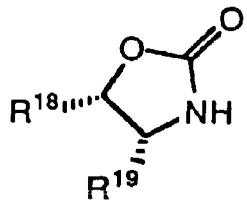

worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, mit einer starken Base und Behandeln des sich daraus ergebenden Anions mit dem Produkt aus Schritt (a),
(c) Enolisieren des Produkts aus Schritt (b) entweder mit:

(i) einem Dialkylbortriflat und einer tertiären Aminbase
(ii) $TiCl_4$ und Tetramethylethylendiamin (TMEDA) oder einem Gemisch aus TMEDA und Triethylamin,

anschließend Kondensieren mit einem Aldehyd der Formel $R_{23}$-G-CHO, worin $R_{23}$-G- wie vorstehend definiert ist,
(d) Hydrolysieren des Produktes von Schritt (c) mit einer Base und Wasserstoffperoxid,
(e) Kondensieren des Produkts von Schritt (d) mit einem Amin der Formel $R_{20}NH_2$, worin $R_{20}$ wie vorstehend definiert ist, durch Behandeln mit einem dehydratisierenden Kupplungsmittel, gegebenenfalls Zusetzen eines Aktivierungsmittels und
(f) Cyclisieren des Produktes von Schritt (e) wie in Verfahren C beschrieben.

**23.** Verfahren zum Herstellen einer Verbindung der in Anspruch 22 definierten Formel II, worin $R_{22}$-F-Wasserstoff ist und $R_{21}$-E- und $R_{23}$-G- die relative trans-Stereochemie besitzen, umfassend:

(a) Enolisieren einer Verbindung der Formel

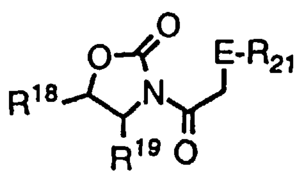

mit $TiCl_4$ und Tetramethylethylendiamin (TMEDA) , anschließend Kondensieren mit einem Imin der Formel $R_{23}$-G-CH=N-$R_{20}$, worin $R_{23}$-G- und $R_{20}$ wie in Anspruch 22 definiert sind und wobei die Gruppe

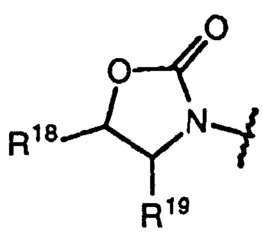

eine chirale Hilfsgruppe der Formel

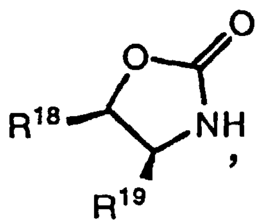 , 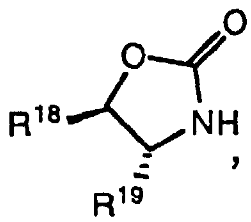 , 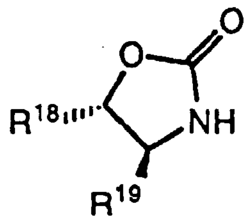 oder 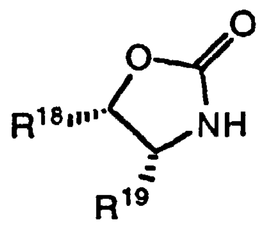

darstellt, worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, und
(b) Cyclisieren des Produktes aus Schritt (a) durch Behandeln mit einer starken nukleophilen Base.

**24.** Verfahren zum Herstellen einer Verbindung der in Anspruch 22 definierten Formel II, umfassend

(a) das Behandeln eines Esters der Formel $R_{21}$-E-CH(F-$R_{22}$)-C(O)O$R^{17}$, worin $R^{17}$ $C_{1-6}$-Alkyl, Menthyl oder 10- (Diisopropylsulfonamido)isobornyl ist, mit einer starken Base und anschließend mit einem Imin der Formel $R_{23}$-G-CH=N-$R_{20}$, worin $R_{23}$-G- und $R_{20}$ wie in Anspruch 22 definiert sind, oder
(b) zum Herstellen einer Verbindung der in Anspruch 22 definierten Formel II, worin $R_{22}$-F- nicht Wasserstoff ist, Umsetzen einer Verbindung der in Anspruch 22 definierten Formel II, worin $R_{22}$-F- Wasserstoff ist, mit einer starken Base und einem Alkylierungsmittel oder einem Acylierungsmittel oder
(c) zum Herstellen einer Verbindung der in Anspruch 22 definierten Formel II, worin $R_{22}$-F- Wasserstoff ist und $R_{21}$-E- und $R_{23}$-G- die relative cis-Stereochemie besitzen, Umsetzen einer Verbindung der in Anspruch 22 definierten Formel II, worin $R_{22}$-F-Wasserstoff ist und $R_{21}$-E- und $R_{23}$-G- die relative trans-Stereochemie besitzen, mit einer starken Base bei -80°C bis -40°C und anschließend mit einer Protonenquelle oder
(d) zum Herstellen einer Verbindung der in Anspruch 22 definierten Formel II, wobei $R_{22}$-F- Wasserstoff ist und $R_{21}$-E- und $R_{23}$-G- die relative trans-Stereochemie besitzen, das Umsetzen einer Verbindung der in Anspruch 22 definierten Formel II, wobei $R_{22}$-F-Wasserstoff ist und $R_{21}$-E- und $R_{23}$-G- die relative cis-Stereochemie besitzen, mit einem Alkalimetall-t-butoxid, anschließend Abbrechen mit Säure oder
(e) Umsetzen eines Carbonsäurederivates der Formel $R_{21}$-E-CH(F-$R_{22}$)-C(O)-Z, worin $R_{21}$-E- und $R_{22}$-F- wie in Anspruch 22 definiert sind und Z Cl,

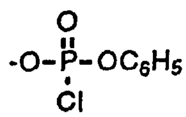 oder 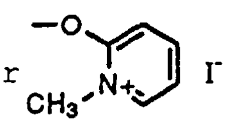

ist, mit einem Imin der Formel $R_{23}$-G-CH=N-$R_{20}$, worin $R_{23}$-G- und $R_{20}$ wie in Anspruch 22 definiert sind, in Anwesenheit einer tertiären Aminbase.

**Patentansprüche für folgenden Vertragsstaat : ES**

**1.** Verfahren zum Herstellen einer Verbindung mit der Strukturformel I

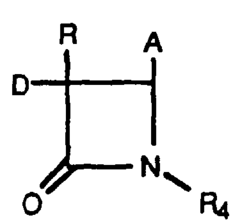

I

worin

A -CH=CH-B;

-C≡C-B;

$-(CH_2)_p$-X-B, worin p 0, 1 oder 2 ist und X eine Bindung, -NH- oder $-S(O)_{0-2}-$ ist;

Heteroaryl, benzokondensiertes Heteroaryl, W-substituiertes Heteroaryl oder W-substituiertes benzokondensiertes Heteroaryl, worin Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroaryl-Gruppen deren N-Oxide, und wobei W 1-3 Substituenten an den Ringkohlenstoffatomen ist, die aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino) -$C_{1-6}$-alkyl, $C_{3-6}$-Alkandioyl, $C_{1-6}$-Alkyl-$C_{3-6}$-alkandioyl, Allyloxy, $-CF_3$, $-OCF_3$, Benzyl, $R_{14}$-Benzyl, Benzyloxy, $R_{14}$-Benzyloxy, Phenoxy, $R_{14}$-Phenoxy, Dioxolanyl, $NO_2$, $-NR_{10}R_{11}$, $NR_{10}R_{11}$ ($C_{1-6}$-Alkyl)-, $NR_{10}R_{11}$ ($C_{1-6}$-Alkoxy)-, OH, Halogen, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-Butyldimethylsilyloxymethyl, $-C(O)R_{12}$ und

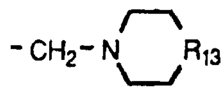

besteht und wobei,

sofern vorhanden, die Substituenten an den substituierten Heteroaryl-Stickstoffatomen aus der Gruppe ausgewählt sind, die aus $C_{1-6}$-Alkyl, $C_{1-6}$-Alkoxy, $-C(O)OR_5$, $-C(O)R_5$, OH, $NR_{10}R_{11}(C_{1-6})$-, $NR_{10}R_{11}$($C_{1-6}$-Alkoxy) -, $-S(O)_2NH_2$ und 2- (Trimethylsilyl)ethoxymethyl besteht;

-C(O)-B oder

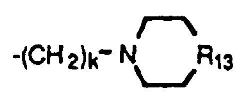

ist, worin k 1 oder 2 ist;

D $B'-(CH_2)_mC(O)-$, worin m 1, 2, 3, 4 oder 5 ist;

$B'-(CH_2)_q-$, worin q 2, 3, 4, 5 oder 6 ist;

$B'-(CH_2)_e-Z-(CH_2)_r-$, worin Z -O-, -C(O)-, Phenylen, $-NR_8-$ oder $-S(O)_{0-2}-$ ist, e 0, 1, 2, 3, 4 oder 5 ist und r 1, 2, 3, 4 oder 5 ist, vorausgesetzt, daß die Summe von e und r 1, 2, 3, 4, 5 oder 6 ist;

B'-($C_{2-6}$-Alkenylen) -, B'-($C_4$-$C_6$-Alkadienylen)-;

$B'-(CH_2)_t$-Z- ($C_2$-$C_6$-Alkenylen)-, worin Z wie vorstehend definiert ist und worin t 0, 1, 2 oder 3 ist, vorausgesetzt; daß die Summe von t und der Zahl der Kohlenstoffatome in der Alkylenkette 2, 3, 4, 5 oder 6 ist;

$B'-(CH_2)_f-V-(CH_2)_g-$, worin V $C_3$-$C_6$-Cycloalkylen ist, f 1, 2, 3, 4 oder 5 ist und g 0, 1, 2, 3, 4 oder 5 ist, vorausgesetzt, daß die Summe von f und g 1, 2, 3, 4, 5 oder 6 ist;

$B'-(CH_2)_t$-V-($C_2$-$C_6$-Alkenylen)- oder B'- ($C_2$-$C_6$-Alkenylen) $-V-(CH_2)_t-$, worin V und t wie vorstehend definiert sind, vorausgesetzt, daß die Summe von t und der Zahl der Kohlenstoffatome in der Alkenylenkette 2, 3, 4, 5 oder 6 ist;

B'- $(CH_2)_a$-Z- $(CH_2)_b$-V-$(CH_2)_d$-, worin Z und V wie vorstehend definiert sind und a, b und d unabhängig 0, 1, 2, 3, 4, 5 oder 6 sind, vorausgesetzt, daß die Summe von a, b und d 0, 1, 2, 3, 4, 5 oder 6 ist;

$T-(CH_2)_s-$, worin T Cycloalkyl mit 3-6 Kohlenstoffatomen ist und s 1, 2, 3, 4, 5 oder 6 ist, oder

Naphthylmethyl, Heteroarylmethyl oder W-substituiertes Heteroarylmethyl ist, worin Heteroaryl und W wie vorstehend definiert sind;

B

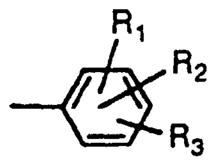

ist;

B' Naphthyl, Heteroaryl oder W-substituiertes Heteroaryl, worin Heteroaryl wie vorstehend definiert ist, oder

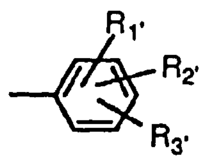

ist;

R Wasserstoff, Fluor, $C_1$-$C_{15}$-Alkyl, $C_2$-$C_{15}$-Alkenyl, $C_2$-$C_{15}$-Alkinyl oder B-$(CH_2)_h$- ist, worin h 0, 1, 2 oder 3 ist;

$R_1$, $R_2$ und $R_3$ unabhängig aus der Gruppe ausgewählt sind, die aus H, $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl, $C_{1-6}$-Alkyl-$C_{1-6}$-alkandioyl, Allyloxy, -$CF_3$, -$OCF_3$, Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$ ($C_{1-6}$-Alkyl)-, $NR_{10}R_{11}$ ($C_{1-6}$-Alkoxy)-, OH, o-Halogen, m-Halogen, -NHC(O)$OR_5$, -NHC(O)$R_5$, $R_6O_2$SNH-, $(R_6O_2S)_2$N-, -$S(O)_2NH_2$, -$S(O)_{0-2}R_{10}$, tert-Butyldimethylsilyloxymethyl, - C(O)$R_{12}$

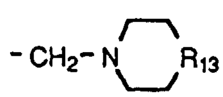 und 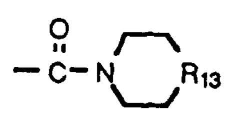

besteht,
oder $R_1$ Wasserstoff ist und $R_2$ und $R_3$ zusammen mit den benachbarten Kohlenstoffatomen, an die sie gebunden sind, einen Dioxolanylring bilden;

$R_{1'}$, $R_{2'}$ und $R_{3'}$ unabhängig aus der Gruppe ausgewählt sind, die aus H, $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl, $C_{1-6}$-Alkyl-$C_{3-6}$-alkandioyl, Allyloxy, -$CF_3$, -$OCF_3$, Benzyl, $R_{14}$-Benzyl, Benzyloxy, $R_{14}$-Benzyloxy, Phenoxy, $R_{14}$-Phenoxy, Dioxolanyl, $NO_2$, -$NR_{10}R_{11}$, $NR_{10}R_{11}$ ($C_{1-6}$-Alkyl)-, $NR_{10}R_{11}$ ($C_{1-6}$-Alkoxy)-, OH, Halogen, -NHC(O)$OR_5$, -NHC(O)$R_5$, $R_6O_2$SNH-, $(R_6O_2S)_2$N-, -$S(O)_2NH_2$, -S $(O)_{0-2}R_{10}$, tert-Butyldimethylsilyloxymethyl, -C(O)$R_{12}$,

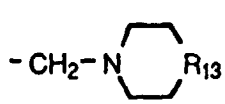 und 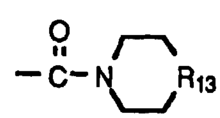

besteht, oder $R_{1'}$ Wasserstoff ist und $R_{2'}$ und $R_{3'}$ zusammen mit den benachbarten Kohlenstoffatomen, an die sie gebunden sind, einen Dioxolanylring bilden;

$R_4$ 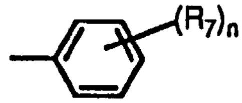 ,

worin n 0, 1, 2 oder 3 ist, Indanyl, Benzofuranyl, Benzodioxolyl, Tetrahydronaphthyl, Pyridyl, Pyrazinyl, Pyrimidinyl oder Chinolyl ist;

$R_5$ $C_{1-6}$-Alkyl, Phenyl, $R_{14}$-Phenyl, Benzyl oder $R_{14}$-Benzyl ist;

$R_6$ OH, $C_{1-6}$-Alkyl, Phenyl, Benzyl, $R_{14}$-Phenyl oder $R_{14}$-Benzyl ist;

$R_7$ $C_{1-6}$-Alkyl, $C_{1-6}$-Alkoxy, OH, Halogen, $-NR_{10}R_{11}$, $-NHC(O)OR_5$, $-NHC(O)R_5$, $NO_2$, -CN, $-N_3$, -SH, $-S(O)_{0-2}-(C_{1-6}$-Alkyl), $-COOR_9$, $-CONR_{10}R_{11}$, $-COR_{12}$, Phenoxy, Benzyloxy, $-OCF_3$ oder tert-Butyldimethylsilyloxy ist und wenn n 2 oder 3 ist, die Gruppen $R_7$ gleich oder verschieden sein können;

$R_8$ H, $C_{1-6}$-Alkyl, Phenyl-$C_{1-6}$-alkyl oder $-C(O)R_9$ ist;

$R_9$ H, $C_{1-6}$-Alkyl oder Phenyl-$C_{1-6}$-alkyl ist;

$R_{10}$ und $R_{11}$ unabhängig aus H und $C_{1-6}$-Alkyl ausgewählt sind;

$R_{12}$ H, OH, $C_{1-6}$-Alkoxy, Phenoxy, Benzyloxy,

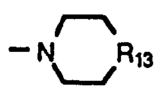

$-NR_{10}R_{11}$, $C_{1-6}$-Alkyl, Phenyl oder $R_{14}$-Phenyl ist;

$R_{13}$ -O-, $-CH_2-$, -NH- oder $-N(C_{1-6}$-Alkyl)- ist und

$R_{14}$ 1-3 Gruppen ist, die unabhängig aus der Gruppe ausgewählt sind, die aus $C_{1-6}$-Alkyl, $C_{1-6}$-Alkoxy, -COOH, $NO_2$, $-NR_{10}R_{11}$, OH oder Halogen besteht,
oder eines pharmazeutisch annehmbaren Salzes derselben,
wobei R H ist und D und A eine relative trans-Stereochemie besitzen, umfassend

Verfahren A: das Cyclisieren eines Hydroxyamids der Formel

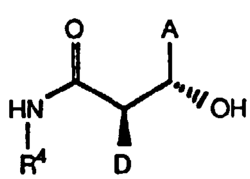 oder 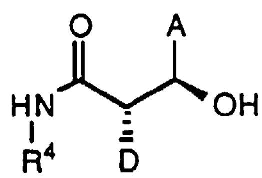

worin D, A und $R^4$ wie vorstehend 1 definiert sind, durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder

(iii)einem Dialkylchlorphosphat, einer wäßrigen Base und einem Phasentransferkatalysator oder
(iv) einem Di- oder Trichlorbenzoylchlorid und einem Alkalimetallhydrid oder

Verfahren B:

(a) das Umsetzen einer Carbonsäure der Formel $DCH_2$-COOH, worin D wie vorstehend definiert ist, mit einem Chlorierungsmittel,
(b) Deprotonieren eines chiralen Oxazolidinons der Formel

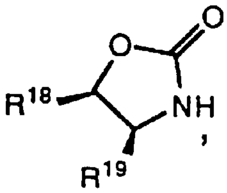 , 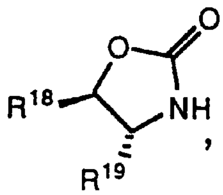 , 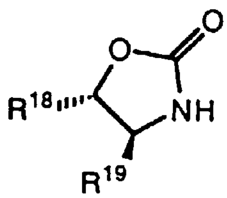 oder 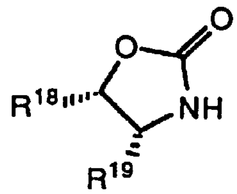

worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, mit einer starken Base und Behandeln des sich daraus ergebenden Anions mit dem Produkt aus Schritt (a),
(c) Enolisieren des Produkts aus Schritt (b) entweder mit:

(i) einem Dialkylbortriflat und einer tertiären Aminbase
(ii) $TiCl_4$ und Tetramethylethylendiamin (TMEDA) oder einem Gemisch aus TMEDA und Triethylamin,

anschließend Kondensieren mit einem Aldehyd der Formel A-CHO, worin A wie vorstehend definiert ist,
(d) Hydrolysieren des Produktes von Schritt (c) mit einer Base und Wasserstoffperoxid,
(e) Kondensieren des Produkts von Schritt (d) mit einem Amin der Formel $R^4NH_2$, worin $R^4$ wie vorstehend definiert ist, durch Behandeln mit einem dehydratisierenden Kupplungsmittel, gegebenenfalls Zusetzen eines Aktivierungsmittels und
(f) Cyclisieren des Produktes wie vorstehend in Verfahren A beschrieben.

**2.** Verfahren zum Herstellen einer Verbindung der in Anspruch 1 definierten Formel I, wobei R Wasserstoff ist und D und A eine relative trans-Stereochemie besitzen, umfassend:

(a) das Enolisieren einer Verbindung der Formel

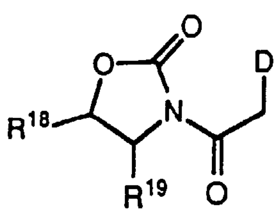

mit $TiCl_4$ und Tetramethylethylendiamin (TMEDA), anschließend Kondensieren mit einem Imin der Formel A-CH=N-$R^4$, wobei A und $R^4$ wie vorstehend definiert sind, und wobei die Gruppe

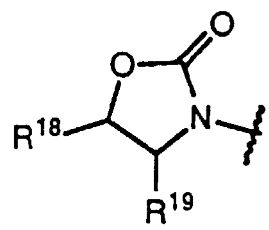

einen chiralen Hilfsstoff der Formel

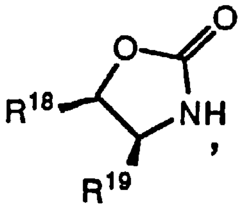 , 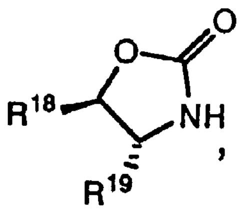 , 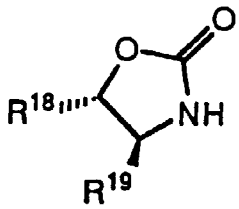 oder 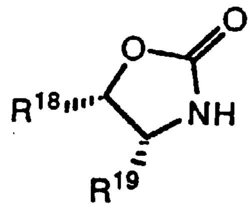

darstellt, worin $R^{18}$ und $R^{19}$ unabhängig aus der Gruppe ausgewählt sind, die aus Wasserstoff, $C_1$-$C_6$-Alkyl, Phenyl, Naphthyl, substituiertem Phenyl, substituiertem Naphthyl und Benzyl besteht, und
(b) Cyclisieren des Produkts aus Schritt (a) durch Behandeln mit einer starken nicht-nukleophilen Base.

**3.** Verfahren zum Herstellen von Verbindungen der in Anspruch 1 definierten Formel I, umfassend:

(a) das Behandeln eines Esters der Formel 1 D-CH(R)-C(O)$OR^{17}$, worin $R^{17}$ $C_{1-6}$-Alkyl, Menthyl oder 10-(Diisopropylsulfonamido) isobornyl ist, mit einer starken Base und anschließend mit einem Imin der Formel A-CH=N-$R^4$, worin A und $R^4$ wie in Anspruch 1 definiert sind, oder
(b) zum Herstellen einer Verbindung der in Anspruch 1 definierten Formel I, worin R nicht Wasserstoff ist, das Umsetzen einer Verbindung der in Anspruch 1 definierten Formel I, worin R Wasserstoff ist, mit einer starken Base und einem Alkylierungsmittel oder einem Acylierungsmittel oder
(c) zum Herstellen einer Verbindung der in Anspruch 1 definierten Formel I, worin R Wasserstoff ist und D und A die relative cis-Stereochemie besitzen, das Umsetzen einer Verbindung der in Anspruch 1 definierten Formel I, worin R Wasserstoff ist und D und A die relative trans-Stereochemie besitzen, mit einer starken Base bei -80°C bis -40°C und anschließend mit einer Protonenquelle oder
(d) zum Herstellen einer Verbindung der in Anspruch 1 definierten Formel I, wobei R Wasserstoff ist und D und A die relative trans-Stereochemie besitzen, das Umsetzen einer Verbindung der in Anspruch 1 definierten Formel I, worin R Wasserstoff ist und D und A die relative cis-Stereochemie besitzen, mit einem Alkalimetall-t-butoxid, anschließend Abbrechen mit Säure oder
(e) Umsetzen eines Carbonsäurederivates der Formel D-CH(R)-C(O)-Z, worin D und R wie in Anspruch 1 definiert sind und Z Cl,

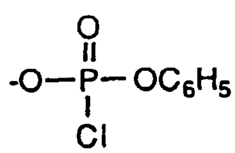 oder 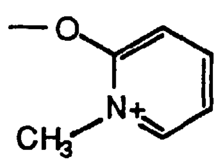 $I^-$

ist, mit einem Imin der Formel -A-CH=N$R^4$, worin A und $R^4$ wie in Anspruch 1 definiert sind, in Anwesenheit einer tertiären Aminbase.

**4.** Verfahren zum Herstellen einer Verbindung der Formel II

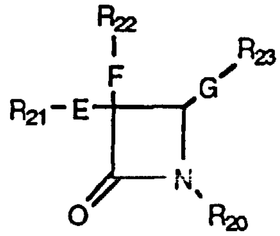

II

worin

$R_{20}$ Phenyl, W-substituiertes Phenyl, Naphthyl, W-substituiertes Naphthyl, Benzodioxolyl, Heteroaryl, W-substituiertes Heteroaryl, benzokondensiertes Heteroaryl und W-substituiertes benzokondensiertes Heteroaryl ist, wobei Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroarylgruppen das N-Oxid derselben ist;

$R_{21}$, $R_{22}$ und $R_{23}$ unabhängig aus H oder $R_{20}$ ausgewählt sind;

W 1 bis 3 Substituenten ist, die unabhängig aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl, $C_{1-6}$-Alkyl-$C_{3-6}$-alkandioyl, Allyloxy, $-CF_3$, $-OCF_3$, Benzyl, $R_{14}$-Benzyl, Benzyloxy, $R_{14}$-Benzyloxy, Phenoxy, $R_{14}$-Phenoxy, Dioxolanyl, $NO_2$, $-NR_{10}R_{11}$, $NR_{10}R_{11}$ ($C_{1-6}$-Alkyl)-, $NR_{10}R_{11}$ ($C_{1-6}$-Alkoxy)-, OH, Halogen, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-Butyldimethylsilyloxymethyl, $-C(O)R_{12}$,

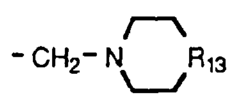

und

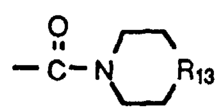

besteht;

E, F und G unabhängig eine Bindung, $C_{3-6}$-Cycloalkylen, $C_I$-$C_{10}$-Alkylen, $C_2$-$C_{10}$-Alkenylen, $C_2$-$C_{10}$-Alkinylen, eine definierte Alkylen-, Alkenylen- oder Alkinylenkette, die durch einen oder mehrere Substituenten substituiert ist, der unabhängig aus der Gruppe ausgewählt sind, die aus Phenyl, W-substituiertem Phenyl, Heteroaryl und W-substituiertem Heteroaryl besteht, wobei Heteroaryl wie vorstehend definiert ist, eine definierte Alkylen-, Alkenylen- oder Alkinylenkette, die durch eine oder mehr Gruppen unterbrochen ist, die unabhängig aus der Gruppe ausgewählt sind, die aus -O-, -S-, -SO-, $-SO_2-$, $-NR_8$, -C(O)-, $C_3$-$C_6$-Cycloalkylen, Phenylen, W-substituiertem Phenylen, Heteroarylen und W-substituiertem Heteroarylen besteht, oder eine definierte unterbrochene Alkylen-, Alkenylen- oder Alkinylengruppe sind, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, oder eines von $R_{21}$-E und $R_{22}$-F aus der Gruppe ausgewählt ist, die aus Halogen, OH, $C_{1-6}$-Alkoxy, $-OC(O)R_5$, $-NR_{10}R_{11}$, -SH oder -S($C_{1-6}$-Alkyl) besteht;

$R_5$ $C_{1-6}$-Alkyl, Phenyl, $R_{14}$-Phenyl, Benzyl oder $R_{14}$-Benzyl ist;

$R_6$ OH, $C_{1-6}$-Alkyl, Phenyl, Benzyl, $R_{14}$-Phenyl oder $R_{14}$-Benzyl ist;

$R_8$ H, $C_{1-6}$-Alkyl, Phenyl-$C_{1-6}$-alkyl oder -C(O)$R_9$ ist;

$R_9$ H, $C_{1-6}$-Alkyl, Phenyl oder Phenyl-$C_{1-6}$-alkyl ist;

$R_{10}$ und $R_{11}$ unabhängig aus H und $C_{1-6}$-Alkyl ausgewählt sind,

$R_{12}$ H, OH, $C_{1-6}$-Alkoxy, Phenoxy, Benzyloxy,

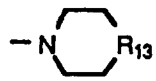 ,

-$NR_{10}R_{11}$, $C_{1-6}$-Alkyl, Phenyl oder $R_{14}$-Phenyl ist;

$R_{13}$ -O-, -$CH_2$-, -NH- oder -N($C_{1-6}$-Alkyl)- ist;

$R_{14}$ 1-3 Gruppen ist, die unabhängig aus der Gruppe ausgewählt sind, die aus $C_{1-6}$-Alkyl, $C_{1-6}$-Alkoxy, -COOH, $NO_2$, -$NR_{10}R_{11}$, OH oder Halogen besteht;
vorausgesetzt, daß wenn G eine Bindung ist, $R_{23}$ nicht H ist und vorausgesetzt, daß wenn $R_{23}$ W-substituiertes Phenyl ist, W nicht p-Halogen ist,
oder eines pharmazeutisch annehmbaren Salzes derselben, wobei $R_{22}$-F H ist und $R_{21}$-E- und $R_{23}$-G- die relative trans-Stereochemie besitzen, umfassend

Verfahren C: das Cyclisieren eines Hydroxyamids der Formel

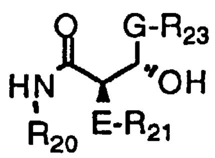 oder 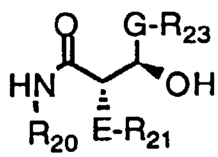

worin $R_{21}$-E-, $R_{23}$-G- und $R_{20}$ wie vorstehend definiert sind, durch Behandeln mit:

(i) einem Azodicarbonsäuredialkylester und einem Trialkylphosphin oder
(ii) einem Di- oder Trichlorbenzoylchlorid, einer wäßrigen Base und einem Phasentransferkatalysator, anschließend Behandeln des sich daraus ergebenden Di- oder Trichlorbenzoesäureesters mit einer wäßrigen Base und einem Phasentransferkatalysator oder
(iii)einem Dialkylchlorphosphat, einer wäßrigen Base und einem Phasentransferkatalysator oder
(iv) einem Di- oder Trichlorbenzoylchlorid und einem Alkalimetallhydrid oder

Verfahren D:

(a) das Umsetzen einer Carbonsäure der Formel $R_{21}$-E-$CH_2$-COOH, wobei $R_{21}$-E- wie vorstehend definiert ist, mit einem Chlorierungsmittel,
(b) Deprotonieren eines chiralen Oxazolidinons der Formel

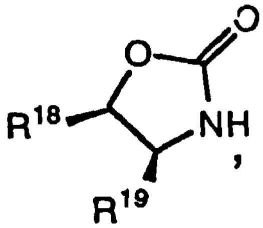 , 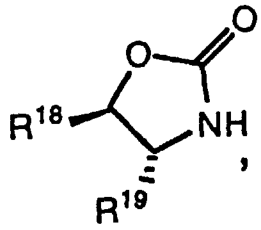 , 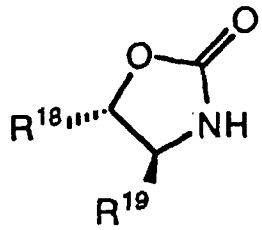 oder 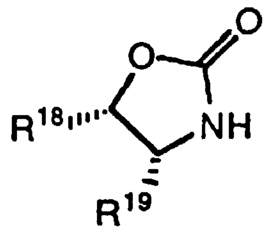 .

worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, mit einer starken Base und Behandeln des sich daraus ergebenden Anions mit dem Produkt aus Schritt (a),

(c) Enolisieren des Produkts aus Schritt (b) entweder mit:

(i) einem Dialkylbortriflat und einer tertiären Aminbase

(ii) $TiCl_4$ und Tetramethylethylendiamin (TMEDA) oder einem Gemisch aus TMEDA und Triethylamin,

anschließend Kondensieren mit einem Aldehyd der Formel $R_{23}$-G-CHO, worin $R_{23}$-G- wie vorstehend definiert ist,

(d) Hydrolysieren des Produktes von Schritt (c) mit einer Base und Wasserstoffperoxid,

(e) Kondensieren des Produkts von Schritt (d) mit einem Amin der Formel $R_{20}NH_2$, worin $R_{20}$ wie vorstehend definiert ist, durch Behandeln mit einem dehydratisierenden Kupplungsmittel, gegebenenfalls Zusetzen eines Aktivierungsmittels und

(f) Cyclisieren des Produktes von Schritt (e) wie in Verfahren C beschrieben.

**5.** Verfahren zum Herstellen einer Verbindung der in Anspruch 4 definierten Formel II, worin $R_{22}$-F- Wasserstoff ist und $R_{21}$-E- und $R_{23}$-G- die relative trans-Stereochemie besitzen, umfassend:

(a) Enolisieren einer Verbindung der Formel

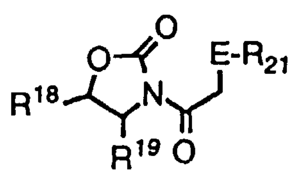

mit $TiCl_4$ und Tetramethylethylendiamin (TMEDA), anschließend Kondensieren mit einem Imin der Formel $R_{23}$-G-CH=N-$R_{20}$, worin $R_{23}$-G- und $R_{20}$ wie in Anspruch 4 definiert sind und wobei die Gruppe

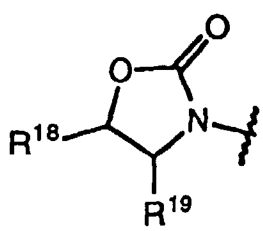

eine chirale Hilfsgruppe der Formel

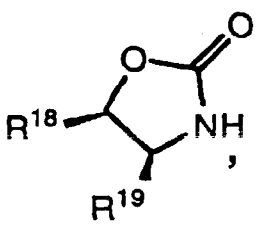 , 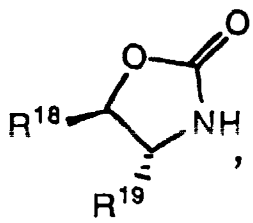 , 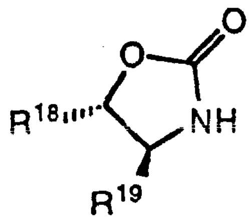 oder 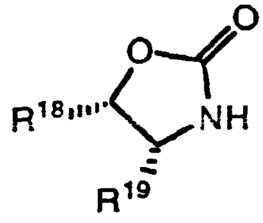

darstellt, worin $R^{18}$ und $R^{19}$ unabhängig aus der Gruppe ausgewählt sind, die aus Wasserstoff, $C_{1-6}$-Alkyl, Phenyl, Naphthyl, substituiertem Phenyl, substituiertem Naphthyl und Benzyl besteht, und
(b) Cyclisieren des Produktes aus Schritt (a) durch Behandeln mit einer starken nukleophilen Base.

**6.** Verfahren zum Herstellen einer Verbindung der in Anspruch 4 definierten Formel II, umfassend

(a) das Behandeln eines Esters der Formel 1 $R_{21}$-E-CH(F-$R_{22}$)-C(O)$OR^{17}$, worin $R^{17}$ $C_{1-6}$-Alkyl, Menthyl oder 10- (Diisopropylsulfonamido)isobornyl ist, mit einer starken Base und anschließend mit einem Imin der Formel $R_{23}$-G-CH=N-$R_{20}$, worin $R_{23}$-G- und $R_{20}$ wie in Anspruch 4 definiert sind, oder
(b) zum Herstellen einer Verbindung der in Anspruch 4 definierten Formel II, worin $R_{22}$-F- nicht Wasserstoff ist, Umsetzen einer Verbindung der in Anspruch 4 definierten Formel II, worin $R_{22}$-F- Wasserstoff ist, mit einer starken Base und einem Alkylierungsmittel oder einem Acylierungsmittel oder
(c) zum Herstellen einer Verbindung der in Anspruch 4 definierten Formel II, worin $R_{22}$-F- Wasserstoff ist und $R_{21}$-E- und $R_{23}$-G- die relative cis-Stereochemie besitzen, Umsetzen einer Verbindung der in Anspruch 4 definierten Formel II, worin $R_{22}$-F-Wasserstoff ist und $R_{21}$-E- und $R_{23}$-G- die relative trans-Stereochemie besitzen, mit einer starken Base bei -80°C bis -40°C und anschließend mit einer Protonenquelle oder
(d) zum Herstellen einer Verbindung der in Anspruch 4 definierten Formel II, worin $R_{22}$-F- Wasserstoff ist und $R_{21}$-E- und $R_{23}$-G-die relative trans-Stereochemie besitzen, das Umsetzen einer Verbindung der in Anspruch 4 definierten Formel II, worin $R_{22}$-F-Wasserstoff ist und $R_{21}$-E- und $R_{23}$-G- die relative cis-Stereochemie besitzen,mit einem Alkalimetall-t-butoxid, anschließend Abbrechen mit Säure oder
(e) Umsetzen eines Carbonsäurederivates der Formel $R_{21}$-E-CH(F-$R_{22}$)-C(O)-Z, worin $R_{21}$-E- und $R_{22}$-F- wie in Anspruch 4 definiert sind und Z Cl,

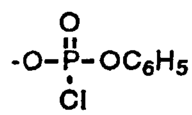 oder 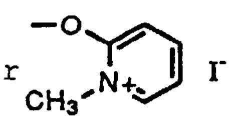

ist, mit einem Imin der Formel $R_{23}$-G-CH=N-$R_{20}$, worin $R_{23}$-G- und $R_{20}$ wie in Anspruch 4 definiert sind, in Anwesenheit einer tertiären Aminbase.

**7.** Verfahren von Anspruch 1, wobei R Wasserstoff ist.

**8.** Verfahren von Anspruch 1 oder 7, wobei D B'-$(CH_2)_q$-, B'- $(CH_2)_e$-Z- $(CH_2)_r$-, B'- ($C_2$-$C_6$-Alkenylen) - oder B'- $(CH_2)_f$- V- $(CH_2)_g$- ist, worin B', Z, V, q, e, r, f und g wie in Anspruch 1 definiert sind.

**9.** Verfahren von Anspruch 8, wobei D B'-$(CH_2)_q$-, worin B' Phenyl ist und q 3 oder 4 ist; B'-$(CH_2)_e$-Z- $(CH_2)_r$-, w worin B' p-Fluorphenyl oder p-Methoxyphenyl ist, e Null ist, Z -O- ist und r 2 ist; B'-($C_2$-$C_6$-Alkenylen)-3-Phenyl-1-propenyl ist oder B'-$(CH_2)_f$-V-$(CH_2)_g$- ist, worin B' Phenyl ist, f 1 ist, V Cyclopropylen ist und g null ist.

**10.** Verfahren von Anspruch 1, 7, 8 oder 9, wobei A -$(CH_2)_p$-X-B ist, worin X, B und p wie in Anspruch 1 definiert sind.

**11.** Verfahren von Anspruch 10, wobei p null ist und X eine Bindung ist.

**12.** Verfahren von Anspruch 10, wobei $R_1$, $R_2$ und $R_3$ aus der Gruppe ausgewählt sind, die aus H, OH, $C_{1-6}$-Alkoxy, $NO_2$, $C_{1-6}$-Alkoxy-$C_{1-6}$-alkoxy, m-Halogen, $C_{1-6}$-Alkyl-$C_{3-6}$-alkandioyl, $NR_{10}R_{11}$($C_{1-6}$-Alkoxy)-, Allyloxy, Phenoxy, $C_{1-6}$-Alkoxycarbonyl-$C_{1-6}$-alkoxy und -C(O)$R_{12}$ besteht.

**13.** Verfahren von Anspruch 1, 7, 8, 9, 10 oder 11, wobei $R_4$ Phenyl, $R_7$-substituiertes Phenyl oder Indanyl ist.

**14.** Verfahren von Anspruch 13, wobei $R_7$ aus der Gruppe ausgewählt ist, die aus $C_{1-6}$-Alkyl, $C_{1-6}$-Alkoxy, Halogen, $-OCF_3$, $C_{1-6}$-Alkylthio, $-NR_{10}R_{11}$, -CN, OH und $-COR_{12}$ besteht.

**15.** Verfahren zum Herstellen einer pharmazeutischen Zusammensetzung zur Verwendung bei der Herstellung eines Arzneimittels zum Erniedrigen von Serumcholesterin, wobei das Verfahren das Vermischen eines pharmazeutisch annehmbaren Trägers mit einer Verbindung mit der Struktur

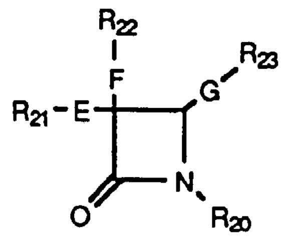

worin

$R_{20}$ Phenyl, W-substituiertes Phenyl, Naphthyl, W-substituiertes Naphthyl, Benzodioxolyl, Heteroaryl, W-substituiertes Heteroaryl, benzokondensiertes Heteroaryl und W-substituiertes benzokondensiertes Heteroaryl, wobei Heteroaryl aus der Gruppe ausgewählt ist, die aus Pyrrolyl, Pyridinyl, Pyrimidinyl, Pyrazinyl, Triazinyl, Imidazolyl, Thiazolyl, Pyrazolyl, Thienyl, Oxazolyl und Furanyl besteht, und bei stickstoffhaltigen Heteroaryl-Gruppen deren N-Oxid ist;

$R_{21}$, $R_{22}$ und $R_{23}$ unabhängig aus H oder $R_{20}$ ausgewählt sind;

W 1 bis 3 Substituenten ist, die unabhängig aus der Gruppe ausgewählt sind, die aus $C_{1-6}$-Alkyl, Hydroxy-$C_{1-6}$-alkyl, $C_{1-6}$-Alkoxy, $C_{1-6}$-Alkoxy-$C_{1-6}$-alkyl, $C_{1-6}$-Alkoxy-$C_{1-6}$-alkoxy, $C_{1-6}$-Alkoxycarbonyl-$C_{1-6}$-alkoxy, ($C_{1-6}$-Alkoxyimino)-$C_{1-6}$-alkyl, $C_{3-6}$-Alkandioyl, $C_{1-6}$-Alkyl-$C_{3-6}$-alkandioyl, Allyloxy, $-CF_3$, $-OCF_3$, Benzyl, $R_{14}$-Benzyl, Benzyloxy, $R_{14}$-Benzyloxy, Phenoxy, $R_{14}$-Phenoxy, Dioxolanyl, $NO_2$, $-NR_{10}R_{11}$, $NR_{10}R_{11}$ ($C_{1-6}$-Alkyl)-, $NR_{10}R_{11}$ ($C_{1-6}$-Alkoxy)-, OH, Halogen, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-Butyldimethylsilyloxymethyl, $-C(O)R_{12}$,

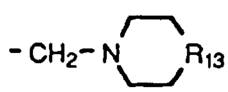 und 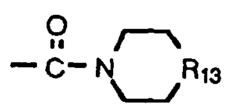

besteht;

E, F und G unabhängig eine Bindung, $C_{3-6}$-Cycloalkylen, $C_1$-$C_{10}$-Alkylen, $C_2$-$C_{10}$-Alkenylen, $C_2$-$C_{10}$-Alkinylen, eine definierte Alkylen-, Alkenylen- oder Alkinylenkette, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, wobei Heteroaryl wie vorstehend definiert ist, eine definierte Alkylen-, Alkenylen- oder Alkinylenkette, die durch eine oder mehr Gruppen unterbrochen ist, die unabhängig aus der Gruppe ausgewählt sind, die aus -O-, -S-, -SO-, $-SO_2-$, $-NR_8$, -C(O)-, $C_3$-$C_6$-Cycloalkylen, Phenylen, W-substituiertem Phenylen, Heteroarylen und W-substituiertem Heteroarylen besteht, oder eine definierte unterbrochene Alkylen-, Alkenylen- oder Alkinylengruppe sind, die durch einen oder mehrere Substituenten substituiert ist, die unabhängig aus der Gruppe ausgewählt sind, die aus Phenyl, W-substituiertem Phenyl, Heteroaryl und W-substituiertem Heteroaryl besteht, oder eines von $R_{21}$-E und $R_{22}$-F aus der Gruppe ausgewählt ist, die aus

Halogen, OH, $C_{1-6}$-Alkoxy, -OC(O)$R_5$, -$NR_{10}R_{11}$, -SH oder -S($C_{1-6}$-Alkyl) besteht;

| | |
|---|---|
| $R_5$ | $C_{1-6}$-Alkyl, Phenyl, $R_{14}$-Phenyl, Benzyl oder $R_{14}$-Benzyl ist; |
| $R_6$ | OH, $C_{1-6}$-Alkyl, Phenyl, Benzyl, $R_{14}$-Phenyl oder $R_{14}$-Benzyl ist; |
| $R_8$ | H, $C_{1-6}$-Alkyl, Phenyl-$C_{1-6}$-alkyl oder -C(O)$R_9$ ist; |
| $R_9$ | H, $C_{1-6}$-Alkyl, Phenyl oder Phenyl-$C_{1-6}$-alkyl ist; |
| $R_{10}$ und $R_{11}$ | unabhängig aus H und $C_{1-6}$-Alkyl ausgewählt sind; |
| $R_{12}$ | H, OH, $C_{1-6}$-Alkoxy, Phenoxy, Benzyloxy, 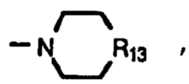 , -$NR_{10}R_{11}$, $C_{1-6}$-Alkyl, Phenyl oder $R_{14}$-Phenyl ist; |
| $R_{13}$ | -O-, -$CH_2$-, -NH- oder -N($C_{1-6}$-Alkyl)- ist; |
| $R_{14}$ | 1-3 Gruppen ist, die unabhängig aus der Gruppe ausgewählt sind, die aus $C_{1-6}$-Alkyl, $C_{1-6}$-Alkoxy, -COOH, $NO_2$, -$NR_{10}R_{11}$, OH oder Halogen besteht; |

vorausgesetzt, daß wenn G eine Bindung ist, $R_{23}$ nicht H ist und vorausgesetzt, daß wenn $R_{23}$ W-substituiertes Phenyl ist, W nicht p-Halogen ist,
oder einem pharmazeutisch annehmbaren Salz derselben in einem pharmazeutisch annehmbaren Träger umfaßt.

**16.** Verfahren von Anspruch 1, wobei die hergestellten Verbindungen aus der Gruppe ausgewählt sind, die aus Verbindungen der Formel

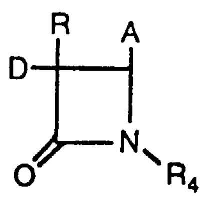

besteht, worin D, R, A und $R_4$ und die relative Stereochemie zwischen D und A wie in der folgenden Tabelle definiert sind:

| D | R | A | relative Stereo-chemie | $R_4$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-$NO_2$-$C_6H_4$- | 3R,4R | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | 3-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 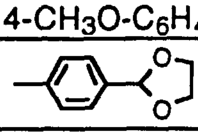 | trans | p-$CH_3O$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | 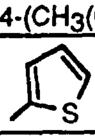 | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($C_6H_5$-O)-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 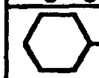 $(CH_2)_3$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-($(CH_3)_2N$-$(CH_2)_3$-O)-$C_6H_4$- | cis | 4-$CH_3O$-$C_6H_4$- |

| | | | | |
|---|---|---|---|---|
| $C_6H_5-(CH_2)_3-$ | H | $4-CH_3O-C_6H_4-$ | cis | $C_6H_5-$ |
| $C_6H_5-(CH_2)_3-$ | H | $4-CH_3O-C_6H_4-$ | 3S,4S | $p-CH_3O-C_6H_4-$ |
| $C_6H_5-(CH_2)_3-$ | H | $p-CH_3O-C_6H_4-$ | 3S,4R | $p-CH_3O-C_6H_4-$ |
| $C_6H_5-(CH_2)_3-$ | H | $4-CH_3O-C_6H_4-$ | trans | $4-F-C_6H_4-$ |
| $C_6H_5-(CH_2)_3-$ | H | $4-CH_3O-C_6H_4-$ | trans | $3-CH_3O-C_6H_4-$ |
| $C_6H_5-(CH_2)_3-$ | H | $4-CH_3O-C_6H_4-$ | trans | $4-Cl-C_6H_4-$ |
| $C_6H_5-(CH_2)_3-$ | H | $4-CH_3O-C_6H_4-$ | trans | $4-(CH_3CH_2-O)-C_6H_4-$ |
| $C_6H_5-(CH_2)_3-$ | H | $4-CH_3O-C_6H_4-$ | trans | $4-CH_3S-C_6H_4-$ |
| $C_6H_5-(CH_2)_3-$ | H | $4-CH_3O-C_6H_4-$ | trans | $4-CH_3-C_6H_4-$ |
| $C_6H_5-(CH_2)_3-$ | H | $4-CH_3O-C_6H_4-$ | trans | $4-(C_6H_5-O)-C_6H_4-$ |
| 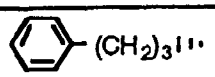 | H | 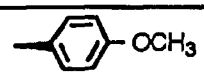 | trans optisch rein | $C_6H_5-$ |
| 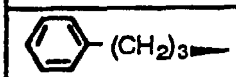 | H | 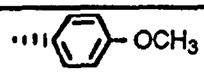 | trans optisch rein | $C_6H_5-$ |
| $C_6H_5-(CH_2)_3-$ | H | $4-(CH_3CH_2-O)-C_6H_4-$ | trans | $4-CH_3O-C_6H_4-$ |
| $C_6H_5-(CH_2)_3-$ | H | $4-(C_6H_5-CH_2-O)-C_6H_4-$ | trans | $4-CH_3O-C_6H_4-$ |
| $C_6H_5-(CH_2)_3-$ | H | $4-(CH_2=CH-CH_2-O)-C_6H_4-$ | trans | $4-CH_3O-C_6H_4-$ |
| $C_6H_5-(CH_2)_3-$ | H | $4-(C_6H_5-O)-C_6H_4-$ | trans | $4-CH_3O-C_6H_4-$ |
| $C_6H_5-O-(CH_2)_2-$ | H | $4-CH_3O-C_6H_4-$ | trans | $4-CH_3O-C_6H_4-$ |
| $4-F-C_6H_4-O-(CH_2)_2-$ | H | $4-CH_3O-C_6H_4-$ | trans | $4-CH_3O-C_6H_4-$ |
| $C_6H_5-(CH_2)_4-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $C_6H_5-$ |
| $C_6H_5-(CH_2)_3-$ | H | $4-CH_3O-C_6H_4-$ | trans | $4-(CH_3CH_2OC(O))-C_6H_4-$ |
| $C_6H_5-(CH_2)_3-$ | H | $4-CH_3O-C_6H_4-$ | trans | $3-NO_2-C_6H_4-$ |
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 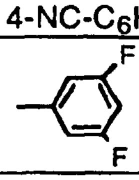 |
| $C_6H_5-(CH_2)_3-$ | H | $4-CH_3O-C_6H_4-$ | trans | $4-(CH_3C(O))-C_6H_4-$ |

| | | | | |
|---|---|---|---|---|
| $C_6H_5$-$(CH_2)_3$- | H | 4-$CH_3O$-$C_6H_4$- | trans | 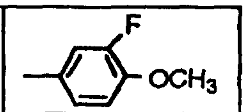 |
| $C_6H_5$-$(CH_2)_3$- | H | 4-$CH_3O$-$C_6H_4$- | trans | 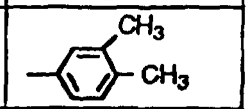 |
| $C_6H_5$-$(CH_2)_3$- | H | 4-($(CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 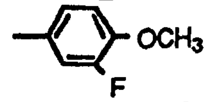 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 4-($(CH_3)_2N$)-$C_6H_4$- | trans | 4-$CH_3O$-$C_6H_4$- |
| $C_6H_5$-$(CH_2)_3$- | H | 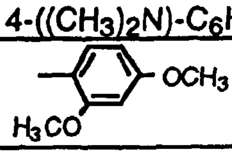 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| 4-$O_2N$-$C_6H_4$-$(CH_2)_3$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | 4-I-$C_6H_4$- |
| $C_6H_5$-$(CH_2)_3$- | H | 4-($CH_3(CH_2)_3$-O)-$C_6H_4$- | trans | 4-$CH_3O$-$C_6H_4$- |
| 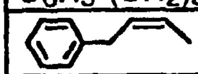 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 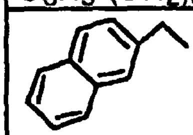 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 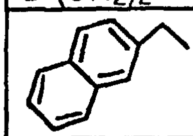 | H | 4-$CH_3O$-$C_6H_4$- | cis | $C_6H_5$- |
| $C_6H_5$-$(CH_2)_3$- | H | 4-$CH_3O$-$C_6H_4$- | trans | 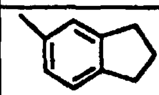 |
| $C_6H_5$-$(CH_2)_3$- | H | 4-$NH_2$-$C_6H_4$- | cis | 4-$CH_3O$-$C_6H_4$- |
| $C_6H_5$-$(CH_2)_3$- | H | 4-$CH_3O$-$C_6H_4$- | trans | 3-$NH_2$-$C_6H_4$- |
| $C_6H_5$-$(CH_2)_3$- | H | 4-OH-$C_6H_4$- | trans | 4-$CH_3O$-$C_6H_4$- |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-$CH_3O$-$C_6H_4$- | 3R,4S | 4-OH-$C_6H_4$- |

| | | | | |
|---|---|---|---|---|
| $C_6H_5$-$(CH_2)_3$- | H | 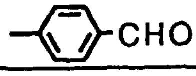 | trans | 4-$CH_3O$-$C_6H_4$- |
| $C_6H_5$-$(CH_2)_3$- | H | 4-$CH_3O$-$C_6H_4$- | trans | 4-($CH_3SO_2$)-$C_6H_4$- |
| $C_6H_5$-$(CH_2)_3$- | H | 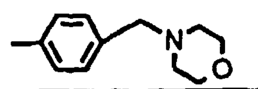 | trans | 4-$CH_3O$-$C_6H_4$- |
| $C_6H_5$-$(CH_2)_3$- | H | 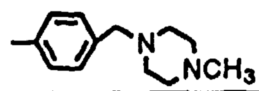 | trans | 4-$CH_3O$-$C_6H_4$- |
| $C_6H_5$-$(CH_2)_3$- | H | 4-($CH_3OCH_2$)-$C_6H_4$- | trans | 4-$CH_3O$-$C_6H_4$- |
| $C_6H_5$-$(CH_2)_3$- | H | 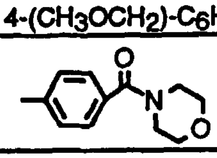 | cis | 4-$CH_3O$-$C_6H_4$- |
| $C_6H_5$-$(CH_2)_3$- | H | 4-($CH_3CH_2O_2C$)-$C_6H_4$- | trans | 4-$CH_3O$-$C_6H_4$- |
| $C_6H_5$-$(CH_2)_3$- | H | 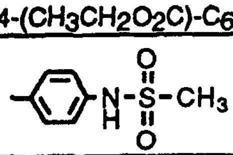 | trans | 4-$CH_3O$-$C_6H_4$- |
| $C_6H_5$-$(CH_2)_3$- | H | 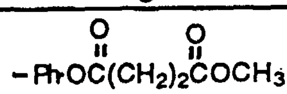 worin 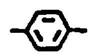 ist | trans | 4-$CH_3O$-$C_6H_4$- |
| $C_6H_5$-$(CH_2)_3$- | H | -Ph-$OCH_2CO_2Et$ worin 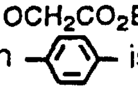 ist und Et -$CH_2CH_3$ ist | 3R,4S | 4-$CH_3O$-$C_6H_4$- |
| $C_6H_5$-$(CH_2)_3$- | H | 4-$CH_3O$-$C_6H_4$- | cis | 4-($CH_3CH_2O$)-$C_6H_4$- |
| 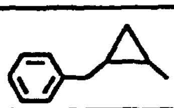 | H | 4-$CH_3O$-$C_6H_4$- | cis | 4-$CH_3O$-$C_6H_4$- |

**17.** Verfahren von Anspruch 1, wobei die hergestellte Verbindung (3R,4S)-1,4-Bis-(4-methoxyphenyl)-3-(3-(phenylpropyl)-2-azetidinon ist.

**18.** Verfahren von Anspruch 15, wobei die verwendete Verbindung aus der Gruppe ausgewählt ist, die aus Verbindungen der Formel

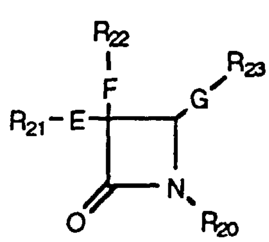

besteht, wobei $R_{21}$-E-, $R_{22}$-F-, $R_{23}$-G-, $R_{20}$ und die relative Stereochemie zwischen $R_{21}$-E- und $R_{23}$-G- wie in der

folgenden Tabelle definiert sind:

| $R_{21}$-E- | $R_{22}$-F- | $R_{23}$-G- | rel. Stereo-chem. | $R_{20}$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| 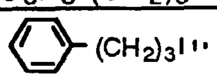 | H | 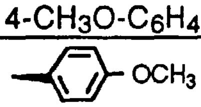 | trans optisch rein | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |

| | | | | |
|---|---|---|---|---|
| $C_6H_5-(CH_2)_3-$ | H | $4-CH_3O-C_6H_4-$ | trans | $4-((CH_3CH_2)_2-N)-C_6H_4-$ |
| $C_6H_5-(CH_2)_3-$ | H | $4-CH_3O-C_6H_4-$ | trans | $4-NC-C_6H_4-$ |
| $C_6H_5-(CH_2)_3-$ | H | $4-CH_3O-C_6H_4-$ | trans | 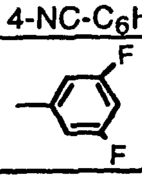 |
| $C_6H_5-(CH_2)_3-$ | H | $4-CH_3O-C_6H_4-$ | trans | $4-(CH_3C(O))-C_6H_4-$ |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 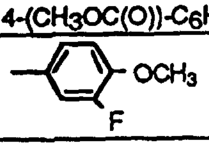 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | 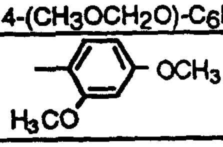 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $C_6H_5-(CH_2)_3-$ | H | $4-CH_3O-C_6H_4-$ | trans | $4-I-C_6H_4-$ |
| 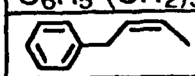 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 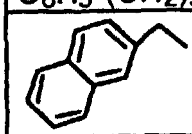 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 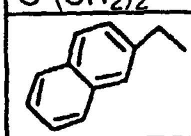 | H | $4-CH_3O-C_6H_4-$ | cis | $C_6H_5-$ |
| $C_6H_5-(CH_2)_3-$ | H | $4-CH_3O-C_6H_4-$ | trans | 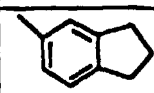 |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | $-Ph-O\overset{O}{\overset{\|}{C}}(CH_2)_2\overset{O}{\overset{\|}{C}}OCH_3$ worin Ph 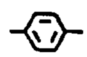 ist | trans | $4-CH_3O-C_6H_4-$ |

| | | | | |
|---|---|---|---|---|
| $C_6H_5-(CH_2)_3-$ | H | $-Ph-OCH_2CO_2Et$ worin Ph 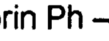 ist und Et $-CH_2CH_3$ ist | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 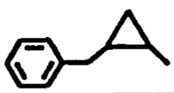 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 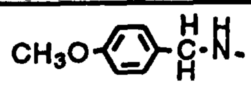 | H | $4-CH_3O-C_6H_4-$ | cis | $C_6H_5-$ |

**19.** Verfahren der Verwendung einer in Anspruch 1 definierten Verbindung zur Herstellung eines Arzneimittels zum Erniedrigen des Serumcholesterins.

**20.** Verfahren der Verwendung einer in Anspruch 1 definierten Verbindung zur Herstellung eines Arzneimittels zum Hemmen des Enzyms Acyl-CoA:Cholesterin-Acyltransferase.

**21.** Verfahren der Verwendung einer in Anspruch 15 definierten Zusammensetzung zur Herstellung eines Arzneimittels zum Erniedrigen der Serumcholesterinwerte in einem Säuger, der einer derartigen Behandlung bedarf.

**22.** Verfahren der Verwendung einer in Anspruch 15 definierten Zusammensetzung zur Herstellung eines Arzneimittels zum Hemmen des Enzyms Acyl-CoA:Cholesterin-Acyltransferase.

**Revendications**

**Revendications pour les Etats contractants suivants : AT, BE, CH, LI, DE, DK, FR, GB, IT, LU, MC, NL, SE, PT**

**1.** Composé ayant la formule de structure

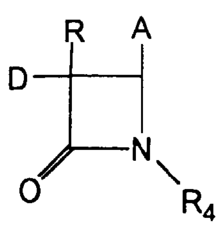

où

A est

$-CH=CH-B$;
$-C\equiv C-B$;
$-(CH_2)_p-X-B$, où p est 0, 1 ou 2 et X est une liaison, -NH- ou $-S(O)_{0-2}-$;
hétéroaryle, hétéroaryle benzofusionné, hétéroaryle W-substitué ou hétéroaryle benzofusionné W-substitué, où hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazolyle et furanyle, et pour les hétéroaryles contenant de l'azote, leurs N-oxydes et où W est 1-3 substituants sur les atomes de carbone du cycle sélectionnés dans le groupe consistant en alkyle $C_{1-6}$, hydroxy alkyle $C_{1-6}$, alcoxy $C_{1-6}$, alcoxy $C_{1-6}$ alkyle $C_{1-6}$, alcoxy $C_{1-6}$ alcoxy $C_{1-6}$, alcoxy $C_{1-6}$ carbonyle alcoxy $C_{1-6}$, (alcoxyimino $C_{1-6}$)alkyle $C_{1-6}$, alcanedioyle $C_{3-6}$, alkyle $C_1$-$_6$-alcanedioyle $C_{3-6}$, allyloxy, $-CF_3$, $-OCF_3$, benzyle, $R_{14}$ benzyle, benzyloxy, $R_{14}$-benzyloxy, phénoxy, $R_{14}$-phénoxy, dioxolanyle, $NO_2$, $-NR_{10}R_{11}$, $NR_{10}R_{11}$ (alkyle $C_{1-6}$)-, $NR_{10}R_{11}$ (alcoxy $C_{1-6}$)-, OH, halogéno, $-NHC(O)OR_5$, $-NHC(O)R_5$, $R_6O_2SNH$-, $(R_6O_2S)_2N$-, $S(O)_2NH_2$, tert-butyldiméthylsilyloxyméthyle, $-C(O)R_{12}$ et

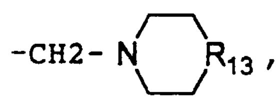

et où les substituants sur les atomes d'azote du cycle hétéroaryle substitué, quand ils sont présents, sont sélectionnés dans le groupe consistant en alkyle $C_{1-6}$, alcoxy $C_{1-6}$, $-C(O)OR_5$, $-C(O)R_5$, OH, $NR_{10}R_{11}$ (alkyle inférieur)-, $NR_{10}R_{11}$ (alcoxy $C_{1-6}$), $-S(O)_2NH_2$ et 2-(triméthylsilyl)éthoxyméthyle;
-C(O)-B; ou

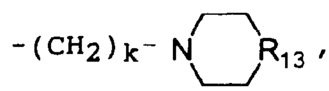

où k est 1 ou 2;

D est

B'-$(CH_2)_mC(O)$-, où m est 1, 2, 3, 4 ou 5;
B'-$(CH_2)_q$-, où q est 2, 3, 4, 5 ou 6;
B'-$(CH_2)_e$-Z-$(CH_2)_r$-, où Z est -O-, -C(O)-, phénylène, $-NR_8$- ou $-S(O)_{0-2}$-, e est 0, 1, 2, 3, 4 ou 5 et r est 1, 2, 3, 4 ou 5, à condition que la somme de e et r soit 1, 2, 3, 4, 5 ou 6.
B'-(alcénylène $C_2$-$C_6$)-;B'-(alcanediénylène $C_4$-$C_6$)-;
B'-$(CH_2)_t$-Z-(alcénylène $C_2$-$C_6$)-, où Z est tel que défini ci-dessus, et où t est 0, 1, 2, ou 3, à condition que la somme de t et du nombre d'atomes de carbone dans la chaîne d'alcénylène soit 2, 3, 4, 5 ou 6;
B'-$(CH_2)_f$-V-$(CH_2)_g$-, où V est cycloalkylène $C_3$-$C_6$, f est 1,2,3,4 ou 5 et g est 0, 1, 2, 3, 4 ou 5, à condition que la somme de f et g soit 1, 2, 3, 4, 5 ou 6;
B'-$(CH_2)_t$-V-(alcénylène $C_2$-$C_6$)- ou B'-(alcénylène $C_2$-$C_6$)-V-$(CH_2)_t$-, où V et t sont tels que définis ci-dessus, à condition que la somme de t et du nombre d'atomes de carbone dans la chaîne d'alcénylène soit 2, 3, 4, 5 ou 6;
B'-$(CH_2)_a$-Z-$(CH_2)_b$-V-$(CH_2)_d$-, où Z et V sont tels que définis ci-dessus et a, b et d sont indépendamment 0, 1, 2, 3, 4, 5 ou 6, à condition que la somme de a, b et d soit 0, 1, 2, 3, 4, 5 ou 6;
T-$(CH_2)_s$-, où T est cycloalkyle de 3-6 atomes de carbone et s est 1, 2, 3, 4, 5 ou 6; ou
naphtylméthyle, hétéroarylméthyle, ou hétéroarylméthyle W-substitué, où hétéroaryle et W sont tels que définis ci-dessus;
B est

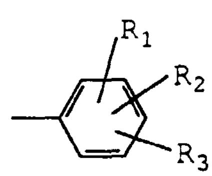

B' est naphtyle, hétéroaryle ou hétéroaryle W-substitué, où hétéroaryle est tel que défini ci-dessus, ou

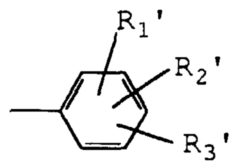

R est hydrogène, fluoro, alkyle $C_1$-$C_{15}$, alcényle $C_2$-$C_{15}$, alkynyle $C_2$-$C_{15}$, ou B-$(CH_2)_h$-, où h est 0, 1, 2 ou 3;
$R_1$, $R_2$ et $R_3$ sont indépendamment sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$-alcoxy $C_{1-6}$, alcoxy $C_{1-6}$ carbonyl-alcoxy $C_{1-6}$, (alcoxyimino $C_{1-6}$)alkyle $C_{1-6}$, alcanedioyle $C_{3-6}$, alkyle $C_{1-6}$ alcanedioyle $C_{3-6}$, alkyle $C_{1-6}$-alcanedioyle $C_{3-6}$) allyloxy, -$CF_3$, -$OCF_3$, benzyle, $R_{14}$ benzyle, benzyloxy, $R_{14}$-benzyloxy, phénoxy, $R_{14}$-phénoxy, dioxolanyle, $NO_2$, -$NR_{10}R_{11}$, $NR_{10}R_{11}$ (alkyle $C_{1-6}$)-, $NR_{10}R_{11}$ (alcoxy $C_{1-6}$)-, OH, o-halogéno, m-halogéno,-NHC (O)$OR_5$,

-NHC(O)$R_5$,$R_6O_2$SNH-, $(R_6O_2S)_2$N-, -S(O)$_2NH_2$, -$CH_2$-S(O)$_2R_{10}$, tert-butyldiméthylsilyloxyméthyle, -C(O)$R_{12}$, -$CH_2$-

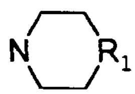 et 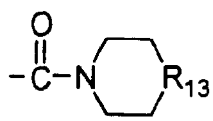 ;

ou bien R1 est hydrogène et R2 et R3, avec les atomes de carbone adjacents auxquels ils sont attachés, forment un cycle de dioxolanyle;
$R_{1'}$, $R_{2'}$ et $R_{3'}$ sont indépendamment sélectionnés dans le groupe consistant en H, alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$-alcoxy $C_{1-6}$, alcoxy $C_{1-6}$-carbonyl-alcoxy $C_{1-6}$, (alcoxyimino $C_{1-6}$) -alkyle $C_{1-6}$, alcanedioyle $C_{3-6}$, alkyle $C_{1-6}$-alcanedioyle $C_{3-6}$, allyloxy, -$CF_3$, -$OCF_3$, benzyle, $R_{14}$-benzyle, benzyloxy, $R_{14}$-benzyloxy, phénoxy, $R_{14}$-phénoxy, dioxolanyle, $NO_2$, -$NR_{10}R_{11}$, $NR_{10}R_{11}$ (alkyle $C_{1-6}$)-, $NR_{10}R_{11}$ (alcoxy $C_{1-6}$)-, OH, halogéno, -NHC(O)$OR_5$, -NHC(O)$R_5$, $R_6O_2$SNH-, $(R_6O_2S)_2$N-, -S(O)$_2NH_2$, -S(O)$_{0-2}R_{10}$, tert-butyldiméthylsiloxyméthyle, C(O)R12,

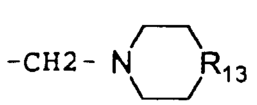 et 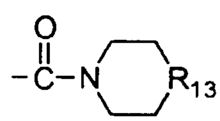 ,

ou bien R1' est hydrogène et $R_{2'}$ et $R_{3'}$, avec les atomes de carbone adjacents auxquels ils sont attachés, forment ensemble un cycle de dioxolanyle;
$R_4$ est

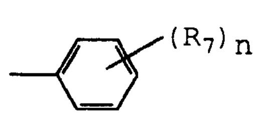 ,

où n est 0, 1, 2 ou 3,
indanyle, benzofuranyle, benzodioxolyle, tétrahydronaphtyle, pyridyle, pyrazinyle, pyrimidinyle ou quinolyle;
$R_5$ est alkyle $C_{1-6}$, phényle, $R_{14}$-phényle, benzyle ou $R_{14}$-benzyle;
$R_6$ est OH, alkyle $C_{1-6}$, phényle, benzyle, $R_{14}$-phényle ou $R_{14}$-benzyle;
$R_7$ est alkyle $C_{1-6}$, alcoxy $C_{1-6}$, OH, halogéno; -$NR_{10}R_{11}$, -NHC(O)$OR_5$, -NHC(O)$R_5$, $NO_2$, -CN, -$N_3$, -SH, -S(O)$_{0-2}$- alkyle $C_{1-6}$, -$COOR_9$, -$CONR_{10}R_{11}$, -$COR_{12}$, phénoxy, benzyloxy, -$OCF_3$, ou tert-butyldiméthylsilyloxy, et quand n est 2 ou 3, les groupes $R_7$ peuvent être identiques ou différents.
$R_8$ est H, alkyle $C_{1-6}$, phényle alkyle $C_{1-6}$, ou -C(O)$R_9$;
$R_9$ est H, alkyle $C_{1-6}$, phényle ou phényle alkyle $C_{1-6}$;

$R_{10}$ et $R_{11}$ sont indépendamment sélectionnés parmi H et alkyle $C_{1-6}$;
$R_{12}$ est H, OH, alcoxy $C_{1-6}$, phénoxy, benzyloxy,

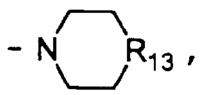

$NR_{10}R_{11}$, alkyle $C_{1-6}$, phényle ou $R_{14}$-phényle;
$R_{13}$ est -O-, $-CH_2-$, -NH- ou -N(alkyle $C_{1-6}$)-; et
$R_{14}$ est 1-3 groupes indépendamment sélectionnés dans le groupe consistant en alkyle $C_{1-6}$, alcoxy $C_{1-6}$, -COOH, $NO_2$, $-NR_{10}R_{11}$, OH ou halogéno;
ou bien un sel pharmaceutiquement acceptable.

2. Composé de la revendication 1 où R est hydrogène.

3. Composé de la revendication 1 ou 2 où D est $B'-(CH_2)_q-$, $B'-(CH_2)_e-Z-(CH_2)_r-$, B'-(alcénylène $C_2$-$C_6$)- ou $B'-(CH_2)_f-V-(CH_2)_g-$, où B', Z, V, q, e, r, f, et g sont tels que définis à la revendication 1.

4. Composé de la revendication 3 où D est B' $-(CH_2)q-$ où B' est phényle et q est 3 ou 4; $B'-(CH_2)_e-Z-(CH_2)_r-$ où B' est p-fluorophényle ou p-méthoxyphényle, e est zéro, Z est-O-, et r est 2; B'-(alcénylène $C_2$-$C_6$)- est 3-phényl-1-propényle; ou bien $B'-(CH_2)_f-V-(CH_2)_g-$ où B' est phényle, f est 1, V est cyclopropylène, et g est zéro.

5. Composé de la revendication 1, 2, 3 ou 4 où A est $-(CH_2)_p-X-B$ où X, B et p sont tels que définis à la revendication 1.

6. Composé de la revendication 5 où p est zéro et X est une liaison.

7. Composé de la revendication 6 où $R_1$, $R_2$ et $R_3$ sont sélectionnés dans le groupe consistant en H, OH, alcoxy $C_{1-6}$, $NO_2$, alcoxy $C_{1-6}$, -alcoxy $C_{1-6}$, m-halogéno, alkyle C1-6, alcanedioyle $C_{3-6}$, $NR_{10}R_{11}$, alcoxy $C_{1-6}$, allyloxy, phénoxy, alcoxy $C_{1-6}$ carbonyle alcoxy $C_{1-6}$ et $-C(O)R_{12}$.

8. Composé de la revendication 1, 2, 3, 4, 5 ou 6 où $R_4$ est phényle, phényle $R_7$-substitué ou indanyle.

9. Composé de la revendication 8 où $R_7$ est sélectionné dans le groupe consistant en alkyle $C_{1-6}$, alcoxy $C_{1-6}$, halogéno, $-OCF_3$, alkylthio $C_{1-6}$, $-NR_{10}R_{11}$, -CN, OH, et $-COR_{12}$.

10. Utilisation d'une composition pharmaceutique pour la fabrication d'un médicament pour abaisser le cholestérol dans le sérum, laquelle composition comprend un composé ayant la structure :

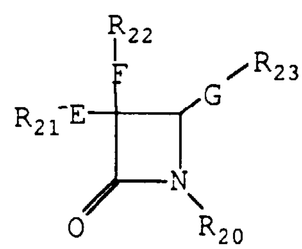

où

$R_{20}$ est phényle, phényle W-substitué, naphtyle, naphtyle W-substitué, benzodioxolyle, hétéroaryle, hétéroaryle W-substitué, hétéroaryle benzofusionné, et hétéroaryle benzofusionné W-substitué, où l'hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazoyle et furanyle, et pour les hétéroaryles contenant de l'azote, leurs N-oxydes;
$R_{21}$, $R_{22}$ et $R_{23}$ sont indépendamment sélectionnés parmi H ou $R_{20}$;
W est 1 à 3 substituants indépendamments sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$ -alcoxy $C_{1-6}$, alcoxy $C_{1-6}$ carbonyle alcoxy $C_{1-6}$,

(alcoxyimino $C_{1-6}$) alkyle $C_{1-6}$, alcanedioyle $C_{3-6}$, alkyle $C_{1-6}$-alcanedioyle $C_{3-6}$) allyloxy, $-CF_3$, $-OCF_3$, benzyle, $R_{14}$-benzyle, benzyloxy, $R_{14}$ -benzyloxy, phénoxy, $R_{14}$ -phénoxy, dioxolanyle, $NO_2$, $-NR_{10}R_{11}$, $NR_{10}R_{11}$ (alkyle $C_{1-6}$)-, $NR_{10}R_{11}$ (alcoxy $C_{1-6}$)-, OH, halogéno, $-NHC(O)OR_5$, $-NHC(O)\ R_5$,
$R_6O_2SNH-$, $(R_6O_2S)_2N-$, $-S(O)_2NH_2$, $-S(O)_{0-2}R_{10}$, tert-butyldiméthylsiloxyméthyle, $-C(O)R_{12}$,

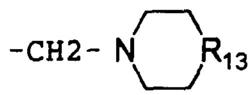

et

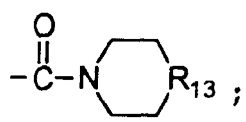

E, F et G sont indépendamment une liaison; cycloalkylène $C_3$-$C_6$; alkylène $C_1$-$C_{10}$; alcénylène $C_2$-$C_{10}$; alkynylène $C_2$-$C_{10}$; une chaîne d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué; ou l'hétéroaryle est tel que défini ci-dessus; une chaîne d'alkylène, alcénylène ou alkynylène telle que définie, interrompue par un ou plusieurs groupes indépendamment sélectionnés dans le groupe consistant en -O-, -S-, -SO-, $-SO_2-$, $-NR_8$, -C(O)-, cycloalkylène $C_3$-$C_6$, phénylène, phénylène W-substitué, hétéroarylène et hétéroarylène W-substitué; ou bien une chaîne interrompue d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué; ou bien l'un de $R_{21}$-E et $R_{22}$-F est sélectionné dans le groupe consistant en halogéno, OH, alcoxy $C_{1-6}$, $-OC(O)R_5$, $-NR_{10}R_{11}$, -SH ou -S (alkyle $C_{1-6}$);
$R_5$ est alkyle $C_{1-6}$, phényle, $R_{14}$-phényle, benzyle ou $R_{14}$-benzyle;
$R_6$ est OH, alkyle $C_{1-6}$, phényle, benzyle, $R_{14}$-phényle ou $R_{14}$-benzyle;
$R_8$ est H, alkyle $C_{1-6}$, phényl alkyl $C_{1-6}$ ou $-C(O)R_9$;
$R_9$ est H, alkyle $C_{1-6}$, phényle ou phényl alkyle $C_{1-6}$;
$R_{10}$ et $R_{11}$ sont indépendamment sélectionnés parmi H et alkyle $C_{1-6}$;
$R_{12}$ est H, OH, alcoxy $C_{1-6}$, phénoxy, benzyloxy,

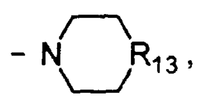

$-NR_{10}R_{11}$, alkyle $C_{1-6}$, phényle ou $R_{14}$-phényle;
$R_{13}$ est -O-, $-CH_2-$, -NH- ou -N(alkyle $C_{1-6}$)-;
$R_{14}$ est 1-3 groupes indépendamment sélectionnés dans le groupe consistant en alkyle $C_{1-6}$, alcoxy $C_{1-6}$, -COOH, $NO_2$, $-NR_{10}R_{11}$, OH ou halogéno;
à condition que quand G est une liaison, $R_{23}$ ne soit pas H, et à condition que quand $R_{23}$ est phényle W-substitué, W ne soit pas p-halogéno;
ou bien un sel pharmaceutiquement acceptable;
dans un support pharmaceutiquement acceptable.

**11.** Composé de la revendication 1 sélectionné dans le groupe consistant en composés de la formule

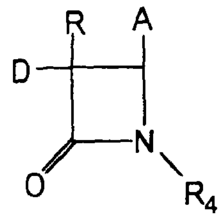

où D, R, A et $R_4$ et la stéréochimie relative entre D et A sont tels que définis au tableau qui suit :

| D | R | A | Stéréochimie relative | $R_4$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-$NO_2$-$C_6H_4$- | 3R,4R | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | 3-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H . | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 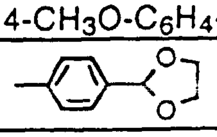 | trans | p-$CH_3O$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | 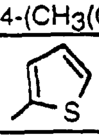 | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($C_6H_5$-O)-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 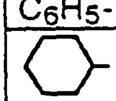 $(CH_2)_3$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-($(CH_3)_2N$-$(CH_2)_3$-O)-$C_6H_4$- | cis | 4-$CH_3O$-$C_6H_4$- |

| | | | | |
|---|---|---|---|---|
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| $C_6H_5$-$(CH_2)_3$- | H | 4-$CH_3O$-$C_6H_4$- | trans | 4-($C_6H_5$-O)-$C_6H_4$- |
| 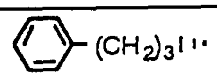 | H | 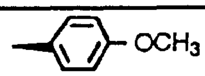 | trans optiquement pur | $C_6H_5$- |
| 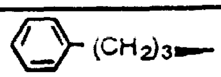 | H | 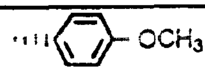 | trans optiquement pur | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($C_6H_5$-$CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| $C_6H_5$-O-$(CH_2)_2$- | H | 4-$CH_3O$-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |
| $C_6H_5$-$(CH_2)_3$- | H | 4-$CH_3O$-$C_6H_4$- | trans | 4-($CH_3CH_2OC(O)$)-$C_6H_4$- |
| $C_6H_5$-$(CH_2)_3$- | H | 4-$CH_3O$-$C_6H_4$- | trans | 3-$NO_2$-$C_6H_4$- |
| $C_6H_5$-$(CH_2)_3$- | H | 4-$CH_3O$-$C_6H_4$- | trans | 4-($(CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 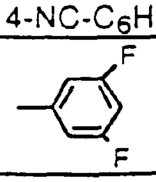 |
| $C_6H_5$-$(CH_2)_3$- | H | 4-$CH_3O$-$C_6H_4$- | trans | 4-($CH_3C(O)$)-$C_6H_4$- |

| | | | | |
|---|---|---|---|---|
| $C_6H_5$-$(CH_2)_3$- | H | 4-$CH_3O$-$C_6H_4$- | trans | 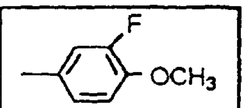 |
| $C_6H_5$-$(CH_2)_3$- | H | 4-$CH_3O$-$C_6H_4$- | trans | 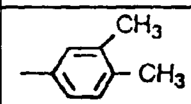 |
| $C_6H_5$-$(CH_2)_3$- | H | 4-($(CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 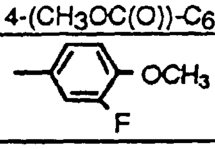 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 4-($(CH_3)_2N$)-$C_6H_4$- | trans | 4-$CH_3O$-$C_6H_4$- |
| $C_6H_5$-$(CH_2)_3$- | H | 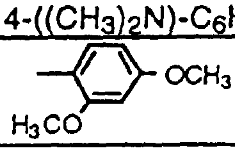 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| 4-$O_2N$-$C_6H_4$-$(CH_2)_3$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | 4-I-$C_6H_4$- |
| $C_6H_5$-$(CH_2)_3$- | H | 4-($CH_3(CH_2)_3$-O)-$C_6H_4$- | trans | 4-$CH_3O$-$C_6H_4$- |
| 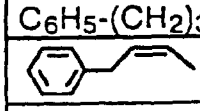 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 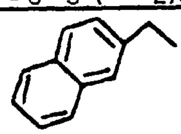 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 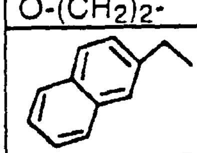 | H | 4-$CH_3O$-$C_6H_4$- | cis | $C_6H_5$- |
| $C_6H_5$-$(CH_2)_3$- | H | 4-$CH_3O$-$C_6H_4$- | trans | 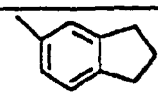 |
| $C_6H_5$-$(CH_2)_3$- | H | 4-$NH_2$-$C_6H_4$- | cis | 4-$CH_3O$-$C_6H_4$- |
| $C_6H_5$-$(CH_2)_3$- | H | 4-$CH_3O$-$C_6H_4$- | trans | 3-$NH_2$-$C_6H_4$- |
| $C_6H_5$-$(CH_2)_3$- | H | 4-OH-$C_6H_4$- | trans | 4-$CH_3O$-$C_6H_4$- |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-$CH_3O$-$C_6H_4$- | 3R,4S | 4-OH-$C_6H_4$- |

| | | | | |
|---|---|---|---|---|
| $C_6H_5$-$(CH_2)_3$- | H | 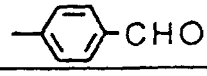 | trans | 4-$CH_3O$-$C_6H_4$- |
| $C_6H_5$-$(CH_2)_3$- | H | 4-$CH_3O$-$C_6H_4$- | trans | 4-$(CH_3SO_2)$-$C_6H_4$- |
| $C_6H_5$-$(CH_2)_3$- | H | 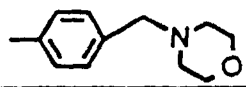 | trans | 4-$CH_3O$-$C_6H_4$- |
| $C_6H_5$-$(CH_2)_3$- | H | 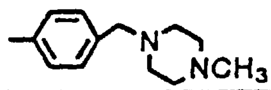 | trans | 4-$CH_3O$-$C_6H_4$- |
| $C_6H_5$-$(CH_2)_3$- | H | 4-$(CH_3OCH_2)$-$C_6H_4$- | trans | 4-$CH_3O$-$C_6H_4$- |
| $C_6H_5$-$(CH_2)_3$- | H | 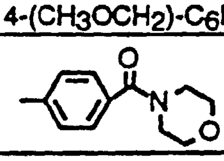 | cis | 4-$CH_3O$-$C_6H_4$- |
| $C_6H_5$-$(CH_2)_3$- | H | 4-$(CH_3CH_2O_2C)$-$C_6H_4$- | trans | 4-$CH_3O$-$C_6H_4$- |
| $C_6H_5$-$(CH_2)_3$- | H | 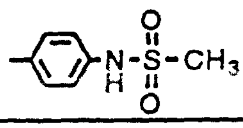 | trans | 4-$CH_3O$-$C_6H_4$- |
| $C_6H_5$-$(CH_2)_3$- | H | $-Ph\text{-}O\overset{O}{\overset{\|}{C}}(CH_2)_2\overset{O}{\overset{\|}{C}}OCH_3$ où Ph est 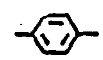 | trans | 4-$CH_3O$-$C_6H_4$- |
| $C_6H_5$-$(CH_2)_3$- | H | $-Ph\text{-}OCH_2CO_2Et$ où Ph est 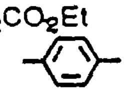 et ET est $-CH_2CH_3$ | 3R,4S | 4-$CH_3O$-$C_6H_4$- |
| $C_6H_5$-$(CH_2)_3$- | H | 4-$CH_3O$-$C_6H_4$- | cis | 4-$(CH_3CH_2O)$-$C_6H_4$- |
| 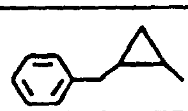 | H | 4-$CH_3O$-$C_6H_4$- | cis | 4-$CH_3O$-$C_6H_4$- |

**12.** Composé de la revendication 1, ou 11 qui est (3R, 4S)-1,4-bis-(4-méthoxyphényl)-3-(3-phénylpropyl)-2-azétidinone.

**13.** Utilisation de la revendication 10 où le composé est sélectionné dans le groupe consistant en composés de la formule

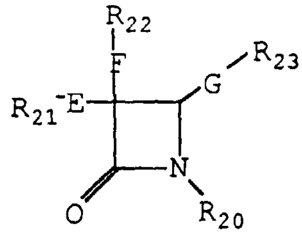

où $R_{21}$-E-, $R_{22}$-F-, $R_{23}$-G-, $R_{20}$ et la stéréochimie relative entre $R_{21}$-E et $R_{23}$-G sont tels que définis au tableau qui suit :

| $R_{21}$-E- | $R_{22}$-F- | $R_{23}$-G- | Stéréochimie relative | $R_{20}$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| 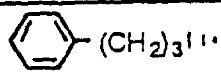 | H | 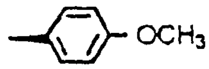 | trans optiquement pur | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |

| | | | | |
|---|---|---|---|---|
| $C_6H_5$-$(CH_2)_3$- | H | 4-$CH_3O$-$C_6H_4$- | trans | 4-(($CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 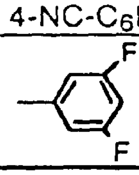 |
| $C_6H_5$-$(CH_2)_3$- | H | 4-$CH_3O$-$C_6H_4$- | trans | 4-($CH_3C(O)$)-$C_6H_4$- |
| $C_6H_5$-$(CH_2)_3$- | H | 4-(($CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 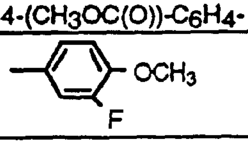 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 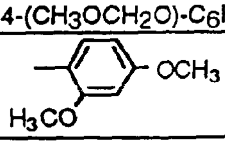 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| $C_6H_5$-$(CH_2)_3$- | H | 4-$CH_3O$-$C_6H_4$- | trans | 4-I-$C_6H_4$- |
| 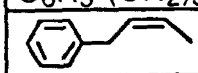 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 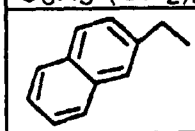 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 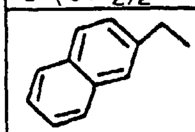 | H | 4-$CH_3O$-$C_6H_4$- | cis | $C_6H_5$- |
| $C_6H_5$-$(CH_2)_3$- | H | 4-$CH_3O$-$C_6H_4$- | trans | 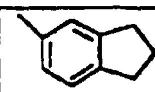 |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-($CH_3CH_2O_2C$)-$C_6H_4$- | trans | 4-$CH_3O$-$C_6H_4$- |
| $C_6H_5$-$(CH_2)_3$- | H | 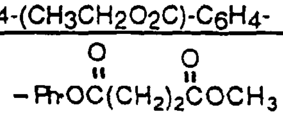 où Ph est 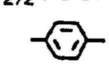 | trans | 4-$CH_3O$-$C_6H_4$- |

| | | | | |
|---|---|---|---|---|
| $C_6H_5-(CH_2)_3-$ | H | $-Ph-OCH_2CO_2Et$<br>où Ph est 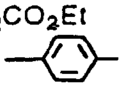<br>et ET est $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$, |
| 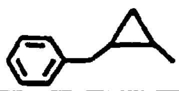 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 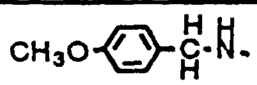 | H | $4-CH_3O-C_6H_4-$ | cis | $C_6H_5-$ |

**14.** Utilisation d'un composé de la revendication 1 pour la fabrication d'un médicament pour abaisser le cholestérol dans le sérum.

**15.** Utilisation d'un composé de la revendication 1 pour la fabrication d'un médicament pour inhiber l'enzyme acyl CoA: cholestérol acyl transférase.

**16.** Procédé pour la préparation d'une composition pharmaceutique telle que définie à la revendication 10 qui comprend le mélange d'un composé tel que défini à la revendication 10 avec un support pharmaceutiquement acceptable.

**17.** Utilisation d'une composition telle que définie à la revendication 10 pour la fabrication d'un médicament pour l'inhibition de l'enzyme acyl CoA: cholestérol acyl tranférase.

**18.** Procédé pour la production d'un composé de la revendication 1, ou est H, et D et A ont une stéréochimie relative trans, comprenant la cyclisation d'une hydroxyamide de la formule

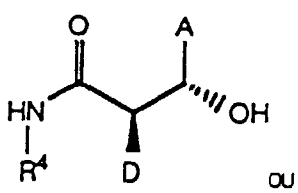

ou

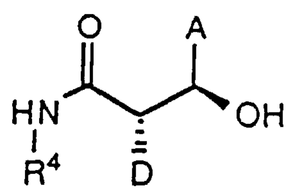

où D, A et $R^4$ sont tels que définis à la revendication 1, par traitement avec :

(i) un dialkylazodicarboxylate et une trialkylphosphine; ou
(ii) un chlorure de di- ou tri-chlorobenzoyle, une base aqueuse et un catalyseur de transfert de phase, puis traitement du di- ou tri-chlorobenzoate résultant avec une base aqueuse et un catalyseur de transfert de phase; ou
(iii) un dialkylchlorophosphate, une base aqueuse et un catalyseur de transfert de phase; ou
(iv) un chlorure de di- ou tri-chlorobenzoyle et un hydrure d'un métal alcalin.

**19.** Procédé de production d'un composé de la revendication 1 ayant la formule

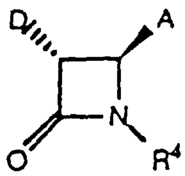

ou

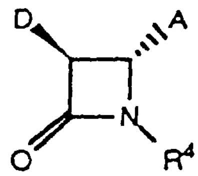

où D, A et $R^4$ sont tels que définis dans la revendication 1, comprenant :

(a) la réaction d'un acide carboxylique de la formule $DCH_2$-COOH, où D est tel que défini ci-dessus, avec un agent de chloruration;
(b) la déprotonation d'une oxazolidinone chirale de la formule

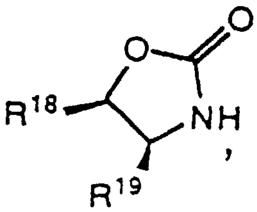
,
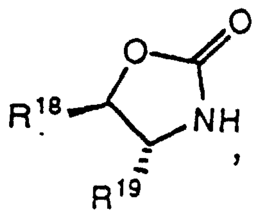
,
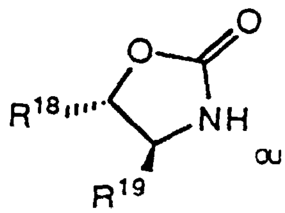
ou
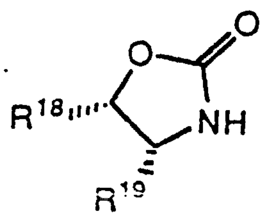

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; avec une base forte et le traitement de l'anion résultant avec le produit de l'étape (a);
(c) l'énolisation du produit de l'étape (b) avec soit:

(i) un triflate de dialkylbore et une base d'amine tertiaire;
(ii) $TiCl_4$ et de la tétraméthyléthylènediamine (TMEDA) ou un mélange de TMEDA et de triéthylamine;

puis la condensation avec un aldéhyde de la formule A-CHO, où A est tel que défini ci-dessus;
(d) l'hydrolyse du produit de l'étape (c) avec une base et du péroxyde d'hydrogène;
(e) la condensation du produit de l'étape (d) avec une amine de la formule $R^4NH_2$, où $R^4$ est tel que défini ci-dessus, par traitement avec un agent de couplage déshydratant, en ajoutant facultativement un agent activant; et
(f) la cyclisation du produit de l'étape (e) par traitement avec :

(i) un dialkylazodicarboxylate et une trialkylphosphine; ou
(ii) un chlorure de di- ou tri-chlorobenzoyle,
une base aqueuse et un catalyseur de transfert de phase, puis traitement du di- ou tri-chlorobenzoate résultant avec une base aqueuse et un catalyseur de transfert de phase; ou
(iii) un dialkylchlorophosphate, une base aqueuse et un catalyseur de transfert de phase; ou
(iv) un chlorure de di- ou tri-chlorobenzoyle et un hydrure d'un métal alcalin.

**20.** Procédé de production d'un composé de la revendication 1, où R est hydrogène et D et A ont une stéréochimie relative trans, comprenant :

(a) l'énolisation d'un composé de formule

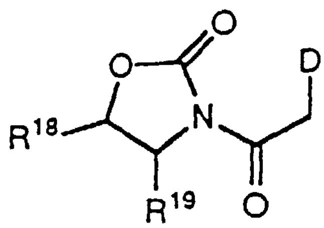

avec $TiCl_4$ et de la tétraméthyléthylènediamine (TMEDA), puis la condensation avec une imine de la formule A-CH=N-$R^4$, où A et $R^4$ sont tels que définis ci-dessus, et où le groupe

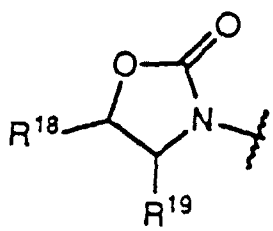

représente un auxiliaire chiral de la formule

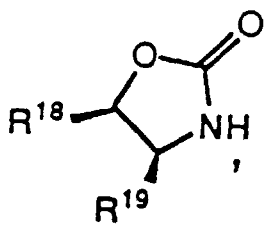 , 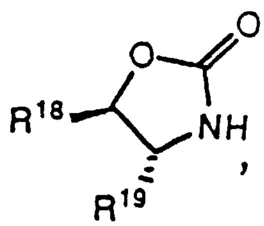 , 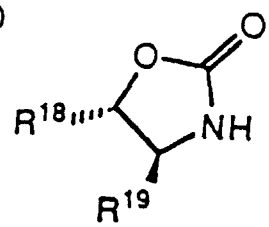 ou 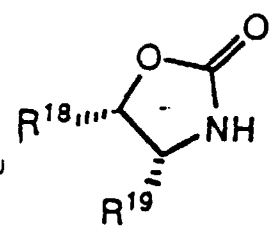

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; et
(b) la cyclisation du produit de l'étape (a) par traitement avec une base non nucléophile forte.

**21.** Procédé de préparation de composés de la revendication 1 comprenant :

(a) le traitement d'un ester de la formule D-CH(R)-C(O)O$R^{17}$, où $R^{17}$ est alkyle $C_{1-6}$, menthyle, ou 10-(diisopropylsulfonamido)-isobornyle, avec une base forte; puis avec une imine de la formule A-CH=N-$R^4$, où A et $R^4$ sont tels que définis à la revendication 1; ou
(b) pour la préparation d'un composé de la revendication 1 où R n'est pas hydrogène, la réaction d'un composé de la revendication 1, où R est hydrogène, avec une base forte et un agent d'alkylation ou un agent d'acylation; ou
(c) pour la préparation d'un composé de la revendication 1, où R est hydrogène, et D et A ont une stéréochimie relative cis, la réaction d'un composé de la revendication 1, où R est hydrogène, et D et A ont une stéréochimie relative trans, avec une base forte entre -80 et -40°C, et puis avec une source de protons; ou
(d) pour la préparation d'un composé de la revendication 1, où R est hydrogène, et D et A ont une stéréochimie relative trans, la réaction d'un composé de la revendication 1, où R est hydrogène, et D et A ont une stéréochimie relative cis, avec un t-butoxyde d'un métal alcalin, puis l'arrêt avec un acide; ou
(e) la réaction d'un dérivé d'acide carboxylique de la formule D-CH(R)-C(O)-Z, où D et R sont tels que définis à la revendication 1 et Z est Cl,

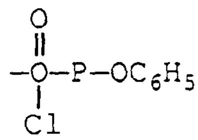

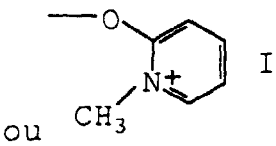

, ou

avec une imine de la formule A-CH=N-$R^4$, où A et $R^4$ sont tels que définis à la revendication 1, en présence d'une base d'amine tertiaire.

**22.** Procédé de préparation d'un composé de formule II

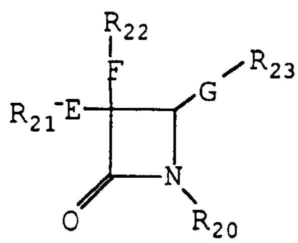

II

où

$R_{20}$ est phényle, phényle W-substitué, naphtyle, naphtyle W-substitué, benzodioxolyle, hétéroaryle, hétéroaryle W-substitué, hétéroaryle benzofusionné et hétéroaryle benzofusionné W-substitué, où hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazolyle et furanyle, et pour les hétéroaryles contenant de l'azote, leurs N-oxydes;

$R_{21}$, $R_{22}$ et $R_{23}$ sont indépendamment sélectionnés parmi H ou $R_{20}$;

W est 1 à 3 substituants indépendamment sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$ -alcoxy $C_{1-6}$, alcoxy $C_{1-6}$-carbonylalcoxy $C_{1-6}$, (alcoxyimino $C_{1-6}$)alkyle $C_{1-6}$, alcanedioyle $C_{3-6}$, alkyle $C_{1-6}$ -alcanedioyle $C_{3-6}$, allyloxy, $-CF_3$, $-OCF_3$, benzyle, $R_{14}$-benzyle, benzyloxy, $R_{14}$-benzyloxy, phénoxy, $R_{14}$-phénoxy, dioxolanyle, $NO_2$, $-NR_{10}R_{11}$, $NR_{10}R_{11}$(alkyle $C_{1-6}$)-, $NR_{10}R_{11}$(alcoxy $C_{1-6}$)-, OH, halogéno, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-butyldiméthylsilyloxyméthyle, $-C(O)R_{12}$,

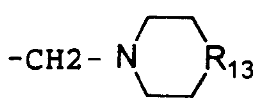

et

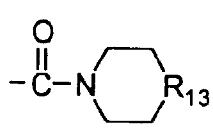

;

E, F et G sont indépendamment une liaison; cycloalkylène $C_3$-$C_6$; alkylène $C_1$-$C_{10}$; alcénylène $C_2$-$C_{10}$; alkynylène $C_2$-$C_{10}$; une chaîne d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué, où hétéroaryle est tel que défini ci-dessus; une chaîne d'alkylène, alcénylène ou alkynylène telle que définie, interrompue par un ou plusieurs groupes indépendamment sélectionnés dans le groupe consistant en -O-, -S-, -SO-, $-SO_2$-, $-NR_8$, -C(O)-, cycloalkylène $C_3$-$C_6$, phénylène,

phénylène W-substitué, hétéroarylène et hétéroarylène W-substitué; ou bien une chaîne interrompue d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué; ou bien l'un de $R_{21}$-E et $R_{22}$-F est sélectionné dans le groupe consistant en halogéno, OH, alcoxy inférieur, -OC(O)$R_5$, - $NR_{10}R_{11}$, -SH ou -S(alkyle $C_{1-6}$);
$R_5$ est alkyle $C_{1-6}$, phényle, $R_{14}$-phényle, benzyle ou $R_{14}$-benzyle;
$R_6$ est OH, alkyle $C_{1-6}$, phényle, benzyle, $R_{14}$-phényle ou $R_{14}$-benzyle;
$R_8$ est H, alkyle $C_{1-6}$, phényle alkyle $C_{1-6}$ ou -C(O)$R_9$;
$R_9$ est H, alkyle $C_{1-6}$, phényle ou phényle alkyle $C_{1-6}$;
$R_{10}$ et $R_{11}$ sont indépendamment sélectionnés parmi H et alkyle $C_{1-6}$;

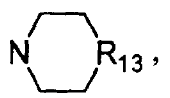

$R_{12}$ est H, OH, alcoxy $C_{1-6}$, phénoxy, benzyloxy, - $NR_{10}R_{11}$, alkyle $C_{1-6}$, phényle ou $R_{14}$-phényle;
$R_{13}$ est -O-, -$CH_2$-, -NH- ou -N(alkyle $C_{1-6}$)-;
$R_{14}$ est 1-3 groupes indépendamment sélectionnés dans le groupe consistant en alkyle $C_{1-6}$, alcoxy $C_{1-6}$, -COOH, $NO_2$, -$NR_{10}R_{11}$, OH ou halogéno;
à condition que quand G est une liaison, $R_{23}$ ne soit pas H, et à condition que quand $R_{23}$ est phényle W-substitué, W ne soit pas p-halogéno;
ou bien un sel pharmaceutiquement acceptable;
où $R_{22}$-F est H, et $R_{21}$-E- et $R_{23}$-G- ont une stéréochimie relative trans, comprenant :

Procédé C: la cyclisation d'une hydroxyamide de la formule

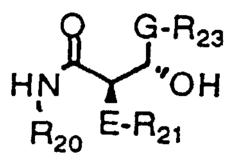 ou 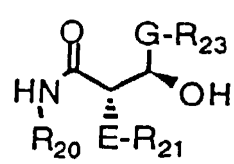

où $R_{21}$-E-, $R_{23}$-G- et $R_{20}$ sont tels que définis ci-dessus, par traitement avec :

(i) un dialkylazodicarboxylate et une trialkylphosphine; ou
(ii) un chlorure de di- ou tri-chlorobenzoyle, une base aqueuse et un catalyseur de transfert de phase, puis traitement du di- ou tri-chlorobenzoate résultant avec une base aqueuse et un catalyseur de transfert de phase; ou
(iii) un dialkylchlorophosphate, une base aqueuse et un catalyseur de transfert de phase; ou
(iv) un chlorure de di- ou tri-chlorobenzoyle et un hydrure d'un métal alcalin; ou

Procédé D:

(a) la réaction d'un acide carboxylique de la formule $R_{21}$-E-$CH_2$-COOH, où $R_{21}$-E- est tel que défini ci-dessus avec un agent de chloruration;
(b) la déprotonation d'une oxazolidinone chirale de la formule

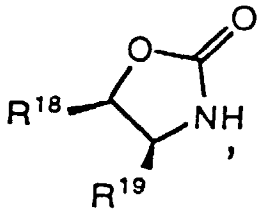, 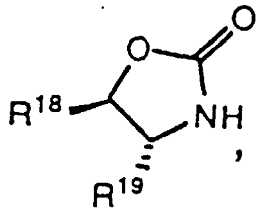, 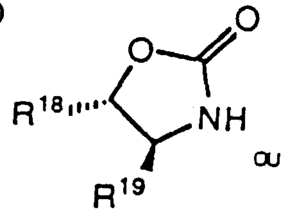 ou 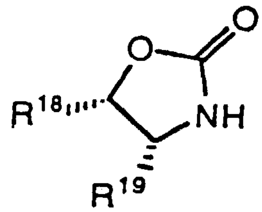

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; avec une base forte et traitement de l'anion résultant avec le produit de l'étape (a);

(c) l'énolisation du produit de l'étape (b) avec soit :

(i) un triflate de dialkylbore et une base d'amine tertiaire;

(ii) $TiCl_4$ et de la tétraméthyléthylènediamine (TMEDA) ou un mélange de TMEDA et de triéthylamine;

puis la condensation avec un aldéhyde de la formule $R_{23}$-G-CHO, où $R_{23}$-G est tel que défini ci-dessus;

(d) l'hydrolyse du produit de l'étape (c) avec une base et du péroxyde d'hydrogène;

(e) la condensation du produit de l'étape (d) avec une amine de la formule $R_{20}NH_2$, où $R_{20}$ est tel que défini ci-dessus, par traitement avec un agent de couplage déshydratant, en ajoutant facultativement un agent activant; et

(f) la cyclisation du produit de l'étape (e) telle que décrite au Procédé C.

**23.** Procédé de production d'un composé de formule II tel que défini dans la revendication 22, où $R_{22}$-F- est hydrogène, et $R_{21}$-E- et $R_{23}$-G- ont une stéréochimie relative trans, comprenant :

(a) l'énolisation d'un composé de la formule

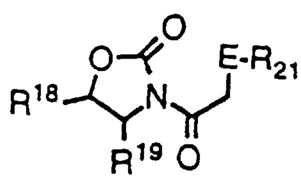

avec $TiCl_4$ et de la tétraméthyléthylènediamine (TMEDA), puis la condensation avec une imine de la formule $R_{23}$-G-CH=N-$R_{20}$, où $R_{23}$-G- et $R_{20}$ sont tels que définis à la revendication 22, et où le groupe

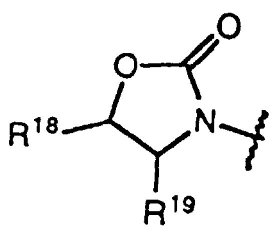

représente un auxiliaire chiral de la formule

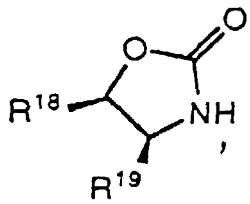 , 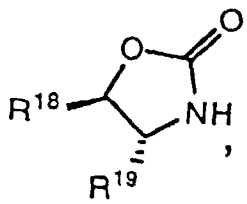 , 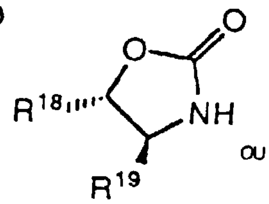 ou 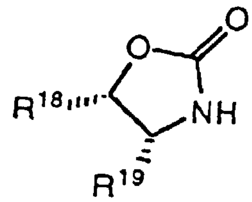

où $R^{18}$ et $R^{19}$ sont indépendamment sélectionnés dans le groupe consistant en hydrogène, alkyle $C_1$-$C_6$, phényle, naphtyle, phényle substitué, naphtyle substitué, et benzyle; et
(b) la cyclisation du produit de l'étape (a) par traitement avec une base non nucléophile forte.

**24.** Procédé de préparation d'un composé de formule II tel que défini à la revendication 22, comprenant

(a) la traitement d'un ester de la formule $R_{21}$-E-CH(F-$R_{22}$)-C(O)O$R^{17}$, où $R^{17}$ est alkyle $C_{1-6}$, menthyle, ou 10-(diisopropylsulfonamido)-isobornyle, avec une base forte, et puis avec un imine de la formule $R_{23}$-G-CH=N-$R_{20}$, où $R_{23}$-G- et $R_{20}$ sont tels que définis à la revendication 22, ou
(b) pour la préparation d'un composé de formule II tel que défini à la revendication 22, où $R_{22}$-F- n'est pas de l'hydrogène, la réaction d'un composé de formule II tel que défini à la revendication 22, où $R_{22}$-F- est de l'hydrogène, avec une base forte et un agent d'alkylation ou un agent d'acylation; ou
(c) pour la préparation d'un composé de formule II tel que défini dans la revendication 22, où $R_{22}$-F- est hydrogène, et $R_{21}$-E- et $R_{23}$-G- ont une stéréochimie relative cis, la réaction d'un composé de la formule II tel que défini à la revendication 22, où $R_{22}$-F- est hydrogène, et $R_{21}$-E-, et $R_{23}$-G- ont une stéréochimie relative trans, avec une base forte à -80° jusqu'à -40°C, et puis avec une source de protons; ou
(d) pour la préparation d'un composé de formule II tel que défini à la revendication 22, où $R_{22}$-F- est hydrogène, et $R_{21}$-E- et $R_{23}$-G- ont une stéréochimie relative trans, la réaction d'un composé de formule II tel que défini à la revendication 22, où $R_{22}$-F- est hydrogène et $R_{21}$-E- et $R_{23}$-G- ont une stéréochimie relative cis avec un t-butoxyde d'un métal alcalin, puis l'arrêt avec un acide; ou
(e) la réaction d'un dérivé d'acide carboxylique de la formule $R_{21}$-E-CH(F-$R_{22}$)-C(O)-Z, où $R_{21}$-E- et $R_{22}$-F- sont tels que définis à la revendication 22, et Z est Cl,

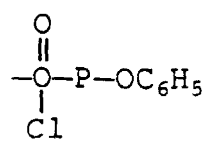 , ou 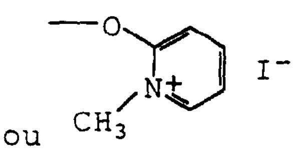 ,

avec une imine de la formule $R_{23}$-G-CH=N-$R_{20}$, où $R_{23}$-G- et $R_{20}$ sont tels que définis à la revendication 22, en présence d'une base d'amine tertiaire.

## Revendications pour l'Etat contractant suivant : GR

**1.** Composé ayant la formule de structure

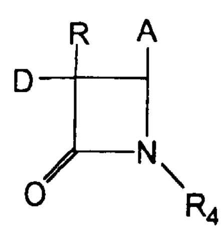

où

A est

-CH=CH-B;
-C≡C-B;
$-(CH_2)_p$-X-B, où p est 0, 1 ou 2 et X est une liaison, -NH- ou $-S(O)_{0-2}$-;
hétéroaryle, hétéroaryle benzofusionné, hétéroaryle W-substitué ou hétéroaryle benzofusionné W-substitué, où hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazolyle et furanyle, et pour les hétéroaryles contenant de l'azote, leurs N-oxydes et où W est 1-3 substituants sur les atomes de carbone du cycle sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$ alcoxy $C_{1-6}$, alcoxy $C_{1-6}$ carbonyl- alcoxy $C_{1-6}$, (alcoximino $C_{1-6}$)alkyle $C_{1-6}$, alcanedioyle $C_{3-6}$, alkyle $C_{1-6}$-alcanedioyle $C_{3-6}$, allyloxy, $-CF_3$, $-OCF_3$, benzyle, $R_{14}$ benzyle, benzyloxy, $R_{14}$-benzyloxy, phénoxy, $R_{14}$-phénoxy, dioxolanyle, $NO_2$, $-NR_{10}R_{11}$, $NR_{10}R_{11}$ (alkyle $C_{1-6}$)-, $NR_{10}R_{11}$ (alcoxy $C_{1-6}$)-, OH, halogéno, $-NHC(O)OR_5$, $-NHC(O)R_5$, $R_6O_2SNH$-, $(R_6O_2S)_2N$-, $S(O)_2NH_2$, tert-butyldiméthylsilyloxyméthyle, $-C(O)R_{12}$ et

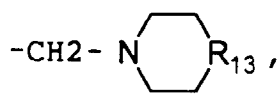

et où les substituants sur les atomes d'azote du cycle hétéroaryle substitué, quand ils sont présents, sont sélectionnés dans le groupe consistant en alkyle $C_{1-6}$, alcoxy $C_{1-6}$, $-C(O)OR_5$, $-C(O)R_5$, OH, $NR_{10}R_{11}$ (alkyle inférieur)-, $NR_{10}R_{11}$ (alcoxy $C_{1-6}$), $-S(O)_2NH_2$ et 2-(triméthylsilyl)éthoxyméthyle;
-C(O)-B; ou

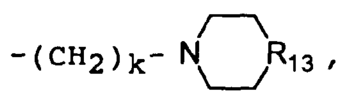

où k est 1 ou 2;

D est

B'-$(CH_2)_m$C(O)-, où m est 1, 2, 3, 4 ou 5;

B'-$(CH_2)_q$-, où q est 2, 3, 4, 5 ou 6;

B'-$(CH_2)_e$-Z-$(CH_2)_r$-, où Z est -O-, -C(O)-, phénylène, $-NR_8$- ou $-S(O)_{0-2}$-, e est 0, 1, 2, 3, 4 ou 5 et r est 1, 2, 3, 4 ou 5, à condition que la somme de e et r soit 1, 2, 3, 4, 5 ou 6.

B'-(alcénylène $C_2$-$C_6$)-;B'-(alcanediénylène $C_4$-$C_6$) -;

B'-$(CH_2)_t$-Z-(alcénylène $C_2$-$C_6$)-, où Z est tel que défini ci-dessus, et où t est 0, 1, 2, ou 3, à condition que la somme de t et du nombre d'atomes de carbone dans la chaîne d'alcénylène soit 2, 3, 4, 5 ou 6;

B'-$(CH_2)_f$-V-$(CH_2)_g$-, où V est cycloalkylène $C_3$-$C_6$, f est 1,2,3,4 ou 5 et g est 0, 1, 2, 3, 4 ou 5, à condition que la somme de f et g soit 1, 2, 3, 4, 5 ou 6;

B'-$(CH_2)_t$-V-(alcénylène $C_2$-$C_6$)- ou B'-(alcénylène $C_2$-$C_6$)-V-$(CH_2)_t$-, où V et t sont tels que définis ci-dessus, à condition que la somme de t et du nombre d'atomes de carbone dans la chaîne d'alcénylène soit 2, 3, 4, 5 ou 6;

B'-$(CH_2)_a$-Z-$(CH_2)_b$-V-$(CH_2)_d$-, où Z et V sont tels que définis ci-dessus et a, b et d sont indépendamment 0, 1, 2, 3, 4, 5 ou 6, à condition que la somme de a, b et d soit 0, 1, 2, 3, 4, 5 ou 6;

$T-(CH_2)_s-$, où T est cycloalkyle de 3-6 atomes de carbone et s est 1, 2, 3, 4, 5 ou 6; ou naphtylméthyle, hétéroarylméthyle, ou hétéroarylméthyle W-substitué, où hétéroaryle et W sont tels que définis ci-dessus;

B est

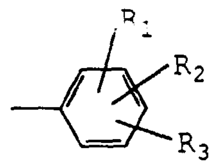

B' est naphtyle, hétéroaryle ou hétéroaryle W-substitué, où hétéroaryle est tel que défini ci-dessus, ou

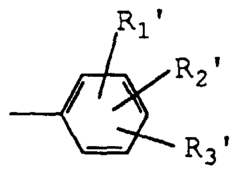

R est hydrogène, fluoro, alkyle $C_1$-$C_{15}$, alcényle $C_2$-$C_{15}$, alkynyle $C_2$-$C_{15}$, ou $B-(CH_2)_h-$, où h est 0, 1, 2 ou 3;

$R_1$, $R_2$ et $R_3$ sont indépendamment sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$-alcoxy $C_{1-6}$, alcoxy-$C_{1-6}$ carbonyl-alcoxy $C_{1-6}$, (alcoxyimino $C_{1-6}$)-alkyle $C_{1-6}$, alcanedioyle $C_{3-6}$, alkyle $C_{1-6}$ alcanedioyle $C_{3-6}$, alkyle $C_{1-6}$-alcanedioyle $C_{3-6}$) allyloxy, $-CF_3$, $-OCF_3$, benzyle, $R_{14}$ benzyle, benzyloxy, $R_{14}$-benzyloxy, phénoxy, $R_{14}$ phénoxy, dioxolanyle, $NO_2$, $-NR_{10}R_{11}$, $NR_{10}R_{11}$ (alkyle $C_{1-6}$)-, $NR_{10}R_{11}$ (alcoxy $C_{1-6}$)-, OH, o-halogéno, m-halogéno, -NHC(O)$OR_5$, -NHC(O)$R_5$,$R_6O_2SNH-$, $(R_6O_2S)_2N-$, $-S(O)_2NH_2$, $-S(O)_2R_{10}$, tert-butyldiméthylsilyloxyméthyle, $-C(O)R_{12}$,

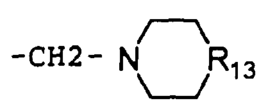 et 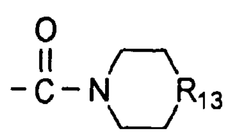,

ou bien R1 est hydrogène et R2 et

R3, avec les atomes de carbone adjacents auxquels ils sont attachés, forment un cycle de dioxolanyle;

$R_{1'}$, $R_{2'}$ et $R_{3'}$ sont indépendamment sélectionnés dans le groupe consistant en H, alkyle $C_{1-6}$, hydroxy alkyle $C_{1-6}$, alcoxy $C_{1-6}$, alcoxy $C_{1-6}$ alkyle $C_{1-6}$, alcoxy $C_{1-6}$-alcoxy $C_{1-6}$, alcoxy $C_{1-6}$-carbonyl-alcoxy $C_{1-6}$, (alcoxyimino $C_{1-6}$)-alkyle $C_{1-6}$, alcanedioyle $C_{3-6}$, alkyle $C_{1-6}$-alcanedioyle $C_{3-6}$, allyloxy, $-CF_3$, $-OCF_3$, benzyle, $R_{14}$-benzyle, benzyloxy, $R_{14}$-benzyloxy, phénoxy, $R_{14}$-phénoxy, dioxolanyle, $NO_2$, $-NR_{10}R_{11}$, $NR_{10}R_{11}$ (alkyle $C_{1-6}$)-, $NR_{10}R_{11}$ (alcoxy $C_{1-6}$)-, OH, halogéno, -NHC(O)$OR_5$, -NHC(O)$R_5$, $R_6O_2SNH-$, $(R_6O_2S)_2N-$, $-S(O)_2NH_2$, $-S(O)_{0-2}R_{10}$, tert-butyldiméthylsiloxyméthyle, $-C(O)R_{12}$,

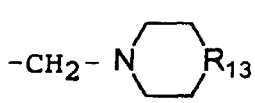

et

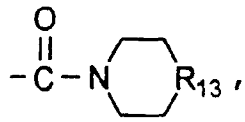

ou bien $R_1$' est hydrogène et $R_2$, et $R_{3'}$, avec les atomes de carbone adjacents auxquels ils sont attachés, forment ensemble un cycle de dioxolanyle;

$R_4$ est

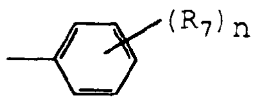

,

où n est 0, 1, 2 ou 3, indanyle, benzofuranyle, benzodioxolyle, tétrahydronaphtyle, pyridyle, pyrazinyle, pyrimidinyle ou quinolyle;

$R_5$ est alkyle $C_{1-6}$, phényle, $R_{14}$-phényle, benzyle ou $R_{14}$-benzyle;

$R_6$ est OH, alkyle $C_{1-6}$, phényle, benzyle, $R_{14}$-phényle ou $R_{14}$-benzyle;

$R_7$ est alkyle $C_{1-6}$, alcoxy $C_{1-6}$, OH, halogéno, $-NR_{10}R_{11}$, $-NHC(O)OR_5$, $-NHC(O)R_5$, $NO_2$, -CN, $-N_3$, -SH, $-S(O)_{0-2}$- alkyle $C_{1-6}$, $-COOR_9$, $-CONR_{10}R_{11}$, $-COR_{12}$, phénoxy, benzyloxy, $-OCF_3$, ou tert-butyldiméthyl-silyloxy, et quand n est 2 ou 3, les groupes $R_7$ peuvent être identiques ou différents.

$R_8$ est H, alkyle $C_{1-6}$, phényle alkyle $C_{1-6}$, ou $-C(O)R_9$;

$R_9$ est H, alkyle $C_{1-6}$, phényle ou phényle alkyle $C_{1-6}$;

$R_{10}$ et $R_{11}$ sont indépendamment sélectionnés parmi H et alkyle $C_{1-6}$;

$R_{12}$ est H, OH, alcoxy $C_{1-6}$, phénoxy, benzyloxy,

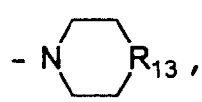

$NR_{10}R_{11}$, alkyle $C_{1-6}$, phényle ou $R_{14}$-phényle;

$R_{13}$ est -O-, $-CH_2$-, -NH- ou -N(alkyle $C_{1-6}$)-; et

$R_{14}$ est 1-3 groupes indépendamment sélectionnés dans le groupe consistant en alkyle $C_{1-6}$, alcoxy $C_{1-6}$, -COOH, $NO_2$, $-NR_{10}R_{11}$, OH ou halogéno;

ou bien un sel pharmaceutiquement acceptable.

2. Composé de la revendication 1 où R est hydrogène.

3. Composé de la revendication 1 ou 2 où D est $B'-(CH_2)_q-$, $B'-(CH_2)_e-Z-(CH_2)_r-$, B'-(alcénylène $C_2$-$C_6$)- ou $B'-(CH_2)_f-V-(CH_2)_g-$, où B', Z, V, q, e, r, f, et g sont tels que définis à la revendication 1.

4. Composé de la revendication 3 où D est $B'-(CH_2)_q-$ où B' est phényle et q est 3 ou 4; $B'-(CH_2)_e-Z-(CH_2)_r-$ où B'

est p-fluorophényle ou p-méthoxyphényle, e est zéro, Z est-O-, et r est 2; B'-(alcénylène $C_2$-$C_6$)- est 3-phényl-1-propényle; ou bien B'-$(CH_2)_f$-V-$(CH2)_g$- où B' est phényle, f est 1, V est cyclopropylène, et g est zéro. .

**5.** Composé de la revendication 1, 2, 3 ou 4 où A est -$(CH_2)_p$-X-B où X, B et p sont tels que définis à la revendication 1.

**6.** Composé de la revendication 5 où p est zéro et X est une liaison.

**7.** Composé de la revendication 6 où $R_1$, $R_2$ et $R_3$ sont sélectionnés dans le groupe consistant en H, OH, alcoxy $C_{1\text{-}6}$, $NO_2$, alcoxy $C_{1\text{-}6}$ -alcoxy $C_{1\text{-}6}$, m-halogéno, alkyle $C_{1\text{-}6}$, alcanedioyle $C_{3\text{-}6}$, $NR_{10}R_{11}$, alcoxy $C_{1\text{-}6}$, allyloxy, phénoxy, alcoxy $C_{1\text{-}6}$ carbonyle alcoxy $C_{1\text{-}6}$ et -C(O)$R_{12}$.

**8.** Composé de la revendication 1, 2, 3, 4, 5 ou 6 où $R_4$ est phényle, phényle $R_7$-substitué ou indanyle.

**9.** Composé de la revendication 8 où $R_7$ est sélectionné dans le groupe consistant en alkyle $C_{1\text{-}6}$, alcoxy $C_{1\text{-}6}$, halogéno, -$OCF_3$, alkylthio $C_{1\text{-}6}$, -$NR_{10}R_{11}$, -CN, OH, et -$COR_{12}$.

**10.** Procédé pour la préparation d'une composition pharmaceutique à utiliser pour la fabrication d'un médicament pour abaisser le cholestérol dans le sérum, lequel procédé consiste à mélanger un support pharmaceutiquement acceptable à un composé ayant la structure

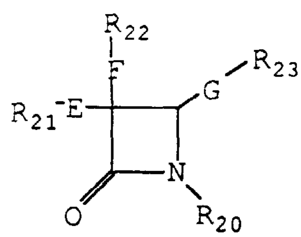

où

$R_{20}$ est phényle, phényle W-substitué, naphtyle, naphtyle W-substitué, benzodioxolyle, hétéroaryle, hétéroaryle W-substitué, hétéroaryle benzofusionné, et hétéroaryle benzofusionné W-substitué, où l'hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazoyle et furanyle, et pour les hétéroaryles contenant de l'azote, leurs N-oxydes;

$R_{21}$, $R_{22}$ et $R_{23}$ sont indépendamment sélectionnés parmi H ou $R_{20}$;

W est 1 à 3 substituants indépendamments sélectionnés dans le groupe consistant en alkyle $C_{1\text{-}6}$, hydroxy alkyle $C_{1\text{-}6}$, alcoxy $C_{1\text{-}6}$, alcoxy $C_{1\text{-}6}$-alkyle $C_{1\text{-}6}$, alcoxy $C_{1\text{-}6}$-alcoxy $C_{1\text{-}6}$, alcoxy $C_{1\text{-}6}$ carbonyl-alcoxy $C_{1\text{-}6}$, (alcoxyimino $C_{1\text{-}6}$) alkyle $C_{1\text{-}6}$, alcanedioyle $C_{3\text{-}6}$, alkyle $C_{1\text{-}6}$-alcanedioyle $C_{3\text{-}6}$ allyloxy, -$CF_3$, -$OCF_3$, benzyle, $R_{14}$ benzyle, benzyloxy, $R_{14}$ -benzyloxy, phénoxy, $R_{14}$-phénoxy, dioxolanyle, $NO_2$, -$NR_{10}R_{11}$, $NR_{10}R_{11}$ (alkyle $C_1$-$_6$)-, $NR_{10}R_{11}$ (alcoxy $C_{1\text{-}6}$)-, OH, halogéno, -NHC(O)$OR_5$, -NHC(O)$R_5$, $R_6O_2SNH$-, $(R_6O_2S)_2N$-, -$S(O)_2NH_2$, -$S(O)_{0\text{-}2}R_{10}$, tert-butyldiméthylsiloxyméthyle, -C(O)$R_{12}$,

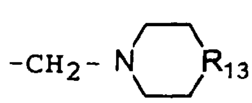

et

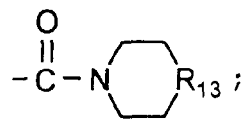

E, F, et G sont indépendamment une liaison; cycloalkylène $C_3$-$C_6$; alkylène $C_1$-$C_{10}$; alkylène $C_2$-$C_{10}$; alkynylène $C_2$-$C_{10}$; une chaîne d'alkylène, alcénylène ou alkynylène telle que définie, substituée par un ou plusieurs substituants indépendamment sélectionnées dans le groupe consistant en phényle, phényle W-substitué, hétéroaryle et hétéroaryle W-substitué, ou l'hétéroaryle est tel que défini ci-dessus; une chaîne d'alkylène, alcénylène ou alkynylène telle que définie, interrompue par un ou plusieurs groupes indépendamment sélectionnés dans le groupe consistant en -O-, -S-, -SO-, $SO_2$-, -$NR_8$, -C(O)-, cycloalkylène $C_3$-$C_6$, phénylène, phénylène W-substitué, hétéroarylène et hétéroarylène W-substitué; ou bien une chaîne interrompue d'alkylène, alcénylène ou alkynylène telle que définie, substituée par un ou plusieurs substitutants indépendamment sélectionnés dans le groupe consistant en phényle, phényle W-substitué, hétéroaryle et hétéroaryle W-substitué; ou bien l'un de $R_{21}$-E et $R_{22}$-F est sélectionné dans le groupe consistant en halogéno, OH, alcoxy $C_1$-$_6$, -OC(O)$R_5$, -$NR_{10}R_{11}$, -SH ou -S(alkyle $C_1$-6);

$R_5$ est alkyle $C_{1-6}$, phényle, $R_{14}$-phényle, benzyle ou $R_{14}$-benzyle;

$R_6$ est OH, alkyle $C_{1-6}$, phényle, benzyle, $R_{14}$-phényle ou $R_{14}$-benzyle;

$R_8$ est H, alkyle $C_{1-6}$, phényl alkyl $C_{1-6}$ ou-C(O)$R_9$;

$R_9$ est H, alkyle $C_{1-6}$, phényle ou phényl alkyle $C_{1-6}$;

$R_{10}$ et $R_{11}$ sont indépendamment sélectionnés parmi H et alkyle $C_{1-6}$;

$R_{12}$ est H, OH, alcoxy $C_{1-6}$, phénoxy, benzyloxy,

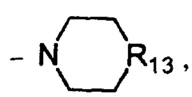

-$NR_{10}R_{11}$, alkyle $C_{1-6}$, phényle ou $R_{14}$-phényle;

$R_{13}$ est -O-, -$CH_2$-, -NH- ou -N(alkyle $C_{1-6}$)-;

$R_{14}$ est 1-3 groupes indépendamment sélectionnés dans le groupe consistant en alkyle $C_{1-6}$, alcoxy $C_{1-6}$, -COOH, $NO_2$, -$NR_{10}R_{11}$, OH ou halogéno;

à condition que quand G est une liaison, $R_{23}$ ne soit pas H, et à condition que quand $R_{23}$ est phényle W-substitué, W ne soit pas p-halogéno;

ou bien un sel pharmaceutiquement acceptable;

dans un support pharmaceutiquement acceptable.

**11.** Composé de la revendication 1 sélectionné dans le groupe consistant en composés de la formule

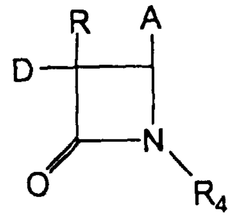

où D, R, A et $R_4$ et la stéréochimie relative entre D et A sont tels que définis au tableau qui suit :

| D | R | A | Stéréochimie relative | R4 |
|---|---|---|---|---|
| $C_6H_5-(CH_2)_3-$ | H | $p-CH_3O-C_6H_4-$ | 3S,4S | $p-CH_3O-C_6H_4-$ |
| $C_6H_5-(CH_2)_3-$ | H | $p-NO_2-C_6H_4-$ | 3R,4R | $p-CH_3O-C_6H_4-$ |
| $C_6H_5-(CH_2)_3-$ | H | $p-OH-C_6H_4-$ | 3S,4S | $p-CH_3O-C_6H_4-$ |
| $C_6H_5-(CH_2)_3-$ | H | $3-CH_3O-C_6H_4-$ | 3S,4S | $p-CH_3O-C_6H_4-$ |
| $C_6H_5-(CH_2)_3-$ | H | $p-CH_3O-C_6H_4-$ | trans | $p-CF_3O-C_6H_4-$ |
| $C_6H_5-(CH_2)_3-$ | H | $4-CH_3O-C_6H_4-$ | cis | $p-Cl-C_6H_4-$ |
| $C_6H_5-(CH_2)_3-$ | H | $4-CH_3O-C_6H_4-$ | cis | $p-CH_3-C_6H_4-$ |
| $C_6H_5-(CH_2)_3-$ | H | 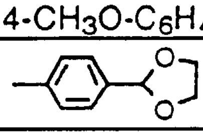 | trans | $p-CH_3O-C_6H_4-$ |
| $C_6H_5-(CH_2)_3-$ | H | $4-((CH_3)_2CH-O)-C_6H_4-$ | cis | $p-CH_3O-C_6H_4-$ |
| $C_6H_5-(CH_2)_3-$ | H | $4-(CH_3(CH)_2-O)-C_6H_4-$ | cis | $p-CH_3O-C_6H_4-$ |
| $C_6H_5-(CH_2)_3-$ | H | 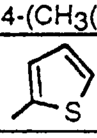 | cis | $p-CH_3O-C_6H_4-$ |
| $C_6H_5-(CH_2)_3-$ | H | $CH_3CH_2-O-C_6H_4-$ | cis | $p-CH_3O-C_6H_4-$ |
| $C_6H_5-(CH_2)_3-$ | H | $CH_3(CH_2)_3-O-C_6H_4-$ | cis | $p-CH_3O-C_6H_4-$ |
| $C_6H_5-(CH_2)_3-$ | H | $4-(C_6H_5-O)-C_6H_4-$ | cis | $p-CH_3O-C_6H_4-$ |
| $C_6H_5-(CH_2)_3-$ | H | $4-(CH_2=CH-CH_2-O)-C_6H_4-$ | cis | $p-CH_3O-C_6H_4-$ |
| $C_6H_5-(CH_2)_3-$ | H | $4-(CH_3CH_2-O)-C_6H_4-$ | cis | $C_6H_5-$ |
| 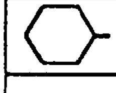 $(CH_2)_3-$ | H | $4-CH_3O-C_6H_4-$ | cis | $4-CH_3O-C_6H_4-$ |
| $4-F-C_6H_4-O-(CH_2)_2-$ | H | $4-CH_3O-C_6H_4-$ | cis | $4-CH_3O-C_6H_4-$ |
| $4-F-C_6H_4-O-(CH_2)_2-$ | H | $4-CH_3O-C_6H_4-$ | cis | $C_6H_5-$ |
| $C_6H_5-(CH_2)_3-$ | H | $4-((CH_3)_2N-(CH_2)_3-O)-C_6H_4-$ | cis | $4-CH_3O-C_6H_4-$ |

| | | | | |
|---|---|---|---|---|
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| $C_6H_5$-$(CH_2)_3$- | H | 4-$CH_3O$-$C_6H_4$- | trans | 4-($C_6H_5$-O)-$C_6H_4$- |
| 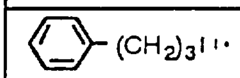 | H | 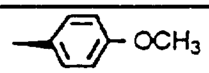 | trans optique pure | $C_6H_5$- |
| 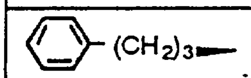 | H | 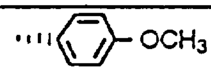 | trans optique pure | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($C_6H_5$-$CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| $C_6H_5$-O-$(CH_2)_2$- | H | 4-$CH_3O$-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |
| $C_6H_5$-$(CH_2)_3$- | H | 4-$CH_3O$-$C_6H_4$- | trans | 4-($CH_3CH_2OC(O)$)-$C_6H_4$- |
| $C_6H_5$-$(CH_2)_3$- | H | 4-$CH_3O$-$C_6H_4$- | trans | 3-$NO_2$-$C_6H_4$- |
| $C_6H_5$-$(CH_2)_3$- | H | 4-$CH_3O$-$C_6H_4$- | trans | 4-($(CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 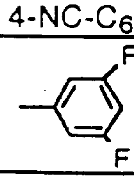 |
| $C_6H_5$-$(CH_2)_3$- | H | 4-$CH_3O$-$C_6H_4$- | trans | 4-($CH_3C(O)$)-$C_6H_4$- |

| | | | | |
|---|---|---|---|---|
| $C_6H_5-(CH_2)_3-$ | H | $4-CH_3O-C_6H_4-$ | trans | 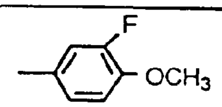 |
| $C_6H_5-(CH_2)_3-$ | H | $4-CH_3O-C_6H_4-$ | trans | 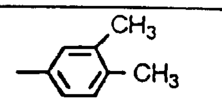 |
| $C_6H_5-(CH_2)_3-$ | H | $4-((CH_3)_2CH-O)-C_6H_4-$ | trans | $4-CH_3O-C_6H_4-$ |
| $C_6H_5-(CH_2)_3-$ | H | $4-(CH_3(CH_2)_2-O)-C_6H_4-$ | trans | $4-CH_3O-C_6H_4-$ |
| $C_6H_5-(CH_2)_3-$ | H | $4-(CH_3OC(O))-C_6H_4-$ | trans | $C_6H_5-$ |
| $C_6H_5-(CH_2)_3-$ | H | 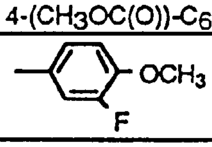 | trans | $4-CH_3O-C_6H_4-$ |
| $C_6H_5-(CH_2)_3-$ | H | $4-(CH_3OCH_2O)-C_6H_4-$ | trans | $C_6H_5-$ |
| $C_6H_5-(CH_2)_3-$ | H | $4-((CH_3)_2N)-C_6H_4-$ | trans | $4-CH_3O-C_6H_4-$ |
| $C_6H_5-(CH_2)_3-$ | H | 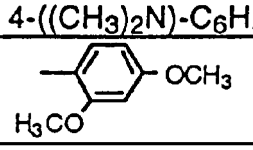 | trans | $4-CH_3O-C_6H_4-$ |
| $C_6H_5-(CH_2)_3-$ | H | $4-(CH_3CH_2-O)-C_6H_4-$ | trans | $C_6H_5-$ |
| $4-O_2N-C_6H_4-(CH_2)_3-$ | H | $4-CH_3O-C_6H_4-$ | trans | $4-CH_3O-C_6H_4-$ |
| $C_6H_5-(CH_2)_3-$ | H | $4-CH_3O-C_6H_4-$ | trans | $4-I-C_6H_4-$ |
| $C_6H_5-(CH_2)_3-$ | H | $4-(CH_3(CH_2)_3-O)-C_6H_4-$ | trans | $4-CH_3O-C_6H_4-$ |
| 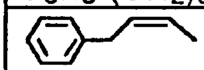 | H | $4-CH_3O-C_6H_4-$ | trans | $4-CH_3O-C_6H_4-$ |
| $C_6H_5-(CH_2)_5-$ | H | $4-CH_3O-C_6H_4-$ | trans | $4-CH_3O-C_6H_4-$ |
| 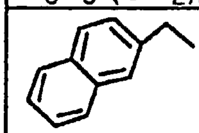 | H | $4-CH_3O-C_6H_4-$ | trans | $C_6H_5-$ |
| $4-(CH_3O)-C_6H_4-O-(CH_2)_2-$ | H | $4-CH_3O-C_6H_4-$ | trans | $C_6H_5-$ |
| 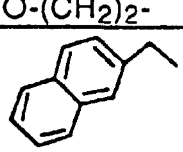 | H | $4-CH_3O-C_6H_4-$ | cis | $C_6H_5-$ |
| $C_6H_5-(CH_2)_3-$ | H | $4-CH_3O-C_6H_4-$ | trans | 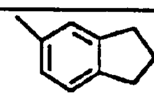 |
| $C_6H_5-(CH_2)_3-$ | H | $4-NH_2-C_6H_4-$ | cis | $4-CH_3O-C_6H_4-$ |
| $C_6H_5-(CH_2)_3-$ | H | $4-CH_3O-C_6H_4-$ | trans | $3-NH_2-C_6H_4-$ |
| $C_6H_5-(CH_2)_3-$ | H | $4-OH-C_6H_4-$ | trans | $4-CH_3O-C_6H_4-$ |
| $C_6H_5-(CH_2)_3-$ | H | $4-CH_3O-C_6H_4-$ | 3R,4S | $4-CH_3O-C_6H_4-$ |
| $C_6H_5-(CH_2)_3-$ | H | $4-OH-C_6H_4-$ | trans | $4-OH-C_6H_4-$ |
| $C_6H_5-(CH_2)_3-$ | H | $4-CH_3O-C_6H_4-$ | 3R,4S | $4-OH-C_6H_4-$ |

| | | | | |
|---|---|---|---|---|
| $C_6H_5-(CH_2)_3-$ | H | 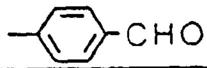 | trans | $4-CH_3O-C_6H_4-$ |
| $C_6H_5-(CH_2)_3-$ | H | $4-CH_3O-C_6H_4-$ | trans | $4-(CH_3SO_2)-C_6H_4-$ |
| $C_6H_5-(CH_2)_3-$ | H | 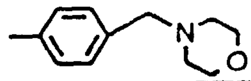 | trans | $4-CH_3O-C_6H_4-$ |
| $C_6H_5-(CH_2)_3-$ | H | 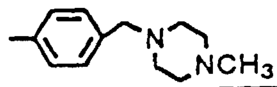 | trans | $4-CH_3O-C_6H_4-$ |
| $C_6H_5-(CH_2)_3-$ | H | $4-(CH_3OCH_2)-C_6H_4-$ | trans | $4-CH_3O-C_6H_4-$ |
| $C_6H_5-(CH_2)_3-$ | H | 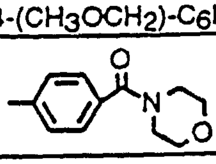 | cis | $4-CH_3O-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 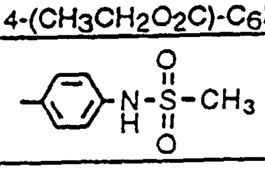 | trans | $4-CH_3O-C_6H_4-$ |
| $C_6H_5-(CH_2)_3-$ | H | $-Ph-O\overset{O}{\overset{\|}{C}}(CH_2)_2\overset{O}{\overset{\|}{C}}OCH_3$ où Ph est 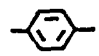 | trans | $4-CH_3O-C_6H_4-$ |
| $C_6H_5-(CH_2)_3-$ | H | $-Ph-OCH_2CO_2Et$ où Ph est 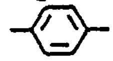 et ET est $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 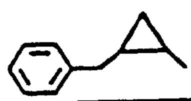 | H | $4-CH_3O-C_6H_4-$ | cis | $4-CH_3O-C_6H_4-$ |

**12.** Composé de la revendication 1, ou 11 qui est (3R, 4S)-1,4-bis-(4-méthoxyphényl)-3-(3-phénylpropyl)-2-azétidinone.

**13.** Utilisation de la revendication 10 où le composé est sélectionné dans le groupe consistant en composés de la formule

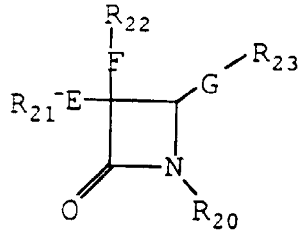

où $R_{21}$-E-, $R_{22}$-F-, $R_{23}$-G-, $R_{20}$ et la stéréochimie relative entre $R_{21}$-E et $R_{23}$-G sont tels que définis au tableau qui suit :

| $R_{21}$-E- | $R_{22}$-F- | $R_{23}$-G- | Stéréochimie relative | $R_{20}$ |
|---|---|---|---|---|
| $C_6H_5-(CH_2)_3-$ | H | p-$CH_3O-C_6H_4-$ | 3S,4S | p-$CH_3O-C_6H_4-$ |
| $C_6H_5-(CH_2)_3-$ | H | p-$OH-C_6H_4-$ | 3S,4S | p-$CH_3O-C_6H_4-$ |
| $C_6H_5-(CH_2)_3-$ | H | p-$CH_3O-C_6H_4-$ | trans | p-$CF_3O-C_6H_4-$ |
| $C_6H_5-(CH_2)_3-$ | H | 4-$CH_3O-C_6H_4-$ | cis | p-$Cl-C_6H_4-$ |
| $C_6H_5-(CH_2)_3-$ | H | 4-$CH_3O-C_6H_4-$ | cis | p-$CH_3-C_6H_4-$ |
| $C_6H_5-(CH_2)_3-$ | H | 4-$((CH_3)_2CH-O)-C_6H_4-$ | cis | p-$CH_3O-C_6H_4-$ |
| $C_6H_5-(CH_2)_3-$ | H | 4-$(CH_3(CH)_2-O)-C_6H_4-$ | cis | p-$CH_3O-C_6H_4-$ |
| $C_6H_5-(CH_2)_3-$ | H | $CH_3CH_2-O-C_6H_4-$ | cis | p-$CH_3O-C_6H_4-$ |
| $C_6H_5-(CH_2)_3-$ | H | $CH_3(CH_2)_3-O-C_6H_4-$ | cis | p-$CH_3O-C_6H_4-$ |
| $C_6H_5-(CH_2)_3-$ | H | 4-$(CH_2=CH-CH_2-O)-C_6H_4-$ | cis | p-$CH_3O-C_6H_4-$ |
| $C_6H_5-(CH_2)_3-$ | H | 4-$(CH_3CH_2-O)-C_6H_4-$ | cis | $C_6H_5-$ |
| 4-$F-C_6H_4-O-(CH_2)_2-$ | H | 4-$CH_3O-C_6H_4-$ | cis | $C_6H_5-$ |
| $C_6H_5-(CH_2)_3-$ | H | 4-$CH_3O-C_6H_4-$ | cis | $C_6H_5-$ |
| $C_6H_5-(CH_2)_3-$ | H | 4-$CH_3O-C_6H_4-$ | 3S,4S | p-$CH_3O-C_6H_4-$ |
| $C_6H_5-(CH_2)_3-$ | H | p-$CH_3O-C_6H_4-$ | 3S,4R | p-$CH_3O-C_6H_4-$ |
| $C_6H_5-(CH_2)_3-$ | H | 4-$CH_3O-C_6H_4-$ | trans | 4-$F-C_6H_4-$ |
| $C_6H_5-(CH_2)_3-$ | H | 4-$CH_3O-C_6H_4-$ | trans | 3-$CH_3O-C_6H_4-$ |
| $C_6H_5-(CH_2)_3-$ | H | 4-$CH_3O-C_6H_4-$ | trans | 4-$Cl-C_6H_4-$ |
| $C_6H_5-(CH_2)_3-$ | H | 4-$CH_3O-C_6H_4-$ | trans | 4-$(CH_3CH_2-O)-C_6H_4-$ |

| | | | | |
|---|---|---|---|---|
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| 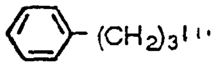 | H | 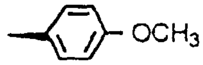 | trans optique pure | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |
| $C_6H_5$-$(CH_2)_3$- | H | 4-$CH_3O$-$C_6H_4$- | trans | 4-(($CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 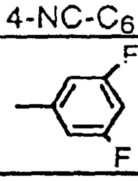 |
| $C_6H_5$-$(CH_2)_3$- | H | 4-$CH_3O$-$C_6H_4$- | trans | 4-($CH_3C$(O))-$C_6H_4$- |
| $C_6H_5$-$(CH_2)_3$- | H | 4-(($CH_3)_2$CH-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC$(O))-$C_6H_4$- | trans | $C_6H_5$- |
| $C_6H_5$-$(CH_2)_3$- | H | 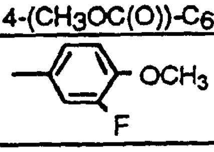 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 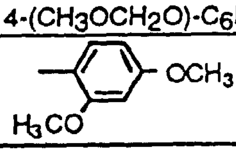 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| $C_6H_5$-$(CH_2)_3$- | H | 4-$CH_3O$-$C_6H_4$- | trans | 4-I-$C_6H_4$- |
| 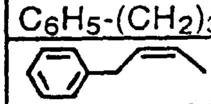 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 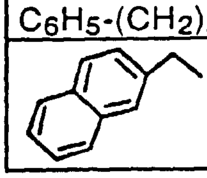 | H | 4-$CH_3O$-$C_6H_4$- | trans | $C_6H_5$- |

| | | | | |
|---|---|---|---|---|
| 4-$(CH_3O)$-$C_6H_4$-O-$(CH_2)_2$- | H | 4-$CH_3O$-$C_6H_4$- | trans | $C_6H_5$- |
| 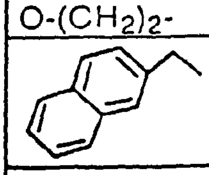 | H | 4-$CH_3O$-$C_6H_4$- | cis | $C_6H_5$- |
| $C_6H_5$-$(CH_2)_3$- | H | 4-$CH_3O$-$C_6H_4$- | trans | 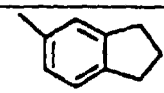 |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-$(CH_3CH_2O_2C)$-$C_6H_4$- | trans | 4-$CH_3O$-$C_6H_4$- |
| $C_6H_5$-$(CH_2)_3$- | H | $-PhO\overset{O}{C}(CH_2)_2\overset{O}{C}OCH_3$ où Ph est 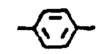 | trans | 4-$CH_3O$-$C_6H_4$- |
| $C_6H_5$-$(CH_2)_3$- | H | $-Ph-OCH_2CO_2Et$ où Ph est 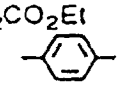 et Et est $-CH_2CH_3$ | 3R,4S | 4-$CH_3O$-$C_6H_4$- |
| $C_6H_5$-$(CH_2)_3$- | H | 4-$CH_3O$-$C_6H_4$- | cis | 4-$(CH_3CH_2O)$-$C_6H_4$- |
| 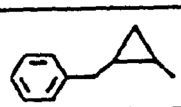 | H | 4-$CH_3O$-$C_6H_4$- | cis | 4-$CH_3O$-$C_6H_4$- |
| $C_6H_5$-$(CH_2)_2$-NH- | H | 4-$CH_3O$-$C_6H_4$- | cis | $C_6H_5$- |
| 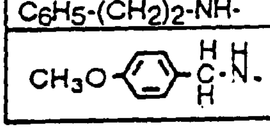 | H | 4-$CH_3O$-$C_6H_4$- | cis | $C_6H_5$- |

**14.** Utilisation d'un composé de la revendication 1 pour la fabrication d'un médicament pour abaisser le cholestérol dans le sérum.

**15.** Utilisation d'un composé de la revendication 1 pour la fabrication d'un médicament pour inhiber l'enzyme acyl CoA: cholestérol acyl transférase.

**16.** Utilisation d'une composition telle que définie à la revendication 10 pour la fabrication d'un médicament pour abaisser les niveaux de cholestérol dans le sérum chez un mammifère nécessitant un tel traitement.

**17.** Utilisation d'une composition telle que définie à la revendication 10 pour la fabrication d'un médicament pour l'inhibition de l'enzyme acyl CoA: cholestérol acyl tranférase.

**18.** Procédé pour la production d'un composé de la revendication 1, où R est H, et D et A ont une stéréochimie relative trans, comprenant la cyclisation d'une hydroxyamide de la formule

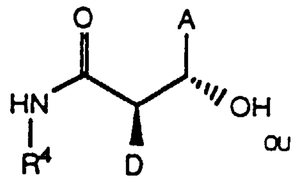

ou

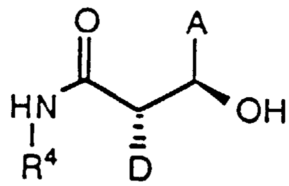

où D, A et $R^4$ sont tels que définis à la revendication 1, par traitement avec :

(i) un dialkylazodicarboxylate et une trialkylphosphine; ou
(ii) un chlorure de di- ou tri-chlorobenzoyle, une base aqueuse et un catalyseur de transfert de phase, puis traitement du di- ou tri-chlorobenzoate résultant avec une base aqueuse et un catalyseur de transfert de phase; ou
(iii) un dialkylchlorophosphate, une base aqueuse et un catalyseur de transfert de phase; ou
(iv) un chlorure de di- ou tri-chlorobenzoyle et un hydrure d'un métal alcalin.

**19.** Procédé de production d'un composé de la revendication 1 ayant la formule

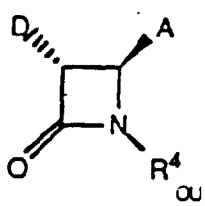

ou

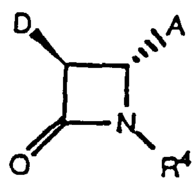

où D, A et $R^4$ sont tels que définis dans la revendication 1, comprenant :

(a) la réaction d'un acide carboxylique de la formule $DCH_2$-COOH, où D est tel que défini ci-dessus, avec un agent de chloruration;

(b) la déprotonation d'une oxazolidinone chirale de la formule

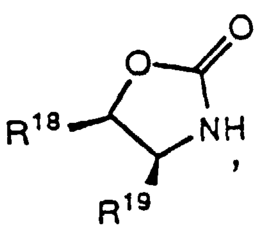

,

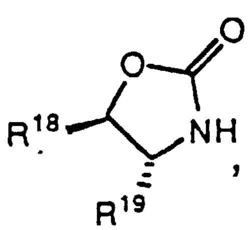

,

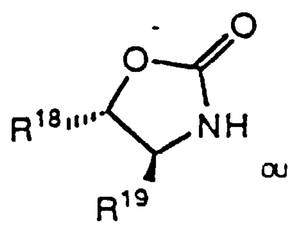

ou

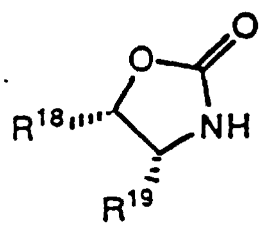

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; avec une base forte et le traitement de l'anion résultant avec le produit de l'étape (a);

(c) l'énolisation du produit de l'étape (b) avec soit:

(i) un triflate de dialkylbore et une base d'amine tertiaire;
(ii) $TiCl_4$ et de la tétraméthyléthylène diamine (TMEDA) ou un mélange de TMEDA et de triéthylamine;

puis la condensation avec un aldéhyde de la formule A -CHO, où A est tel que défini ci-dessus;

(d) l'hydrolyse du produit de l'étape (c) avec une base et du péroxyde d'hydrogène;

(e) la condensation du produit de l'étape (d) avec une amine de la formule $R^4NH_2$, où $R^4$ est tel que défini ci-dessus; par traitement avec un agent de couplage déshydratant, en ajoutant facultativement un agent activant; et

(f) la cyclisation du produit de l'étape (e) par traitement avec :

(i) un dialkylazodicarboxylate et une trialkylphosphine; ou

(ii) un chlorure de di- ou tri-chlorobenzoyle, une base aqueuse et un catalyseur de transfert de phase, puis traitement du di- ou tri-chlorobenzoate résultant avec une base aqueuse et un catalyseur de transfert de phase; ou

(iii) un dialkylchlorophosphate, une base aqueuse et un catalyseur de transfert de phase; ou

(iv) un chlorure de di- ou tri-chlorobenzoyle et un hydrure d'un métal alcalin.

**20.** Procédé de production d'un composé de la revendication 1, où R est hydrogène et D et A ont une stéréochimie relative trans, comprenant :

(a) l'énolisation d'un composé de formule

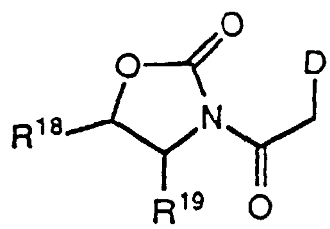

avec $TiCl_4$ et de la tétraméthyléthylènediamine (TMEDA), puis la condensation avec une imine de la formule $A-CH=N-R^4$, où A et $R^4$ sont tels que définis ci-dessus, et où le groupe

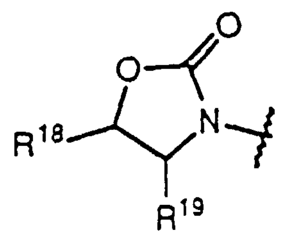

représente un auxiliaire chiral de la formule

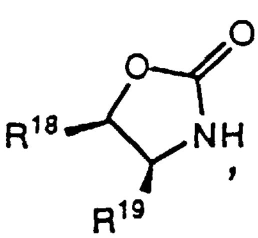 , 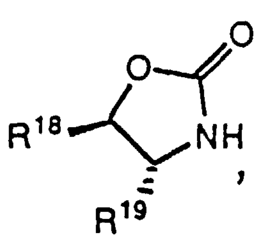 , 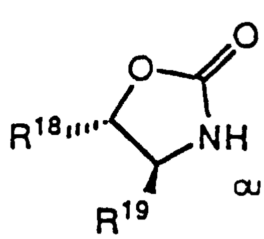 ou 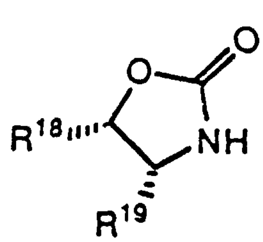

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; et

(b) la cyclisation du produit de l'étape (a) par traitement avec une base non nucléophile forte.

**21.** Procédé de préparation de composés de la revendication 1 comprenant :

(a) le traitement d'un ester de la formule D-CH(R)-C(O)OR$^{17}$, où R$^{17}$ est alkyle $C_{1-6}$, menthyle, ou 10-(diisopropylsulfonamido) isobornyle, avec une base forte, puis avec une imine de la formule A-CH=N-R$^4$, où A et R$^4$ sont tels que définis à la revendication 1; ou

(b) pour la préparation d'un composé de la revendication 1 où R n'est pas hydrogène, la réaction d'un composé de la revendication 1, où R est hydrogène, avec une base forte et un agent d'alkylation ou un agent d'acylation; ou

(c) pour la préparation d'un composé de la revendication 1, où R est hydrogène, et D et A ont une stéréochimie relative cis, la réaction d'un composé de la revendication 1, où R est hydrogène, et D et A ont une stéréochimie relative trans, avec une base forte entre -80 et -40°C, et puis avec une source de protons; ou

(d) pour la préparation d'un composé de la revendication 1, où R est hydrogène, et D et A ont une stéréochimie relative trans, la réaction d'un composé de la revendication 1, où R est hydrogène, et D et A ont une stéréochimie relative cis, avec un t-butoxyde d'un métal alcalin, puis l'arrêt avec un acide; ou

(e) la réaction d'un dérivé d'acide carboxylique de la formule D-CH(R)-C(O)-Z, où D et R sont tels que définis à la revendication 1 et Z est Cl,

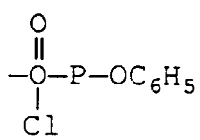

, ou

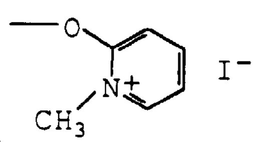

avec une imine de la formule A-CH=N-R$^4$, où A et R$^4$ sont tels que définis à la revendication 1, en présence d'une base d'amine tertiaire.

**22.** Procédé de préparation d'un composé de formule II

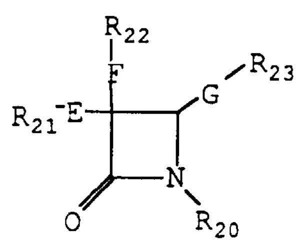

II

où

$R_{20}$ est phényle, phényle W-substitué, naphtyle, naphtyle W-substitué, benzodioxolyle, hétéroaryle, hétéroaryle W-substitué, hétéroaryle benzofusionné et hétéroaryle benzofusionné W-substitué, où hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazolyle et furanyle, et pour les hétéroaryles contenant de l'azote, leurs N-oxydes;

$R_{21}$, $R_{22}$ et $R_{23}$ sont indépendamment sélectionnés parmi H ou $R_{20}$;

W est 1 à 3 substituants indépendamment sélectionnés dans le groupe consistant en alkyle $C_{1-6}$, hydroxy alkyle $C_{1-6}$, alcoxy $C_{1-6}$, alcoxy $C_{1-6}$ alkyle $C_{1-6}$, alcoxy $C_{1-6}$ -alcoxy $C_{1-6}$, alcoxy $C_{1-6}$-carbonyl-alcoxy $C_{1-6}$, (alcoxyimino $C_{1-6}$)alkyle $C_{1-6}$, alcanedioyle $C_{3-6}$, alkyle $C_{1-6}$-alcanedioyle $C_{3-6}$, allyloxy, $-CF_3$, $-OCF_3$, benzyle,

$R_{14}$-benzyle, benzyloxy, $R_{14}$-benzyloxy, phénoxy, $R_{14}$-phénoxy, dioxolanyle, $NO_2$, $-NR_{10}R_{11}$, $NR_{10}R_{11}$(alkyle $C_{1-6}$), $NR_{10}R_{11}$(alcoxy $C_{1-6}$)-, OH, halogéno, $-NHC(O)OR_5$, $-NHC(O)R_5$, $R_6O_2SNH$-, $(R_6O_2S)_2N$-, $-S(O)_2NH_2$, $-S(O)_{0-2}R_{10}$, tert-butyldiméthylsilyloxyméthyle, $-C(O)R_{12}$,

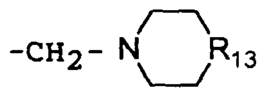 et 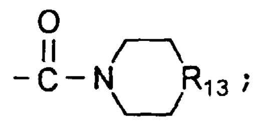

E, F et G sont indépendamment une liaison; cycloalkylène $C_3$-$C_6$; alkylène $C_1$-$C_{10}$; alcénylène $C_2$-$C_{10}$; alkynylène $C_2$-$C_{10}$; une chaîne d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué, où hétéroaryle est tel que défini ci-dessus; une chaîne d'alkylène, alcénylène ou alkynylène telle que définie, interrompue par un ou plusieurs groupes indépendamment sélectionnés dans le groupe consistant en -O-, -S-, -SO-, $-SO_2$-, $-NR_8$, -C(O)-, cycloalkylène $C_3$-$C_6$, phénylène, phénylène W-substitué, hétéroarylène et hétéroarylène W-substitué; ou bien une chaîne interrompue d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué; ou bien l'un de $R_{21}$-E et $R_{22}$-F est sélectionné dans le groupe consistant en halogéno, OH, alcoxy inférieur, $-OC(O)R_5$, $-NR_{10}R_{11}$, -SH ou -S(alkyle $C_{1-6}$);

$R_5$ est alkyle $C_{1-6}$, phényle, $R_{14}$-phényle, benzyle ou $R_{14}$-benzyle;

$R_6$ est OH, alkyle $C_{1-6}$, phényle, benzyle, $R_{14}$-phényle ou $R_{14}$-benzyle;

$R_8$ est H, alkyle $C_{1-6}$, phényle alkyle $C_{1-6}$ ou $-C(O)R_9$;

$R_9$ est H, alkyle $C_{1-6}$, phényle ou phényle alkyle $C_{1-6}$;

$R_{10}$ et $R_{11}$ sont indépendamment sélectionnés parmi H et alkyle $C_{1-6}$;

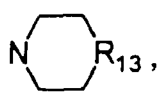

$R_{12}$ est H, OH, alcoxy $C_{1-6}$, phénoxy, benzyloxy, $-NR_{10}R_{11}$, alkyle $C_{1-6}$, phényle ou $R_{14}$-phényle;

$R_{13}$ est -O-, $-CH_2$-, -NH- ou -N(alkyle $C_{1-6}$)-;

$R_{14}$ est 1-3 groupes indépendamment sélectionnés dans le groupe consistant en alkyle $C_{1-6}$, alcoxy $C_{1-6}$, -COOH, $NO_2$, $-NR_{10}R_{11}$, OH ou halogéno;

à condition que quand G est une liaison, $R_{23}$ ne soit pas H, et à condition que quand $R_{23}$ est phényle W-substitué, W ne soit pas p-halogéno;

ou bien un sel pharmaceutiquement acceptable;

où $R_{22}$-F est H, et $R_{21}$-E- et $R_{23}$-G- ont une stéréochimie relative trans, comprenant

Procédé C: la cyclisation d'une hydroxyamide de la formule

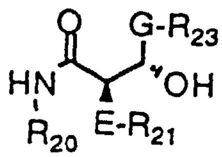

ou

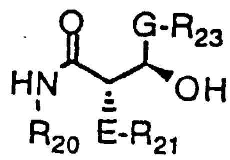

où $R_{21}$-E-, $R_{23}$-G- et $R_{20}$ sont tels que définis ci-dessus, par traitement avec :

(i) un dialkylazodicarboxylate et une trialkylphosphine; ou

(ii) un chlorure de di- ou tri-chlorobenzoyle, une base aqueuse et un catalyseur de transfert de phase, puis traitement du di- ou tri-chlorobenzoate résultant avec une base aqueuse et un catalyseur de transfert de phase; ou

(iii) un dialkylchlorophosphate, une base aqueuse et un catalyseur de transfert de phase; ou

(iv) un chlorure de di- ou tri-chlorobenzoyle et un hydrure d'un métal alcalin;

ou
Procédé D:

(a) la réaction d'un acide carboxylique de la formule $R_{21}$-E-$CH_2$-COOH, où $R_{21}$-E- est tel que défini ci-dessus avec un agent de chloruration;

(b) la déprotonation d'une oxazolidinone chirale de la formule

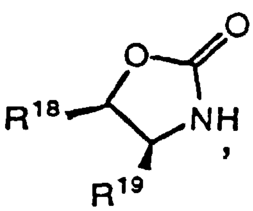
,
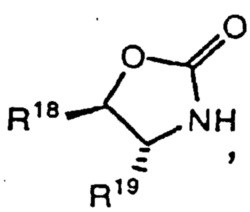
,
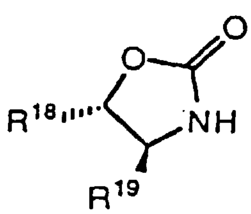
ou
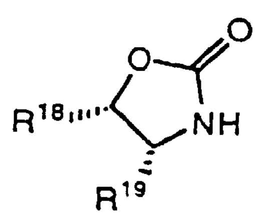

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; avec une base forte et traitement de l'anion résultant avec le produit de l'étape (a);

(c) l'énolisation du produit de l'étape (b) avec soit :

(i) un triflate de dialkylbore et une base d'amine tertiaire;

(ii) $TiCl_4$ et de la tétraméthyléthylènediamine (TMEDA) ou un mélange de TMEDA et de triéthylamine;

puis la condensation avec un aldéhyde de la formule $R_{23}$-G-CHO, où $R_{23}$-G est tel que défini ci-dessus;

(d) l'hydrolyse du produit de l'étape (c) avec une base et du péroxyde d'hydrogène;

(e) la condensation du produit de l'étape (d) avec une amine de la formule $R_{20}NH_2$, où $R_{20}$ est tel que défini ci-dessus, par traitement avec un agent de couplage déshydratant, en ajoutant facultativement un agent activant; et

(f) la cyclisation du produit de l'étape (e) telle que décrite au Procédé C.

**23.** Procédé de production d'un composé de formule II tel que défini dans la revendication 22, où $R_{22}$-F- est hydrogène, et $R_{21}$-E- et $R_{23}$-G- ont une stéréochimie relative trans, comprenant :

(a) l'énolisation d'un composé de la formule

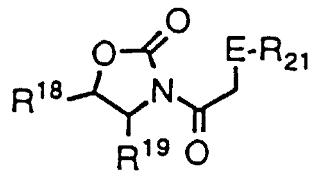

avec $TiCl_4$ et de la tétraméthyléthylènediamine (TMEDA), puis la condensation avec une imine de la formule $R_{23}$-G-CH=N-$R_{20}$, où $R_{23}$-G- et $R_{20}$ sont tels que définis à la revendication 22, et où le groupe

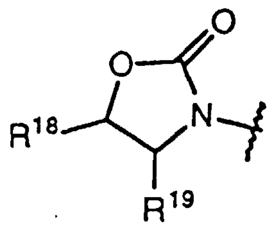

représente un auxiliaire chiral de la formule

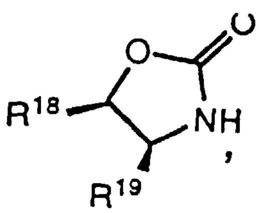 , 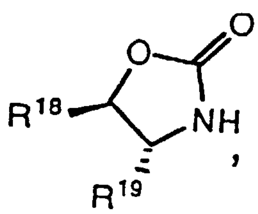 , 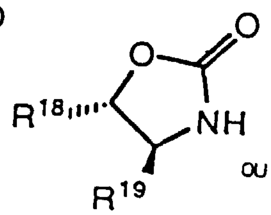 ou 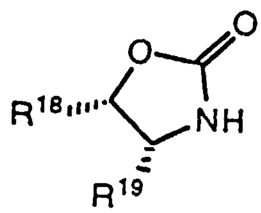

où $R^{18}$ et $R^{19}$ sont indépendamment sélectionnés dans le groupe consistant en hydrogène, alkyle $C_1$-$C_6$, phényle, naphtyle, phényle substitué, naphtyle substitué, et benzyle; et

(b) la cyclisation du produit de l'étape (a) par traitement avec une base non nucléophile forte.

**24.** Procédé de préparation d'un composé de formule II tel que défini à la revendication 22, comprenant

(a) la traitement d'un ester de la formule $R_{21}$-E-CH(F-$R_{22}$)-C(O)O$R^{17}$, où $R^{17}$ est alkyle $C_{1-6}$, menthyle, ou l0-(diisopropylsulfonamido)-isobornyle, avec une base forte, et puis avec un imine de la formule $R_{23}$-G-CH=N-$R_{20}$, où $R_{23}$-G- et $R_{20}$ sont tels que définis à la revendication 22, ou

(b) pour la préparation d'un composé de formule II tel que défini à la revendication 22, où $R_{22}$-F- n'est pas de l'hydrogène, la réaction d'un composé de formule II tel que défini à la revendication 22, où $R_{22}$-F- est de l'hydrogène, avec une base forte et un agent d'alkylation ou un agent d'acylation; ou

(c) pour la préparation d'un composé de formule II tel que défini dans la revendication 22, où $R_{22}$-F- est hydrogène, et $R_{21}$-E- et $R_{23}$-G- ont une stéréochimie relative cis, la réaction d'un composé de la formule II tel que défini à la revendication 22, où $R_{22}$-F- est hydrogène, et $R_{21}$-E-, et $R_{23}$-G- ont une stéréochimie relative trans, avec une base forte à -80° jusqu'à -40°C, et puis avec une source de protons; ou

(d) pour la préparation d'un composé de formule II tel que défini à la revendication 22, où $R_{22}$-F- est hydrogène, et $R_{21}$-E- et $R_{23}$-G- ont une stéréochimie relative trans, la réaction d'un composé de formule II tel que défini à la revendication 22, où $R_{22}$-F- est hydrogène et $R_{21}$-E- et $R_{23}$-G- ont une stéréochimie relative cis avec un t-butoxyde d'un métal alcalin, puis l'arrêt avec un acide; ou

(e) la réaction d'un dérivé d'acide carboxylique de la formule $R_{21}$-E-CH(F-$R_{22}$)-C(O)-Z, où $R_{21}$-E- et $R_{22}$-F- sont tels que définis à la revendication 22, et Z est Cl,

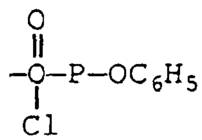

, ou

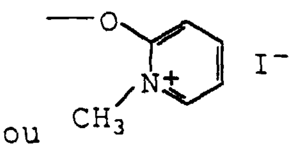

,

avec une imine de la formule $R_{23}$-G-CH=N-$R_{20}$, où $R_{23}$-G- et $R_{20}$ sont tels que définis à la revendication 22, en présence d'une base d'amine tertiaire.

**Revendications pour l'Etat contractant suivant : ES**

**1.** Un procédé de production d'un composé ayant la formule de structure I

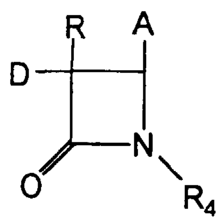

I

où

A est

-CH=CH-B;
-C≡C-B;
-$(CH_2)_p$-X-B, où p est 0, 1 ou 2 et X est une liaison, -NH- ou -$S(O)_{0-2}$-;
hétérearyle, hétéroaryle benzofusionné, hétéroaryle W substitué ou hétéroaryle benzofusionné W-substitué, où hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazolyle et furanyle, et pour les hétéroaryles contenant de l'azote, leurs N-oxydes et où W est 1-3 substituants sur les atomes de carbone du cycle sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$-alcoxy $C_{1-6}$, alcoxy $C_{1-6}$-carbonylalcoxy $C_{1-6}$, (alcoxyamino $C_{1-6}$)alkyle $C_{1-6}$, alcanedioyle $C_{3-6}$, alkyl $C_{1-6}$-alcanedioyle $C_{3-6}$, allyloxy, -$CF_3$, - $OCF_3$, benzyle, $R_{14}$ benzyle, benzyloxy, $R_{14}$-benzyloxy, phénoxy, $R_{14}$-phénoxy, dioxolanyle, $NO_2$, -$NR_{10}R_{11}$, $NR_{10}R_{11}$ (alkyle $C_{1-6}$)-, $NR_{10}R_{11}$ (alcoxy $C_{1-6}$)-, OH, halogéno, -NHC(O)$OR_5$, -NHC(O)$R_5$, $R_6O_2SNH$-, $(R_6O_2S)_2N$-, $S(O)_2NH_2$, tert-butyldiméthylsilyloxyméthyle, -C(O)$R_{12}$ et

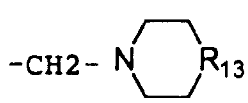

,

et où les substituants sur les atomes d'azote du cycle hétéroaryle substitué, quand ils sont présents, sont

sélectionnés dans le groupe consistant en alkyle $C_{1-6}$, alcoxy $C_{1-6}$, $-C(O)OR_5$, $-C(O)R_5$, OH, $NR_{10}R_{11}$ (alkyle inférieur)-, $NR_{10}R_{11}$ (alcoxy $C_{1-6}$), $-S(O)_2NH_2$ et 2-(triméthylsilyl)éthoxyméthyle;
-C(O)-B; ou

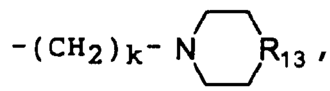

où k est 1 ou 2;

D est

$B'-(CH_2)_mC(O)-$, où m est 1, 2, 3, 4 ou 5;

$B'-(CH_2)_q-$, où q est 2, 3, 4, 5 ou 6;

$B'-(CH_2)_e-Z-(CH_2)_r-$, où Z est -O-, -C(O)-, phénylène, $-NR_8-$ ou $-S(O)_{0-2}-$, e est 0, 1, 2, 3, 4 ou 5 et r est 1, 2, 3, 4 ou 5, à condition que la somme de e et r soit 1, 2, 3, 4, 5 ou 6.

B'-(alcénylène $C_2$-$C_6$)-;B'-(alcanediénylène $C_4$-$C_6$)-;

$B'-(CH_2)_t-Z-$(alcénylène $C_2$-$C_6$)-, où Z est tel que défini ci-dessus, et où t est 0, 1, 2, ou 3, à condition que la somme de t et du nombre d'atomes de carbone dans la chaîne d'alcénylène soit 2, 3, 4, 5 ou 6;

$B'-(CH_2)_f-V-(CH_2)_g-$, où V est cycloalkylène $C_3$-$C_6$, f est 1,2,3,4 ou 5 et g est 0, 1, 2, 3, 4 ou 5, à condition que la somme de f et g soit 1, 2, 3, 4, 5 ou 6;

$B'-(CH_2)_t-V-$(alcénylène $C_2$-$C_6$)- ou B'-(alcénylène $C_2$-$C_6$) $-V-(CH_2)_t-$, où V et t sont tels que définis ci-dessus, à condition que la somme de t et du nombre d'atomes de carbone dans la chaîne d'alcénylène soit 2, 3, 4, 5 ou 6;

$B'-(CH_2)_a-Z-(CH_2)_b-V-(CH_2)_d-$, où Z et V sont tels que définis ci-dessus et a, b et d sont indépendamment 0, 1, 2, 3, 4, 5 ou 6, à condition que la somme de a, b et d soit 0, 1, 2, 3, 4, 5 ou 6;

$T-(CH_2)_s-$, où T est cycloalkyle de 3-6 atomes de carbone et s est 1, 2, 3, 4, 5 ou 6; ou

naphtylméthyle, hétéroarylméthyle, ou hétéroarylméthyle W-substitué, où hétéroaryle et W sont tels que définis ci-dessus;

B est

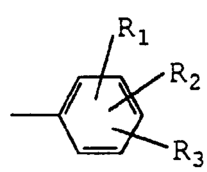

B' est naphtyle, hétéroaryle ou hétéroaryle W-substitué, où hétéroaryle est tel que défini ci-dessus, ou

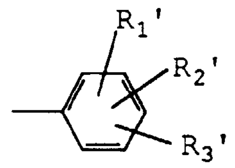

R est hydrogène, fluoro, alkyle $C_1$-$C_{15}$, alcényle $C_2$-$C_{15}$, alkynyle $C_2$-$C_{15}$, ou B-$(CH_2)_h$-, où h est 0, 1, 2 ou 3;

$R_1$, $R_2$ et $R_3$ sont indépendamment sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$-alcoxy $C_{1-6}$, alcoxy $C_{1-6}$-carbonyl-alcoxy $C_{1-6}$, (alcoxyimino $C_{1-6}$)alkyle $C_{1-6}$, alcanedioyle $C_{3-6}$, alkyle $C_{1-6}$ alcanedioyle $C_{3-6}$, alkyle $C_{1-6}$-alcanedioyle $C_{3-6}$ allyloxy, -$CF_3$, -$OCF_3$, benzyle, $R_{14}$ benzyle, benzyloxy, $R_{14}$-benzyloxy, phénoxy, $R_{14}$ phénoxy, dioxolanyle, $NO_2$, - $NR_{10}R_{11}$, $NR_{10}R_{11}$ (alkyle $C_{1-6}$)-, $NR_{10}R_{11}$ (alcoxy $C_{1-6}$)-, OH, o-halogéno, m-halogéno, -NHC (O)$OR_5$, -NHC(O)$R_5$,$R_6O_2$SNH-, $(R_6O_2S)_2$N-, -S$(O)_2NH_2$, -S$(O)_2R_{10}$, tert-butyldiméthylsilyloxyméthyle, -C(O)$R_{12}$,

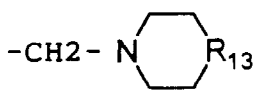 et 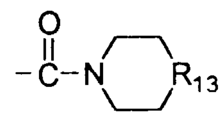 ,

ou bien $R_1$' est hydrogène et $R_2$' et $R_3$', avec les atomes de carbone adjacents auxquels ils sont attachés, forment un cycle de dioxolanyle;

$R_{1'}$, $R_{2'}$ et $R_{3'}$ sont indépendamment sélectionnés dans le groupe consistant en H, alkyle $C_{1-6}$, hydroxy alkyle $C_{1-6}$,- alcoxy $C_{1-6}$, alcoxy $C_{1-6}$-alkyle $C_{1-6}$, alcoxy $C_{1-6}$-alcoxy $C_{1-6}$, alcoxy $C_{1-6}$-carbonyl-alcoxy $C_{1-6}$, (alcoxyimino $C_{1-6}$)-alkyle $C_{1-6}$, alcanedioyle $C_{3-6}$, alkyle $C_{1-6}$ alcanedioyle $C_{3-6}$, allyloxy, -$CF_3$, -$OCF_3$, benzyle, $R_{14}$-benzyle, benzyloxy, $R_{14}$-benzyloxy, phénoxy, $R_{14}$-phénoxy, dioxolanyle, $NO_2$, -$NR_{10}R_{11}$, $NR_{10}R_{11}$ (alkyle $C_{1-6}$)-, $NR_{10}R_{11}$ (alcoxy $C_{1-6}$)-, OH, halogéno, -NHC(O)$OR_5$, -NHC(O)$R_5$, $R_6O_2$SNH-, $(R_6O_2S)_2$N-, -S$(O)_2NH_2$, -S$(O)_{0-2}R_{10}$, tert-butyldiméthyl-siloxyméthyle, -C(O)$R_{12}$,

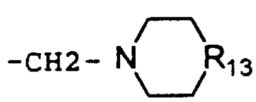

et

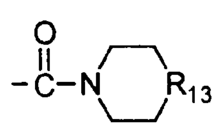 ,

ou bien $R_1$' est hydrogène et $R_{2'}$ et $R_{3'}$, avec les atomes de carbone adjacents auxquels ils sont attachés, forment ensemble un cycle de dioxolanyle;

$R_4$ est

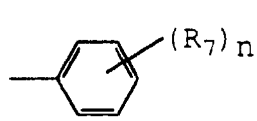 ,

où n est 0, 1, 2 ou 3, indanyle, benzofuranyle, benzodioxolyle, tétrahydronaphtyle, pyridyle, pyrazinyle, pyrimidinyle ou quinolyle;

$R_5$ est alkyle $C_{1-6}$, phényle, $R_{14}$-phényle, benzyle ou $R_{14}$-benzyle;

$R_6$ est OH, alkyle $C_{1-6}$, phényle, benzyle, $R_{14}$-phényle ou $R_{14}$-benzyle;

$R_7$ est alkyle $C_{1-6}$, alcoxy $C_{1-6}$, OH, halogéno, $-NR_{10}R_{11}$, $-NHC(O)OR_5$, $-NHC(O)R_5$, $NO_2$, -CN, $-N_3$, -SH, $-S(O)_{0-2}$- alkyle $C_{1-6}$, $-COOR_9$, $-CONR_{10}R_{11}$, $-COR_{12}$, phénoxy, benzyloxy, $-OCF_3$, ou tert-butyldiméthylsilyloxy, et quand n est 2 ou 3, les groupes $R_7$ peuvent être identiques ou différents.

$R_8$ est H, alkyle $C_{1-6}$, phényle alkyle $C_{1-6}$, ou $-C(O)R_9$;

$R_9$ est H, alkyle $C_{1-6}$, phényle ou phényle alkyle $C_{1-6}$;

$R_{10}$ et $R_{11}$ sont indépendamment sélectionnés parmi H et alkyle $C_{1-6}$;

$R_{12}$ est H, OH, alcoxy $C_{1-6}$, phénoxy, benzyloxy,

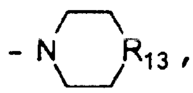

$-NR_{10}R_{11}$, alkyle $C_{1-6}$, phényle ou $R_{14}$-phényle;

$R_{13}$ est -O-, $-CH_2$-, -NH- ou -N(alkyle $C_{1-6}$)-; et

$R_{14}$ est 1-3 groupes indépendamment sélectionnés dans le groupe consistant en alkyle $C_{1-6}$, alcoxy $C_{1-6}$, -COOH, $NO_2$, $-NR_{10}R_{11}$, OH ou halogéno;

ou bien un sel pharmaceutiquement acceptable,

où R est H, et D et A ont une stochiométrie relative trans,
comprenant

Procédé A: la cyclisation d'une hydroxyamine de la formule

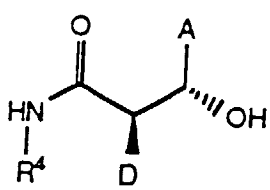

o

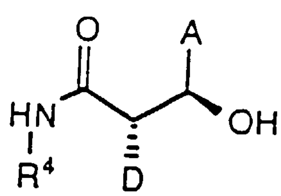

où D,A et $R^4$ sont tels que définis ci-dessus, par traitement avec:

(i) un dialkylazodicarboxylate et une trialkylphosphine; ou

(ii) un chlorure de di- ou tri-chlorobenzoyle, et une base aqueuse et un catalyseur de transfert de phase, puis traitement du di- ou tri-chlorobenzoate résultant avec une base aqueuse et un catalyseur de transfert de phase; ou

(iii) un dialkylchlorophosphate, une base aqueuse et un catalyseur de transfert de phase; ou

(iv) un chlorure de di- ou tri-chlorobenzoyle et un hydrure d'un métal alcalin;

ou

Procédé B:

(a) la réaction d'un acide carboxylique de la formule $DCH_2$-COOH, où D est tel que défini ci-dessus, avec un agent de chloruration;

(b) la déprotonation d'une oxazolidinone chirale de la formule

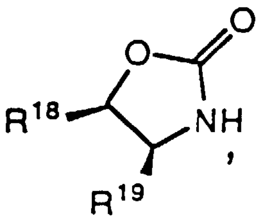 , 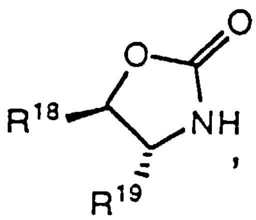 , 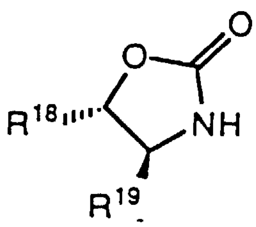 ou 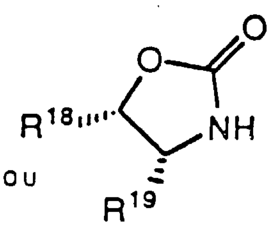

où $R^{18}$ et $R^{19}$ sont indépendamment sélectionnés dans le groupe consistant en: hydrogène, alkyle $C_1$-$C_6$, phényle, naphtyle, phényle substitué, naphtyle substitué, et benzyle; avec une base forte et traitement de l'anion résultant avec le produit de l'étape (a);

(c) l'énolisation du produit de l'étape (b) avec soit:

(i) un triflate de dialkylbore et une base d'amine tertiaire;

(ii) TiC14 et de la tétraméthyléthylènediamine (TMEDA) ou un mélange de TMEDA et de triéthylamine; puis la condensation avec un aldéhyde de la formule A-CHO, où A est tel que défini ci-dessus;

(d) l'hydrolyse de produit de l'étape (c) avec une base et du péroxyde d'hydrogène;

(e) la condensation du produit de l'étape (d) avec une amine de la formule $R^4NH_2$, où $R^4$ est tel que défini ci-dessus, par traitement avec un agent de couplage déshydratant en ajoutant facultativement un agent activant; et

(f) la cyclisation du produit tel que décrit au Procédé A, ci-dessus.

**2.** Procédé de production d'un composé de la formule 1, où R est hydrogène et D et A ont une stéréochimie relative trans, comprenant :

(a) l'énolisation d'un composé de formule

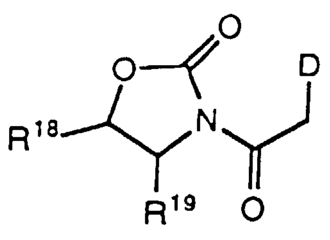

avec $TiCl_4$ et de la tétraméthyléthylènediamine (TMEDA), puis la condensation avec une imine de la formule A-CH=N-$R^4$, où A et $R^4$ sont tels que définis ci-dessus, et où le groupe

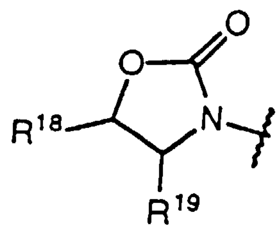

représente un auxiliaire chiral de la formule

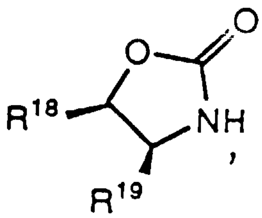 , 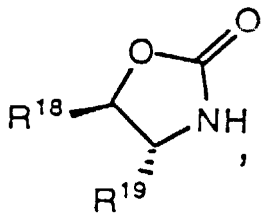 , 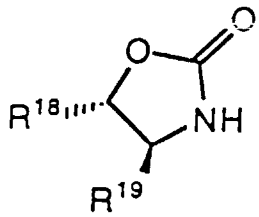 or 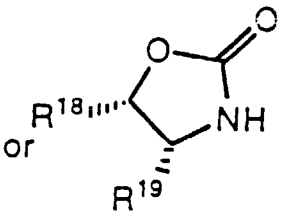

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; et

(b) la cyclisation du produit de l'étape (a) par traitement avec une base non nucléophile forte.

**3.** Procédé de préparation des composés de formule I tels que définis à la revendication 1 comprenant :

(a) le traitement d'un ester de la formule D-CH(R)-C(O)OR$^{17}$, où $R^{17}$ est alkyle $C_{1-6}$, menthyle, ou 10-(diisopropylsulfonamido)isobornyle, avec une base forte, puis avec une imine de la formule A-CH=N-R$^4$, où A et $R^4$ sont tels que définis à la revendication 1, ou

(b) pour la préparation d'un composé de formule I tel que défini à la revendication 1 où R n'est pas hydrogène, la réaction d'un composé de la revendication 1, où R est hydrogène, avec une base forte et un agent d'alkylation ou un agent d'acylation; ou

(c) pour la préparation d'un composé de formule I tel que défini à la revendication 1, où R est hydrogène, et D et A ont une stéréochimie relative cis, la réaction d'un composé de la formule I de la revendication 1, où R est hydrogène, et D et A ont une stéréochimie relative trans, avec une base forte entre -80 et -40°C, et puis avec une source de protons; ou

(d) pour la préparation d'un composé de formule I tel que défini à la revendication 1, où R est hydrogène, et D et A ont une stéréochimie relative trans, la réaction d'un composé de formule I tel que défini à la revendication 1, où R est hydrogène, et D et A ont une stéréochimie relative cis, avec un t-butoxyde d'un métal alcalin, puis l'arrêt avec un acide; ou

(e) la réaction d'un dérivé d'acide carboxylique de la formule D-CH(R)-C(O)-Z, où D et R sont tels que définis à la revendication 1 et Z est Cl,

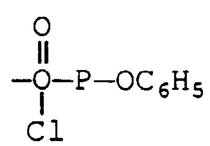 , ou 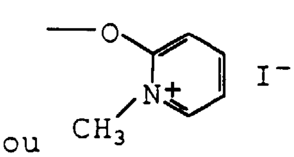

avec une imine de la formule A-CH=N-R$^4$, où A et $R^4$ sont tels que définis à la revendication 1, en présence

d'une base d'amine tertiaire.

**4.** Procédé de préparation d'un composé de formule II

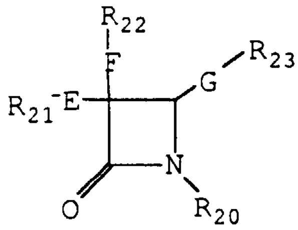

II

où

$R_{20}$ est phényle, phényle W-substitué, naphtyle, naphtyle W-substitué, benzodioxolyle, hétéroaryle, hétéroaryle W-substitué, hétéroaryle benzofusionné et hétéroaryle benzofusionné W-substitué, où hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazolyle et furanyle, et pour les hétéroaryles contenant de l'azote, leurs N-oxydes;

$R_{21}$, $R_{22}$ et $R_{23}$ sont indépendamment sélectionnés parmi H ou $R_{20}$;

W est 1 à 3 substituants indépendamment sélectionnés dans le groupe consistant en alkyle $C_{1-6}$, hydroxy alkyle $C_{1-6}$, alcoxy $C_{1-6}$, alcoxy $C_{1-6}$-alkyle $C_{1-6}$, alcoxy $C_{1-6}$ -alcoxy $C_{1-6}$, alcoxy $C_{1-6}$-carbonylalcoxy $C_{1-6}$, (alcoxyimino $C_{1-6}$)alkyle $C_{1-6}$, alcanedioyle $C_{3-6}$, alkyle $C_{1-6}$-alcanedioyle $C_{3-6}$, allyloxy, $-CF_3$, $-OCF_3$, benzyle, $R_{14}$-benzyle, benzyloxy, $R_{14}$-benzyloxy, phénoxy, $R_{14}$-phénoxy, dioxolanyle, $NO_2$, $-NR_{10}R_{11}$, $NR_{10}R_{11}$(alkyle $C_{1-6}$)-, $NR_{10}R_{11}$(alcoxy $C_{1-6}$)-, OH, halogéno, $-NHC(O)OR_5$, $-NHC(O)R_5$, $R_6O_2SNH-$, $(R_6O_2S)_2N-$, $-S(O)_2NH_2$, $-S(O)_{0-2}R_{10}$, tert-butyldiméthylsilyloxyméthyle, $-C(O)R_{12}$,

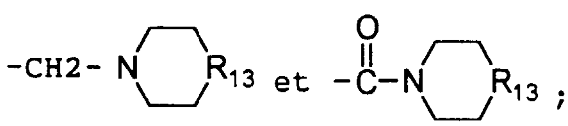

E, F et G sont indépendamment une liaison; cycloalkylène $C_3$-$C_6$; alkylène $C_1$-$C_{10}$; alcénylène $C_2$-$C_{10}$; alkynylène $C_2$-$C_{10}$; une chaîne d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué, où hétéroaryle est tel que défini ci-dessus; une chaîne d'alkylène, alcénylène ou alkynylène telle que définie, interrompue par un ou plusieurs groupes indépendamment sélectionnés dans le groupe consistant en -O-, -S-, -SO-, $-SO_2-$, $-NR_8$, -C(O)-, cycloalkylène $C_3$-$C_6$, phénylène, phénylène W-substitué, hétéroarylène et hétéroarylène W-substitué; ou bien une chaîne interrompue d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué; ou bien l'un de $R_{21}$-E et $R_{22}$-F est sélectionné dans le groupe consistant en halogéno, OH, alcoxy inférieur, $-OC(O)R_5$, $-NR_{10}R_{11}$, -SH ou -S(alkyle $C_{1-6}$);

$R_5$ est alkyle $C_{1-6}$, phényle, $R_{14}$-phényle, benzyle ou $R_{14}$-benzyle;

$R_6$ est OH, alkyle $C_{1-6}$, phényle, benzyle, $R_{14}$-phényle ou $R_{14}$-benzyle;

$R_8$ est H, alkyle $C_{1-6}$, phényle alkyle $C_{1-6}$ ou $-C(O)R_9$;

$R_9$ est H, alkyle $C_{1-6}$, phényle ou phényle alkyle $C_{1-6}$;

$R_{10}$ et $R_{11}$ sont indépendamment sélectionnés parmi H et alkyle $C_{1-6}$;

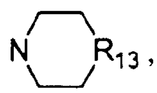

$R_{12}$ est H, OH, alcoxy $C_{1-6}$, phénoxy, benzyloxy, $-NR_{10}R_{11}$, alkyle $C_{1-6}$, phényle ou $-R_{14}$ phényle;

$R_{13}$ est -O-, $-CH_2-$, -NH- ou -N(alkyle $C_{1-6}$)-;

$R_{14}$ est 1-3 groupes indépendamment sélectionnés dans le groupe consistant en alkyle $C_{1-6}$, alcoxy $C_{1-6}$, -COOH, $NO_2$, $-NR_{10}R_{11}$, OH ou halogéno;

à condition que quand G est une liaison, $R_{23}$ ne soit pas H, et à condition que quand $R_{23}$ est phényle W-substitué, W ne soit pas p-halogéno;

où bien un sel pharmaceutiquement acceptable;

où $R_{22}$-F est H, et $R_{21}$-E- et $R_{23}$-G- ont une stéréochimie relative trans, comprenant

Procédé C: la cyclisation d'une hydroxyamide de la formule

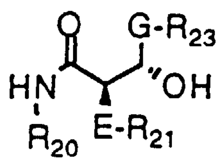

or

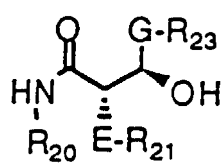

où $R_{21}$-E-, $R_{23}$-G- et $R_{20}$ sont tels que définis ci-dessus, par traitement avec :

(i) un dialkylazodicarboxylate et une trialkylphosphine; ou

(ii) un chlorure de di- ou tri-chlorobenzoyle, une base aqueuse et un catalyseur de transfert de phase, puis traitement du di- ou tri-chlorobenzoate résultant avec une base aqueuse et un catalyseur de transfert de phase; ou

(iii) un dialkylchlorophosphate, une base aqueuse et un catalyseur de transfert de phase; ou (iv) un chlorure de di- ou tri-chlorobenzoyle et un hydrure d'un métal alcalin;
ou

Procédé D:

(a) la réaction d'un acide carboxylique de la formule $R_{21}-E-CH_2-COOH$, où $R_{21}$-E- est tel que défini ci-dessus avec un agent de chloruration;

(b) la déprotonation d'une oxazolidinone chirale de la formule

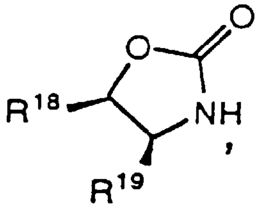 , 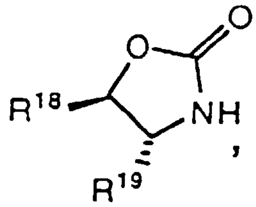 , 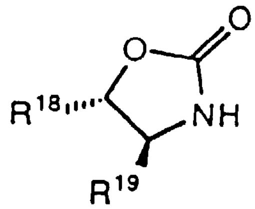 or 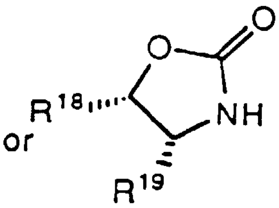

où $R^{18}$ et $R^{19}$ sont indépendamment sélectionnés dans le groupe consistant en : hydrogène, alkyle $C_1$-$C_6$, phényle, naphtyle, phényle substitué, naphtyle substitué, et benzyle; avec une base forte et traitement de l'anion résultant avec le produit de l'étape (a);

(c) l'énolisation du produit de l'étape (b) avec soit :

(i) un triflate de dialkylbore et une base d'amine tertiaire;

(ii) $TiCl_4$ et de la tétraméthyléthylènediamine (TMEDA) ou un mélange de TMEDA et de triéthylamine; puis la condensation avec un aldéhyde de la formule $R_{23}$-G-CHO, où $R_{23}$-G est tel que défini ci-dessus;

(d) l'hydrolyse du produit de l'étape (c) avec une base et du péroxyde d'hydrogène;

(e) la condensation du produit de l'étape (d) avec une amine de la formule $R_{20}NH_2$, où $R_{20}$ est tel que défini ci-dessus, par traitement avec un agent de couplage déshydratant, en ajoutant facultativement un agent activant; et

(f) la cyclisation du produit de l'étape (e) telle que décrite au Procédé C.

**5.** Procédé de production d'un composé de formule II tel que défini dans la revendication 4, où $R_{22}$-F- est hydrogène, et $R_{21}$-E- et $R_{23}$-G- ont une stéréochimie relative trans, comprenant :

(a) l'énolisation d'un composé de la formule

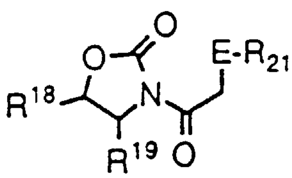

avec $TiCl_4$ et de la tétraméthyléthylènediamine (TMEDA), puis la condensation avec une imine de la formule $R_{23}$-G-CH=N-$R_{20}$, où $R_{23}$-G- et $R_{20}$ sont tels que définis à la revendication 4, et où le groupe

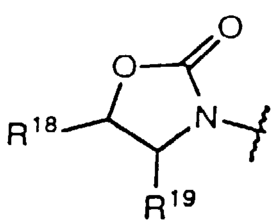

représente un auxiliaire chiral de la formule

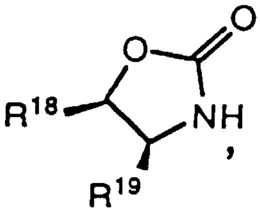, 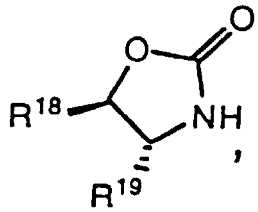, 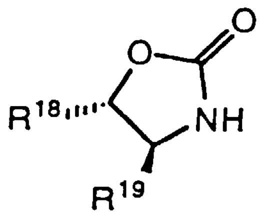 or 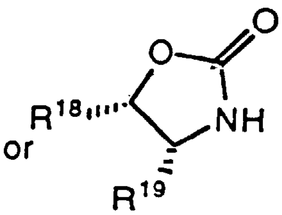

où $R^{18}$ et $R^{19}$ sont indépendamment sélectionnés dans le groupe consistant en hydrogène, alkyle $C_1$-$C_6$, phényle, naphtyle, phényle substitué, naphtyle substitué, et benzyle; et

(b) la cyclisation du produit de l'étape (a) par traitement avec une base non nucléophile forte.

**6.** Procédé de préparation d'un composé de formule II tel que défini à la revendication 4, comprenant

(a) la traitement d'un ester de la formule $R_{21}$-E-CH(F-$R_{22}$)-C(O)O$R^{17}$, où $R^{17}$ est alkyle $C_{1-6}$, menthyle, ou I0-(diisopropylsulfonamido)-isobornyle, avec une base forte, et puis avec un imine de la formule $R_{23}$-G-CH=N-$R_{20}$, où $R_{23}$-G- et $R_{20}$ sont tels que définis à la revendication 4, ou

(b) pour la préparation d'un composé de formule II tel que défini à la revendication 22, où $R_{22}$-F- n'est pas de l'hydrogène, la réaction d'un composé de formule II tel que défini à la revendication 22, où $R_{22}$-F- est de l'hydrogène, avec une base forte et un agent d'alkylation ou un agent d'acylation; ou

(c) pour la préparation d'un composé de formule II tel que défini dans la revendication 22, où $R_{22}$-F- est hydrogène, et $R_{21}$-E- et $R_{23}$-G- ont une stéréochimie relative cis, la réaction d'un composé de la formule II tel que défini à la revendication 4, où $R_{22}$-F- est hydrogène, et $R_{21}$-E-, et $R_{23}$-G- ont une stéréochimie relative trans avec une base forte à -80° jusqu'à -40°C, et puis avec une source de protons; ou

(d) pour la préparation d'un composé de formule II tel que défini à la revendication 4, où $R_{22}$-F- est hydrogène, et $R_{21}$-E- et $R_{23}$-G- ont une stéréochimie relative trans, la réaction d'un composé de formule II tel que défini à la revendication 22, où $R_{22}$-F- est hydrogène et $R_{21}$-E- et $R_{23}$-G- ont une stéréochimie relative cis, avec un t-butoxyde d'un métal alcalin, puis l'arrêt avec un acide; ou

(e) la réaction d'un dérivé d'acide carboxylique de la formule $R_{21}$-E-CH(F-$R_{22}$)-C(O)-Z, où $R_{21}$-E- et $R_{22}$-F- sont tels que définis à la revendication 4, et Z est Cl,

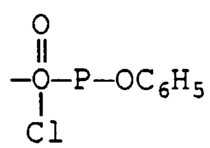 , ou 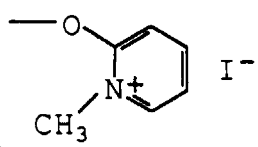 ,

avec une imine de la formule $R_{23}$-G-CH=N-$R_{20}$, où $R_{23}$-G- et $R_{20}$ sont tels que définis à la revendication 4, en présence d'une base d'amine tertiaire.

**7.** Procédé de la revendication 1 où R est hydrogène.

**8.** Procédé de la revendication 1 ou 7 où D est B'-$(CH_2)_q$-, B'-$(CH_2)_e$-Z-$(CH_2)_r$-, B'-(alcénylène $C_2$-$C_6$)- ou B'-$(CH_2)_f$-V-$(CH_2)_g$-, où B', Z, V, q, e, r, f, et g sont tels que définis à la revendication 1.

**9.** Procédé de la revendication 8 où D est B'-$(CH_2)_q$- où B' est phényle et q est 3 ou 4; B'-$(CH_2)_e$-Z-$(CH_2)_r$- où B' est p-fluorophényle ou p-méthoxyphényle, e est zéro, Z est-O-, et r est 2; B'-(alcénylène $C_2$-$C_6$)- est 3-phényl-1-propényle; ou bien B'-$(CH_2)_f$-V-(CH2)$_g$- où B' est phényle, f est 1, V est cyclopropylène, et g est zéro.

**10.** Procédé de la revendication 1, 7, 8 ou 9 où A est $-(CH_2)_p$-X-B où X, B et p sont tels que définis à la revendication 1.

**11.** Procédé de la revendication 10 où p est zéro et X est une liaison.

**12.** Procédé de la revendication 11 où $R_1$, $R_2$ et $R_3$ sont sélectionnés dans le groupe consistant en H, OH, alcoxy $C_{1-6}$, $NO_2$, alcoxy $C_{1-6}$-alcoxy $C_{1-6}$, m-halogéno, alkyle $C_{1-6}$, alcanedioyle $C_{3-6}$, $NR_{10}R_{11}$, alcoxy $C_{1-6}$, allyloxy, phénoxy, alcoxy $C_{1-6}$ carbonyle alcoxy $C_{1-6}$ et $C(O)R_{12}$.

**13.** Procédé de la revendication 1, 7, 8, 9, 10 ou 11 où $R_4$ est phényle, phényle $R_7$-substitué ou indanyle.

**14.** Procédé de la revendication 3 où $R_7$ est sélectionné dans le groupe consistant en alkyle $C_{1-6}$, alcoxy $C_{1-6}$, halogéno, $-OCF_3$, alkylthio $C_{1-6}$, $-NR_{10}R_{11}$, -CN, OH, et $-COR_{12}$.

**15.** Procédé de préparation d'une composition pharmaceutique à utiliser dans la fabrication d'un médicament pour abaisser le cholestérol dans le sérum, lequel procédé consiste à mélanger un support pharmaceutique à utiliser dans la fabrication d'un médicament pour abaisser le cholestérol dans le sérum, lequel procédé consiste à mélanger un support pharmaceutiquement acceptable avec un composé ayant la structure

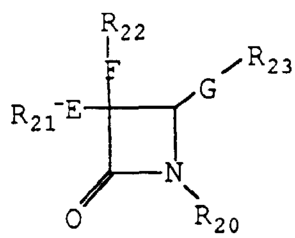

où

$R_{20}$ est phényle, phényle W-substitué, naphtyle, naphtyle W-substitué, benzodioxolyle, hétéroaryle, hétéroaryle W-substitué, hétéroaryle benzofusionné, et hétéroaryle benzofusionné W-substitué, où l'hétéroaryle est sélectionné dans le groupe consistant en pyrrolyle, pyridinyle, pyrimidinyle, pyrazinyle, triazinyle, imidazolyle, thiazolyle, pyrazolyle, thiényle, oxazoyle et furanyle, et pour les hétéroaryles contenant de l'azote, leurs N-oxydes;

$R_{21}$, $R_{22}$ et $R_{23}$ sont indépendamment sélectionnés parmi H ou $R_{20}$;

W est 1 à 3 substituants indépendamments sélectionnés dans le groupe consistant en alkyle $C_{1-6}$, hydroxy alkyle $C_{1-6}$, alcoxy $C_{1-6}$, alcoxy $C_{1-6}$ alkyle $C_{1-6}$, alcoxy $C_{1-6}$ alcoxy $C_{1-6}$, alcoxy $C_{1-6}$ carbonyle alcoxy $C_{1-6}$, (alcoxyimino $C_{1-6}$) alkyle $C_{1-6}$, alcanedioyle $C_{3-6}$, alkyle $C_{1-6}$-alcanedioyle $C_{3-6}$ allyloxy, $-CF_3$, $-OCF_3$, benzyle, $R_{14}$ benzyle, benzyloxy, $R_{14}$ -benzyloxy, phénoxy, $R_{14}$ - phénoxy, dioxolanyle, $NO_2$, $-NR_{10}R_{11}$, $NR_{10}R_{11}$ (alkyle $C_{1-6}$ )-, $NR_{10}R_{11}$ (alcoxy $C_{1-6}$)-, OH, halogéno, $-NHC(O)OR_5$, $-NHC(O)R_5$,

$R_6O_2SNH-$, $(R_6O_2S)_2N-$, $-S(O)_2NH_2$, $-S(O)_{0-2}R_{10}$, tert-butyldiméthylsiloxyméthyle, $-C(O)R_{12}$,

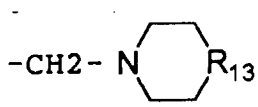

et

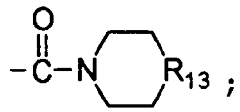

E, F et G sont indépendamment une liaison; cycloalkylène $C_3$-$C_6$; alkylène $C_1$-$C_{10}$; alcénylène $C_2$-$C_{10}$; alkynylène $C_2$-$C_{10}$; une chaîne d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué, ou l'hétéroaryle est tel que défini ci-dessus; une chaîne d'alkylène, alcénylène ou alkynylène telle que définie, interrompue par un ou plusieurs groupes indépendamment sélectionnés dans le groupe consistant en -O-, -S-, -SO-, $-SO_2-$, $-NR_8$, -C(O)-, cycloalkylène $C_3$-$C_6$, phénylène, phénylène W-substitué, hétéroarylène et hétéroarylène W-substitué; ou bien une chaîne interrompue d'alkylène, alcénylène ou alkynylène telle que définie, substituée par un ou plusieurs substituants indépendamment sélectionnés dans le groupe consistant en phényle, phényle W-substitué, hétéroaryle et hétéroaryle W-substitué; ou bien l'un de $R_{21}$-E et $R_{22}$-F est sélectionné dans le groupe consistant en halogéno, OH, alcoxy $C_{1-6}$, $-OC(O)R_5$, $-NR_{10}R_{11}$, -SH ou -S (alkyle $C_{1-6}$);

$R_5$ est alkyle $C_{1-6}$, phényle, $R_{14}$-phényle, benzyle ou $R_{14}$-benzyle;

$R_6$ est OH, alkyle $C_{1-6}$, phényle, benzyle, $R_{14}$-phényle ou $R_{14}$-benzyle;

$R_8$ est H, alkyle $C_{1-6}$, phényl alkyl $C_{1-6}$ ou $-C(O)R_9$;

$R_9$ est H, alkyle $C_{1-6}$, phényle ou phényl alkyle $C_{1-6}$;

$R_{10}$ et $R_{11}$ sont indépendamment sélectionnés parmi H et alkyle $C_{1-6}$;

$R_{12}$ est H, OH, alcoxy $C_{1-6}$, phénoxy, benzyloxy,

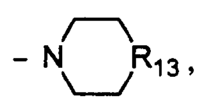

$-NR_{10}R_{11}$, alkyle $C_{1-6}$, phényle ou $R_{14}$-phényle;

$R_{13}$ est -O-, $-CH_2-$, -NH- ou -N(alkyle $C_{1-6}$)-;

$R_{14}$ est 1-3 groupes indépendamment sélectionnés dans le groupe consistant en alkyle $C_{1-6}$, alcoxy $C_{1-6}$, -COOH, $NO_2$, $-NR_{10}R_{11}$, OH ou halogéno;

à condition que quand G est une liaison, $R_{23}$ ne soit pas H, et à condition que quand $R_{23}$ est phényle W-substitué, W ne soit pas p-halogéno;

ou bien un sel pharmaceutiquement acceptable;

dans un support pharmaceutiquement acceptable.

**16.** Procédé de la revendication 1 où les composés préparés sont sélectionnés dans le groupe consistant en composés de la formule

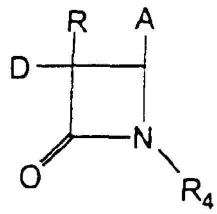

où D,R,A et $R_4$ et la stéréochimie relative entre D et A sont tels que définis au tableau qui suit:

| D | R | A | Stéréochimie relative | $R_4$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-$NO_2$-$C_6H_4$- | 3R,4R | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | 3-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 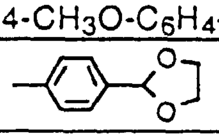 | trans | p-$CH_3O$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | 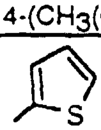 | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($C_6H_5$-O)-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 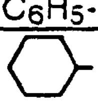 -$(CH_2)_3$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | 4-$CH_3O$-$C_6H_4$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-($(CH_3)_2N$-$(CH_2)_3$-O)-$C_6H_4$- | cis | 4-$CH_3O$-$C_6H_4$- |

| | | | | |
|---|---|---|---|---|
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| $C_6H_5$-$(CH_2)_3$- | H | 4-$CH_3O$-$C_6H_4$- | trans | 4-($C_6H_5$-O)-$C_6H_4$- |
| 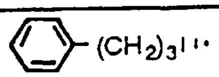 | H | 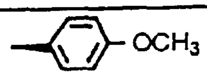 | trans optique pure | $C_6H_5$- |
| 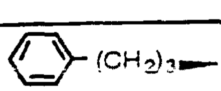 | H | 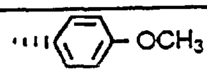 | trans optique pure | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($C_6H_5$-$CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| $C_6H_5$-O-$(CH_2)_2$- | H | 4-$CH_3O$-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |
| $C_6H_5$-$(CH_2)_3$- | H | 4-$CH_3O$-$C_6H_4$- | trans | 4-($CH_3CH_2OC(O)$)-$C_6H_4$- |
| $C_6H_5$-$(CH_2)_3$- | H | 4-$CH_3O$-$C_6H_4$- | trans | 3-$NO_2$-$C_6H_4$- |
| $C_6H_5$-$(CH_2)_3$- | H | 4-$CH_3O$-$C_6H_4$- | trans | 4-($(CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 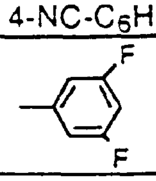 |
| $C_6H_5$-$(CH_2)_3$- | H | 4-$CH_3O$-$C_6H_4$- | trans | 4-($CH_3C(O)$)-$C_6H_4$- |

| | | | | |
|---|---|---|---|---|
| $C_6H_5$-$(CH_2)_3$- | H | 4-$CH_3O$-$C_6H_4$- | trans | 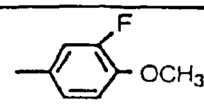 |
| $C_6H_5$-$(CH_2)_3$- | H | 4-$CH_3O$-$C_6H_4$- | trans | 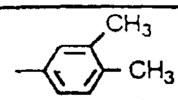 |
| $C_6H_5$-$(CH_2)_3$- | H | 4-($(CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 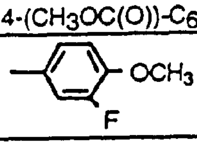 | trans | 4-$CH_3O$-$C_6H_4$- |
| $C_6H_5$-$(CH_2)_3$- | H | 4-($CH_3OCH_2O$)-$C_6H_4$- | trans | $C_6H_5$ |
| $C_6H_5$-$(CH_2)_3$- | H | 4-($(CH_3)_2N$)-$C_6H_4$- | trans | 4-$CH_3O$-$C_6H_4$- |
| $C_6H_5$-$(CH_2)_3$- | H | 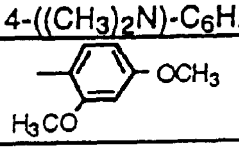 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| 4-$O_2N$-$C_6H_4$-$(CH_2)_3$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | 4-I-$C_6H_4$- |
| $C_6H_5$-$(CH_2)_3$- | H | 4-($CH_3(CH_2)_3$-O)-$C_6H_4$- | trans | 4-$CH_3O$-$C_6H_4$- |
| 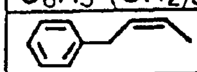 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 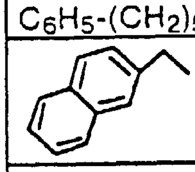 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 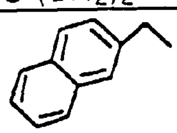 | H | 4-$CH_3O$-$C_6H_4$- | cis | $C_6H_5$- |
| $C_6H_5$-$(CH_2)_3$- | H | 4-$CH_3O$-$C_6H_4$- | trans | 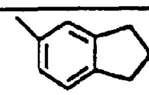 |
| $C_6H_5$-$(CH_2)_3$- | H | 4-$NH_2$-$C_6H_4$- | cis | 4-$CH_3O$-$C_6H_4$- |
| $C_6H_5$-$(CH_2)_3$- | H | 4-$CH_3O$-$C_6H_4$- | trans | 3-$NH_2$-$C_6H_4$- |
| $C_6H_5$-$(CH_2)_3$- | H | 4-OH-$C_6H_4$- | trans | 4-$CH_3O$-$C_6H_4$- |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-$CH_3O$-$C_6H_4$- | 3R,4S | 4-OH-$C_6H_4$- |

| | | | | |
|---|---|---|---|---|
| $C_6H_5-(CH_2)_3-$ | H | 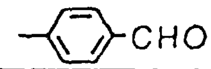 | trans | $4-CH_3O-C_6H_4-$ |
| $C_6H_5-(CH_2)_3-$ | H | $4-CH_3O-C_6H_4-$ | trans | $4-(CH_3SO_2)-C_6H_4-$ |
| $C_6H_5-(CH_2)_3-$ | H | 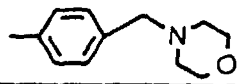 | trans | $4-CH_3O-C_6H_4-$ |
| $C_6H_5-(CH_2)_3-$ | H | 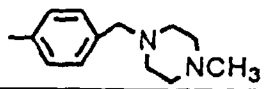 | trans | $4-CH_3O-C_6H_4-$ |
| $C_6H_5-(CH_2)_3-$ | H | $4-(CH_3OCH_2)-C_6H_4-$ | trans | $4-CH_3O-C_6H_4-$ |
| $C_6H_5-(CH_2)_3-$ | H | 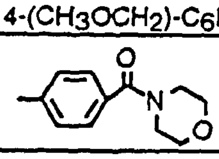 | cis | $4-CH_3O-C_6H_4-$ |
| $C_6H_5-(CH_2)_3-$ | H | $4-(CH_3CH_2O_2C)-C_6H_4-$ | trans | $4-CH_3O-C_6H_4-$ |
| $C_6H_5-(CH_2)_3-$ | H | 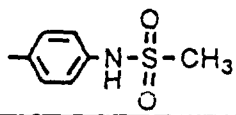 | trans | $4-CH_3O-C_6H_4-$ |
| $C_6H_5-(CH_2)_3-$ | H | $-Ph-O\overset{O}{\overset{\|}{C}}(CH_2)_2\overset{O}{\overset{\|}{C}}OCH_3$ où Ph est 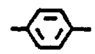 | trans | $4-CH_3O-C_6H_4-$ |
| $C_6H_5-(CH_2)_3-$ | H | $-Ph-OCH_2CO_2Et$ où Ph est 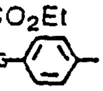 et ET est $-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 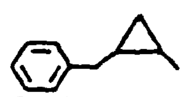 | H | $4-CH_3O-C_6H_4-$ | cis | $4-CH_3O-C_6H_4-$ |

**17.** Procédé de la revendication 1 où le composé préparé est (3R,45)-1,4-bis-(4-méthoxyphényl)-3-(3-phénylpropyl)-2-azétidinone.

**18.** Procédé de la revendication 15, où le composé utilisé est sélectionné dans le groupe consistant en composés de la formule

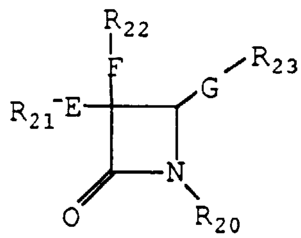

où $R_{21}$-E-, $R_{22}$-F-, $R_{23}$-G-, $R_{20}$ et la stéréochimie relative entre $R_{21}$-E- et $R_{23}$-G- sont tels que définis au tableau qui suit:

| $R_{21}$-E- | $R_{22}$-F- | $R_{23}$-G- | Stéréochimie relative | $R_{20}$ |
|---|---|---|---|---|
| $C_6H_5$-$(CH_2)_3$- | H | p-$CH_3O$-$C_6H_4$- | 3S,4S | p-$CH_3O$-$C_6H_4$- |
| $C_6H_5$-$(CH_2)_3$- | H | p-OH-$C_6H_4$- | 3S,4S | p-$CH_3O$-$C_6H_4$- |
| $C_6H_5$-$(CH_2)_3$- | H | p-$CH_3O$-$C_6H_4$- | trans | p-$CF_3O$-$C_6H_4$- |
| $C_6H_5$-$(CH_2)_3$- | H | 4-$CH_3O$-$C_6H_4$- | cis | p-Cl-$C_6H_4$- |
| $C_6H_5$-$(CH_2)_3$- | H | 4-$CH_3O$-$C_6H_4$- | cis | p-$CH_3$-$C_6H_4$- |
| $C_6H_5$-$(CH_2)_3$- | H | 4-($(CH_3)_2CH$-O)-$C_6H_4$- | cis | p-$CH_3O$-$C_6H_4$- |
| $C_6H_5$-$(CH_2)_3$- | H | 4-($CH_3(CH)_2$-O)-$C_6H_4$- | cis | p-$CH_3O$-$C_6H_4$- |
| $C_6H_5$-$(CH_2)_3$- | H | $CH_3CH_2$-O-$C_6H_4$- | cis | p-$CH_3O$-$C_6H_4$- |
| $C_6H_5$-$(CH_2)_3$- | H | $CH_3(CH_2)_3$-O-$C_6H_4$- | cis | p-$CH_3O$-$C_6H_4$- |
| $C_6H_5$-$(CH_2)_3$- | H | 4-($CH_2$=CH-$CH_2$-O)-$C_6H_4$- | cis | p-$CH_3O$-$C_6H_4$- |
| $C_6H_5$-$(CH_2)_3$- | H | 4-($CH_3CH_2$-O)-$C_6H_4$- | cis | $C_6H_5$- |
| 4-F-$C_6H_4$-O-$(CH_2)_2$- | H | 4-$CH_3O$-$C_6H_4$- | cis | $C_6H_5$- |
| $C_6H_5$-$(CH_2)_3$- | H | 4-$CH_3O$-$C_6H_4$- | cis | $C_6H_5$- |
| $C_6H_5$-$(CH_2)_3$- | H | 4-$CH_3O$-$C_6H_4$- | 3S,4S | p-$CH_3O$-$C_6H_4$- |
| $C_6H_5$-$(CH_2)_3$- | H | p-$CH_3O$-$C_6H_4$- | 3S,4R | p-$CH_3O$-$C_6H_4$- |
| $C_6H_5$-$(CH_2)_3$- | H | 4-$CH_3O$-$C_6H_4$- | trans | 4-F-$C_6H_4$- |
| $C_6H_5$-$(CH_2)_3$- | H | 4-$CH_3O$-$C_6H_4$- | trans | 3-$CH_3O$-$C_6H_4$- |
| $C_6H_5$-$(CH_2)_3$- | H | 4-$CH_3O$-$C_6H_4$- | trans | 4-Cl-$C_6H_4$- |
| $C_6H_5$-$(CH_2)_3$- | H | 4-$CH_3O$-$C_6H_4$- | trans | 4-($CH_3CH_2$-O)-$C_6H_4$- |
| $C_6H_5$-$(CH_2)_3$- | H | 4-$CH_3O$-$C_6H_4$- | trans | 4-$CH_3S$-$C_6H_4$- |
| $C_6H_5$-$(CH_2)_3$- | H | 4-$CH_3O$-$C_6H_4$- | trans | 4-$CH_3$-$C_6H_4$- |
| 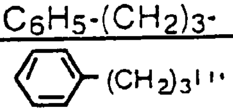 | H | 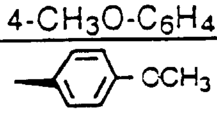 | trans optique pure | $C_6H_5$- |
| $C_6H_5$-$(CH_2)_3$- | H | 4-($CH_3CH_2$-O)-$C_6H_4$- | trans | 4-$CH_3O$-$C_6H_4$- |
| $C_6H_5$-$(CH_2)_3$- | H | 4-($CH_2$=CH-$CH_2$-O)-$C_6H_4$- | trans | 4-$CH_3O$-$C_6H_4$- |
| $C_6H_5$-$(CH_2)_3$- | H | 4-($C_6H_5$-O)-$C_6H_4$- | trans | 4-$CH_3O$-$C_6H_4$- |
| 4-F-$C_6H_4$-O-$(CH_2)_2$- | H | 4-$CH_3O$-$C_6H_4$- | trans | 4-$CH_3O$-$C_6H_4$- |
| $C_6H_5$-$(CH_2)_4$- | H | 4-$CH_3O$-$C_6H_4$- | trans | 4-$CH_3O$-$C_6H_4$- |
| $C_6H_5$-$(CH_2)_3$- | H | 4-$CH_3O$-$C_6H_4$- | trans | $C_6H_5$- |

| | | | | |
|---|---|---|---|---|
| $C_6H_5$-$(CH_2)_3$- | H | 4-$CH_3O$-$C_6H_4$- | trans | 4-($(CH_3CH_2)_2$-N)-$C_6H_4$- |
| $C_6H_5$-$(CH_2)_3$- | H | 4-$CH_3O$-$C_6H_4$- | trans | 4-NC-$C_6H_4$- |
| $C_6H_5$-$(CH_2)_3$- | H | 4-$CH_3O$-$C_6H_4$- | trans | 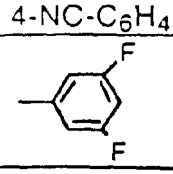 |
| $C_6H_5$-$(CH_2)_3$- | H | 4-$CH_3O$-$C_6H_4$- | trans | 4-($CH_3C(O)$)-$C_6H_4$- |
| $C_6H_5$-$(CH_2)_3$- | H | 4-($(CH_3)_2CH$-O)-$C_6H_4$- | trans | 4-$CH_3O$-$C_6H_4$- |
| $C_6H_5$-$(CH_2)_3$- | H | 4-($CH_3(CH_2)_2$-O)-$C_6H_4$- | trans | 4-$CH_3O$-$C_6H_4$- |
| $C_6H_5$-$(CH_2)_3$- | H | 4-($CH_3OC(O)$)-$C_6H_4$- | trans | $C_6H_5$- |
| $C_6H_5$-$(CH_2)_3$- | H | 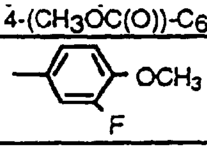 | trans | 4-$CH_3O$-$C_6H_4$- |
| $C_6H_5$-$(CH_2)_3$- | H | 4-($CH_3OCH_2O$)-$C_6H_4$- | trans | $C_6H_5$- |
| $C_6H_5$-$(CH_2)_3$- | H | 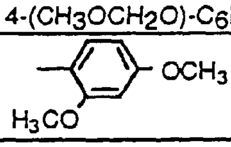 | trans | 4-$CH_3O$-$C_6H_4$- |
| $C_6H_5$-$(CH_2)_3$- | H | 4-($CH_3CH_2$-O)-$C_6H_4$- | trans | $C_6H_5$- |
| $C_6H_5$-$(CH_2)_3$- | H | 4-$CH_3O$-$C_6H_4$- | trans | 4-I-$C_6H_4$- |
| 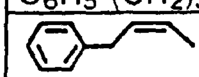 | H | 4-$CH_3O$-$C_6H_4$- | trans | 4-$CH_3O$-$C_6H_4$- |
| $C_6H_5$-$(CH_2)_5$- | H | 4-$CH_3O$-$C_6H_4$- | trans | 4-$CH_3O$-$C_6H_4$- |
| 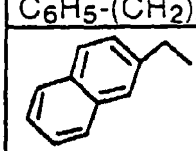 | H | 4-$CH_3O$-$C_6H_4$- | trans | $C_6H_5$- |
| 4-($CH_3O$)-$C_6H_4$-O-$(CH_2)_2$- | H | 4-$CH_3O$-$C_6H_4$- | trans | $C_6H_5$- |
| 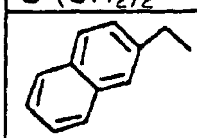 | H | 4-$CH_3O$-$C_6H_4$- | cis | $C_6H_5$- |
| $C_6H_5$-$(CH_2)_3$- | H | 4-$CH_3O$-$C_6H_4$- | trans | 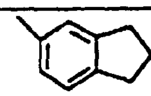 |
| $C_6H_5$-$(CH_2)_3$- | H | 4-$CH_3O$-$C_6H_4$- | 3R,4S | 4-$CH_3O$-$C_6H_4$- |
| $C_6H_5$-$(CH_2)_3$- | H | 4-OH-$C_6H_4$- | trans | 4-OH-$C_6H_4$- |
| $C_6H_5$-$(CH_2)_3$- | H | 4-($CH_3CH_2O_2C$)-$C_6H_4$- | trans | 4-$CH_3O$-$C_6H_4$- |
| $C_6H_5$-$(CH_2)_3$- | H | -PhOC(O)$(CH_2)_2$C(O)$OCH_3$ où Ph est 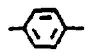 | trans | 4-$CH_3O$-$C_6H_4$- |

| | | | | |
|---|---|---|---|---|
| $C_6H_5-(CH_2)_3-$ | H | $-Ph-OCH_2CO_2Et$ où Ph est 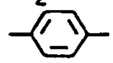 et ET est$-CH_2CH_3$ | 3R,4S | $4-CH_3O-C_6H_4-$ |
| $C_6H_5-(CH_2)_3-$ | H | $4-CH_3O-C_6H_4-$ | cis | $4-(CH_3CH_2O)-C_6H_4-$ |
| 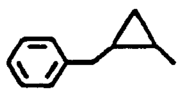 | H | $4-CH_3O-C_6H_4-$ | cis | $4-CH_3O-C_6H_4-$ |
| $C_6H_5-(CH_2)_2-NH-$ | H | $4-CH_3O-C_6H_4-$ | cis | $C_6H_5-$ |
| 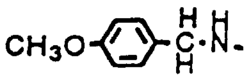 | H | $4-CH_3O-C_6H_4-$ | cis | $C_6H_5-$ |

**19.** Méthode d'utilisation d'un composé défini à la revendication 1 pour la fabrication d'un médicament pour abaisser le cholestérol dans le sérum.

**20.** Méthode d'utilisation d'un composé défini à la revendication 1 pour la fabrication d'un médicament pour inhiber l'enzyme acyl CoA: cholestérol acyl transférase.

**21.** Méthode d'utilisation d'une composition définie à la revendication 15 pour la fabrication d'un médicament pour abaisser les niveaux de cholestérol dans le sérum chez un mammifère nécessitant un tel traitement.

**22.** Méthode d'utilisation d'une composition définie à la revendication 15 pour la fabrication d'un médicament pour inhiber l'enzyme acyl CoA:cholestérol acyl transférase.